# EUROPEAN PATENT APPLICATION

(11) **EP 2 143 796 A2**
(43) Date of publication of application: **13.01.2010**
(21) Application number: 09160573.3
(22) Date of filing: 28.02.2001
(51) Int. Cl.: C12N 15/12, C07K 14/705, C12Q 1/68, G01N 33/53, G01N 33/68

(54) **1983, 52881, 2398, 45449, 50289, and 52872, G protein-coupled receptors and uses therefor**

(30) Priority: 29.02.2000 US 186059 P
(62) Divisional of application: 01916306.2
(71) Applicant: Millennium Pharmaceuticals, Inc., Cambridge, MA 02139 (US)
(72) Inventor: Silos-Santiago, Inmaculada, Del Mar, CA 92014 (US); Galvin, Katherine M., Jamaica Plain, MA 02130 (US); Glucksmann, Maria Alexandra, Lexington, MA 02173 (US)
(74) Representative: Williams, Gareth Owen

(57) **Abstract**

The invention provides isolated nucleic acids molecules, designated 1983, 52881, 2398, 45449, 50289, and 52872 nucleic acid molecules, which encode G protein-coupled receptor members. The invention also provides antisense nucleic acid molecules, recombinant expression vectors containing 1983, 52881, 2398, 45449, 50289, or 52872 nucleic acid molecules, host cells into which the expression vectors have been introduced, and nonhuman transgenic animals in which a 1983, 52881, 2398, 45449, 50289, or 52872 gene has been introduced or disrupted. The invention still further provides isolated 1983, 52881, 2398, 45449, 50289, or 52872 proteins, fusion proteins, antigenic peptides and anti-1983, 52881, 2398, 45449, 50289, or 52872 antibodies. Diagnostic methods utilizing compositions of the invention are also provided.

## Description

### Related Application

This application claims priority to U.S. provisional application number 60/186,059, filed on February 29, 2000, the content of which is incorporated herein by reference.

### Background of the Invention

G-protein coupled receptors (GPCRs) are seven transmembrane domain proteins that mediate signal transduction of a diverse number of ligands through heterotrimeric G proteins (Strader, C. D. et al. (1994) Annu. Rev. Biochem. 63: 101-132). G protein-coupled receptors (GPCRs), along with G-proteins and effector proteins (e.g., intracellular enzymes and channels), are the components of a modular signaling system. Upon ligand binding to an extracellular portion of a GPCR, different G proteins are activated, which in turn modulate the activity of different intracellular effector enzymes and ion channels (GutlGind, J.S. (1998) J. Biol. Chem. 273: 1839-1842; Selbie, L.A. and Hill, S.J. (1998) Trends Pharmacol. Sci. 19:87-93).

G proteins represent a family of heterotrimeric proteins composed of α, β and γ subunits, which bind guanine nucleotides. These proteins are usually linked to cell surface receptors (e.g., a GPCR). Following ligand binding to a GPCR, a conformational change is transmitted to the G protein, which causes the α-subunit to exchange a bound GDP molecule for a GTP molecule and to dissociate from the βγ-subunits. The GTP-bound form of the α-subunit typically functions as an effector-modulating moiety, leading to the production of second messengers, such as cyclic AMP (e.g., by activation of adenylate cyclase), diacylglycerol or inositol phosphates. Over 20 different types of α-subunits are known in man, which associate with a smaller pool of β and γ subunits. Examples of mammalian G proteins include Gᵢ, Gₒ, G_{q}, Gₛ and Gₜ (Lodish H. et al. Molecular Cell Biology, Scientific American Books Inc., New York, N.Y., 1995).

One subfamily of seven transmembrane receptors is the rhodopsin family. Proteins of this family can be expressed in photoreceptor cells. They generally contain a prosthetic group, 11-cis-retinal. Absorption of light by retinal causes an isomerization in the molecule and consequently a conformational change in the rhodopsin protein. This structural change is transmitted to a signaling cascade by means of the coupled G protein.

GPCRs are of critical importance to several systems including the endocrine system, the central nervous system and peripheral physiological processes. The GPCR genes and gene-products are also believed to be causative agents of disease (Spiegel et al. (1993) J. Clin. Invest. 92:1119-1125); McKusick and Amberger (1993) J. Med. Genet. 30:1-26). Given the important biological roles and properties of GPCRs, there exists a need for the identification of novel genes encoding such proteins as well as for the discovery of modulators of such molecules for use in regulating a variety of normal and/or pathological cellular processes.

### Summary of the Invention

The present invention is based, in part, on the discovery of novel G-protein coupled receptors and nucleic acids encoding these receptors, referred to herein collectively as "GPCRs," or by the individual clone name "1983, 52881, 2398, 45449, 50289, and 52872." The nucleotide sequence of a cDNA encoding 1983 is shown in SEQ ID NO:1, and the amino acid sequence of a 1983 polypeptide is shown in SEQ ID NO:2. In addition, the nucleotide sequence of the coding region of a 1983 polypeptide is depicted in SEQ ID NO:3. The nucleotide sequence of a cDNA encoding a 52881 polypeptide is shown in SEQ ID NO:4, and the amino acid sequence of a 52881 polypeptide is shown in SEQ ID NO:5. In addition, the nucleotide sequence of the coding region of a 52881 polypeptide is depicted in SEQ ID NO:6. The nucleotide sequence of a cDNA encoding 2398 polypeptide is shown in SEQ ID NO:7, and the amino acid sequence of a 2398 polypeptide is shown in SEQ ID NO:8. In addition, the nucleotide sequence of the coding region of a 2398 polypeptide is depicted in SEQ ID NO:9. The nucleotide sequence of a cDNA encoding a 45449 polypeptide is shown in SEQ ID NO:10, and the amino acid sequence of a 45449 polypeptide is shown in SEQ ID NO:11. In addition, the nucleotide sequence of the coding region of a 45449 polypeptide is depicted in SEQ ID NO:12. The nucleotide sequence of a cDNA encoding a 50289 polypeptide is shown in SEQ ID NO:13, and the amino acid sequence of a 50289 polypeptide is shown in SEQ ID NO:14. In addition, the nucleotide sequence of the coding region of a 50289 polypeptide is depicted in SEQ ID NO:15. The nucleotide sequence of a cDNA encoding a 52872 polypeptide is shown in SEQ ID NO:16, and the amino acid sequence of a 52872 polypeptide is shown in SEQ ID NO:17. In addition, the nucleotide sequence of the coding region of a 52872 polypeptide is depicted in SEQ ID NO:18.

Accordingly, in one aspect, the invention features a nucleic acid molecule which encodes a 1983, 52881, 2398, 45449, 50289, or 52872 protein or polypeptide, e.g., a biologically active portion of the 1983, 52881, 2398, 45449, 50289, or 52872 protein. In a preferred embodiment the isolated nucleic acid molecule encodes a polypeptide having the amino acid sequence of SEQ ID NO:2, SEQ ID NO:5, SEQ ID NO:8, SEQ ID NO: 11, SEQ ID NO:14, SEQ ID NO:17. In other embodiments, the invention provides isolated 1983, 52881, 2398, 45449, 50289, or 52872 nucleic acid molecules having the nucleotide sequence shown in SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, or the sequence of the DNA insert of the plasmid deposited with ATCC Accession Number _, ATCC Accession Number _, ATCC Accession Number _, ATCC Accession Number _, ATCC Accession Number _, or ATCC Accession Number _. In still other embodiments, the invention provides nucleic acid molecules that are substantially identical (e.g., naturally occurring allelic variants) to the nucleotide sequence shown in SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, or the sequence of the DNA insert of the plasmid deposited with ATCC Accession Number _, ATCC Accession Number _, ATCC Accession Number _, ATCC Accession Number _, ATCC Accession Number _, or ATCC Accession Number _. In other embodiments, the invention provides a nucleic acid molecule which hybridizes under a stringent hybridization condition described herein to a nucleic acid molecule comprising the nucleotide sequence of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO: 13, SEQ ID NO:15, SEQ ID NO:16, SEQ ID NO:18, or the sequence of the DNA insert of the plasmid deposited with ATCC Accession Number _, ATCC Accession Number _, ATCC Accession Number _, ATCC Accession Number _, ATCC Accession Number _, or ATCC Accession Number _, wherein the nucleic acid encodes a full length 1983, 52881, 2398, 45449, 50289, or 52872 protein or an active fragment thereof. In a preferred embodiment, the 1983, 52881, 2398, 45449, 50289, or 52872 nucleic acid has a nucleotide sequence identical to, or substantially identical to, SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, or the sequence of the DNA insert of the plasmid deposited with ATCC Accession Number _, ATCC Accession Number _, ATCC Accession Number _, ATCC Accession Number _, ATCC Accession Number _, or ATCC Accession Number _. In other embodiments, the 1983, 52881, 2398, 45449, 50289, or 52872 nucleic acid is a fragment of at least 50, 100, 150, 200, 250, 300, 350, 400, 450, 500, 600, 700, 800, 900 or more contiguous nucleotides of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO: 16, SEQ ID NO:18, or the sequence of the DNA insert of the plasmid deposited with ATCC Accession Number _, ATCC Accession Number _, ATCC Accession Number _, ATCC Accession Number _, ATCC Accession Number _, or ATCC Accession Number _.

In a related aspect, the invention further provides nucleic acid constructs which include a 1983, 52881, 2398, 45449, 50289, or 52872 nucleic acid molecule described herein. In certain embodiments, the nucleic acid molecules of the invention are operatively linked to native or heterologous regulatory sequences. Also included, are vectors and host cells containing the 1983, 52881, 2398, 45449, 50289, or 52872 nucleic acid molecules of the invention e.g., vectors and host cells suitable for producing 1983, 52881, 2398, 45449, 50289, or 52872 nucleic acid molecules and polypeptides.

In another related aspect, the invention provides nucleic acid fragments suitable as primers or hybridization probes for the detection of 1983, 52881, 2398, 45449, 50289, or 52872-encoding nucleic acids.

In still another related aspect, isolated nucleic acid molecules that are antisense to a 1983, 52881, 2398, 45449, 50289, or 52872 encoding nucleic acid molecule are provided.

In another aspect, the invention features, 1983, 52881, 2398, 45449, 50289, or 52872 polypeptides, and biologically active or antigenic fragments thereof that are useful, e.g., as reagents or targets in assays applicable to treatment and diagnosis of 1983, 52881, 2398, 45449, 50289, or 52872-mediated or 1983, 52881, 2398, 45449, 50289, or 52872-related disorders. In another embodiment, the invention provides 1983, 52881, 2398, 45449, 50289, or 52872 polypeptides having a 1983, 52881, 2398, 45449, 50289, or 52872 activity. Preferred polypeptides are 1983, 52881, 2398, 45449, 50289, or 52872 proteins including at least one seven transmembrane domain domain or at least one ANF receptor ligand binding domain, and, preferably, having a 1983, 52881, 2398, 45449, 50289, or 52872 activity, e.g., a 1983, 52881, 2398, 45449, 50289, or 52872 activity as described herein.

In other embodiments, the invention provides 1983, 52881, 2398, 45449, 50289, or 52872 polypeptides, e.g., a 1983, 52881, 2398, 45449, 50289, or 52872 polypeptide having the amino acid sequence shown in SEQ ID NO:2, SEQ ID NO:5, SEQ ID NO:8, SEQ ID NO:11, SEQ ID NO:14, SEQ ID NO:17, or an amino acid sequence encoded by the cDNA insert of the plasmid deposited with ATCC Accession Number ATCC Accession Number _, ATCC Accession Number _, ATCC Accession Number _, ATCC Accession Number _, or ATCC Accession Number _; an amino acid sequence that is substantially identical to the amino acid sequence shown in SEQ ID NO:2, SEQ ID NO:5, SEQ ID NO:8, SEQ ID NO:11, SEQ ID NO:14, SEQ ID NO:17, or an amino acid sequence encoded by the cDNA insert of the plasmid deposited with ATCC Accession Number _, ATCC Accession Number _, ATCC Accession Number _, ATCC Accession Number _, ATCC Accession Number _, or ATCC Accession Number _; or an amino acid sequence encoded by a nucleic acid molecule having a nucleotide sequence which hybridizes under a stringent hybridization condition described herein to a nucleic acid molecule comprising the nucleotide sequence of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, SEQ ID NO:18, or the sequence of the DNA insert of the plasmid deposited with ATCC Accession Number _, ATCC Accession Number _, ATCC Accession Number _, ATCC Accession Number _, ATCC Accession Number _, or ATCC Accession Number _, wherein the nucleic acid encodes a full length 1983, 52881, 2398, 45449, 50289, or 52872 protein or an active fragment thereof.

In a preferred embodiment, the 1983, 52881, 2398, 45449, 50289, or 52872 polypeptide has an amino acid sequence identical to, or substantially identical to, SEQ ID NO:2, SEQ ID NO:5, SEQ ID NO:8, SEQ ID NO:11, SEQ ID NO:14, SEQ ID NO:17; or an amino acid sequence encoded by the cDNA insert of the plasmid deposited with ATCC Accession Number _, ATCC Accession Number _, ATCC Accession Number _, ATCC Accession Number _, ATCC Accession Number _, or ATCC Accession Number _. In other embodiments, the 1983, 52881, 2398, 45449, 50289, or 52872 polypeptide is a fragment of at least 15, 20, 50, 100, 150, 200, 250, 300 or more contiguous amino acids of SEQ ID NO:2, SEQ ID NO:5, SEQ ID NO:8, SEQ ID NO:11, SEQ ID NO:14, SEQ ID NO:17; or an amino acid sequence encoded by the cDNA insert of the plasmid deposited with ATCC Accession Number _, ATCC Accession Number _, ATCC Accession Number _, ATCC Accession Number _, ATCC Accession Number _, or ATCC Accession Number _.

In a related aspect, the invention further provides nucleic acid constructs which include a 1983, 52881, 2398, 45449, 50289, or 52872 nucleic acid molecule described herein.

In a related aspect, the invention provides 1983, 52881, 2398, 45449, 50289, or 52872 polypeptides or fragments operatively linked to non-52881 polypeptides to form fusion proteins.

In another aspect, the invention features antibodies and antigen-binding fragments thereof, that react with, or more preferably specifically bind 1983, 52881, 2398, 45449, 50289, or 52872 polypeptides.

In another aspect, the invention provides methods of screening for compounds that modulate the expression or activity of the 1983, 52881, 2398, 45449, 50289, or 52872 polypeptides or nucleic acids.

In still another aspect, the invention provides a process for modulating 1983, 52881, 2398, 45449, 50289, or 52872 polypeptide or nucleic acid expression or activity, e.g. using the screened Compounds. In certain embodiments, the methods involve treatment of conditions related to activity or expression of the 1983, 52881, 2398, 45449, 50289, or 52872 polypeptides or nucleic acids, such as cardiovascular disorders, angiogenesis-related disorders, neural disorders, conditions involving pain response, aberrant or altered pain responses, pain related disorders, or inflammatory responses.

Examples of cardiovascular disorders include e.g., atherosclerosis, thrombosis, heart failure, ischemic heart disease, angina pectoris, myocardial infarction, sudden cardiac death, hypertensive heart disease; non-coronary vessel disease, such as arteriolosclerosis, small vessel disease, nephropathy, hypertriglyceridemia, hypercholesterolemia, hyperlipidemia, xanthomatosis, asthma, hypertension, emphysema and chronic pulmonary disease; or a cardiovascular condition associated with interventional procedures ("procedural vascular trauma"), such as restenosis following angioplasty, placement of a shunt, stet, stent, synthetic or natural excision grafts, indwelling catheter, valve or other implantable devices.

In one embodiment, the cardiovascular disorder is caused by aberrant fatty acid metabolism. Examples of disorders involving aberrant fatty acid metabolism include, but are not limited to, atherosclerosis, arteriolosclerosis, hypertriglyceridemia, obesity, diabetes, hypercholesterolemia, xanthomatosis, and hyperlipidemia. Most preferable, the disorder is atherosclerosis.

In the cardiovascular applications, an agent is administered alone or in combination with a cholesterol-lowering agent. Examples of cholesterol lowering agents include bile acid sequestering resins (e.g. colestipol hydrochloride or cholestyramine), fibric acid derivatives (e.g. clofibrate, fenofibrate, or gemfibrozil), thiazolidenediones (e.g. troglitazone), or hydroxymethylglutaryl coenzyme A reductase (HMG-CoA reductase) inhibitors (e.g. statins, such as fluvastatin sodium, lovastatin, pravastatin sodium, or simvastatin), an ApoAII-lowering agent, a VLDL lowering agent, an ApoAI-stimulating agent, as well as inhibitors of, nicotinic acid, niacin, or probucol. Preferred cholesterol lowering agents include inhibitors of IIMG-CoA reductase (e.g., statins), nicotinic acid, and niacin.

The cholesterol-lowering agent can be administered prior to, at the same time, or after administration of the agent, in single or multiple administration schedules. For example, the cholesterol lowering agent and the agents of the invention can be administered continually over a preselected period of time, or administered in a series of spaced doses, i.e., intermittently, for a period of time.

In preferred embodiments, the agent, alone or in combination with, the cholesterol lowering agent, inhibit (block or reduce) atherosclerotic lesion formation or development, e.g., so as to inhibit lipid accumulation, increase plaque stability or promote lesion regression.

In a preferred embodiment, the agent, administered alone or in combination with the cholesterol lowering agent, results in a favorable plasma lipid profile (e.g., increased HDL and/or reduced LDL).

In a preferred embodiment, the agent modulates (e.g., decreases or increases) the activity or expression of a 1983, 52881, 2398, 45449, 50289, or 52872 polypeptide or nucleic acid.

In a preferred embodiment, the agent modulates (e.g., increases or decreases) expression of the 1983, 52881, 2398, 45449, 50289, or 52872 nucleic acid by, e.g., modulating transcription, mRNA stability, etc.

In preferred embodiments, the agent is a peptide, a phosphopeptide, a small molecule, e.g., a member of a combinatorial or natural product library, or an antibody, or any combination thereof.

In additional preferred embodiments, the agent is an antisense, a ribozyme, or a triple helix molecule, or a 1983, 52881, 2398, 45449, 50289, or 52872 nucleic acid or a fragment thereof, or any combination thereof.

In a preferred embodiment, the subject is a patient undergoing a therapeutic or prophylactic protocol. Preferably, the subject is a human suffering from, or at risk of a cardiovascular disease, e.g., atherosclerosis, thrombosis, heart failure, ischemic heart disease, angina pectoris, myocardial infarction, sudden cardiac death, hypertensive heart disease; non-coronary vessel disease, such as arteriolosclerosis, small vessel disease, nephropathy, hypertriglyceridemia, obesity, diabetes, hypercholesterolemia, hyperlipidemia, xanthomatosis, asthma, hypertension, emphysema and chronic pulmonary disease; or a cardiovascular condition associated with interventional procedures ("procedural vascular trauma"), such as restenosis following angioplasty, placement of a shunt, stet, stent, synthetic or natural excision grafts, indwelling catheter, valve or other implantable devices.

In a preferred embodiment, the subject is a human suffering from, or at risk of a disorder involving aberrant fatty acid metabolism. Examples of such disorders include, but are not limited to, atherosclerosis, arteriolosclerosis, hypertriglyceridemia, obesity, diabetes, hypercholesterolemia, xanthomatosis and hyperlipidemia. Most preferable, the disorder is atherosclerosis.

In other embodiments, the subject is a non-human animal, e.g., an experimental animal.

In yet another aspect, the invention features a method of treating or preventing a cardiovascular disorder (e.g., atherosclerosis), in a subject. The method includes administering to the subject an agent that modulates the activity or expression of a 1983, 52881, 2398, or 45449 polypeptide or nucleic acid, in an amount effective to treat or prevent the cardiovascular disorder.

In yet another aspect, the invention features a method of treating or preventing a disease related to angiogenesis or neovascularization in a subject. The method includes administering to the subject an agent that modulates the activity or expression of a 1983, 52881, 2398, or 45449, 50289, or 52872 polypeptide or nucleic acid, in an amount effective to treat or prevent the disorder. Diseases in which angiogenesis or neovascularization play a role include neoplastic disease, retinopathy (e.g., diabetic retinopathy), and macular degeneration.

The invention also features a method of diagnosing a disorder, e.g., a cardiovascular disorder (e.g., atherosclerosis) or angiogenesis-related disorder, in a subject. The method includes evaluating the expression or activity of a 1983, 52881, 2398, 45449, 50289, or 52872 nucleic acid or a 1983, 52881, 2398, 45449, 50289, or 52872 polypeptide, such that, a difference in the level of 1983, 52881, 2398, 45449, 50289, or 52872 nucleic acid or 1983, 52881, 2398, 45449, 50289, or 52872 polypeptide relative to a normal subject or a cohort of normal subjects is indicative of the disorder.

In a preferred embodiment, the subject is a human.

In a preferred embodiment, the evaluating step occurs *in vitro* or *ex vivo*. For example, a sample, e.g., a blood sample, is obtained from the subject.

In a preferred embodiment, the evaluating step occurs *in vivo*. For example, by administering to the subject a detectably labeled agent that interacts with the 1983, 52881, 2398, 4544.9, 50289, or 52872 nucleic acid or polypeptide, such that a signal is generated relative to the level of activity or expression of the 1983, 52881, 2398, 45449, 50289, or 52872 nucleic acid or polypeptide.

In a preferred embodiment, the disorder is a cardiovascular disorder, e.g., a cardiovascular disorder as described herein.

In a preferred embodiment, the disorder is atherosclerosis.

The invention also provides assays for determining the activity of or the presence or absence of 1983, 52881, 2398, 45449, 50289, or 52872 polypeptides or nucleic acid molecules in a biological sample, including for disease diagnosis.

In a further aspect, the invention provides assays for determining the presence or absence of a genetic alteration in a 1983, 52881, 2398, 45449, 50289, or 52872 polypeptide or nucleic acid molecule, including for disease diagnosis.

In yet another aspect, the invention features a method for identifying an agent, e.g., a compound, which modulates the activity of a 1983, 52881, 2398, 45449, 50289, or 52872 polypeptide, e.g., a 1983, 52881, 2398, 45449, 50289, or 52872 polypeptide as described herein, or the expression of a 1983, 52881, 2398, 45449, 50289, or 52872 nucleic acid, e.g., a 1983, 52881, 2398, 45449, 50289, or 52872 nucleic acid as described herein, including contacting the 1983, 52881, 2398, 45449, 50289, or 52872 polypeptide or nucleic acid with a test agent (e.g., a test compound); and determining the effect of the test compound on the activity of the polypeptide or nucleic acid to thereby identify a compound which modulates the activity of the polypeptide or nucleic acid. Such agents are useful for treating or preventing a 1983, 52881, 2398, 45449, 50289, or 52872-mediated disorders, e.g., cardiovascular disorders (e.g., atherosclerosis).

In a preferred embodiment, the contacting step occurs *in vitro* or *ex vivo*. For example, a sample, e.g., a blood sample, is obtained from the subject.

In a preferred embodiment, the contacting step occurs *in vivo*. For example, by administering to the subject a detectably labeled agent that interacts with the 1983, 52881, 2398, 45449, 50289, or 52872 nucleic acid or polypeptide, such that a signal is generated relative to the level of activity or expression of the 1983, 52881, 2398, 45449, 50289, or 52872 nucleic acid or polypeptide.

In a preferred embodiment, the agent is an inhibitor (partial or complete inhibitor) of 1983, 52881, 2398, 45449, 50289, or 52872 polypeptide activity or expression. For example, inhibiting 1983, 52881, 2398, 45449, 50289, or 52872 expression and/or activity may promote the growth of blood vessels through the process of angiogenesis.

In a preferred embodiment, the agent is an agonist of 1983, 52881, 2398, 45449, 50289, or 52872 polypeptide activity or expression. For example, increasing 1983, 52881, 2398, 45449, 50289, or 52872 expression and/or activity may inhibit the process of angiogenesis. Such an agent would be particularly useful in inhibiting unwanted angiogenesis, e.g., angiogenesis associated with tumor growth.

In preferred embodiments, the agent is a peptide, a phosphopeptide, a small molecule, e.g., a member of a combinatorial library, or an antibody, or any combination thereof.

In additional preferred embodiments, the agent is an antisense, a ribozyme, a triple helix molecule, or a 1983, 52881, 2398, 45449, 50289, or 52872 nucleic acid, or any combination thereof.

In still another aspect, the invention features a method of modulating (e.g., enhancing or inhibiting) a pain response or an inflammatory response. The method includes contacting a cell with an agent that modulates the activity or expression of a 1983, 52881, 2398, 45449, 50289, or 52872 polypeptide or nucleic acid, in an amount effective to modulate the pain response or inflammatory response.

In a preferred embodiment, the agent modulates (e.g., increases or decreases) signaling through a pain associated receptor, e.g., a 1983, 52881, 2398, 45449, 50289, or 52872 polypeptide described herein.

In a preferred embodiment, the agent modulates (e.g., increases or decreases) expression of the 1983, 52881, 2398, 45449, 50289, or 52872 nucleic acid by, e.g., modulating transcription, mRNA stability, etc.

In preferred embodiments, the agent is a peptide, a phosphopeptide, a small molecule, e.g., a member of a combinatorial library, or an antibody, or any combination thereof. The antibody can be conjugated to a therapeutic moiety selected from the group consisting of a cytotoxin, a cytotoxic agent and a radioactive metal ion.

In additional preferred embodiments, the agent is an antisense molecule, a ribozyme, a triple helix molecule, or a 1983, 52881, 2398, 45449, 50289, or 52872 nucleic acid, or any combination thereof.

In a preferred embodiment, the agent is administered in combination with a cytotoxic agent.

In a preferred embodiment, the cell, e.g., the 1983, 52881, 2398, 45449, 50289, or 52872-expressing cell, is a neural cell, e.g., central or peripheral nervous system cell (e.g., a cell in an area involved in pain control, e.g., a cell in the substantia gelatinosa of the spinal cord, or a cell in the periaqueductal gray matter).

In a preferred embodiment, the agent and the 1983, 52881, 2398, 45449, 50289, or 52872-polypeptide or nucleic acid are contacted *in vitro* or *ex vivo*.

In a preferred embodiment, the contacting step is effected *in vivo* in a subject, e.g., as part of a therapeutic or prophylactic protocol. Preferably, the subject is a human, e.g., a patient with pain or a pain-associated disorder disclosed herein. For example, the subject can be a patient with pain elicited from tissue injury, e.g., inflammation, infection, ischemia; pain associated with musculoskeletal disorders, e.g., joint pain; tooth pain; headaches, e.g., migrane; pain associated with surgery; pain related to inflammation, e.g., irritable bowel syndrome; or chest pain. The subject can be a patient with complex regional pain syndrome (CRPS), reflex sympathetic dystrophy (RSD), causalgia, neuralgia, central pain and dysesthesia syndrome, carotidynia, neurogenic pain, refractory cervicobrachial pain syndrome, myofascial pain syndrome, craniomandibular pain dysfunction syndrome, chronic idiopathic pain syndrome, Costen's pain-dysfunction, acute chest pain syndrome, gynecologic pain syndrome, patellofemoral pain syndrome, anterior knee pain syndrome, recurrent abdominal pain in children, colic, low back pain syndrome, neuropathic pain, phantom pain from amputation, phantom tooth pain, or pain asymbolia. The subject can be a cancer patient, e.g., a patient with brain cancer, bone cancer, or prostate cancer. In other embodiments, the subject is a non-human animal, e.g., an experimental animal, e.g., an arthritic rat model of chronic pain, a chronic constriction injury (CCI) rat model of neuropathic pain, or a rat model of unilateral inflammatory pain by intraplantar injection of complete Freund's adjuvant (CFA).

The contacting step(s) can be repeated.

In preferred embodiments, the agent is a peptide, a phosphopeptide, a small molecule, e.g., a member of a combinatorial library, or an antibody, or any combination thereof. The antibody can be conjugated to a therapeutic moiety selected from the group consisting of a cytotoxin, a cytotoxic agent and a radioactive metal ion.

In additional preferred embodiments, the agent is an antisense, a ribozyme, or a triple helix molecule, or a 1983, 52881, 2398, 45449, 50289, or 52872 nucleic acid, or any combination thereof.

In a preferred embodiment, the agent is administered in combination with a cytotoxic agent.

The administration of the agent and/or protein can be repeated.

In still another aspect, the invention features a method for evaluating the efficacy of a treatment of a disorder, e.g., a disorder disclosed herein, in a subject. The method includes treating a subject with a protocol under evaluation; assessing the expression of a 1983, 52881, 2398, 45449, 50289, or 52872 nucleic acid or 1983, 52881, 2398, 45449, 50289, or 52872 polypeptide, such that a change in the level of 1983, 52881, 2398, 45449, 50289, or 52872 nucleic acid or 1983, 52881, 2398, 45449, 50289, or 52872 polypeptide after treatment, relative to the level before treatment, is indicative of the efficacy of the treatment of the disorder.

In a preferred embodiment, the disorder is pain or a pain related disorder.

In a preferred embodiment, the subject is a human.

The invention also features a method of diagnosing a disorder, e.g., a disorder disclosed herein, in a subject. The method includes evaluating the expression or activity of a 1983, 52881, 2398, 45449, 50289, or 52872 nucleic acid or a 1983, 52881, 2398, 45449, 50289, or 52872 polypeptide, such that, a difference in the level of 1983, 52881, 2398, 45449, 50289, or 52872 nucleic acid or 1983, 52881, 2398, 45449, 50289, or 52872 polypeptide relative to a normal subject or a cohort of normal subjects is indicative of the disorder.

In a preferred embodiment, the disorder is a neurological disorder.

In a preferred embodiment, the disorder is pain or a pain related disorder.

In a preferred embodiment, the subject is a human.

In a preferred embodiment, the evaluating step occurs *in vitro* or *ex vivo*. For example, a sample, e.g., a blood sample, is obtained from the subject.

In a preferred embodiment, the evaluating step occurs *in vivo*. For example, by administering to the subject a detectably labeled agent that interacts with the 1983, 52881, 2398, 45449, 50289, or 52872 nucleic acid or polypeptide, such that a signal is generated relative to the level of activity or expression of the 1983, 52881, 2398, 45449, 50289, or 52872 nucleic acid or polypeptide.

In another aspect, the invention features a two dimensional array having a plurality of addresses, each address of the plurality being positionally distinguishable from each other address of the plurality, and each address of the plurality having a unique capture probe, e.g., a nucleic acid or peptide sequence. At least one address of the plurality has a capture probe that recognizes a 1983, 52881, 2398, 45449, 50289, or 52872 molecule. In one embodiment, the capture probe is a nucleic acid, e.g., a probe complementary to a 1983, 52881, 2398, 45449, 50289, or 52872 nucleic acid sequence. In another embodiment, the capture probe is a polypeptide, e.g., an antibody specific for 1983, 52881, 2398, 45449, 50289, or 52872 polypeptides. Also featured is a method of analyzing a sample by contacting the sample to the aforementioned array and detecting binding of the sample to the array.

Other features and advantages of the invention will be apparent from the following detailed description, and from the claims.

### Brief Description of the Drawings

*Figures 1A-1D* depict a cDNA sequence (SEQ ID NO:1) and predicted amino acid sequence (SEQ ID NO:2) of human 1983. The methionine-initiated open reading frame of human 1983 (without the 5' and 3' untranslated regions) is shown as coding sequence SEQ ID NO:3.

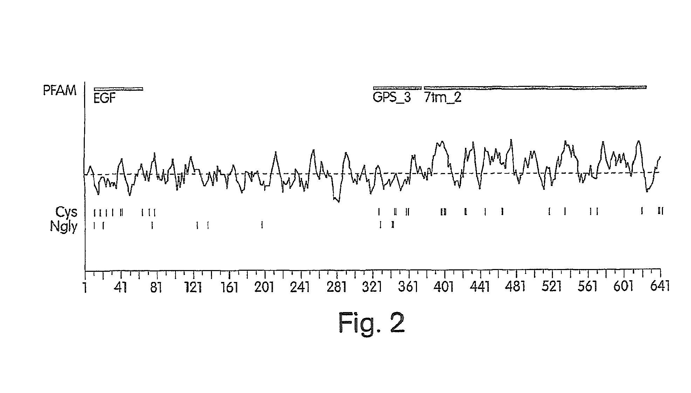

*Figure 2* depicts a hydropathy plot of human 1983 receptor. Relative hydrophobic residues are shown above the dashed horizontal line, and relative hydrophilic residues are below the dashed horizontal line. The location of the transmembrane domains is also indicated. The cysteine residues (cys) and N-glycosylation sites (Ngly) are indicated by short vertical lines just below the hydropathy trace. The numbers corresponding to the amino acid sequence of human 1983 receptor are indicated. Polypeptides of the invention include fragments which include: all or part of a hydrophobic sequence, i.e., a sequence above the dashed line, e.g., the sequence from about amino acid 210-220, from about 290-300, and from about 365-375 of SEQ ID NO:2; all or part of a hydrophilic sequence, i.e., a sequence below the dashed line, e.g., the sequence of from about amino acid 15-35, from about 195-205, and from about 275-285 of SEQ ID NO:2; a sequence which includes a Cys, or a glycosylation site.
*Figure* 3 depicts an alignment of the seven transmembrane (7tm) domain of human 1983 with a consensus amino acid sequence derived from a hidden Markov model. The upper sequence is the consensus amino acid sequence (SEQ ID NO:19), while the lower amino acid sequence corresponds to amino acids 379 to 626 of SEQ ID NO:2.
*Figure 4* depicts an alignment of the EGF-lilce domain of human 1983 with a consensus amino acid sequence derived from a hidden Markov model. The upper sequence is the consensus amino acid sequence (SEQ ID NO:20), while the lower amino acid sequence corresponds to amino acids 17 to 54 of SEQ ID NO:2.
*Figure 5* depicts an alignment of the latrophilin/CL-1-like GPS domain of human 1983 with a consensus amino acid sequence derived from a hidden Markov model. The upper sequence is the consensus amino acid sequence (SEQ ID NO:21), while the lower amino acid sequence corresponds to amino acids 321 to 373 of SEQ ID NO:2.
*Figures 6A-6D* depict a cDNA sequence (SEQ ID NO:4) and predicted amino acid sequence (SEQ ID NO:5) of human 52881. The methionine-initiated open reading frame of human 52881 (without the 5' and 3' untranslated regions) is shown as coding sequence SEQ ID NO:6.

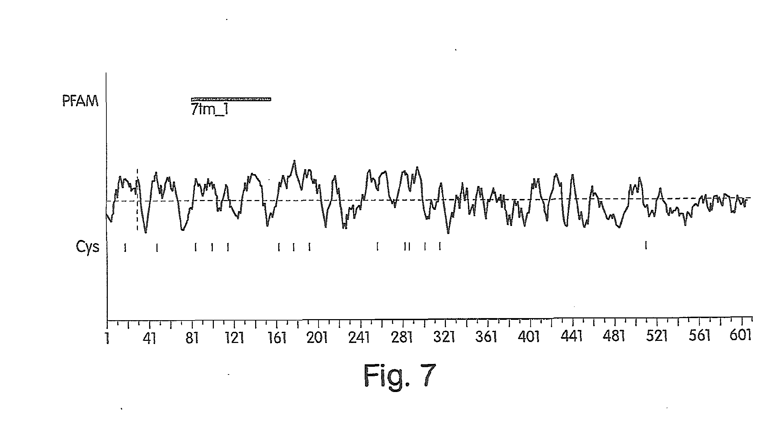

*Figure 7* depicts a hydropathy plot of human 52881. Relative hydrophobic residues are shown above the dashed horizontal line, and relative hydrophilic residues are below the dashed horizontal line. The location of the transmembrane domains is also indicated. The cysteine residues (cys) and N-glycosylation sites (Ngly) are indicated by short vertical lines just below the hydropathy trace. The numbers corresponding to the amino acid sequence of human 52881 receptor are indicated. Polypeptides of the invention include fragments which include: all or part of a hydrophobic sequence, i.e., a sequence above the dashed line, e.g., the sequence from about amino acid 280-300, from about 420-430, and from about 495-505 of SEQ ID NO:5; all or part of a hydrophilic sequence, i.e., a sequence below the dashed line, e.g., the sequence of from about amino acid 225-240, from about 475-490, and from about 540-555 of SEQ ID NO:5; a sequence which includes a Cys, or a glycosylation site.
*Figure 8* depicts an alignment of the seven transmembrane (7tm) domain of human 52881 with a consensus amino acid sequence derived from a hidden Markov model. The upper sequence is the consensus amino acid sequence (SEQ ID NO:22), while the lower amino acid sequence corresponds to amino acids 80 to 154 of SEQ ID NO:5.
*Figures 9A-9B* depict a cDNA sequence (SEQ ID NO:7) and predicted amino acid sequence (SEQ ID NO:8) of human 2398. The methionine-initiated open reading frame of human 2398 (without the 5' and 3' untranslated regions) is shown as coding sequence SEQ ID NO:9.

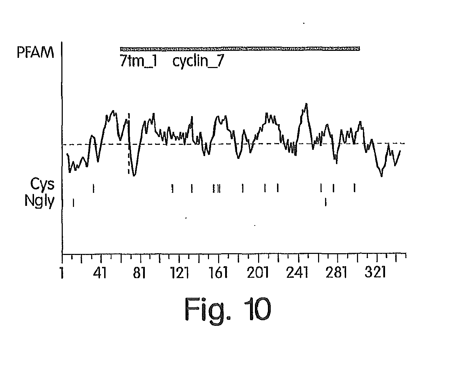

*Figure 10* depicts a hydropathy plot of human 2398. Relative hydrophobic residues are shown above the dashed horizontal line, and relative hydrophilic residues are below the dashed horizontal line. The location of the transmembrane domains is also indicated. The cysteine residues (cys) and N-glycosylation sites (Ngly) are indicated by short vertical lines just below the hydropathy trace. The numbers corresponding to the amino acid sequence of human 2398 receptor are indicated. Polypeptides of the invention include fragments which include: all or part of a hydrophobic sequence, i.e., a sequence above the dashed line, e.g., the sequence from about amino acid 265-275 and from about 285-295 of SEQ ID NO:8; all or part of a hydrophilic sequence, i.e., a sequence below the dashed line, e.g., the sequence of from about amino acid 1-25, from about 70-80, and from about 320-330 of SEQ ID NO:8; a sequence which includes a Cys, or a glycosylation site.
*Figure 11* depicts an alignment of the seven transmembrane (7tm) domain of human 2398 with a consensus amino acid sequence derived from a hidden Markov model. The upper sequence is the consensus amino acid sequence (SEQ ID NO:23), while the lower amino acid sequence corresponds to amino acids 58 to 303 of SEQ ID NO:8.
*Figures 12A-12B* depict a cDNA sequence (SEQ ID NO:10) and predicted amino acid sequence (SEQ ID NO:11) of human 45449. The methionine-initiated open reading frame of human 45449 (without the 5' and 3' untranslated regions) is shown as coding sequence SEQ ID NO:12.

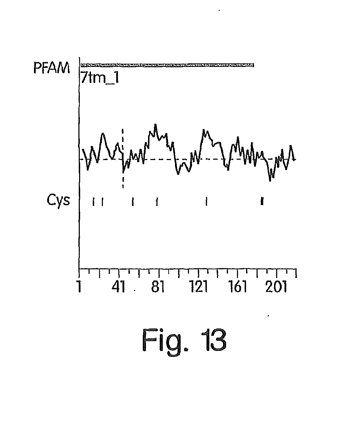

*Figure 13* depicts a hydropathy plot of human 45449. Relative hydrophobic residues are shown above the dashed horizontal line, and relative hydrophilic residues are below the dashed horizontal line. The location of the transmembrane domains are also indicated. The cysteine residues (cys) and N-glycosylation sites (Ngly) are indicated by short vertical lines just below the hydropathy trace. The numbers corresponding to the amino acid sequence of human 45449 receptor are indicated. Polypeptides of the invention include fragments which include: all or part of a hydrophobic sequence, i.e., a sequence above the dashed line, e.g., the sequence from about amino acid 160-170 of SEQ ID NO:11; all or part of a hydrophilic sequence, i.e., a sequence below the dashed line, e.g., the sequence of from about amino acid 100-110 and from about 195-205 of SEQ ID NO: 11; a sequence which includes a Cys, or a glycosylation site.
*Figure 14* depicts an alignment of the seven transmembrane (7tm) domain of human 45449 with a consensus amino acid sequence derived from a hidden Markov model. The upper sequence is the consensus amino acid sequence (SEQ ID NO:24), while the lower amino acid sequence corresponds to amino acids 1 to 176 of SEQ ID NO:11.
*Figures 15A-15E* depict a cDNA sequence (SEQ ID NO:13) and predicted amino acid sequence (SEQ ID NO:14) of human 50289. The methionine-initiated open reading frame of human 50289 (without the 5' and 3' untranslated regions) is shown as coding sequence SEQ ID NO:15.

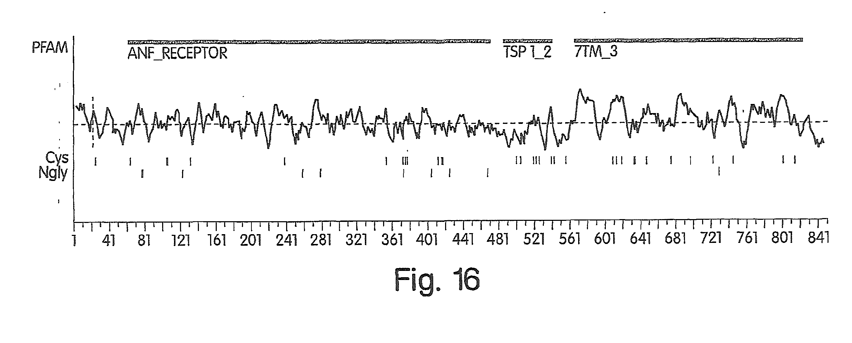

*Figure 16* depicts a hydropathy plot of human 50289. Relative hydrophobic residues are shown above the dashed horizontal line, and relative hydrophilic residues are below the dashed horizontal line. The location of the transmembrane domains is also indicated. The cysteine residues (cys) and N-glycosylation sites (Ngly) are indicated by short vertical lines just below the hydropathy trace. The numbers corresponding to the amino acid sequence of human 50289 receptor are indicated. Polypeptides of the invention include fragments which include: all or part of a hydrophobic sequence, i.e., a sequence above the dashed line, e.g., the sequence from about amino acid 70-80, from about 150-165, and from about 220-240 of SEQ ID NO:14; all or part of a hydrophilic sequence, i.e., a sequence below the dashed line, e.g., the sequence of from about amino acid 50-60, from about 480-510, and from about 545-560 of SEQ ID NO:14; a sequence which includes a Cys, or a glycosylation site.
*Figure 17* depicts an alignment of the ANF receptor ligand binding domain of human 50289 with a consensus amino acid sequence derived from a hidden Markov model. The upper sequence is the consensus amino acid sequence (SEQ ID NO:25), while the lower amino acid sequence corresponds to amino acids 61 to 470 of SEQ ID NO:14.
*Figure 18A-18B* depict a cDNA sequence (SEQ ID NO:16) and predicted amino acid sequence (SEQ ID NO:17) of human 52872. The metlionine-initiated open reading frame of human 52872 (without the 5' and 3' untranslated regions) is shown as coding sequence SEQ ID NO:18.

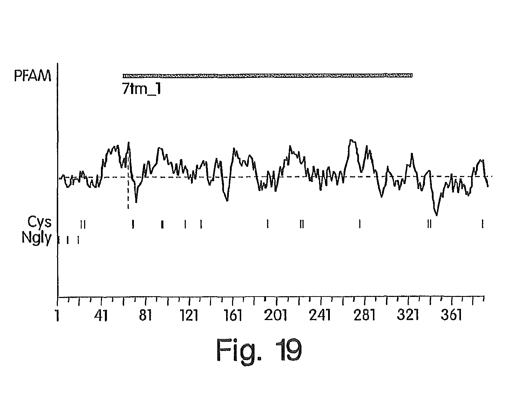

*Figures 19* depicts a hydropathy plot of human 52872. Relative hydrophobic residues are shown above the dashed horizontal line, and relative hydrophilic residues are below the dashed horizontal line. The cysteine residues (cys) are indicated by short vertical lines just below the hydropathy trace. The numbers corresponding to the amino acid sequence of human 52872 are indicated. Polypeptides of the invention include fragments which include: all or part of a hydrophobic sequence, i.e., a sequence above the dashed line, e.g., the sequence from about amino acid 45-65, from about 165-180, and from about 210-225 of SEQ ID NO:17; all or part of a hydrophilic sequence, i.e., a sequence below the dashed line, e.g., the sequence of from about amino acid 295-300, from about 345-360, and from about 370-380 of SEQ ID NO:17; a sequence which includes a Cys, or a glycosylation site.
*Figure 20* depicts an alignment of the seven transmembrane receptor domain of human 52872 with a consensus amino acid sequence derived from a hidden Markov model (HMM) from PFAM. The upper sequence is the consensus amino acid sequence (SEQ ID NO:23), while the lower amino acid sequence corresponds to amino acids 59 to 323 of SEQ ID NO:17.
*Figure 21* depicts relative 52872 mRNA levels in tissue samples derived from human adrenal gland, brain, heart, kidney, liver, lung, mammary gland, placenta, prostate, pituitary gland, muscle, small intestine, spleen, stomach, testes, thymus, trachea, uterus, spinal cord, skin, and dorsal root ganglion (DRG).
*Figure 22* depicts relative 52872 mRNA levels in tissue samples derived from human brain, spinal cord, heart, kidney, liver, lung, DRG, spinal cord, and skin.
*Figure 23* depicts relative 52872 mRNA levels in monkey tissue samples (cortex, DRG, spinal cord, sciatic nerve, kidney, hairy skin, heart, and liver) and in human tissue samples (brain, spinal cord, heart, kidney, liver, and lung).
*Figure 24* depicts the expression of 52872 in DRG following CFA injection, axotomy, and CCI at various days (D) following the treatment.
*Figure 25* depicts the expression of 52872 in spinal cord following CFA injection, axotomy, and CCI at various days (D) following the treatment.
*Figure 26* is a bar graph depicting relative 52881 expression as determined by hybridization on mRNA derived from the 293 cell line 293 (lane 1) and human umbilical vein endothelial cells (HUVEC) treated with: no added growth factors (lane 2); IL-1β (lane 3); or VEGF (lane 4). In lanes 5-7, HUVEC were plated and grown on Matrigel and expression was determined 2 hours after plating (lane 5), 6 hours after plating (lane 6), and 16 hours after plating (lane 7).
*Figure 27* depicts relative 1983 mRNA levels in normal and diseased tissue samples.
*Figure 28* depicts relative 1983 mRNA levels in normal human tissues.
*Figure 29* depicts relative 1983 mRNA levels in tissues and cell samples.
*Figures 30* depicts relative 1983 mRNA levels in mouse angiogenic tissues.
*Figure 31* depicts relative 1983 mRNA levels in an angiogenesis panel.
*Figure 32* depicts relative 1983 mRNA levels in the mouse hindlimb.
*Figure 33* depicts relative 2398 mRNA levels in tissues and cell samples.
*Figures 34* depicts relative 2398 mRNA levels in tissues and cell samples.
*Figure 35* depicts relative 45449 mRNA levels in tissues and cell samples.
*Figure 36* depicts relative 50289 mRNA levels in tissues and cell samples.
*Figures 37* depicts relative 50289 mRNA levels in tissues and cell samples.
*Figure 38* depicts relative 50289 mRNA levels in tissues and cell samples.

### Detailed Description of the Invention

### Human 1983

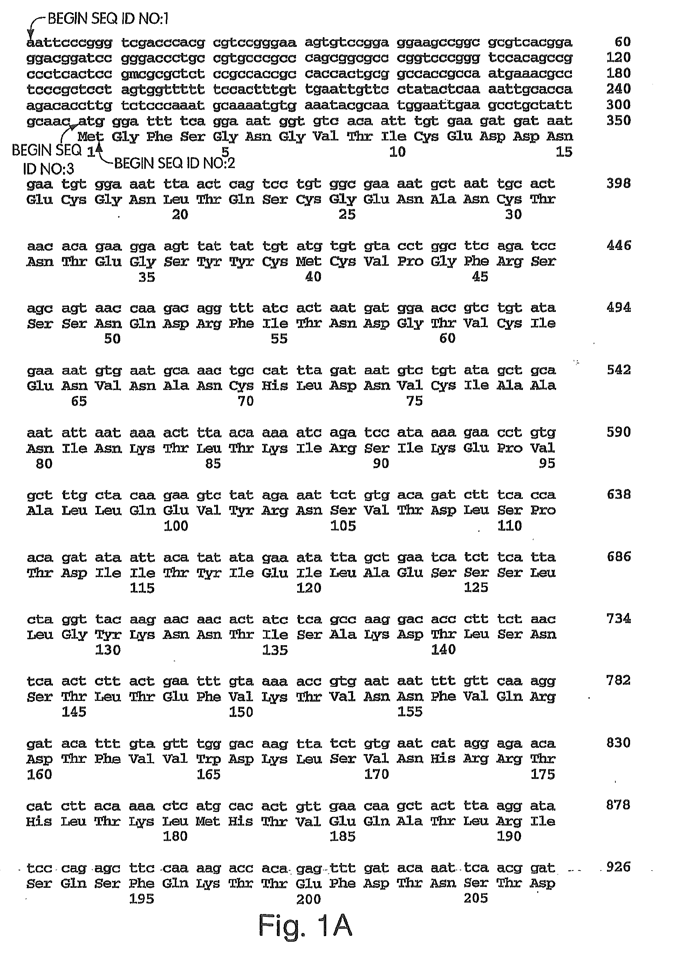

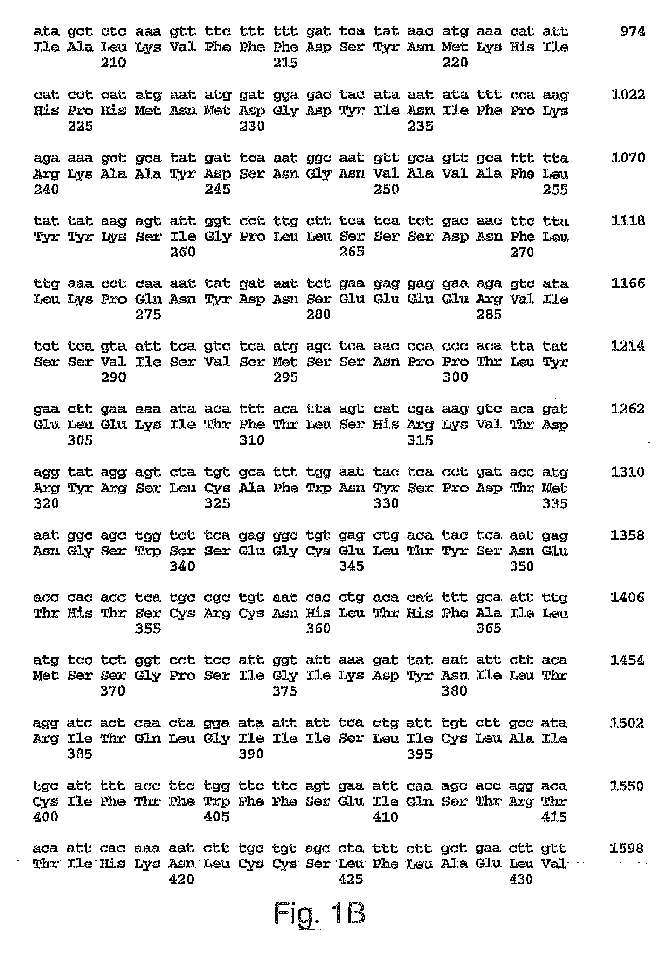

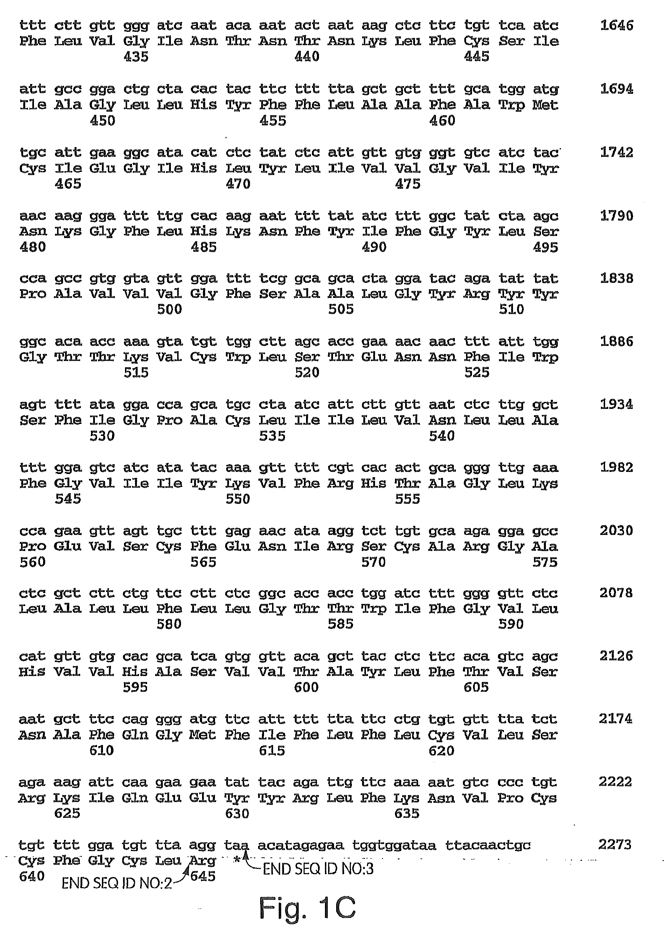

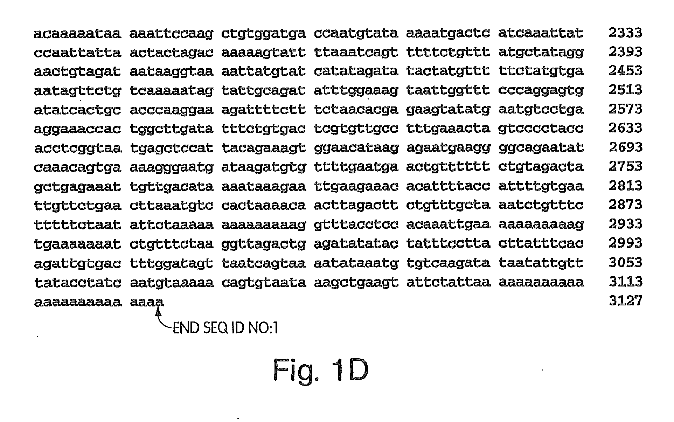

The human 1983 nucleotide sequence (Figures 1A-1D; SEQ ID NO:1), which is approximately 3127 nucleotides long including untranslated regions, contains a predicted methionine-initiated coding sequence of about 1938 nucleotides, including the termination codon (nucleotides indicated as coding of SEQ ID NO:1 in Figures 1A-1D; SEQ ID NO:3). The coding sequence encodes a 645 amino acid protein (Figure 2; SEQ ID NO:2).

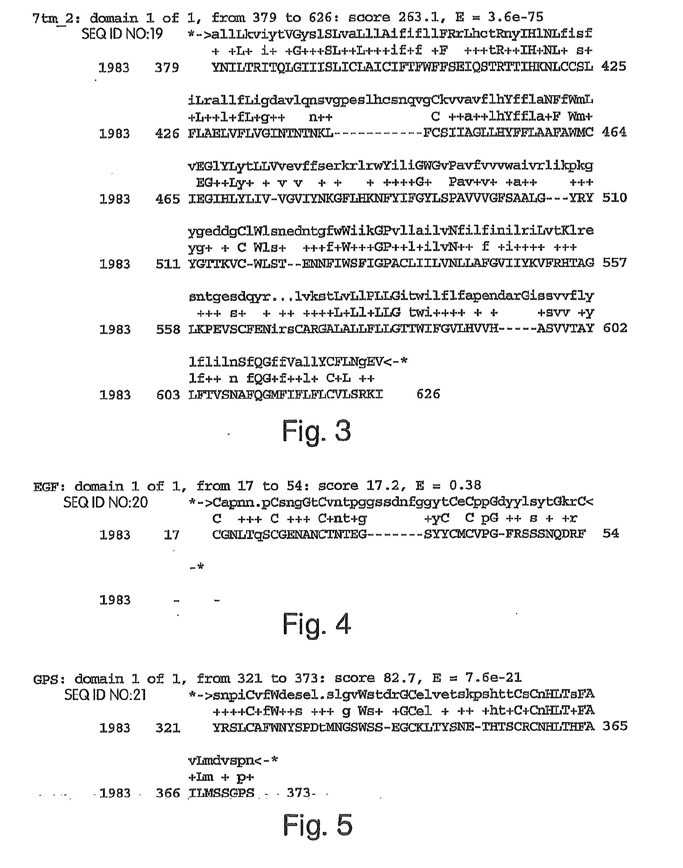

The human 1983 protein contains a predicted seven transmembrane (7TM) domain located at about amino acids 379 to 626 of SEQ ID NO:2 (Figure 3). Human 1983 additionally includes a predicted extracellular domain which extends from about amino acid 1 to about amino acid 387 of SEQ ID NO:2. The extracellular domain of the 1983 protein includes an EGF-like domain located at about amino acids 17-54 of SEQ ID NO:2 (Figure 4). The extracellular domain of the 1983 protein additionally includes a latrophilin CL-1-like GPS domain located at about amino acids 321-373 of SEQ ID NO:2 (Figure 5).

The seven transmembrane domain of the 1983 protein shows homology to members of the secretin family. Predicted transmembrane domains extend from about amino acid 388 (extracellular end) to about amino acid 407 (cytoplasmic end) of SEQ ID NO:2; from about amino acid 420 (cytoplasmic end) to about amino acid 436 (extracellular end) of SEQ ID NO:2; from about amino acid 455 (extracellular end) to about amino acid 479 (cytoplasmic end) of SEQ ID NO:2; from about amino acid 488 (cytoplasmic end) to about amino acid 508 (extracellular end) of SEQ ID NO:2; from about amino acid 525 (extracellular end) to about amino acid 549 (cytoplasmic end) of SEQ ID NO:2; from about amino acid 574 (cytoplasmic end) to about amino acid 591 (extracellular end) of SEQ ID NO:2; and from about amino acid 598 (extracellular end) to about amino acid 622 (cytoplasmic end) of SEQ ID NO:2; three cytoplasmic loops are located at about amino acids 408-419, 480-487 and 550-573 of SEQ ID NO:2; three extracellular loops are located at about amino acid 437-454, 509-524 and 590-597 of SEQ ID NO:2; and a C-terminal cytoplasmic domain is located at about amino acid residues 623-645 of SEQ ID NO:2.

The 1983 receptor protein additionally contains one predicted EF-hand calcium binding domain (PS00018) from about amino acids 108-120 of SEQ ID NO:2; ten predicted protein kinase C phosphorylation sites (PS00005) from about amino acids 90-92, 136-138, 188-190, 313-315, 318-320, 355-357, 412-414, 440-442, 513-515, and 623-625 of SEQ ID NO:2; fifteen predicted casein kinase II phosphorylation sites (PS00006) from about amino acids acids 9-12, 23-26, 31-34, 49-52, 90-93, 105-108, 110-113, 116-119, 136-139, 145-148, 199-202, 265-268, 280-283, 301-304, and 563-566 of SEQ ID NO:2; seven predicted N-myristoylation sites (PS00008) from about amino acids 5-10, 35-40, 337-342, 343-348, 389-394, 435-440, and 476-481 of SEQ ID NO:2; eight predicted N-glycosylation sites (PS00001) from about amino acids 19-22, 29-32, 82-85, 132-135, 143-146, 204-207, 336-339, and 350-353 of SEQ ID NO:2; one predicted glycosaminoglycan attachment site (PS00002) from about amino acid 4-7 of SEQ ID NO:2; one predicted cAMP/cGMP phosporylation site (PS00004) located at about amino acid 315-318 of SEQ ID NO:2; one tyrosine kinase phosphorylation site (PS00007) located at about amino acid 624-631 of SEQ ID NO:2; and one aspartic acid and asparagine hydroxylation site (PS00010) located at about amino acid 30-41 of SEQ ID NO:2.

A plasmid containing the nucleotide sequence encoding human 1983 (clone Fbh1983FL) was deposited with American Type Culture Collection (ATCC), 10801 University Boulevard, Manassas, VA 20110-2209, on _ and assigned Accession Number _. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

For general information regarding PFAM identifiers, PS prefix and PF prefix domain identification numbers, refer to Sonnhammer *et al*. (1997) *Protein* 28:405-420 and http://www.psc.edu/general/ software/packages/pfam/pfam.html.

### Human 52881

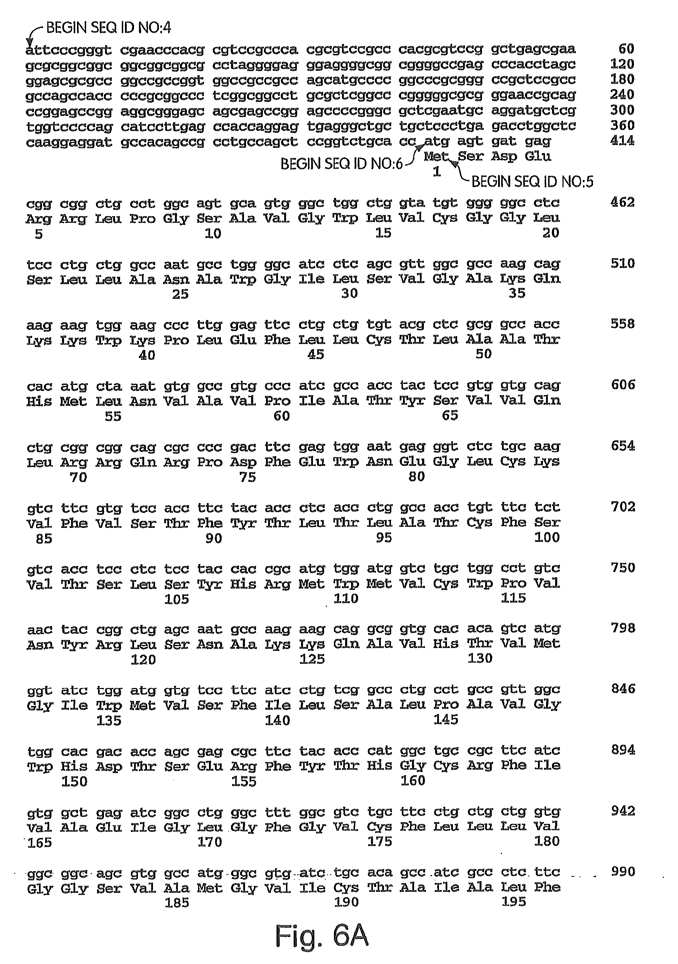

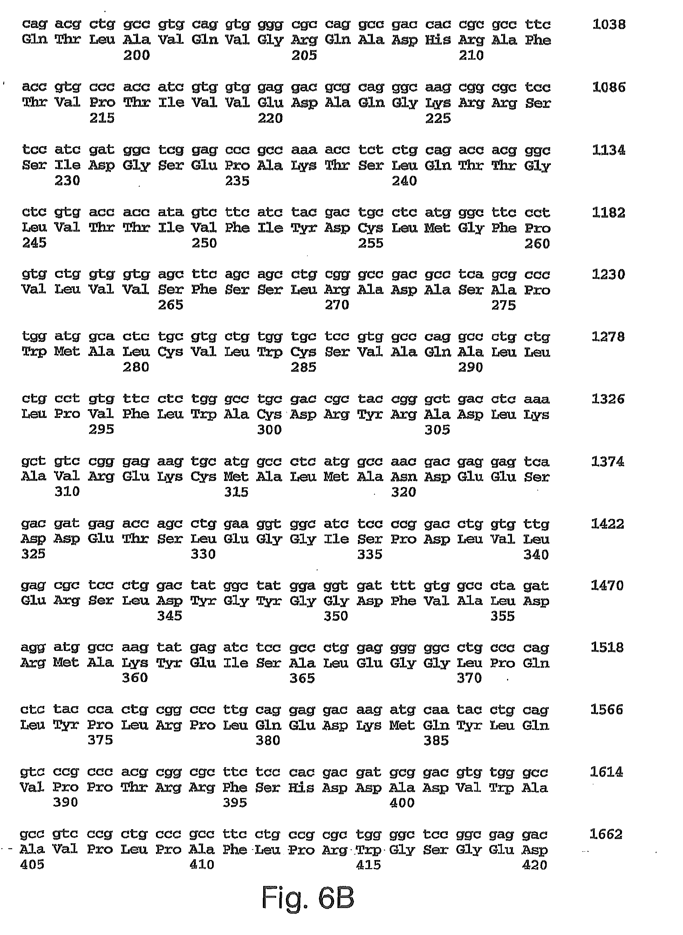

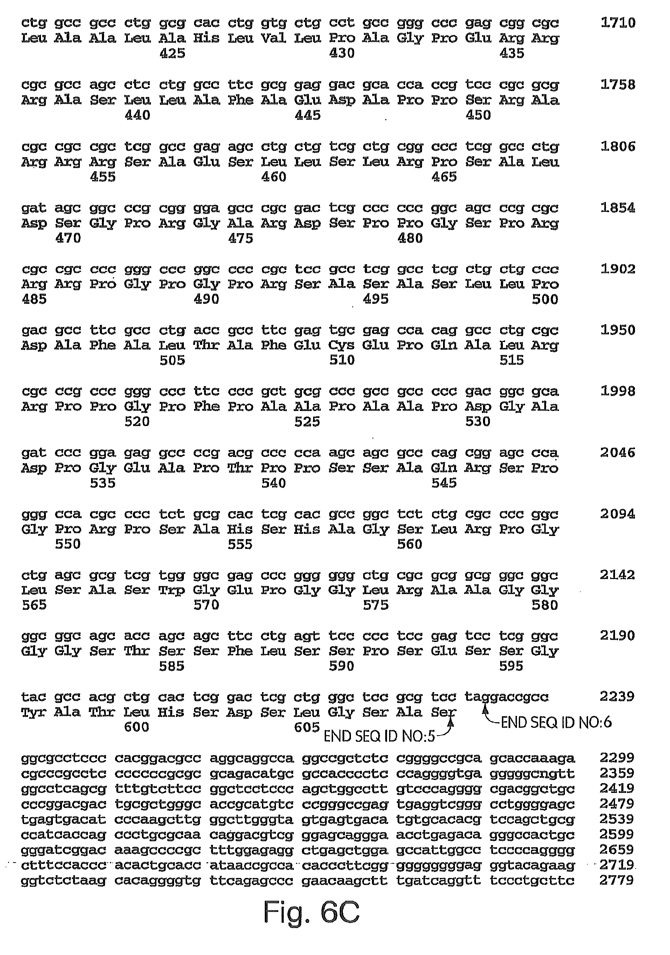

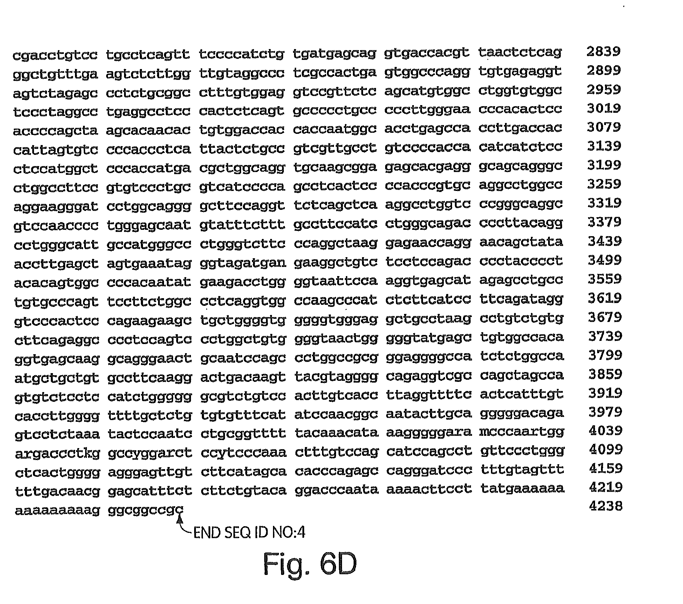

The human 52881 sequence (Figures 6A-6D; SEQ ID NO:4), which is approximately 4238 nucleotides long including untranslated regions, contains a predicted methionine-initiated coding sequence of about 1830 nucleotides, including the termination codon (nucleotides indicated as coding of SEQ ID NO:4 in Figures 6A-6D; SEQ ID NO:6). The coding sequence encodes a 609 amino acid protein (Figure 7; SEQ ID NO:5).

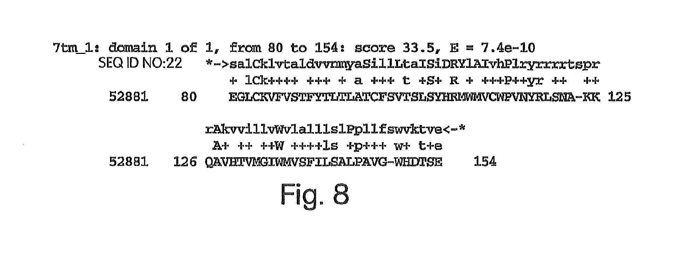

The 52881 protein contains a predicted seven transmembrane (7TM) domain located at about amino acids 80 to 154 of SEQ ID NO:5 (Figure 8). The seven transmembrane domain shows homology to members of the rhodopsin family. Predicted transmembrane domains extend from about amino acids 11-34, 44-67, 85-106, 127-149, 172-196, and 245-269 of SEQ ID NO:5 (Figure 2). Predicted non-transmembrane domains extend from about amino acids 1-10, 35-43, 68-84, 107-126, 150-171, 197-244, and 270-609 of SEQ ID NO:5.

The 52881 protein additionally contains: four predicted cAMP/cGMP phosporylation sites (PS00004) located at about amino acids 225-228, 393-396, 436-439, and 453-456 of SEQ ID NO:5; six predicted protein kinase C phosphorylation sites (PS00005) located at about amino acids 153-155, 268-270, 392-394, 462-464, 482-484, and 560-562 of SEQ ID NO:5; 10 predicted casein kinase II phosphorylation sites (PS00006) located at about amino acids 228-231, 324-327, 328-331, 364-367, 396-399, 417-420, 466-469, 506-509, 568-571, and 590-593 of SEQ ID NO:5; one predicted tyrosine kinase phosphorylation site (PS00007) located at about amino acids 342-348 of SEQ ID NO:5; 10 predicted N-myristoylation sites (PS00008) located at about amino acids 9-14, 169-174, 181-186, 187-192, 232-237, 244-249, 531-536, 564-569, 573-578 and 579-584 of SEQ ID NO:5; and one predicted amidation site (PS00009) from about amino acids 223-226 of SEQ ID NO:5.

A plasmid containing the nucleotide sequence encoding human 52881 (clone Fbh52881FL) was deposited with American Type Culture Collection (ATCC), 10801 University Boulevard, Manassas, VA 20110-2209, on _ and assigned Accession Number _. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

### Human 2398

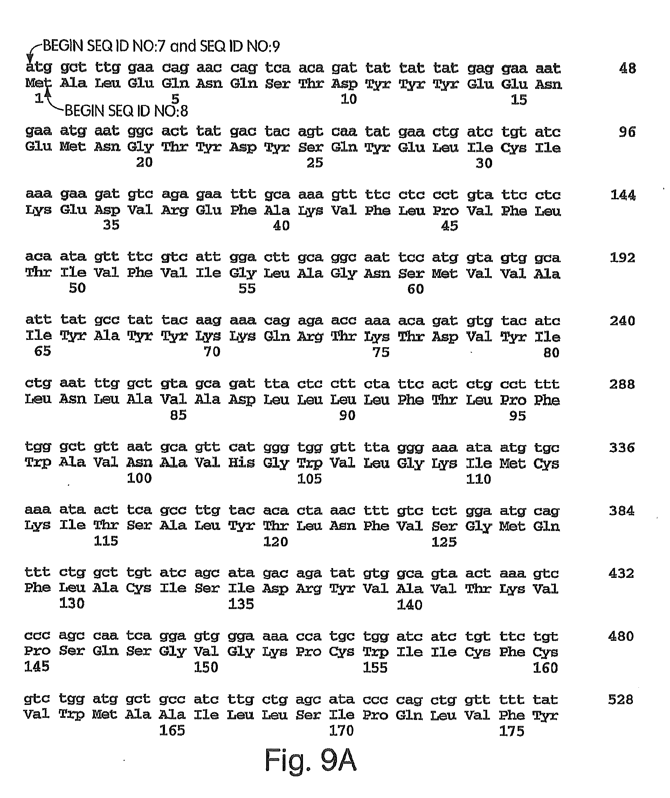

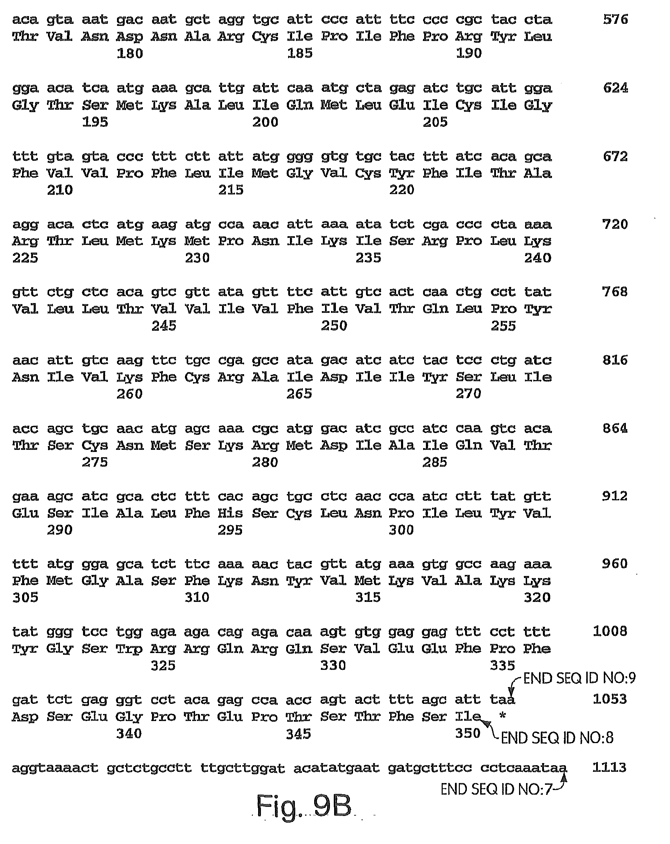

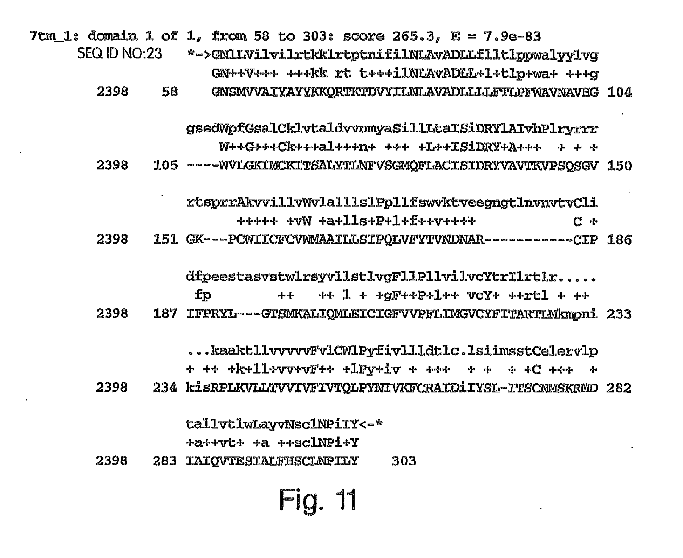

The human 2398 nucleotide sequence (Figures 9A-9B; SEQ ID NO:7), which is approximately 1113 nucleotides long including untranslated regions, contains a predicted methionine-initiated coding sequence of about 1053 nucleotides, including the termination codon (nucleotides indicated as coding of SEQ ID NO:7 in Figures 9A-9B; SEQ ID NO:9). The coding sequence encodes a 350 amino acid protein (Figure 10; SEQ ID NO:8). The 2398 protein contains a G-protein receptor signature (PS00237) located at about amino acids 125-141 of SEQ ID NO:8. The 2398 protein also includes a predicted seven transmembrane (7TM) domain located at about amino acids 58 to 303 of SEQ ID NO:8 (Figure 11). The seven transmembrane domain shows homology to members of the rhodopsin family. An extracellular domain extends from about amino acids 1-41 of SEQ ID NO:8. Predicted transmembrane domains (Figure 10) extend from about amino acid 42 (extracellular end) to about amino acid 66 (cytoplasmic end) of SEQ ID NO:8; from about amino acid 78 (cytoplasmic end) to about amino acid 99 (extracellular end) of SEQ ID NO:8; from about amino acid 114 (extracellular end) to about amino acid 135 (cytoplasmic end) of SEQ ID NO:8; from about amino acid 154 (cytoplasmic end) to about amino acid 176 (extracellular end) of SEQ ID NO:8; from about amino acid 202 (extracellular end) to about amino acid 224 (cytoplasmic end) of SEQ ID NO:8; from about amino acid 241 (cytoplasmic end) to about amino acid 259 (extracellular end) of SEQ ID NO:8; and from about amino acid 291 (extracellular end) to about amino acid 310 (cytoplasmic end) of SEQ ID NO:8; three cytoplasmic loops are located at about amino acids 67-77, 136-153, and 225-240 of SEQ ID NO:8; three extracellular loops are located at about amino acid 100-113, 177-201, and 260-290 of SEQ ID NO:8; and a C-terminal cytoplasmic domain is located at about amino acid residues 311-350 of SEQ ID NO:8.

The 2398 receptor protein additionally contains five predicted protein kinase C phosphorylation sites (PS00005) from about amino acids 195-197, 223-225, 278-280, 309-311 and 323-325 of SEQ ID NO:8; four predicted casein kinase II phosphorylation sites (PS00006) from about amino acids 25-28, 74-77, 177-180, and 330-333 of SEQ ID NO:8; one predicted glycosaminoglycan attachment site (PS00002) located at about amino acids 148-151 of SEQ ID NO:8; one predicted N-myristoylation site (PS00008) from about amino acids 55-60 of SEQ ID NO:8; and one tyrosine kinase phosphorylation site (PS00007) located at about amino acid 263-269 of SEQ ID NO:8.

A plasmid containing the nucleotide sequence encoding human 2398 (clone Fbh2398FL) was deposited with American Type Culture Collection (ATCC), 10801 University Boulevard, Manassas, VA 20110-2209, on _ and assigned Accession Number _. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

### Human 45449

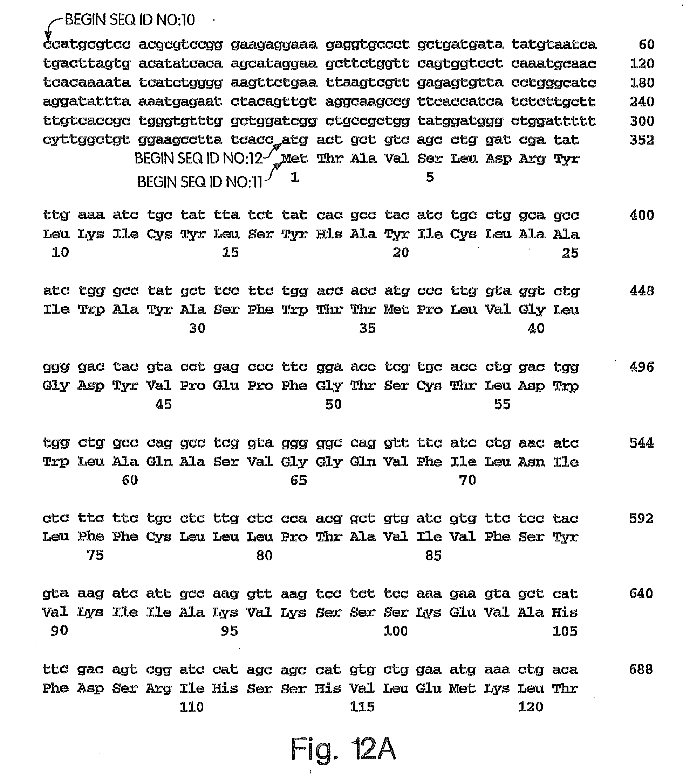

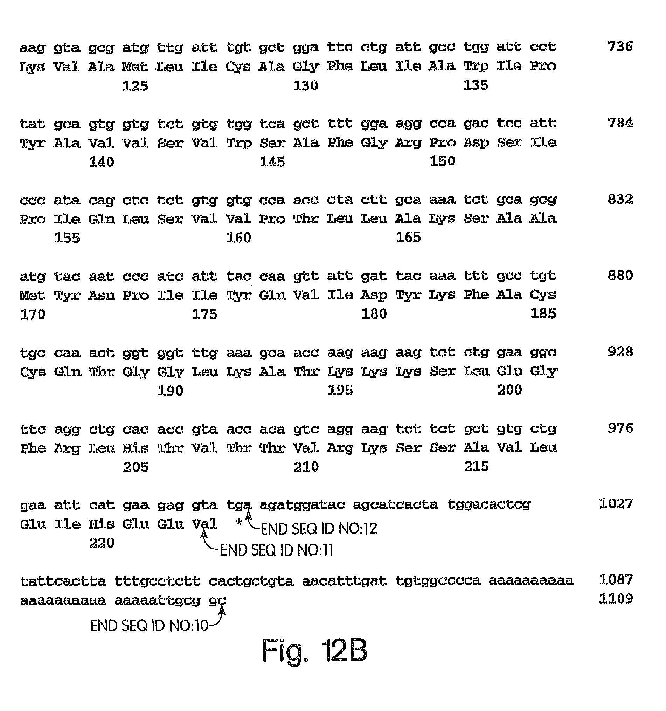

The human 45449 nucleotide sequence (Figures 12A-12B; SEQ ID NO:10), which is approximately 1109 nucleotides long including untranslated regions, contains a predicted methionine-initiated coding sequence of about 672 nucleotides, including the termination codon (nucleotides indicated as coding of SEQ ID NO:10 in Figures 12A-12B; SEQ ID NO:12). The coding sequence encodes a 223 amino acid protein (Figure 13; SEQ ID NO:11).

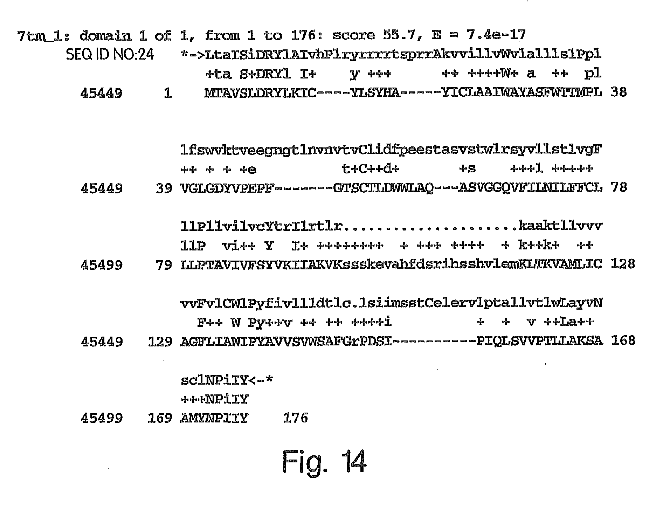

The 45449 protein contains a predicted seven transmembrane (7TM) domain located at about amino acids 1 to 176 of SEQ ID NO:1 I (Figure 14). The seven transmembrane domain shows homology to members of the rhodopsin family. An N-terminal domain extends from about amino acids 1-11 of SEQ ID NO:11. Predicted transmembrane domains (Figure 13) extend from about amino acid 12-33, 68-90, and 123-147 of SEQ ID NO:11. Predicted non-transmembrane domains extend from about amino acids 91-122 and 34-67 of SEQ ID NO:11. A C-terminal domain is located at about amino acid residues 148-324 of SEQ ID NO:11.

The 45449 receptor protein additionally contains: one predicted opsin retinal binding site located at about amino acids 165-183 of SEQ ID NO:11; three predicted protein kinase C phosphorylation sites (PS00005) from about amino acids 99-101, 194-196, and 209-211 of SEQ ID NO:11; one predicted casein kinase II phosphorylation sites (PS00006) from about amino acid 99-102 of SEQ ID NO:11; two predicted N-myristoylation sites (PS00008) from about amino acids 50-55 and 189-194 of SEQ ID NO:11; and two predicted cAMP/cGMP dependent protein kinase phosphorylation site located at about amino acids 195-198 and 211-214 of SEQ ID NO:11.

A plasmid containing the nucleotide sequence encoding human 45449 (clone Fbh45449FL) was deposited with American Type Culture Collection (ATCC), 10801 University Boulevard, Manassas, VA 20110-2209, on_____ and assigned Accession Number ______. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

### Human 50289

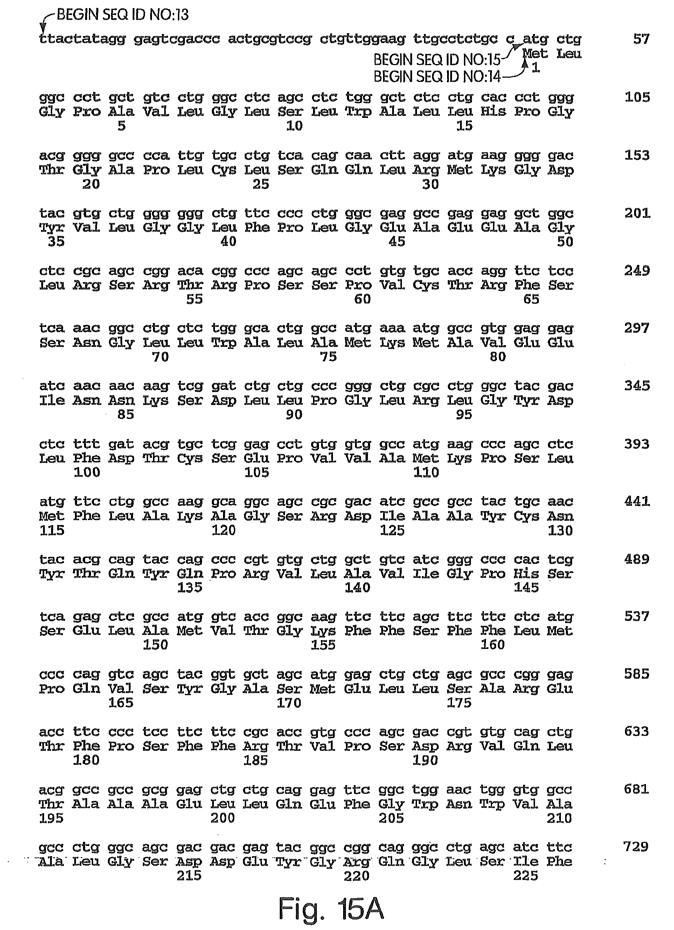

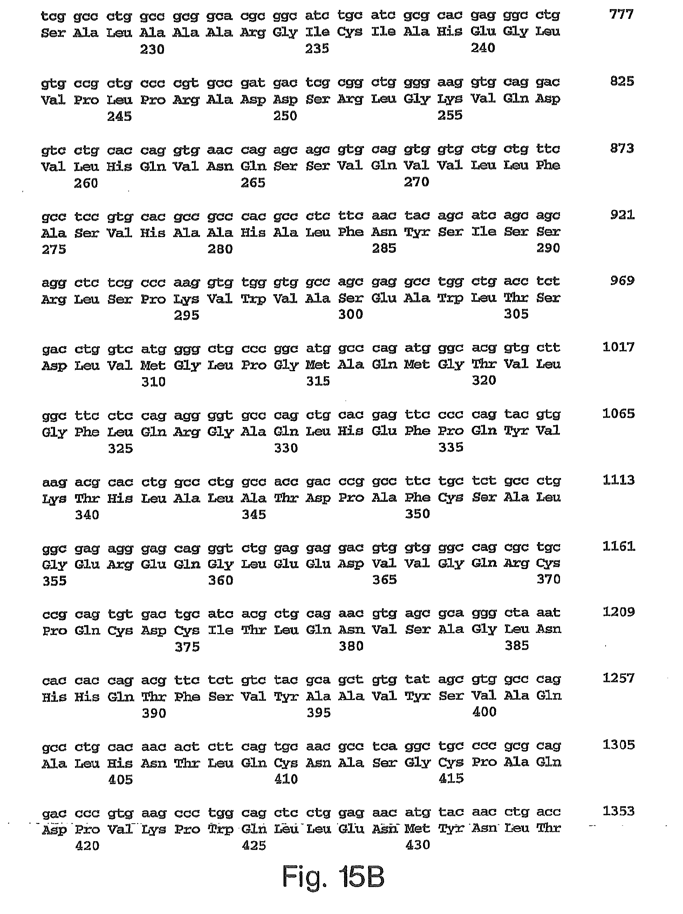

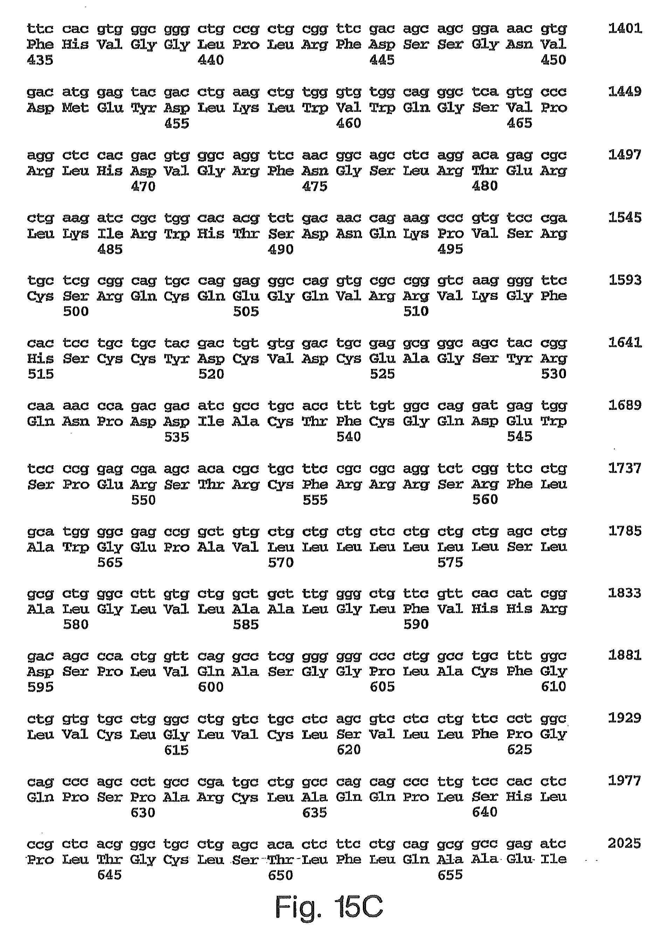

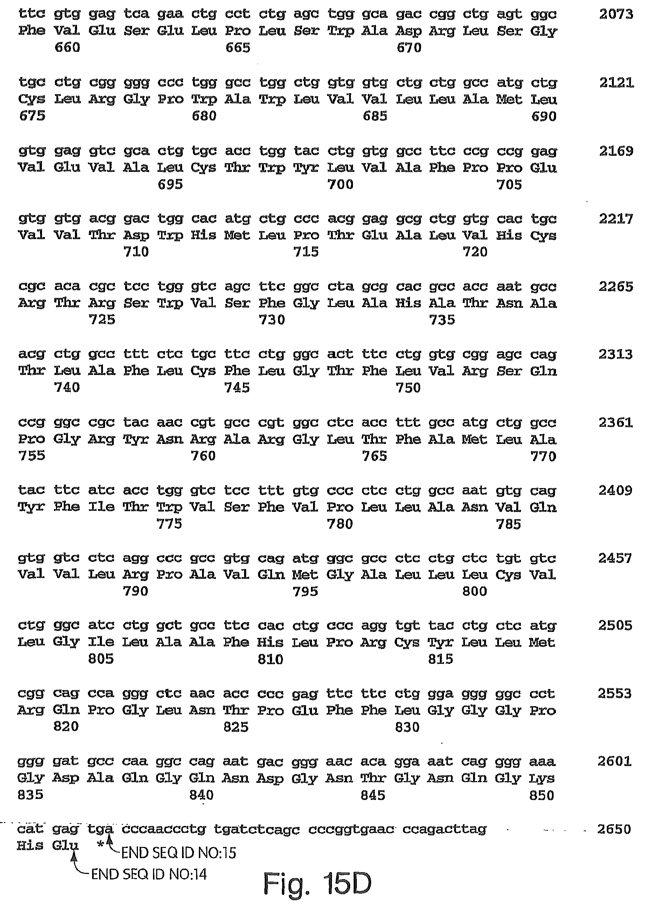

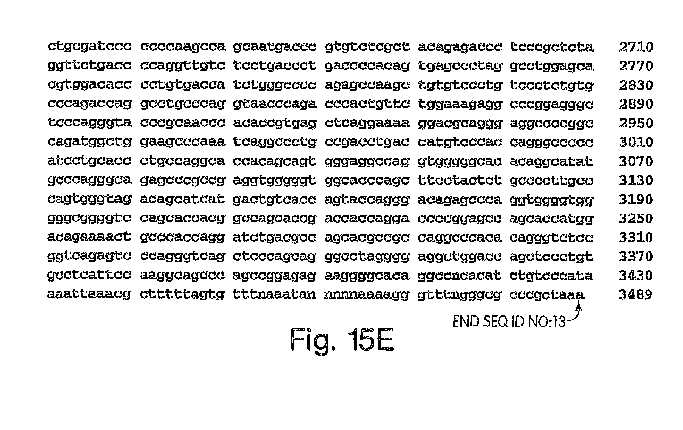

The human 50289 nucleotide sequence (Figures 15A-15E; SEQ ID NO:13), which is approximately 3489 nucleotides long including untranslated regions, contains a predicted methionine-initiated coding sequence of about 2559 nucleotides, including the termination codon (nucleotides indicated as coding of SEQ ID NO:13 in Figures 15A-15E; SEQ ID NO:15). The coding sequence encodes a 852 amino acid protein (Figure 16; SEQ ID NO:14).

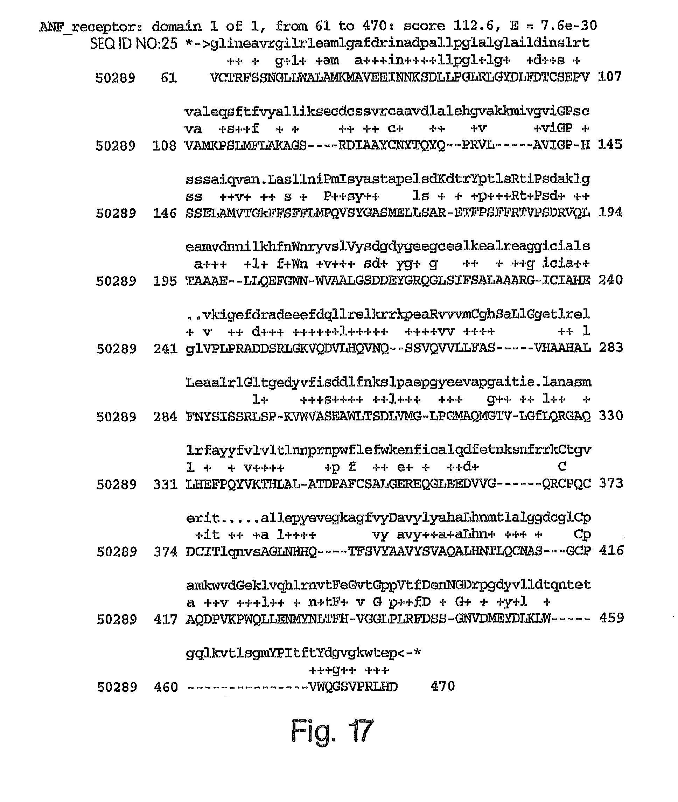

The mature protein is approximately 832 amino acid residues in length (from about amino acid 21 to amino acid 852 of SEQ ID NO:14). The mature 50289 protein contains a natriuretic peptide (ANF) ligand binding domain located at about amino acids 61 to 470 of SEQ ID NO:14 (Figure 17). The ANF domain is located at the extracellular domain of the human 50289, which extends from about amino acid 1-546 of SEQ ID NO:14. Predicted transmembrane domains (Figure 16) extend from about amino acid 567 (extracellular end) to about amino acid 590 (cytoplasmic end) of SEQ ID NO:14; from about amino acid 600 (cytoplasmic end) to about amino acid 623 (extracellular end) of SEQ ID NO:14; from about amino acid 641 (extracellular end) to about amino acid 659 (cytoplasmic end) of SEQ ID NO:14; from about amino acid 679 (cytoplasmic end) to about amino acid 702 (extracellular end) of SEQ ID NO:14; from about amino acid 726 (extracellular end) to about amino acid 750 (cytoplasmic end) of SEQ ID NO:14; from about amino acid 762 (cytoplasmic end) to about amino acid 782 (extracellular end) of SEQ ID NO:14; and from about amino acid 799 (extracellular end) to about amino acid 810 (cytoplasmic end) of SEQ ID NO:14; three extracellular loops located at about amino acids 624-640, 703-678, and 751-761 of SEQ ID NO:14; three cytoplasmic loops located at about amino acid 591-599, 660-678, and 703-725 of SEQ ID NO:14; and a C-terminal cytoplasmic domain is found at about amino acid residues 811-851 of SEQ ID NO:14.

The 50289 receptor protein additionally contains: one GPCR family 3 signature 2 domain located at about amino acids 516-540 of SEQ ID NO:14; nine predicted glycosylation sites located at about amino acids 85-88, 130-133, 264-267, 285-288, 380-383, 411-414, 432-435, 474-478, and 736-739 of SEQ ID NO:14; nine predicted protein kinase C phosphorylation sites (PS00005) from about amino acids 153-155, 175-177, 189-191, 289-291, 293-295, 477-479, 480-482, 527-529 and 550-552 of SEQ ID NO:14; three predicted casein kinase II phosphorylation sites (PS00006) from about amino acid 102-105, 175-178, and 214-217 of SEQ ID NO:14; fourteen predicted N-myristoylation sites (PS00008) from about amino acids 20-25, 69-74, 92-97, 234-239, 319-324, 476-481, 580-585, 602-607, 645-650, 730-735, 762-767, 803-808, 830-835, and 838-843 of SEQ ID NO:14; and one predicted cAMP/cGMP dependent protein kinase phosphorylation site located at about amino acids 555-558 of SEQ ID NO:14.

A plasmid containing the nucleotide sequence encoding human 50289 (clone Fbh50289FL) was deposited with American Type Culture Collection (ATCC), 10801 University Boulevard, Manassas, VA 20110-2209, on _____ and assigned Accession Number _____. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

### Human 52872

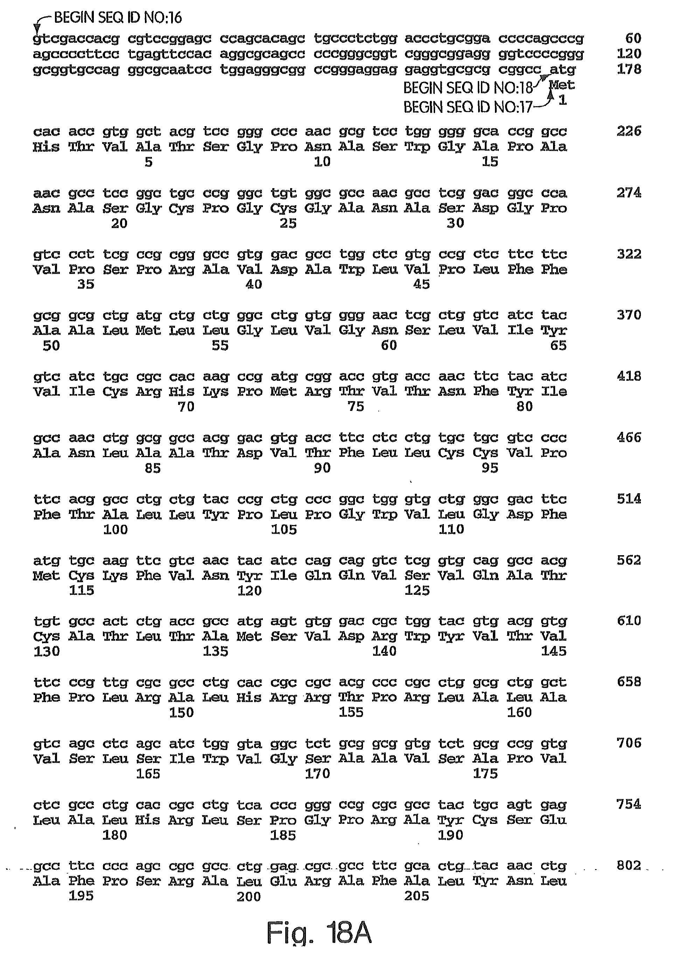

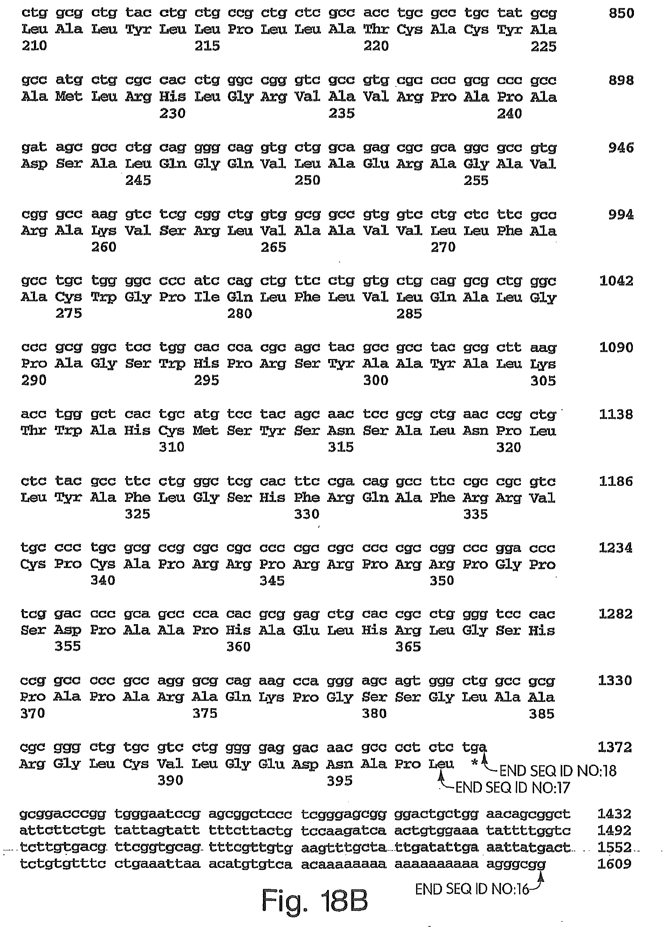

The human 52872 sequence (Figures 18A-18B; SEQ ID NO:16), which is approximately 1609 nucleotides long including untranslated regions, contains a predicted methionine-initiated coding sequence of about 1197 nucleotides, including the termination codon (nucleotides indicated as coding of SEQ ID NO:16 in Figures 18A-18B; SEQ ID NO:18). The coding sequence encodes a 398 amino acid protein (Figure 19; SEQ ID NO:17).

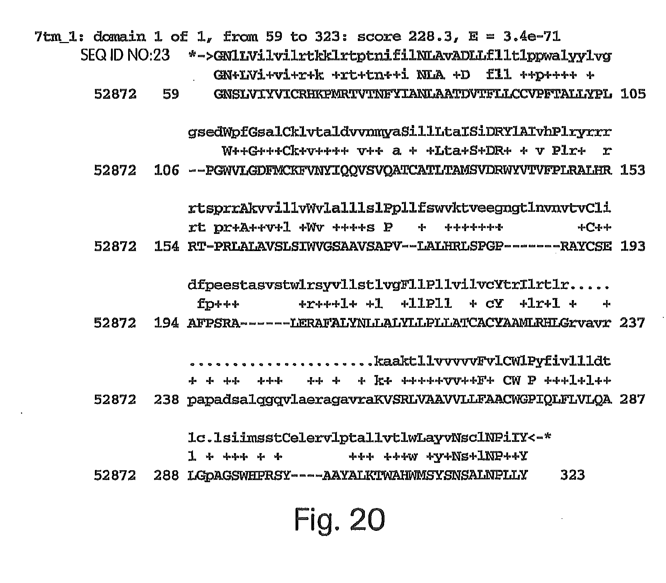

The 52872 protein contains a predicted seven transmembrane (7TM) domain (PFAM Accession Number PF00001) located at about amino acids 59 to 323 of SEQ ID NO:17 (Figure 20). The seven transmembrane domain shows homology to members of the rhodopsin family. An extracellular domain extends from about amino acids 1-42 of SEQ ID NO:17. Predicted transmembrane domains extend from about amino acid 43 (extracellular end) to about amino acid 67 (cytoplasmic end) of SEQ ID NO:17; from about amino acid 76 (cytoplasmic end) to about amino acid 110 (extracellular end) of SEQ ID NO:17; from about amino acid 117 (extracellular end) to about amino acid 136 (cytoplasmic end) of SEQ ID NO:17; from about amino acid 158 (cytoplasmic end) to about amino acid 180 (extracellular end) of SEQ ID NO:17; from about amino acid 204 (extracellular end) to about amino acid 228 (cytoplasmic end) of SEQ ID NO:17; from about amino acid 264 (cytoplasmic end) to about amino acid 285 (extracellular end) of SEQ ID NO:17; and from about amino acid 310 (extracellular end) to about amino acid 326 (cytoplasmic end) of SEQ ID NO:17; three cytoplasmic loops at about amino acids 68-75, 137-157, and 229-263 of SEQ ID NO:17; three extracellular loops at about amino acid 111-116, 181-203, and 286-309 of SEQ ID NO:17; and a C-terminal cytoplasmic domain at about amino acid residues 327-398 of SEQ ID NO:17.

The 52872 receptor protein additionally contains: three predicted N-glycosylation sites (PS00001) from about amino acids 10-13, 18-21, and 28-31 of SEQ ID NO:17; two predicted Protein Kinase C phosphorylation sites (PS00005) at about amino acids 36-38 and 155-157 of SEQ ID NO:17; and five predicted N-myristylation sites (PS00008) from about 14-19, 21-26, 56-61, 247-252, and 255-260 of SEQ ID NO:17.

A plasmid containing the nucleotide sequence encoding human 52872 (clone Fbh52872PL) was deposited with American Type Culture Collection (ATCC), 10801 University Boulevard, Manassas, VA 20110-2209, on _____ and assigned Accession Number _____. This deposit will be maintained under the terms of the Budapest Treaty on the International Recognition of the Deposit of Microorganisms for the Purposes of Patent Procedure. This deposit was made merely as a convenience for those of skill in the art and is not an admission that a deposit is required under 35 U.S.C. §112.

**Table 1 Summary of Sequence Information for GPCR Polypeptides**

| **Gene** | **DNA** | **Polypeptide** | **ORF** | **Figure** |
|---|---|---|---|---|
| 1983 | SEQ ID NO:1 | SEQ ID NO:2 | SEQ ID NO:3 | Figs. 1A-1D |
| 52881 | SEQ ID NO:4 | SEQ ID NO:5 | SEQ ID NO:6 | Figs. 6A-6D |
| 2398 | SEQ ID NO:7 | SEQ ID NO:8 | SEQ ID NO:9 | Figs. 9A-9B |
| 45449 | SEQ ID NO:10 | SEQ ID NO:11 | SEQ ID NO:12 | Figs. 12A-12B |
| 50289 | SEQ ID NO:13 | SEQ ID NO:14 | SEQ ID NO:15 | Figs. 15A-15E |
| 52872 | SEQ ID NO:16 | SEQ ID NO:17 | SEQ ID NO:18 | Figs. 18A-18B |

The 1983, 52881, 2398, 45449, 50289, and 52872 proteins contains a significant number of structural characteristics in common with members of the G protein-coupled receptor family. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologues of non-human origin, e.g., rat or mouse proteins. Members of a family can also have common functional characteristics.

The G-protein coupled receptor family of proteins is an extensive group of proteins, which transduce extracellular signals triggered by, e.g., hormones, neurotransmitters, odorants and light, by interaction with guanine nucleotide-binding (G) proteins. G-protein coupled receptors typically have seven hydrophobic membrane spanning regions. The N-terminus of a G-protein coupled receptor is typically located on the extracellular side of the membrane and is often glycosylated, while the C-terminus is cytoplasmic and generally phosphorylated. Three extracellular loops alternate with three intracellular loops to link the seven transmembrane regions. Some G-protein coupled receptors possess a signal peptide. Generally, the most conserved portions of G-protein coupled receptors are the transmembrane regions and the first two cytoplasmic loops. A conserved acidic-arginine-aromatic triplet is present in the N-terminal extremity of the second cytoplasmic loop and may be implicated in the interaction with G proteins. An alignment of the transmembrane domains of 44 representative GPCRs can be found at <http://mgdkk1.nidll.nih.gov:8000/extended.htm1>.

Based on structural similarities, members of the GPCR family have been classified into various subfamilies, including: Subfamily I, which comprises receptors typified by rhodopsin and the beta2-adrenergic receptor and currently contains over 200 unique members (reviewed by Dohlman et al. (1991) Annu. Rev. Biochem. 60:653-688); Subfamily II, which includes the parathyroid hormone/calcitonin/secretin receptor family (Juppner et al. (1991) Science 254:1024-1026; Lin et al. (1991) Science 254:1022-1024); Subfamily III, which includes the metabotropic glutamate receptor family in mammals, such as the GABA receptors (Nakanishi et al. (1992) Science 258: 597-603); Subfamily IV, which includes the cAMP receptor family that is known to mediate the chemotaxis and development of *D. discoideum* (Klein et al. (1988) Science 241:1467-1472); and Subfamily V, which includes the fungal mating pheromone receptors such as STE2 (reviewed by Kurjan I et al. (1992) Annu. Rev. Biochem. 61:1097-1129). Within each family, distinct, highly conserved motifs have been identified. These motifs have been suggested to be critical for the structural integrity of the receptor, as well as for coupling to G proteins.

Based upon the results of the HMM analysis (HMMER Version 2.1.1), the 52881, 2398, 45449, and 52872 polypeptides appear to belong to the rhodopsin subfamily of GPCRs (Subfamily I). 1983 appears to belong to the secretin subfamily of GPCRs (Subfamily II).

A 52881, 2398, 45449 or 52872 polypeptide can include a "rhodopsin-related seven transmembrane receptor domain" or regions homologous with a "rhodopsin-related seven transmembrane receptor domain".

As used herein, the term "rhodopsin-related seven transmembrane receptor domain" includes an amino acid sequence of about 40-300 amino acid residues in length and having a bit score for the alignment of the sequence to the rhodopsin-related seven transmembrane receptor domain (HMM) of at least 15 or greater. Preferably, the rhodopsin-related seven transmembrane receptor domain includes an amino acid sequence which is about 50-280 amino acids, more preferably about 70-270 amino acids in length, and has a bit score for the alignment of the sequence to the rhodopsin-related seven transmembrane receptor domain (HMM) of at least 20 or greater, preferably 30 or greater. A 52881 protein preferably contains an amino acid sequence of about 75 amino acid residues in length, having a bit score for the alignment of the sequence to the rhodopsin-related seven transmembrane receptor domain at least 30. A 2398 protein preferably contains an amino acid sequence of about 246 amino acid residues in length, having a bit score for the alignment of the sequence to the rhodopsin-related seven transmembrane receptor domain at least 260. A 45449 protein preferably contains an amino acid sequence of about 176 amino acid residues in length, having a bit score for the alignment of the sequence to the rhodopsin-related seven transmembrane receptor domain at least 50. A 52872 protein preferably contains an amino acid sequence of about 265 amino acid residues in length, having a bit score for the alignment of the sequence to the rhodopsin-related seven transmembrane receptor domain at least 220.

The rhodopsin-related seven transmembrane receptor domain (HMM) has been assigned the PFAM Accession Number PF00001 (http;//genome.wustl.edu/Pfam/.html). An alignment of the rhodopsin-related seven transmembrane receptor domain (amino acids 80 to 154 of SEQ ID NO:5) of human 52881 with a consensus amino acid sequence (SEQ ID NO:22) derived from a hidden Markov model is depicted in Figure 8. An alignment of the rhodopsin-related seven transmembrane receptor domain (amino acids 5 8 to 303 of SEQ ID NO:8) of human 2398 with a consensus amino acid sequence (SEQ ID NO:23) derived from a hidden Markov model is depicted in Figure 11. An alignment of the rhodopsin-related seven transmembrane receptor domain (amino acids 1 to 176 of SEQ ID NO:11) of human 45449 with a consensus amino acid sequence (SEQ ID NO:24) derived from a hidden Markov model is depicted in Figure 14. An alignment of the rhodopsin-related seven transmembrane receptor domain (amino acids 59 to 323 of SEQ ID NO:17) of human 52872 with a consensus amino acid sequence (SEQ ID NO:23) derived from a hidden Markov model is depicted in Figure 20.

In a preferred embodiment, a 52881, 2398, 45449 or 52872 polypeptide or protein has a "rhodopsin-related seven transmembrane receptor domain" or a region which includes at least about 40-300 amino acid residues in length, preferably about 50-280 amino acids, more preferably about 70-270 amino acids and has at least about 50%, 60%, 70% 80% 90% 95%, 99%, or 100% homology with a "rhodopsin-related seven transmembrane receptor domain," e.g., the rhodopsin-related seven transmembrane receptor domain of human 52881, 2398, 45449 or 52872 (e.g., amino acids 80 to 154 of SEQ ID NO:5, amino acids 58 to 303 of SEQ ID NO:8, amino acids 1 to 176 of SEQ ID NO:11, or amino acids 59 to 323 of SEQ ID NO:17).

To identify the presence of a "rhodopsin-related seven transmembrane receptor domain" in a 52881, 2398, 45449 or 52872 protein sequence, and make the determination that a polypeptide or protein of interest has a particular profile, the amino acid sequence of the protein can be searched against a database of HMMs (e.g., the Pfam database, release 2.1) using the default parameters (http://www.sanger.ac.uk/Software/Pfam/HMM_search). For example, the hmmsf program, which is available as part of the HMMBR package of search programs, is a family specific default program for MILPAT0063 and a score of 15 is the default threshold score for determining a hit. Alternatively, the threshold score for determining a hit can be lowered (e.g., to 8 bits). A description of the Pfam database can be found in Sonhammer et al. (1997) Proteins 28(3):405-420 and a detailed description of HMMs can be found, for example, in Gribskov et al.(1990) Meth. Enzymol. 183:146-159; Gribskov et al.(1987) Proc. Natl. Acad. Sci. USA 84:4355-4358; Krogh et al.(1994) J. Mol. Biol. 235:1501-1531; and Stultz et al.(1993) Protein Sci. 2:305-314, the contents of which are incorporated herein by reference. A search was performed against the HMM database resulting in the identification of a "rhodopsin-related seven transmembrane receptor domain" domain in the amino acid sequence of human 52881, 2398, 45449 and 52872 (at about amino acids 80 to 154 of SEQ ID NO:5, amino acids 58 to 303 of SEQ ID NO:8, amino acids 1 to 176 of SEQ ID NO:11, and amino acids 59 to 323 of SEQ ID NO:17).

A 1983 polypeptide can include a "secretin-related seven transmembrane receptor domain" or regions homologous with a "rhodopsin-related seven transmembrane receptor domain".

As used herein, the term "secretin-related seven transmembrane receptor domain" includes an amino acid sequence of about 50-300 amino acid residues in length, preferably about 100-280 amino acids, preferably about 150-260 amino acids, more preferably about 248 amino acids and having a bit score for the alignment of the sequence to the secretin-related seven transmembrane receptor domain (HMM) of at least 200 or greater, preferably 250 or greater.

The secretin-related seven transmembrane receptor domain (HMM) has been assigned the PFAM Accession Number PF00002. An alignment of the secretin-related seven transmembrane receptor domain (amino acids 379 to 626 of SEQ ID NO:2) of human 1983 with a consensus amino acid sequence (SEQ ID NO:19) derived from a hidden Markov model is depicted in Figure 3.

In a preferred embodiment, a 1983 polypeptide or protein has a "secretin-related seven transmembrane receptor domain" or a region which includes at least about 40-300 amino acid residues, preferably about 50-300 amino acids, preferably about 100-280 amino acids, preferably about 150-260 amino acids and has at least about 50%, 60%, 70% 80% 90% 95%, 99%, or 100% homology with a "secretin-related seven transmembrane receptor domain," e.g., the secretin-related seven transmembrane receptor domain of human 1983 (e.g., amino acids 379 to 626 of SEQ ID NO:2).

To identify the presence of a "secretin-related seven transmembrane receptor domain" in a 1983 protein sequence, and make the determination that a polypeptide or protein of interest has a particular profile, the amino acid sequence of the protein can be searched against a database of HMMs (e.g., the Pfam database, release 2.1) using the default parameters (http://www.sanger.ac.uk/Software/Pfam/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the Pfam database can be found in Sonhammer et al. (1997) Proteins 28(3):405-420 and a detailed description of HMMs can be found, for example, in Gribskov et al.(1990) Meth. Enzynol. 183:146-159; Gribskov et al.(1987) Proc. Natl. Acad. Sci. USA 84:4355-4358; Krogh et al.(1994) J. Mol. Biol. 235:1501-1531; and Stultz et al.(1993) Protein Sci. 2:305-314, the contents of which are incorporated herein by reference. A search was performed against the HMM database resulting in the identification of a "secretin-related seven transmembrane receptor domain" domain in the amino acid sequence of human 1983 (at about amino acids 379 to 626 of SEQ ID NO:2).

In one embodiment, a 1983 protein includes at least one at least one EGF-like domains. Preferably, the EGF-like domain is found in the extracellular domain of a 1983 protein. As used herein, an "EGF-like domain" refers to an amino acid sequence of about 25 to 50, preferably about 30 to 45, and more preferably 30 to 40 amino acid residues in length. An EGF domain further contains at least about 2 to 10, preferably, 3 to 9, 4 to 8, or 6 to 7 conserved cysteine residues. A consensus EGF-like domain sequence includes six cysteines, all of which are thought to be involved in disulfide bonds having the following amino acid aequence: Xaa(4)-Cys-Xaa(0, 48)-Cys-Xaa(3, 12)-Cys-Xaa(1, 70)-Cys-Xaa(1, 6)-Cys-Xaa(2)-Gly-Aro-Xaa(0, 21)-Gly-Xaa(2)-Cys-Xaa, where Xaa is any amino acid and Aro is any aromatic amino acid. The region between the fifth and the sixth cysteine typically contains two conserved glycines of which at least one is present in most EGF-like domains. Proteins having such domains may play a role in mediating protein-protein interactions, and thus can influence a wide variety of biological processes, including cell surface recognition; modulation of cell-cell contact; modulation of cell fate determination; and modulation of wound healing and tissue repair. The EGF-like domain (HMM) has been assigned the PFAM Accession Number PF00008.

In a preferred embodiment, a 1983 polypeptide or protein has at least one EGF-like domain of about 25 to 50, preferably about 30 to 45, and more preferably 30 to 40 amino acid residues in length, and has at least about 60%, 70% 80% 90% 95%, 99%, or 100% homology with an "EGF-like domain," e.g., at least one EGF-like domain of human 1983 (e.g., residues13-63 of SEQ ID NO:2; Figure 4).

In another embodiment, a 1983 protein includes at least one latrophilin CL-1-like GPS domain. As used herein, a "latrophilin CL-1-like GPS" domain refers to an amino acid sequence of about 25-120 amino acids, preferably about 40-80, and most preferably, about 50 amino acids which is capable of binding alpha-Latrotoxin, a potent excitatory neurotoxin. The latrophilin CL-1-like GPS domain (HMM) has been assigned the PFAM Accession Number PF01825.

In a preferred embodiment, a 1983 polypeptide or protein has at least one latrophilin CL-1-like GPS domain of about 25-120 amino acids, preferably about 40-80, and most preferably, about 50 amino acid residues in length, and has at least about 60%, 70% 80% 90% 95%, 99%, or 100% homology with an " latrophilin CL-1-like GPS domain," e.g., at least one latrophilin CL-1-like GPS domain of human 1983 (e.g., residues 321 to 373 of SEQ ID NO:2; Figure 5).

In one embodiment, a 50289 protein includes at least one at least one ANF ligand binding domain. Preferably, the ANF ligand binding domain is found in the extracellular domain of a 50289 protein. As used herein, an "ANF ligand binding domain" refers to an amino acid sequence of about 100 to 600, preferably, 200-500, more preferably, 300-450, and most preferably, about 409 amino acids which is preferably located outside a cell or extracellularly. Preferably, the ANF ligand binding domain interacts (e.g., binds to) a natriuretic peptide (i.e., a hormone involved in the regulation of fluid and electrolyte homeostasis). Preferred, ANF ligand binding domains mediate the intracellular production of a second messenger, e.g., cGMP, thereby transducing an extracellular signal. Preferred ANF ligand binding domain are involved in modulating a cellular activity, e.g., the regulation of fluid and electrolyte homeostasis. The ANF ligand binding domain (HMM) has been assigned the PFAM Accession Number PF01094.

In a preferred embodiment, a 50289 polypeptide or protein has at least one ANF ligand binding domain of about 100 to 600, preferably, 200-500, more preferably, 300-450, and most preferably, about 409 amino acid residues in length, and has at least about 60%, 70% 80% 90% 95%, 99%, or 100% homology with an "ANF ligand binding domain," e.g., at least one ANF ligand binding domain of human 50289 (e.g., residues 61 to 470 of SEQ ID NO:14; Figure 17).

In one embodiment, a 1983, 52881, 2398, 45449, 50289 or 52872 protein includes at least one, two, three, four, five, six, or preferably, seven transmembrane domains. As used herein, the term "transmembrane domain" includes an amino acid sequence of about 15 amino acid residues in length which spans the plasma membrane. More preferably, a transmembrane domain includes about at least 16, 18, 20, 25, 30, 35 or 40 amino acid residues and spans the plasma membrane. Transmembrane domains are rich in hydrophobic residues, and typically have an α-helical structure. In a preferred embodiment, at least 50%, 60%, 70%, 80%, 90%, 95% or more of the amino acids of a transmembrane domain are hydrophobic, e.g., leucines, isoleucines, tyrosines, or tryptophans. Transmembrane domains are described in, for example, htto://pfam.wustl.edu/cgi-bin/getdesc?name=7tm-1, and Zagotta W.N. et al, (1996) Annual Rev. Neuronsci. 19: 235-63, the contents of which are incorporated herein by reference.

In a preferred embodiment, a 1983, 52881, 2398, 45449, 50289 or 52872 polypeptide or protein has at least one transmembrane domain or a region which includes at least 16, 18, 20, 25 30, 35 or 40 amino acid residues and has at least about 60%, 70% 80% 90% 95%, 99%, or 100% homology with a "transmembrane domain," e.g., at least one transmembrane domain of human 1983, 52881, 2398, 45449, 50289 or 52872 (e.g., amino acid residues 388-407, 420-436, 455-479, 488-508, 525-549, 574-591, and 598-622 of SEQ ID NO:2; amino acid residues 11-34, 44-67, 85-106, 127-149, 172-196, and 245-269 of SEQ ID NO:5; amino acid residues 42-66, 78-99, 114-135, 154-176, 202-224, 241-259, 291-310 of SEQ ID NO:8; amino acid residues 12-33, 68-90, and 123-147 of SEQ ID NO:11; amino acid residues 567-590, 600-623, 641-659, 679-702, 726-750, 762-782, and 799-810 of SEQ ID NO:14; and amino acid residues 43-67, 76-110, 117-136, 158-180, 204-228, 264-285, and 310-326 of SEQ ID NO:17). Preferably, the transmembrane domain transduces a signal, e.g., an extracellular signal across a cell membrane, and/or activates a signal transduction pathway.

In another embodiment, a 1983, 2398, 50289, or 52872 protein includes at least one extracellular domain. When located at the N-terminal domain the extracellular domain is referred to herein as an "N-terminal extracellular domain", or as an N-terminal extracellular loop in the amino acid sequence of the protein. As used herein, an "N-terminal extracellular domain" includes an amino acid sequence having about 1-600, preferably about 1-500, preferably about 1-400, preferably about 1-300, preferably about 1-100, more preferably about 1-70, more preferably about 1-60, more preferably about 1-50, or even more preferably about 1-45 amino acid residues in length and is located outside of a cell or extracellularly. The C-terminal amino acid residue of a "N-terminal extracellular domain" is adjacent to an N-terminal amino acid residue of a transmembrane domain in a naturally-occurring 1983, 2398, 50289, or 52872, or 1983, 2398, 50289, or 52872-like protein. For example, an N-terminal cytoplasmic domain is located at about amino acid residues 1-387 of SEQ ID NO:2,1-41 of SEQ ID NO:8, 1-546 of SEQ ID NO:14, and 1-42 of SEQ ID NO:17.

In a preferred embodiment, a 1983, 2398, 50289, or 52872 polypeptide or protein has an "N-terminal extracellular domain" or a region which includes at least about 1-600, preferably about 1-500, preferably about 1-400, preferably about 1-300, preferably about 1-100, more preferably about 1-70, more preferably about 1-60, more preferably about 1-50, or even more preferably about 1-45 amino acid residues and has at least about 60%, 70% 80% 90% 95%, 99%, or 100% homology with an "N-terminal extracellular domain," e.g., the N-terminal extracellular domain of human 1983, 2398, 50289, or 52872 (e.g., residues 1-387 of SEQ ID NO:2, 1-41 of SEQ ID NO:8, 1-546 of SEQ ID NO:14, and 1-42 of SEQ ID NO:17). Preferably, the N-terminal extracellular domain is capable of interacting (e.g., binding to) with an extracellular signal, for example, a ligand or a cell surface receptor. Most preferably, the N-terminal extracellular domain mediates protein-protein interactions, signal transduction and/or cell adhesion. For example, an EGF-like domain of a 1983 polypeptide may mediate protein-protein interactions. Similarly, an ANF binding domain of a 50289 receptor may mediate ligand binding and/or transduction of an extracellular signal.

In another embodiment, a 1983, 2398, 50289 or 52872 protein include at least one extracellular loop. As defined herein, the term "loop" includes an amino acid sequence having a length of at least about 4, preferably about 5-10, and more preferably about 10-20 amino acid residues, and has an amino acid sequence that connects two transmembrane domains within a protein or polypeptide. Accordingly, the N-terminal amino acid of a loop is adjacent to a C-terminal amino acid of a transmembrane domain in a naturally-occurring a 1983, 2398, 50289 or 52872, or a 1983, 2398, 50289 or 52872-like molecule, and the C-terminal amino acid of a loop is adjacent to an N-terminal amino acid of a transmembrane domain in a naturally-occurring 1983, 2398, 50289 or 52872, or a 1983, 2398, 50289 or 52872-like molecule. As used herein, an "extracellular loop" includes an amino acid sequence located outside of a cell, or extracellularly. For example, an extracellular loop can be found at about amino acids 437-454, 509-524 and 590-597 of SEQ ID NO:2; at about amino acids 100-113, 177-201, and 260-290 of SEQ ID NO:8; at about amino acids 624-640, 703-678, and 751-761 of SEQ ID NO:14; and at about amino acids 111-116, 181-203, and 286-309 of SEQ ID NO:17.

In a preferred embodiment, a 1983, 2398, 50289 or 52872 polypeptide or protein has at least one extracellular loop or a region which includes at least about 4, preferably about 5-10, preferably about 10-20, and more preferably about 20-30 amino acid residues and has at least about 60%, 70% 80% 90% 95%, 99%, or 100% homology with an "extracellular loop," e.g., at least one extracellular loop of human 1983, 2398, 50289 or 52872 (e.g., residues 437-454, 509-524 and 590-597 of SEQ ID NO:2; residues 100-113, 177-201, and 260-290 of SEQ ID NO:8; residues 624-640, 703-678, and 751-761 of SEQ ID NO:14; and residues 111-116, 181-203, and 286-309 of SEQ ID NO:17).

In another embodiment, a 1983, 2398, 50289 or 52872 protein includes at least one cytoplasmic loop, also referred to herein as a cytoplasmic domain. As used herein, a "cytoplasmic loop" includes an amino acid sequence having a length of at least about 5, preferably about 5-10, and more preferably about 10-20 amino acid residues located within a cell or within the cytoplasm of a cell. For example, a cytoplasmic loop is found at about amino acids 408-419, 480-487 and 550-573 of SEQ ID NO:2; at about amino acids 67-77, 136-153, and 225-240 of SEQ ID NO:8; at about amino acids 591-599, 660-678, and 703-725 of SEQ ID NO:14; and at about amino acids 68-75, 137-157, and 229-263 of SEQ ID NO:17.

In a preferred embodiment, a 1983, 2398, 50289 or 52872 polypeptide or protein has at least one cytoplasmic loop or a region which includes at least about 5, preferably about 5-10, and more preferably about 10-20 amino acid residues and has at least about 60%, 70% 80% 90% 95%, 99%, or 100% homology with an "cytoplasmic loop," e.g., at least one cytoplasmic loop of human 1983, 2398, 50289 or 52872 (e.g., residues 408-419, 480-487 and 550-573 of SEQ ID NO:2; residues 67-77, 136-153, and 225-240 of SEQ ID NO:8; residues 591-599, 660-678, and 703-725 of SEQ ID NO:14; or residues 68-75, 137-157, and 229-263 of SEQ ID NO:17).

In another embodiment, a 1983, 2398, 50289 or 52872 protein includes a "C-terminal cytoplasmic domain", also referred to herein as a C-terminal cytoplasmic tail, in the sequence of the protein. As used herein, a "C-terminal cytoplasmic domain" includes an amino acid sequence having a length of at least about 50, preferably about 50-100, more preferably about 70-93 amino acid residues and is located within a cell or within the cytoplasm of a cell. Accordingly, the N-terminal amino acid residue of a "C-terminal cytoplasmic domain" is adjacent to a C-terminal amino acid residue of a transmembrane domain in a naturally-occurring 1983, 2398, 50289 or 52872 or 1983, 2398, 50289 or 52872-like protein. For example, a C-terminal cytoplasmic domain is found at about amino acid residues 623-645 of SEQ ID NO:2; at about amino acid residues 311-350 of SEQ ID NO:8; at about amino acid residues 811-851 of SEQ ID NO:14; and at about amino acid residues 327-398 of SEQ ID NO:17.

In a preferred embodiment, a 1983, 2398, 50289 or 52872 polypeptide or protein has a C-terminal cytoplasmic domain or a region which includes at least about 50, preferably about 50-100, more preferably about 70-93 amino acid residues and has at least about 60%, 70% 80% 90% 95%, 99%, or 100% homology with an "C-terminal cytoplasmic domain," e.g., the C-terminal cytoplasmic domain of human 1983, 2398, 50289 or 52872 (e.g., residues 623-645 of SEQ ID NO:2; residues 311-350 of SEQ ID NO:8; residues 811-851 of SEQ ID NO:14; or residues 327-398 of SEQ ID NO:17).

In one embodiment, a 52881 or 45449 protein includes at least one N-terminal domain. As used herein, an "N-terminal domain" includes an amino acid sequence having about 1-50 or more preferably about 1-10 amino acids, located at the N-terminus of the protein. The C-terminal amino acid residue of a "N-terminal domain" is adjacent to an N-terminal amino acid residue of a transmembrane domain in a naturally-occurring 52881 or 45449-like protein. For example, an N-terminal domain is located at about amino acid residues 1-10 of SEQ ID NO:5 and amino acid residues 1-11 of SEQ ID NO:11.

In a preferred embodiment, a 52881 or 45449 polypeptide or protein has an "N-terminal domain" or a region which includes at least about 1-50, or 1-10 amino acid residues and has at least about 60%, 70% 80% 90% 95%, 99%, or 100% homology with an "N-terminal domain," e.g., the N-terminal domain of human 52881 or 45449 (e.g., residues 1-10 of SEQ ID NO:5 or residues 1-11 of SEQ ID NO:11).

In another embodiment, a 52881 or 45449 protein includes a "C-terminal domain", also referred to herein as a C-terminal tail, in the sequence of the protein. As used herein, a "C-terminal domain" includes an amino acid sequence having a length of at least about 50, preferably about 100-500, more preferably about 200-450, most preferably about 403 amino acid residues. Accordingly, the N-terminal amino acid residue of a "C-terminal domain" is adjacent to a C-terminal amino acid residue of a transmembrane domain in a naturally-occurring 52881 or 45449-like protein. For example, a C-terminal domain is found at about amino acid residues 270-609 of SEQ ID NO:5 and 148-324 of SEQ ID NO:11.

In a preferred embodiment, a 52881 or 45449 polypeptide or protein has a C-terminal domain or a region which includes at least about 50, preferably about 100-500, more preferably about 200-450 amino acid residues and has at least about 60%, 70% 80% 90% 95%, 99%, or 100% homology with an "C-terminal domain," e.g., the C-terminal domain of human 52881 or 45449 (e.g., residues 270-609 of SEQ ID NO:2 or residues 148-324 of SEQ ID NO:11).

In another embodiment, a 52881 or 45449 protein include at least one non-transmembrane loop. As defined herein, the term "loop" includes an amino acid sequence having a length of at least about 4, preferably about 5-100, and more preferably about 9-50 amino acid residues, and has an amino acid sequence that connects two transmembrane domains within a protein or polypeptide.

In a preferred embodiment, a 52881 or 45449 polypeptide or protein has at least one non-transmembrane loop or a region which includes at least about 4, preferably about 5-100, preferably about 9-50, and has at least about 60%, 70% 80% 90% 95%, 99%, or 100% homology with a "non-transmembrane loop," e.g., at least one non-transmembrane loop of human 52881 or 45449 (e.g., residues 35-43, 68-84, 107-126, 150-171, or 197-244 of SEQ ID NO:5 or residues 91-122 or 34-67 of SEQ ID NO:11).

In one embodiment of the invention, a 1983 polypeptide includes at least one, and preferably six or seven, transmembrane domains and/or at least one cytoplasmic loop, and/or at least one extracellular loop. In another embodiment, a 1983 polypeptide further includes an N-terminal extracellular domain and/or a C-terminal cytoplasmic domain. In another embodiment, a 1983 polypeptide can include seven transmembrane domains, three cytoplasmic loops, three extracellular loops and can further include an N-terminal extracellular domain and/or a C-terminal cytoplasmic domain.

In one embodiment of the invention, a 52881 protein includes at least one, and preferably two, three, four, five, or six transmembrane domains and/or at least one, and preferably two, three, four, or five non-transmembrane loops. In another embodiment, the 52881 protein further includes an N-terminal domain and/or a C-terminal domain. The 52881 molecules of the present invention can further include at least one, two, three, and preferably four cAMP/cGMP phosporylation sites. The 52881 molecules can additionally include at least one, two, three, four, five, and preferably six protein kinase C phosphorylation sites. The 52881 molecules can additionally include at least one, two, three, four, five, six, seven, eight, nine, and preferably 10 casein kinase II phosphorylation sites. The 52881 molecules can additionally include at least one tyrosine kinase phosphorylation site. The 52881 molecules can additionally include at least one, two, three, four, five, six, seven, eight, nine, and preferably 10 N-myristoylation sites. The 52881 molecules can further include at least one amidation site.

In one embodiment of the invention, a 2398 polypeptide includes at least one, and preferably six or seven, transmembrane domains and/or at least one cytoplasmic loop, and/or at least one extracellular loop. In another embodiment, a 2398 polypeptide further includes an N-terminal extracellular domain and/or a C-terminal cytoplasmic domain. In another embodiment, a 2398 polypeptide can include seven transmembrane domains, three cytoplasmic loops, three extracellular loops and can further include an N-terininal extracellular domain and/or a C-terminal cytoplasmic domain.

In one embodiment of the invention, a 45449 protein includes at least one, and preferably two, or three transmembrane domains and/or at least one, and preferably two non-transmembrane loops. In another embodiment, the 45449 protein further includes an N-terminal domain and/or a C-terminal domain.

In one embodiment of the invention, a 50289 polypeptide includes at least one, and preferably six or seven, transmembrane domains and/or at least one cytoplasmic loop, and/or at least one extracellular loop. In another embodiment, a 50289 polypeptide further includes an N-terminal extracellular domain and/or a C-terminal cytoplasmic domain. In another embodiment, a 50289 polypeptide can include seven transmembrane domains, three cytoplasmic loops, three extracellular loops and can further include an N-terminal extracellular domain and/or a C-terminal cytoplasmic domain.

In one embodiment of the invention, a 52872 polypeptide includes at least one, and preferably six or seven, transmembrane domains and/or at least one cytoplasmic loop, and/or at least one extracellular loop. In another embodiment, a 52872 polypeptide further includes an N-terminal extracellular domain and/or a C-terminal cytoplasmic domain. In another embodiment, a 52872 polypeptide can include seven transmembrane domains, three cytoplasmic loops, three extracellular loops and can further include an N-terminal extracellular domain and/or a C-terminal cytoplasmic domain. The 52872 molecules of the present invention can further include at least one, two, and preferably three N-glycosylation sites. The 52872 molecules can additionally include at least one, preferably two protein kinase C phosphorylation sites. The 52872 molecules can further include at least one, two, three, four and preferably five N-myristylation sites.

Based on the above-described sequence similarities, the 1983, 52881, 2398, 45449, 50289, and 52872molecules of the present invention are predicted to have similar biological activities as members of the GPCR family. The response mediated by a 1983, 52881, 2398, 45449, 50289, or 52872 receptor protein can depend on the type of cell. For example, in some cells, binding of a ligand to the receptor protein may stimulate an activity such as release of compounds, gating of a channel, cellular adhesion, migration, differentiation, etc., through phosphatidylinositol or cyclic AMP metabolism and turnover while in other cells, the binding of the ligand can produce a different result. Regardless of the cellular activity/response modulated by the receptor protein, it is universal that the protein is a GPCR and interacts with G proteins to produce one or more secondary signals, in a variety of intracellular signal transduction pathways, e.g., through phosphatidylinositol or cyclic AMP metabolism and turnover, in a cell. As used herein, a "signaling transduction pathway" refers to the modulation (e.g., stimulation or inhibition) of a cellular function/activity upon the binding of a ligand to the GPCR (52872 protein). Examples of such functions include mobilization of intracellular molecules that participate in a signal transduction pathway, e.g., phosphatidylinositol 4,5-bisphosphate (PIP₂), inositol 1,4,5-triphosphate (IP₃) and adenylate cyclase.

As used herein, "phosphatidylinositol turnover and metabolism" refers to the molecules involved in the turnover and metabolism of phosphatidylinositol 4,5-bisphosphate (PIP₂) as well as to the activities of these molecules. PIP₂ is a phospholipid found in the cytosolic leaflet of the plasma membrane. Binding of ligand to the receptor activates, in some cells, the plasma-membrane enzyme phospholipase C that in turn can hydrolyze PIP₂ to produce 1,2-diacylglycerol (DAG) and inositol 1,4,5-triphosphate (IP₃). Once formed IP₃ can diffuse to the endoplasmic reticulum surface where it can bind an IP₃ receptor, e.g., a calcium channel protein containing an IP₃ binding site. IP₃ binding can induce opening of the channel, allowing calcium ions to be released into the cytoplasm. IP₃ can also be phosphorylated by a specific kinase to form inositol 1,3,4,5-tetraphosphate (IP₄), a molecule which can cause calcium entry into the cytoplasm from the extracellular medium. IP₃ and IP₄ can subsequently be hydrolyzed very rapidly to the inactive products inositol 1,4-biphosphate (IP₂) and inositol 1,3,4-triphosphate, respectively. These inactive products can be recycled by the cell and used to synthesize PIP₂. The other second messenger produced by the hydrolysis of PIP₂, namely 1,2-diacylglycerol (DAG), remains in the cell membrane where it can serve to activate the enzyme protein kinase C. Protein kinase C is usually found soluble in the cytoplasm of the cell, but upon an increase in the intracellular calcium concentration, this enzyme can move to the plasma membrane where it may be activated by DAG. The activation of protein kinase C in different cells results in various cellular responses such as the phosphorylation of glycogen synthase, or the phosphorylation of various transcription factors, e.g., NF-κB. The language "phosphatidylinositol activity", as used herein, refers to an activity of PIP₂ or one of its metabolites.

Another signaling pathway in which the receptor may participate is the cAMP turnover pathway. As used herein, "cyclic AMP turnover and metabolism" refers to the molecules involved in the turnover and metabolism of cyclic AMP (cAMP) as well as to the activities of these molecules. Cyclic AMP is a second messenger produced in response to ligand-induced stimulation of certain G protein coupled receptors. In the cAMP signaling pathway, binding of a ligand to a GPCR can lead to the activation of the enzyme adenyl cyclase, which catalyzes the synthesis of cAMP. The newly synthesized cAMP can in turn activate a cAMP-dependent protein kinase. This activated kinase can phosphorylate a voltage-gated potassium channel protein, or an associated protein, and lead to the inability of the potassium channel to open during an action potential. The inability of the potassium channel to open results in a decrease in the outward flow of potassium, which normally repolarizes the membrane of a neuron, leading to prolonged membrane depolarization.

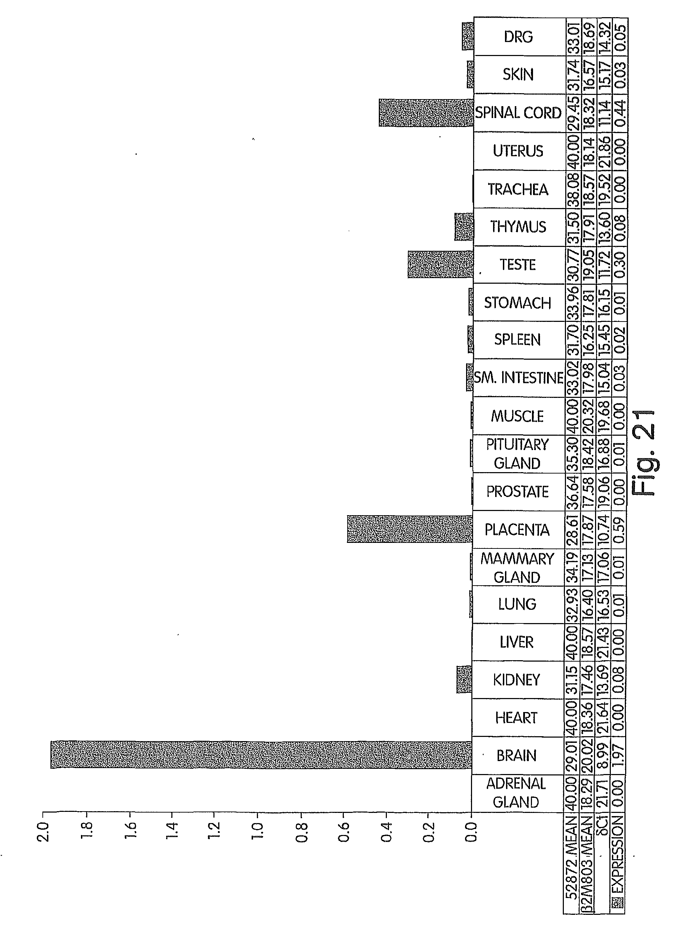

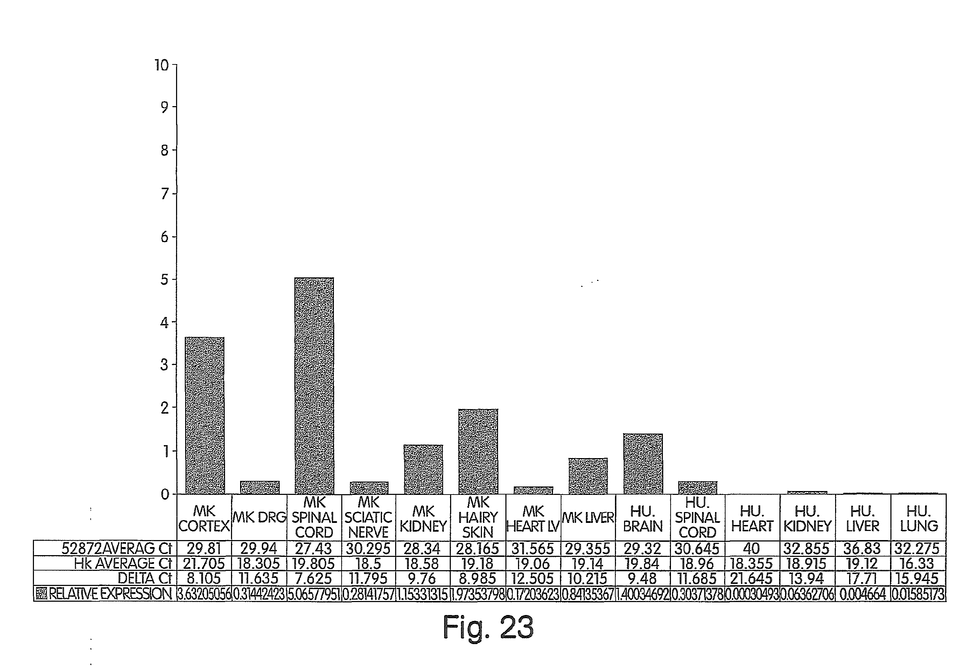

52872 is highly expressed in the central and peripheral nervous system. Figures 21-23 show that 52872 mRNA is expressed at high levels, relative to other tissues tested, in the human brain and spinal cord. Expression was also detected in placenta, testes, thymus, and dorsal root ganglion (DRG). In the monkey, high level 52872 expression was detected in the cortex and the spinal cord (Figure 23). In situ hybridization showed expression of 52872 in the brain cortex, stratum, thalamus, spinal cord, and dorsal horni. Low levels of expression were detected in a small population of medium size DRG neurons.

Animal models of pain response include, but are not limited to: axotomy, the cutting or severing of an axon (Gustafsson et al. (2000) Neuroreport 11:3345-48); chronic constriction injury (CCI), also known as the Bennett model, a model of neuropathic pain which involves ligation of the sciatic nerve in rodents, e.g., rats (Eaton et al. (2000) Cell Transplant. 9:637-56); or intraplantar complete Freund's adjuvant (CFA) injection as a model of arthritic pain (Fraser et al. (2000) Br. J. Pharmacol. 129:1668-72). Other animal models of pain response are described in, e.g., ILAR Journal (1999) Volume 40, Number 3 (entire issue).

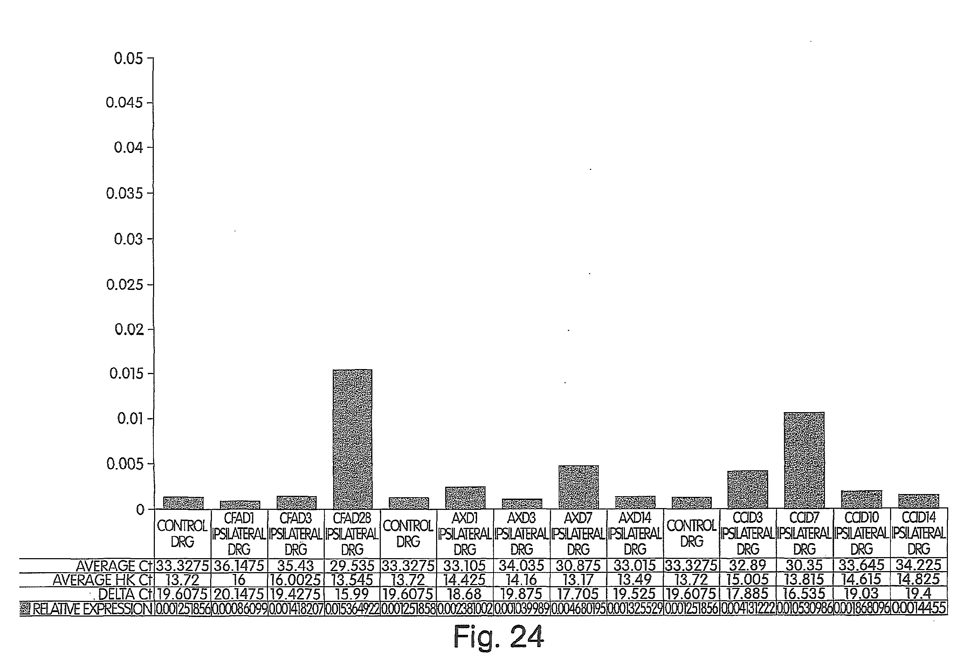

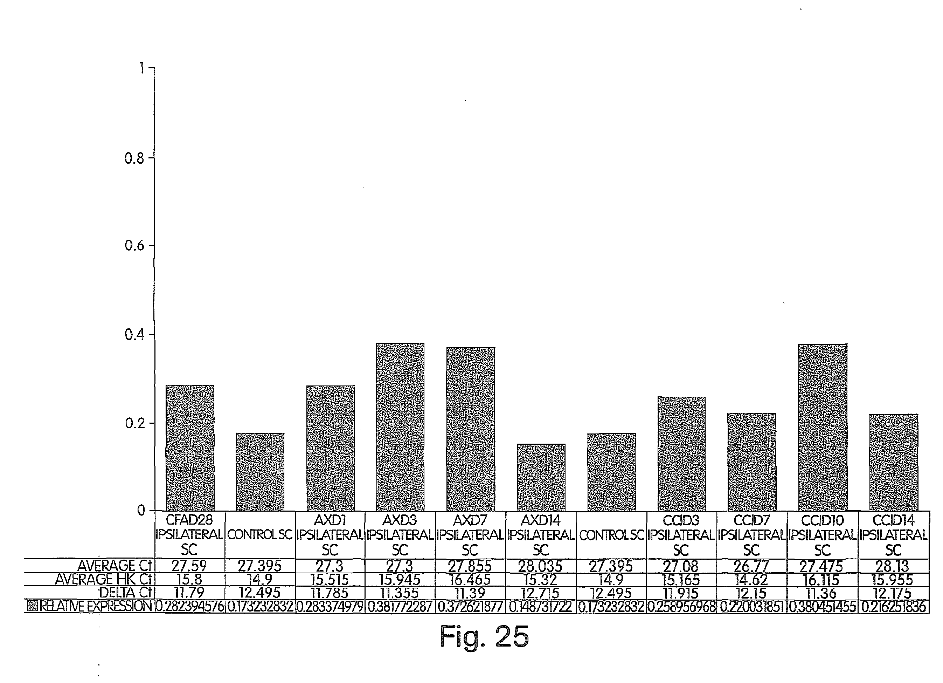

52872 expression was shown to be regulated in three different pain response models. Specifically, the upregulation of 52872 expression was detected in DRG following CFA injection (28 days), axotomy (7 days), and CCI (7 days) (Figure 24). The upregulation of 52872 expression was also detected in the spinal cord following CFA injection (28 days), axotomy (1-7 days), and CCI (1-14 days) (Figure 25).

52872 shows homology to the human galanin receptor type 2 (GAL2-R) (GenBank™ Accession No. 043603). GAL2-R is expressed abundantly within the central nervous system in both the hypothalamus and hippocampus. GAL2-R is a receptor for the hormone galanin, a 29 amino acid neoropeptide that is present in sensory and spinal dorsal horn neurons. Conditions associated with chronic pain such as peripheral nerve injury and inflammation are associated with upregulated synthesis of galanin, e.g., in sensory neurons and spinal cord neurons. Endogenous galanin has been proposed to function as a modulator of nociceptive input, e.g., at the spinal level. The administration of exogenous galanin exerts complex effects on spinal nociceptive transmission, although inhibitory action appears to predominate (Xu et al. (2000) Neuropeptides 34:137-47). Despite these observations, the precise role of galanin in pain processing remains a subject of debate (liu et al. (2000) Brain Res. 886:67-72). Galanin may participate in nociceptive processing by mediating interrelated inhibitory and excitatory effects (Kerr et al. (2000) Eur. J. Neuroses 12:793-802).

Based upon the expression patterns of 52872, the regulated expression in pain models, and its homology to the galanin receptor type 2, 52872 is likely a receptor for a neuropeptide, e.g., a neuropeptide involved in nociception.

52872 associated disorders can detrimentally affect regulation and modulation of the pain response, vasoconstriction, inflammatory response and pain therefrom. Examples of disorders in which the 52872 molecules of the invention may be directly or indirectly involved include pain, pain syndromes, and inflammatory disorders, including inflammatory pain as described in more detail below.

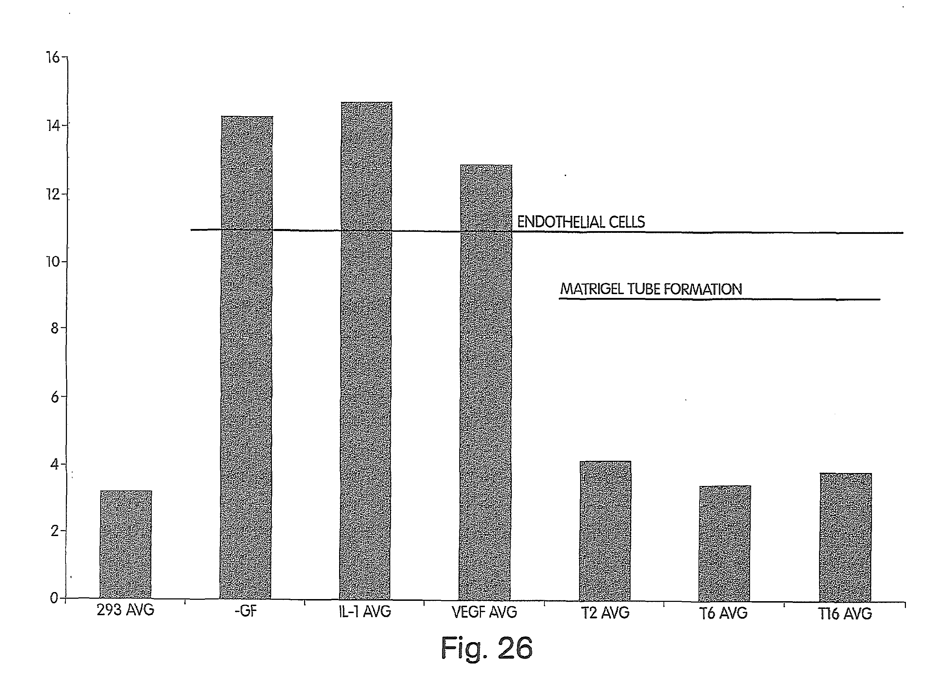

52881 mRNA is expressed in cultured endothelial cells and its expression is downregulated during the formation of vascular tube-like structures (Figure 26). This regulation of 52881 expression suggests that the 52881 protein may inhibit vascular tube formation, a process thought to be similar to angiogenesis. This observation also suggests that 52881 may participate in atherosclerosis and/or the control of vascular tone, as endothelial cell phenotype plays an important role in both of these processes. For example, the expression of cyclooxygenase-2 and endothelin-1, two genes with established relevance to atherosclerosis and the control of vascular tone, have been shown to be regulated in models similar to those described in Figure 26. Based upon the regulated endothelial cell expression of 52881, the polypeptides of the invention may be useful for developing novel diagnostic and therapeutic agents for 52881-mediated or related disorders, e.g., cardiovascular disorders and angiogenesis-related disorders.

Based upon the 1983, 2398, 45449 expression in cardiovascular tissues (e.g., the heart and endothelial cells), it is likely that these molecules are involved in cardiovascular disorders, including hyperproliferative vascular diseases (e.g., hypertension, vascular restenosis and atherosclerosis, ischaemia reperfusion injury, cardiac hypertrophy, coronary artery disease, myocardial infarction, arrythmia, cardiomyopathies, and congestive heart failure), as described in more detail below.

The 1983 molecules of the invention may be involved in skin disorders, such as hyperproliferative skin disorder (e.g., psoriasis; eczema; lupus associated skin lesions; psoriatic arthritis; rheumatoid arthritis that involves hyperproliferation and inflammation of epithelial-related cells lining the joint capsule; dermatitides such as seborrheic dermatitis and solar dermatitis; keratoses such as seborrheic keratosis, senile keratosis, actinic keratosis, photo-induced keratosis, and keratosis follicularis; acne vulgaris; keloids and prophylaxis against keloid formation; nevi; warts including verruca, condyloma or condyloma acuminatum, and human papilloma viral (HPV) infections such as venereal warts; leukoplakia; lichen planus; and keratitis). Similarly, 1983 molecules are expressed liver cells, e.g., hemangiomas, and thus may be involved in mediating liver disorders (as described in more detail below). Accordingly, 1983 molecules can act as novel diagnostic targets and therapeutic agents for controlling disorders involving aberrant activities of these cells.

Similarly, expression of 52872, 1983, 2398, 45449 and 50289 is detected in the neural tissues, e.g., the brain. Accordingly, these molecules can act as novel diagnostic targets and therapeutic agents for controlling neurological disorders. 50289 mRNA expression is also detected in the testis, small intestine and the pituitary. 45449 mRNA expression is also detected in granulocytes and liver cells. Thus, it is likely that 50289 and 45449 molecules are involved in disorders involving aberrant activities of these cells.

As the 1983, 52881, 2398, 45449, 50289, or 52872 polypeptides of the invention may modulate 1983, 52881, 2398, 45449, 50289, or 52872-mediated activities, they may be useful as of for developing novel diagnostic and therapeutic agents for 1983, 52881, 2398, 45449, 50289, or 52872-mediated or related disorders, as described below.

As used herein, a"1983, 52881,2398, 45449, 50289, or 52872 activity", "biological activity of 1983, 52881, 2398, 45449, 50289, or 52872" or "functional activity of 1983, 52881, 2398, 45449, 50289, or 52872", refers to an activity exerted by a 1983, 52881, 2398, 45449, 50289, or 52872 protein, polypeptide or nucleic acid molecule on e.g., a 1983, 52881, 2398, 45449, 50289, or 52872-responsive cell or on a 1983, 52881, 2398, 45449, 50289, or 52872 substrate, e.g., a protein substrate, as determined *in vivo* or *in vitro.* In one embodiment, a 1983, 52881, 2398, 45449, 50289, or 52872 activity is a direct activity, such as an association with a 52872 target molecule. A "target molecule" or "binding partner" is a molecule with which a 1983, 52881,2398, 45449, 50289, or 52872 protein binds or interacts in nature. In an exemplary embodiment, 1983, 52881, 2398, 45449, 50289, or 52872 is a receptor, e.g., a receptor for a neuropeptide.

A 1983, 52881, 2398, 45449, 50289, or 52872 activity can also be an indirect activity, e.g., a cellular signaling activity mediated by interaction of the 1983, 52881, 2398, 45449, 50289, or 52872 protein with a 1983, 52881, 2398, 45449, 50289, or 52872 receptor. Based on the above-described sequence similarities, the 1983, 52881, 2398, 45449, 50289, or 52872 molecules of the present invention are predicted to have similar biological activities as G protein-coupled receptor family members, e.g., neuropeptide receptors. For example, the 1983, 52881, 2398, 45449, 50289, or 52872 proteins of the present invention can have one or more of the following activities: (1) regulating, sensing and/or transmitting an extracellular signal into a cell, for example, transmitting a pain related signal from a neuropeptide; (2) signaling to G proteins; (3) modulating a pain or inflammation response; (4) modulating angiogenesis and/or the control of vascular tone; (5) interacting with (e.g., binding to) an extracellular signal, e.g., a neuropeptide, or a cell surface receptor; (6) mobilizing an intracellular molecule that participates in a signal transduction pathway (e.g., adenylate cyclase or phosphatidylinositol 4,5-bisphosphate (PIP₂), inositol 1,4,5-triphosphate (IP₃)); (7) controlling production or secretion of molecules; (8) altering the structure of a cellular component; (9) modulating cell proliferation, e.g., synthesis of DNA; or (10) modulating cell migration, cell differentiation; and cell survival

As the 1983, 52881, 2398, 45449, 50289, or 52872 polypeptides of the invention may modulate 1983, 52881, 2398, 45449, 50289, or 52872 -mediated activities, they may be useful for developing novel diagnostic and therapeutic agents for 1983, 52881, 2398, 45449, 50289, or 52872-mediated or related disorders. For example, the 1983, 52881, 2398, 45449, 50289, or 52872 molecules can act as novel diagnostic targets and therapeutic agents controlling cardiovascular disorders.

Preferred examples of cardiovascular disorders or diseases include e.g., atherosclerosis, thrombosis, heart failure, ischemic heart disease, angina pectoris, myocardial infarction, sudden cardiac death, hypertensive heart disease; non-coronary vessel disease, such as arteriolosclerosis, small vessel disease, nephropathy, hypertriglyceridemia, hypercholesterolemia, hyperlipidemia, asthma, hypertension, emphysema and chronic pulmonary disease; or a cardiovascular condition associated with interventional procedures ("procedural vascular trauma"), such as restenosis following angioplasty, placement of a shunt, stet, stint, synthetic or natural excision grafts, indwelling catheter, valve or other implantable devices.

The term "cardiovascular disorders" or "disease" includes heart disorders, as well as disorders of the blood vessels of the circulation system caused by, e.g., abnormally high concentrations of lipids in the blood vessels.

Disorders involving the heart, include but are not limited to, heart failure, including but not limited to, cardiac hypertrophy, left-sided heart failure, and right-sided heart failure; ischemic heart disease, including but not limited to angina pectoris, myocardial infarction, chronic ischemic heart disease, and sudden cardiac death; hypertensive heart disease, including but not limited to, systemic (left-sided) hypertensive heart disease and pulmonary (right-sided) hypertensive heart disease; valvular heart disease, including but not limited to, valvular degeneration caused by calcification, such as calcific aortic stenosis, calcification of a congenitally bicuspid aortic valve, and mitral annular calcification, and myxomatous degeneration of the mitral valve (mitral valve prolapse), rheumatic fever and rheumatic heart disease, infective endocarditis, and noninfected vegetations, such as nonbacterial thrombotic endocarditis and endocarditis of systemic lupus erythematosus (Libman-Sacks disease), carcinoid heart disease, and complications of artificial valves; myocardial disease, including but not limited to dilated cardiomyopathy, hypertrophic cardiomyopathy, restrictive cardiomyopathy, and myocarditis; pericardial disease, including but not limited to, pericardial effusion and hemopericardium and pericarditis, including acute pericarditis and healed pericarditis, and rheumatoid heart disease; neoplastic heart disease, including but not limited to, primary cardiac tumors, such as myxoma, lipoma, papillary fibroelastoma, rhabdomyoma, and sarcoma, and cardiac effects of noncardiac neoplasms; congenital heart disease, including but not limited to, left-to-right shunts--late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia, and disorders involving cardiac transplantation.

Disorders involving blood vessels include, but are not limited to, responses of vascular cell walls to injury, such as endothelial dysfunction and endothelial activation and intimal thickening; vascular diseases including, but not limited to, congenital anomalies, such as arteriovenous fistula, atherosclerosis, and hypertensive vascular disease, such as hypertension; inflammatory disease--the vasculitides, such as giant cell (temporal) arteritis, Takayasu arteritis, polyarteritis nodosa (classic), Kawasaki syndrome (mucocutaneous lymph node syndrome), microscopic polyanglitis (microscopic polyarteritis, hypersensitivity or leukocytoclastic anglitis), Wegener granulomatosis, thromboanglitis obliterans (Buerger disease), vasculitis associated with other disorders, and infectious arteritis; Raynaud disease; aneurysms and dissection, such as abdominal aortic aneurysms, syphilitic (luetic) aneurysms, and aortic dissection (dissecting hematoma); disorders of veins and lymphatics, such as varicose veins, thrombophlebitis and phlebothrombosis, obstruction of superior vena cava (superior vena cava syndrome), obstruction of inferior vena cava (inferior vena cava syndrome), and lymphangitis and lymphedema; tumors, including benign tumors and tumor-like conditions, such as hemangioma, lymphangioma, glomus tumor (glomangioma), vascular ectasias, and bacillary angiomatosis, and intermediate-grade (borderline low-grade malignant) tumors, such as Kaposi sarcoma and hemangloendothelioma, and malignant tumors, such as angiosarcoma and hemangiopericytoma; and pathology of therapeutic interventions in vascular disease, such as balloon angioplasty and related techniques and vascular replacement, such as coronary artery bypass graft surgery.

As used herein, the term "atherosclerosis" is intended to have its clinical meaning. This term refers to a cardiovascular condition occurring as a result of narrowing down of the arterial walls. The narrowing is due to the formation of plaques (raised patches) or streaks in the inner lining of the arteries. These plaques consist of foam cells of low-density lipoproteins, oxidized-LDL, decaying muscle cells, fibrous tissue, clumps of blood platelets, cholesterol, and sometimes calcium. They tend to form in regions of turbulent blood flow and are found most often in people with high concentrations of cholesterol in the bloodstream. The number and thickness of plaques increase with age, causing loss of the smooth lining of the blood vessels and encouraging the formation of thrombi (blood clots). Sometimes fragments of thrombi break off and form emboli, which travel through the bloodstream and block smaller vessels. The blood supply is restricted to the heart, eventually forming a blood clot leading to death. The major causes of atherosclerosis are hypercholesterolemia (and low HDL), hypoalphoproteinemia, and hyperlipidemia marked by high circulating cholesterol and high lipids like LDL-cholesterol and triglycerides in the blood. These lipids are deposited in the arterial walls, obstructing the blood flow and forming atherosclerotic plaques leading to death.

As used herein the term "hypercholesterolemia" is a condition with elevated levels of circulating total cholesterol, LDL-cholesterol and VLDL-cholesterol as per the guidelines of the Expert Panel Report of the National Cholesterol Educational Program (NCEP) of Detection, Evaluation of Treatment of high cholesterol in adults (see, Arch. Int. Med. (1988) 148, 36-39).

As used herein the term "hyperlipidemia" or "hyperlipemia" is a condition where the blood lipid parameters are elevated in the blood. This condition manifests an abnormally high concentration of fats. The lipid fractions in the circulating blood are, total cholesterol, low density lipoproteins, very low density lipoproteins and triglycerides.

As used herein the term "lipoprotein" such as VLDL, LDL and HDL, refers to a group of proteins found in the serum, plasma and lymph and are important for lipid transport. The chemical composition of each lipoprotein differs in that the HDL has a higher proportion of protein versus lipid, whereas the VLDL has a lower proportion of protein versus lipid.

As used herein, the term "triglyceride" means a lipid or neutral fat consisting of glycerol combined with three fatty acid molecules.

As used herein the term "xanthomatosis" is a disease evidenced by a yellowish swelling or plaques in the skin resulting from deposits of fat. The presence of xanthomas are usually accompanied by raised blood cholesterol levels.

As used herein the term "apolipoprotein B" or "apoprotein B" or "Apo B" refers to the protein component of the LDL cholesterol transport proteins. Cholesterol synthesized de novo is transported from the liver and intestine to peripheral tissues in the form of lipoproteins. Most of the apolipoprotein B is secreted into the circulatory system as VLDL.

As used herein the term "apolipoprotein A" or "apoprotein A" or "Apo A" refers to the protein component of the HDL cholesterol transport proteins.

"Procedural vascular trauma" includes the effects of surgical/medical-mechanical interventions into mammalian vasculature, but does not include vascular trauma due to the organic vascular pathologies listed hereinabove, or to unintended traumas, such as due to an accident. Thus, procedural vascular traumas within the scope of the present treatment method include (1) organ grafting or transplantation, such as transplantation and grafting of heart, kidney, liver and the like, e.g., involving vessel anastomosis; (2) vascular surgery, such as coronary bypass surgery, biopsy, heart valve replacement, atheroectomy, thrombectomy, and the like; (3) transcatheter vascular therapies (TVT) including angioplasty, e.g., laser angioplasty and PTCA procedures discussed hereinbelow, employing balloon catheters, or indwelling catheters; (4) vascular grafting using natural or synthetic materials, such as in saphenous vein coronary bypass grafts, dacron and venous grafts used for peripheral arterial reconstruction, etc.; (5) placement of a mechanical shunt, such as a PTFE hemodialysis shunt used for arteriovenous communications; and (6) placement of an intravascular stent, which may be metallic, plastic or a biodegradable polymer. See U.S. patent application Ser. No. 08/389,712, filed Feb. 15, 1995, which is incorporated by reference herein. For a general discussion of implantable devices and biomaterials from which they can be formed, see H. Kambic et al., "Biomaterials in Artificial Organs", Chem. Eng. News, 30 (Apr. 14, 1986), the disclosure of which is incorporated by reference herein.

Small vessel disease includes, but is not limited to, vascular insufficiency in the limbs, peripheral neuropathy and retinopathy, e.g., diabetic retinopathy.

In some embodiments, the therapeutic and prophylactic uses of the compositions of the invention, further include the administration of cholesterol lowering agents as a combination drug therapies. The term "combination therapy" as used herein refers to the administration to a subject (concurrently or sequentially) of two or more cholesterol lowering agents. Current combination therapy therapies using combinations of niacin and statins are being used with positive results to treat hyperlipidemia (Guyton, JR. (1999) Curr Cardiol Rep. 1(3):244-250; Otto, C. et al. (1999) Internist (Berl) 40(12):1338-45). Other useful drug combinations include those derived by addition of fish oil, bile acid binding resins, or stanol esters, as well as nonstatin combinations susn as niacin-resin or fibrate-niacin (Guyton, JR. (1999) supra). For examples of dosages and administration schedules of the cholesterol lowering agents, the teachings of Guyton, JR. (1999) supra, Otto, C. et al. (1999) supra, Guyton, JR et al. (1998) Am J Cardiol 82(12A):82U-86U; Guyton, JR et al. (1998) Am J Cardiol. 82(6):737-43; Vega, GL et al. (1998) Am J. Cardiol. 81(4A):36B-42B; Schectman, G. (1996) Ann Intern Med. 125(12):990-1000; Nakamura, H. et al. (1993) Nippon Rinsho 51(8):2101-7; Goldberg, A. et al. (2000) Am J Cardiol 85(9):1100-5; Morgan, JM et al. (1996) J Cardiovasc. Pharmac. Ther. 1(3):195-202; Stein, EA et al. (1996) J Cardiovasc Pharmacol Ther 1(2):107-116; and Goldberg, AC (1998) Am J Cardiol 82(12A):35U-41U, are expressly incorporated by reference.

As used herein, "cholesterol lowering agents" include agents which are useful for lowering serum cholesterol such as for example bile acid sequestering resins (e.g. colestipol hydrochloride or cholestyramine), fish oil, stanol esters, an ApoAII-lowering agent, a VLDL lowering agent, an ApoAI-stimulating agent, fibric acid derivatives (e.g. clofibrate, fenofibrate, or gemfibrozil), thiazolidenediones (e.g. troglitazone), or HMG-CoA reductase inhibitors (e.g. statins, such as fluvastatin sodium, lovastatin, pravastatin sodium, or simvastatin), as well as nicotinic acid, niacin, or probucol. "VLDL-lowering agent" includes an agent which decreases the hepatic synthesis of triglyceride-rich lipoproteins or increases the catabolism of triglyceride-rich lipoproteins, e.g., fibrates such as gemfibrozil, or the statins, increases the expression of the apoE-mediated clearance pathway, or improves insulin sensitivity in diabetics, e.g., the thiazolidene diones.

As the 1983, 52881, 2398, 45449, 50289, or 52872 polypeptides of the invention may modulate 1983, 52881, 2398, 45449, 50289, or 52872-mediated activities, they may be useful for developing novel diagnostic and therapeutic agents for 1983, 52881, 2398, 45449, 50289, or 52872-mediated or related disorders. For example, the 1983, 52881, 2398, 45449, 50289, or 52872 molecules can act as novel diagnostic targets and therapeutic agents controlling pain, pain disorders, and inflammatory disorders. For example, a 1983, 52881, 2398, 45449, 50289, or 52872 inhibitor can be useful in the treatment of pain, as 1983, 52881, 2398, 45449, 50289, or 52872 inhibition could increase the endogenous levels of enkephalins and thereby increase the associated analgesic response.

Examples of pain conditions include, but are not limited to, pain elicited during various forms of tissue injury, e.g., inflammation, infection, and ischemia; pain associated with musculoskeletal disorders, e.g., joint pain, or arthritis; tooth pain; headaches, e.g., migrane; pain associated with surgery; pain related to inflammation, e.g., irritable bowel syndrome; chest pain; or hyperalgesia, e.g., excessive sensitivity to pain (described in, for example, Fields (1987) Pain, New York:McGraw-Hill). Other examples of pain disorders or pain syndromes include, but are not limited to, complex regional pain syndrome (CRPS), reflex sympathetic dystrophy (RSD), causalgia, neuralgia, central pain and dysesthesia syndrome, carotidynia, neurogenic pain, refractory cervicobrachial pain syndrome, myofascial pain syndrome, craniomandibular pain dysfunction syndrome, chronic idiopathic pain syndrome, Costen's pain-dysfunction, acute chest pain syndrome, nonulcer dyspepsia, interstitial cystitis, gynecologic pain syndrome, patellofemoral pain syndrome, anterior knee pain syndrome, recurrent abdominal pain in children, colic, low back pain syndrome, neuropathic pain, phantom pain from amputation, phantom tooth pain, or pain asymbolia (the inability to feel pain). Other examples of pain conditions include pain induced by parturition, or post partum pain.

Agents that modulate 1983, 52881, 2398, 45449, 50289, or 52872 polypeptide or nucleic acid activity or expression can be used to treat pain elicited by any medical condition. A subject receiving the treatment can be additionally treated with a second agent, e.g., an anti-inflammatory agent, an antibiotic, or a chemotherapeutic agent, to further ameliorate the condition.

The 1983, 52881, 2398, 45449, 50289, or 52872 molecules can also act as novel diagnostic targets and therapeutic agents controlling pain caused by other disorders, e.g., cancer, e.g., prostate cancer.

As used herein, the terms "cancer", "hyperproliferative", and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as those affecting lung, breast, thyroid, lymphoid, gastrointestinal, and the genito-urinary tract. The terms "cancer" or "neoplasms" also includes adenocarcinomas that include malignancies such as most colon cancers, renal-cell carcinoma, prostate cancer and/or testicular tumors, non-small cell carcinoma of the lung, cancer of the small intestine, and cancer of the esophagus.

The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon, and ovary. The term also includes carcinosarcomas, e.g., malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

Examples of intestinal (e.g., small intestinal) disorders include, but are not limited to, congenital anomalies, such as atresia and stenosis, Meckel diverticulum, congenital aganglionic megacolon-Hirschsprung disease; enterocolitis, such as diarrhea and dysentery, infectious enterocolitis, including viral gastroenteritis, bacterial enterocolitis, necrotizing enterocolitis, antibiotic-associated colitis (pseudomembranous colitis), and collagenous and lymphocytic colitis, miscellaneous intestinal inflammatory disorders, including parasites and protozoa, acquired immunodeficiency syndrome, transplantation, drug-induced intestinal injury, radiation enterocolitis, neutropenic colitis (typhlitis), and diversion colitis; idiopathic inflammatory bowel disease, such as Crohn disease and ulcerative colitis; tumors of the colon, such as non-neoplastic polyps, adenomas, familial syndromes, colorectal carcinogenesis, colorectal carcinoma, and carcinoid tumors. Disorders involving the small intestine include the malabsorption syndromes such as, celiac sprue, tropical sprue (postinfectious sprue), whipple disease, disaccharidase (lactase) deficiency, abetalipoproteinemia, and tumors of the small intestine including adenomas and adenocarcinoma.

Disorders involving the liver include, but are not limited to, hepatic injury; jaundice and cholestasis, such as bilirubin and bile formation; hepatic failure and cirrhosis, such as cirrhosis, portal hypertension, including ascites, portosystemic shunts, and splenomegaly; infectious disorders, such as viral hepatitis, including hepatitis A-E infection and infection by other hepatitis viruses, clinicopathologic syndromes, such as the carrier state, asymptomatic infection, acute viral hepatitis, chronic viral hepatitis, and fulminant hepatitis; autoimmune hepatitis; drug- and toxin-induced liver disease, such as alcoholic liver disease; inborn errors of metabolism and pediatric liver disease, such as hemochromatosis, Wilson disease, *a*₁-antitrypsin deficiency, and neonatal hepatitis; intrahepatic biliary tract disease, such as secondary biliary cirrhosis, primary biliary cirrhosis, primary sclerosing cholangitis, and anomalies of the biliary tree; circulatory disorders, such as impaired blood flow into the liver, including hepatic artery compromise and portal vein obstruction and thrombosis, impaired blood flow through the liver, including passive congestion and centrilobular necrosis and peliosis hepatis, hepatic vein outflow obstruction, including hepatic vein thrombosis (Budd-Chiari syndrome) and venoocclusive disease; hepatic disease associated with pregnancy, such as preeclampsia and eclampsia, acute fatty liver of pregnancy, and intrehepatic cholestasis of pregnancy; hepatic complications of organ or bone marrow transplantation, such as drug toxicity after bone marrow transplantation, graft-versus-host disease and liver rejection, and nonimmunologic damage to liver allografts; tumors and tumorous conditions, such as nodular hyperplasias, adenomas, and malignant tumors, including primary carcinoma of the liver and metastatic tumors.

Disorders involving the testis and epididymis include, but are not limited to, congenital anomalies such as cryptorchidism, regressive changes such as atrophy, inflammations such as nonspecific epididymitis and orchitis, granulomatous (autoimmune) orchitis, and specific inflammations including, but not limited to, gonorrhea, mumps, tuberculosis, and syphilis, vascular disturbances including torsion, testicular tumors including germ cell tumors that include, but are not limited to, seminoma, spermatocytic seminoma, embryonal carcinoma, yolk sac tumor choriocarcinoma, teratoma, and mixed tumors, tumore of sex cord-gonadal stroma including, but not limited to, Leydig (interstitial) cell tumors and sertoli cell tumors (androblastoma), and testicular lymphoma, and miscellaneous lesions of tunica vaginalis.

Examples of immune disorders include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

The 1983, 52881, 2398, 45449, 50289, or 52872 protein, fragments thereof, and derivatives and other variants of the sequence in SEQ ID NO:2, SEQ ID NO:5, SEQ ID NO:8, SEQ ID NO:11, SEQ ID NO:14, or SEQ ID NO:17 thereof are collectively referred to as "polypeptides or proteins of the invention" or "1983, 52881, 2398, 45449, 50289, or 52872 polypeptides or proteins". Nucleic acid molecules encoding such polypeptides or proteins are collectively referred to as "nucleic acids of the invention" or "1983, 52881, 2398, 45449, 50289, or 52872 nucleic acids." 1983, 52881, 2398, 45449, 50289, or 52872 molecules refer to 1983, 52881, 2398, 45449, 50289, or 52872 nucleic acids, polypeptides, and antibodies.

As used herein, the term "nucleic acid molecule" includes DNA molecules (e.g., a cDNA or genomic DNA) and RNA molecules (e.g., an mRNA) and analogs of the DNA or RNA generated, e.g., by the use of nucleotide analogs. The nucleic acid molecule can be single-stranded or double-stranded, but preferably is double-stranded DNA.

The term "isolated or purified nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in Current Protocols in Molecular Biology, John Wiley & Sons, N.Y. (1989), 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50 °C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

Preferably, an isolated nucleic acid molecule of the invention that hybridizes under stringent conditions to the sequence of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, corresponds to a naturally-occurring nucleic acid molecule.

As used herein, a "naturally-occurring" nucleic acid molecule refers to an RNA or DNA molecule having a nucleotide sequence that occurs in nature (e.g., encodes a natural protein).

As used herein, the terms "gene" and "recombinant gene" refer to nucleic acid molecules which include an open reading frame encoding a 1983, 52881, 2398, 45449, 50289, or 52872 protein, preferably a mammalian 1983, 52881, 2398, 45449, 50289, or 52872 protein, and can further include non-coding regulatory sequences, and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 1983, 52881, 2398, 45449, 50289, or 52872 protein having less than about 30%, 20%, 10% and more preferably 5% (by dry weight), of non-1983, 52881, 2398, 45449, 50289, or 52872 protein (also referred to herein as a "contaminating protein"), or of chemical precursors or non-1983, 52881, 2398, 45449, 50289, or 52872 chemicals. When the 1983, 52881, 2398, 45449, 50289, or 52872 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 1983, 52881, 2398, 45449, 50289, or 52872 (e.g., the sequence of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18 without abolishing or more preferably, without substantially altering a biological activity, whereas an "essential" amino acid residue results in such a change. For example, amino acid residues that are conserved among the polypeptides of the present invention, e.g., those present in a seven transmembrane domain, are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a 1983, 52881, 2398, 45449, 50289, or 52872 protein is preferably replaced with another amino acid residue from the same side chain family. Alternatively, in another embodiment, mutations can be introduced randomly along all or part of a 1983, 52881, 2398, 45449, 50289, or 52872 coding sequence, such as by saturation mutagenesis, and the resultant mutants can be screened for 1983, 52881, 2398, 45449, 50289, or 52872 biological activity to identify mutants that retain activity. Following mutagenesis of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, the encoded protein can be expressed recombinantly and the activity of the protein can be determined.

As used herein, a "biologically active portion" of a 1983, 52881, 2398, 45449, 50289, or 52872 protein includes a fragment of a 1983, 52881, 2398, 45449, 50289, or 52872 protein which participates in an interaction between a 1983, 52881, 2398, 45449, 50289, or 52872 molecule and a non-1983, 52881, 2398, 45449, 50289, or 52872 molecule. Biologically active portions of a 1983, 52881, 2398, 45449, 50289, or 52872 protein include peptides comprising amino acid sequences sufficiently homologous to or derived from the amino acid sequence of the 1983, 52881, 2398, 45449, 50289, or 52872 protein, e.g., the amino acid sequence shown in SEQ ID NO:2, SEQ ID NO:5, SEQ ID NO:8, SEQ ID NO:11, SEQ ID NO:14, or SEQ ID NO:17, which include less amino acids than the full length 1983, 52881, 2398, 45449, 50289, or 52872 proteins, and exhibit at least one activity of a 1983, 52881, 2398, 45449, 50289, or 52872 protein. Typically, biologically active portions comprise a domain or motif with at least one activity of the 1983, 52881, 2398, 45449, 50289, or 52872 protein, e.g., a domain or motif capable of regulating, sensing and/or transmitting an extracellular signal into a cell, for example, an endothelial cell; a domain or motif capable of interacting with (e.g., binding to) an extracellular signal or a cell surface receptor; a domain or motif capable of mobilizing an intracellular molecule that participates in a signal transduction pathway (e.g., adenylate cyclase or phosphatidylinositol 4,5-bisphosphate (PIP₂), inositol 1,4,5-triphosphate (IP₃)); a domain or motif capable of regulating polarization of the plasma membrane; a domain or motif capable of controlling production or secretion of molecules; a domain or motif capable of altering the structure of a cellular component; a domain or motif capable of modulating cell proliferation, e.g., synthesis of DNA; and/or a domain or motif capable of modulating migration, proliferation and/or differentiation of a cell. A biologically active portion of a 1983, 52881, 2398, 45449, 50289, or 52872 protein can be a polypeptide which is, for example, 10, 25, 50, 100, 200 or more amino acids in length. Biologically active portions of a 1983, 52881, 2398, 45449, 50289, or 52872 protein can be used as targets for developing agents which modulate a 1983, 52881, 2398, 45449, 50289, or 52872 mediated activity, e.g., a biological activity described herein.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence (e.g., when aligning a second sequence to the 52881 amino acid sequence of SEQ ID NO:5 having 75 amino acid residues, at least 22, preferably at least 30, more preferably at least 37, even more preferably at least 45, and even more preferably at least 52, 60, or 67 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453 ) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to 1983, 52881, 2398, 45449, 50289, or 52872 nucleic acid molecules of the invention. BLAST protein searches can be performed with the XBLAST program, score = 50, wordlength = 3 to obtain amino acid sequences homologous to 1983, 52881, 2398, 45449, 50289, or 52872 protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used. See http://www.ncbi.nlm.nih.gov.

Particular 1983, 52881, 2398, 45449, 50289, or 52872 polypeptides of the present invention have an amino acid sequence sufficiently identical to the amino acid sequence of SEQ ID NO:2, SEQ ID NO:5, SEQ ID NO:8, SEQ ID NO:11, SEQ ID NO:14, or SEQ ID NO:17. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 90%. 91%, 92%, 93%, 94%, 95%, 96%, 97%, 98% or 99% identity to SEQ ID NO:2, SEQ ID NO:5, SEQ ID NO:8, SEQ ID NO:11, SEQ ID NO:14, or SEQ ID NO:17 are termed sufficiently or substantially identical. In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 60%, or 65% identity, likely 75% identity, more likely 85%, 90%, 91%, 92%, 93%, 94%, 95%, 96%, 97%, 98% or 99% identity to SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18 are termed substantially identical.

"Misexpression or aberrant expression", as used herein, refers to a non-wild type pattern of gene expression, at the RNA or protein level. It includes: expression at non-wild type levels, i.e., over or under expression; a pattern of expression that differs from wild type in terms of the time or stage at which the gene is expressed, e.g., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject," as used herein, refers to human and non-human animals. The term "non-human animals" of the invention includes all vertebrates, e.g., mammals, such as non-human primates (particularly higher primates), sheep, dog, rodent (e.g., mouse or rat), guinea pig, goat, pig, cat, rabbits, cow, and non-mammals, such as chickens, amphibians, reptiles, etc. In a preferred embodiment, the subject is a human. In another embodiment, the subject is an experimental animal or animal suitable as a disease model.

A "purified preparation of cells", as used herein, refers to, in the case of plant or animal cells, an in vitro preparation of cells and not an entire intact plant or animal. In the case of cultured cells or microbial cells, it consists of a preparation of at least 10% and more preferably 50% of the subject cells.

Various aspects of the invention are described in further detail below.

### Isolated Nucleic Acid Molecules

In one aspect, the invention provides, an isolated or purified, nucleic acid molecule that encodes a 1983, 52881, 2398, 45449, 50289, or 52872 polypeptide described herein, e.g., a full length 1983, 52881, 2398, 45449, 50289, or 52872 protein or a fragment thereof, e.g., a biologically active portion of 1983, 52881, 2398, 45449, 50289, or 52872 protein. Also included is a nucleic acid fragment suitable for use as a hybridization probe, which can be used, e.g., to identify a nucleic acid molecule encoding a polypeptide of the invention, 1983, 52881, 2398, 45449, 50289, or 52872 mRNA, and fragments suitable for use as primers, e.g., PCR primers for the amplification or mutation of nucleic acid molecules.

In one embodiment, an isolated nucleic acid molecule of the invention includes the nucleotide sequence shown in SEQ ID NO:1, SEQ ID NO:4, SEQ ID NO:7, SEQ ID NO:10, SEQ ID NO:13, or SEQ ID NO:16, or a portion of any of these nucleotide sequences. In one embodiment, the nucleic acid molecule includes sequences encoding the human 1983, 52881, 2398, 45449, 50289, or 52872 protein (i.e., "the coding region" of SEQ ID NO:1, SEQ ID NO:4, SEQ ID NO:7, SEQ ID NO:10, SEQ ID NO:13, or SEQ ID NO:16, as shown in SEQ ID NO:3, SEQ ID NO:6, SEQ ID NO:9, SEQ ID NO:12, SEQ ID NO:15, or SEQ ID NO:18), as well as 5' untranslated sequences. Alternatively, the nucleic acid molecule can include only the coding region of SEQ ID NO:1, SEQ ID NO:4, SEQ ID NO:7, SEQ ID NO:10, SEQ ID NO:13, or SEQ ID NO:16 (e.g., SEQ ID NO:3, SEQ ID NO:6, SEQ ID NO:9, SEQ ID NO:12, SEQ ID NO:15, or SEQ ID NO:18) and, e.g., no flanking sequences which normally accompany the subject sequence. In another embodiment, the nucleic acid molecule encodes a sequence corresponding to a fragment of the protein from about amino acids 379 to 626 of SEQ ID NO:2, amino acids 80 to 154 of SEQ ID NO:5, amino acids 58 to 303 of SEQ ID NO:8, amino acids 1 to 176 of SEQ ID NO:11, amino acids 59 to 323 of SEQ ID NO:17, or amino acids 61 to 470 of SEQ ID NO:14.

In another embodiment, an isolated nucleic acid molecule of the invention includes a nucleic acid molecule which is a complement of the nucleotide sequence shown in SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, or a portion of any of these nucleotide sequences. In other embodiments, the nucleic acid molecule of the invention is sufficiently complementary to the nucleotide sequence shown in SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, such that it can hybridize to the nucleotide sequence shown in SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, thereby forming a stable duplex.

In one embodiment, an isolated nucleic acid molecule of the present invention includes a nucleotide sequence which is at least about: 60%, 65%, 70%, 75%, 80%, 85%, 90%, 91%, 92%, 93%, 94%, 95%, 96%, 97%, 98%, 99%, or more homologous to the entire length of the nucleotide sequence shown in SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, or a portion, preferably of the same length, of any of these nucleotide sequences.

### 1983, 52881, 2398, 45449, 50289, or 52872 Nucleic Acid Fragments

A nucleic acid molecule of the invention can include only a portion of the nucleic acid sequence of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18. For example, such a nucleic acid molecule can include a fragment which can be used as a probe or primer or a fragment encoding a portion of a 1983, 52881, 2398, 45449, 50289, or 52872 protein, e.g., an immunogenic or biologically active portion of a 1983, 52881, 2398, 45449, 50289, or 52872 protein. A fragment can comprise those nucleotides of SEQ ID NO:1, SEQ ID NO:4, SEQ ID NO:7, SEQ ID NO:10, SEQ ID NO:13, or SEQ ID NO:16 which encode a domain described herein, e.g., a seven transmembrane domain or an ANF receptor ligand binding domain. The nucleotide sequence determined from the cloning of the 1983, 52881, 2398, 45449, 50289, or 52872 gene allows for the generation of probes and primers designed for use in identifying and/or cloning other 1983, 52881, 2398, 45449, 50289, or 52872 family members, or fragments thereof, as well as 1983, 52881, 2398, 45449, 50289, or 52872 homologues, or fragments thereof, from other species.

In another embodiment, a nucleic acid includes a nucleotide sequence that includes part, or all, of the coding region and extends into either (or both) the 5' or 3' noncoding region. Other embodiments include a fragment which includes a nucleotide sequence encoding an amino acid fragment described herein. Nucleic acid fragments can encode a specific domain or site described herein or fragments thereof, particularly fragments thereof which are at least 100, preferably 150, 200, 250, or 300 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, a 1983, 52881, 2398, 45449, 50289, or 52872 nucleic acid fragment can include a sequence corresponding to a seven transmembrane domain or an ANF receptor ligand binding domain.

52881 probes and primers are provided. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30, 35, 40, 45, 50, 55, 60, 65, or 75 consecutive nucleotides of a sense or antisense sequence of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, or of a naturally occurring allelic variant or mutant of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes: a seven transmembrane domain which extends from about amino acids 379 to 626 of SEQ ID NO:2, amino acids 80 to 154 of SEQ ID NO:5, amino acids 58 to 303 of SEQ ID NO:8, amino acids 1 to 176 of SEQ ID NO:11, or amino acids 59 to 323 of SEQ ID NO:17; an ANF receptor ligand binding domain which extends from about amino acids 61 to 470 of SEQ ID NO:14; or a transmembrane domain which extends from about amino acid residues 388-407, 420-436, 455-479, 488-508, 525-549, 574-591, or 598-622 of SEQ ID NO:2; amino acid residues 11-34, 44-67, 85-106, 127-149, 172-196, or 245-269 of SEQ ID NO:5; amino acid residues 42-66, 78-99, 114-135, 154-176, 202-224, 241-259, or 291-310 of SEQ ID NO:8; amino acid residues 12-33, 68-90, or 123-147 of SEQ ID NO:11; amino acid residues 567-590, 600-623, 641-659, 679-702, 726-750, 762-782, or 799-810 of SEQ ID NO:14; or amino acid residues 43-67, 76-110, 117-136, 158-180, 204-228, 264-285, or 310-326 of SEQ ID NO:17.

In another embodiment a set of primers is provided, e.g., primers suitable for use in a PCR, which can be used to amplify a selected region of a 1983, 52881, 2398, 45449, 50289, or 52872 sequence, e.g., a domain, region, site or other sequence described herein. The primers should be at least 5, 10, or 50 base pairs in length and less than 100, or less than 200, base pairs in length. The primers should be identical, or differs by one base from a sequence disclosed herein or from a naturally occurring variant. For example, primers suitable for amplifying all or a portion of any of the following regions are provided: a seven transmembrane domain; an ANF receptor ligand binding domain; a transmembrane domain; and a non-transmembrane domain.

A nucleic acid fragment can encode an epitope bearing region of a polypeptide described herein.

A nucleic acid fragment encoding a "biologically active portion of a 1983, 52881, 2398, 45449, 50289, or 52872 polypeptide" can be prepared by isolating a portion of the nucleotide sequence of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, which encodes a polypeptide having a 1983, 52881, 2398, 45449, 50289, or 52872 biological activity (e.g., the biological activities of the 1983, 52881, 2398, 45449, 50289, or 52872 proteins are described herein), expressing the encoded portion of the 1983, 52881, 2398, 45449, 50289, or 52872 protein (e.g., by recombinant expression *in vitro*) and assessing the activity of the encoded portion of the 1983, 52881, 2398, 45449, 50289, or 52872 protein. For example, a nucleic acid fragment encoding a biologically active portion of 52881 includes seven transmembrane domain or an ANF receptor ligand binding domain, e.g., amino acids 379 to 626 of SEQ ID NO:2, amino acids 80 to 154 of SEQ ID NO:5, amino acids 58 to 303 of SEQ ID NO:8, amino acids 1 to 176 of SEQ ID NO:11, amino acids 59 to 323 of SEQ ID NO:17, or amino acids 61 to 470 of SEQ ID NO:14. A nucleic acid fragment encoding a biologically active portion of a 1983, 52881, 2398, 45449, 50289, or 52872 polypeptide, may comprise a nucleotide sequence which is greater than 300, 400, 500, or more nucleotides in length.

In preferred embodiments, a nucleic acid includes a nucleotide sequence which is about 300, 400, 500, 600, 700, 800, 900, 1000, 1100, 1200, 1300 or more nucleotides in length and hybridizes under stringent hybridization conditions to a nucleic acid molecule of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18.

### 1983, 52881, 2398, 45449, 50289, or 52872 Nucleic Acid Variants

The invention further encompasses nucleic acid molecules that differ from the nucleotide sequence shown in SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18. Such differences can be due to degeneracy of the genetic code (and result in a nucleic acid which encodes the same 1983, 52881, 2398, 45449, 50289, or 52872 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2, SEQ ID NO:5, SEQ ID NO:8, SEQ ID NO:11, SEQ ID NO:14, or SEQ ID NO:17. If alignment is needed for this comparison the sequences should be aligned for maximum homology. "Looped" out sequences from deletions or insertions, or mismatches, are considered differences.

Nucleic acids of the inventor can be chosen for having codons, which are preferred, or non preferred, for a particular expression system. E.g., the nucleic acid can be one in which at least one codon, at preferably at least 10%, or 20% of the codons has been altered such that the sequence is optimized for expression in *E. coli*, yeast, human, insect, or CHO cells.

Nucleic acid variants can be naturally occurring, such as allelic variants (same locus), homologs (different locus), and orthologs (different organism) or can be non naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18, e.g., as follows: by at least one but less than 10, 20, 30, or 40 nucleotides; at least one but less than 1%, 5%, 10% or 20% of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the nucleotide sequence shown in SEQ ID NO:2, SEQ ID NO:5, SEQ ID NO:8, SEQ ID NO:11, SEQ ID NO:14, or SEQ ID NO:17 or a fragment of this sequence. Such nucleic acid molecules can readily be identified as being able to hybridize under stringent conditions, to the nucleotide sequence shown in SEQ ID NO 2 or a fragment of the sequence. Nucleic acid molecules corresponding to orthologs, homologs, and allelic variants of the 1983, 52881, 2398, 45449, 50289, or 52872 cDNAs of the invention can further be isolated by mapping to the same chromosome or locus as the 1983, 52881, 2398, 45449, 50289, or 52872 gene.

Preferred variants include those that are correlated with any of the 1983, 52881, 2398, 45449, 50289, or 52872 biological activities described herein, e.g., regulating, sensing and/or transmitting an extracellular signal into a cell; interacting with (e.g., binding to) an extracellular signal or a cell surface receptor; mobilizing an intracellular molecule that participates in a signal transduction pathway; regulating polarization of the plasma membrane; controlling production or secretion of molecules; altering the structure of a cellular component; modulating cell proliferation, e.g., synthesis of DNA; and modulating cell migration, cell differentiation and cell survival.

Allelic variants of 1983, 52881, 2398, 45449, 50289, or 52872, e.g., human 1983, 52881, 2398, 45449, 50289, or 52872, include both functional and non-functional proteins. Functional allelic variants are naturally occurring amino acid sequence variants of the 1983, 52881, 2398, 45449, 50289, or 52872 protein within a population that maintain any of the 1983, 52881, 2398, 45449, 50289, or 52872biological activities described herein, e.g., regulating, sensing and/or transmitting an extracellular signal into a cell; interacting with (e.g., binding to) an extracellular signal or a cell surface receptor; mobilizing an intracellular molecule that participates in a signal transduction pathway; regulating polarization of the plasma membrane; controlling production or secretion of molecules; altering the structure of a cellular component; modulating cell proliferation, e.g., synthesis of DNA; and modulating cell migration, cell differentiation and cell survival.

Functional allelic variants will typically contain only conservative substitution of one or more amino acids of SEQ ID NO:2, SEQ ID NO:5, SEQ ID NO:8, SEQ ID NO:11, SEQ ID NO:14, or SEQ ID NO:17, or substitution, deletion or insertion of non-critical residues in non-critical regions of the protein. Non-functional allelic variants are naturally-occurring amino acid sequence variants of the 1983, 52881, 2398, 45449, 50289, or 52872, e.g., human 1983, 52881, 2398, 45449, 50289, or 52872, protein within a population that do not have any of the 1983, 52881,2398, 45449, 50289, or 52872 biological activities described herein, e.g., regulating, sensing and/or transmitting an extracellular signal into a cell; interacting with (e.g., binding to) an extracellular signal or a cell surface receptor; mobilizing an intracellular molecule that participates in a signal transduction pathway; regulating polarization of the plasma membrane; controlling production or secretion of molecules; altering the structure of a cellular component; modulating cell proliferation, e.g., synthesis of DNA; and modulating cell migration, cell differentiation and cell survival. Non-functional allelic variants will typically contain a non-conservative substitution, a deletion, or insertion, or premature truncation of the amino acid sequence of SEQ ID NO:2, SEQ ID NO:5, SEQ ID NO:8, SEQ ID NO:11, SEQ ID NO:14, or SEQ ID NO:17, or a substitution, insertion, or deletion in critical residues or critical regions of the protein.

Moreover, nucleic acid molecules encoding other 1983, 52881, 2398, 45449, 50289, or 52872 family members and, thus, which have a nucleotide sequence which differs from the 1983, 52881, 2398, 45449, 50289, or 52872 sequences of SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18 are intended to be within the scope of the invention.

### Antisense Nucleic Acid Molecules Ribozymes and Modified 1983, 52881, 2398, 45449, 50289, or 52872 Nucleic Acid Molecules

In another aspect, the invention features, an isolated nucleic acid molecule which is antisense to 1983, 52881, 2398, 45449, 50289, or 52872. An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire 1983, 52881, 2398, 45449, 50289, or 52872 coding strand, or to only a portion thereof (e.g., the coding region of human 1983, 52881, 2398, 45449, 50289, or 52872 corresponding to SEQ ID NO:3). In another embodiment, the antisense nucleic acid molecule is antisense to a "noncoding region" of the coding strand of a nucleotide sequence encoding 1983, 52881, 2398, 45449, 50289, or 52872 (e.g., the 5' and 3' untranslated regions).

An antisense nucleic acid can be designed such that it is complementary to the entire coding region of 1983, 52881, 2398, 45449, 50289, or 52872 mRNA, but more preferably is an oligonucleotide which is antisense to only a portion of the coding or noncoding region of 1983, 52881, 2398, 45449, 50289, or 52872 mRNA. For example, the antisense oligonucleotide can be complementary to the region surrounding the translation start site of 1983, 52881, 2398, 45449, 50289, or 52872 mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject (e.g., by direct injection at a tissue site), or generated *in situ* such that they hybridize with or bind to cellular mRNA and/or genomic DNA encoding a 1983, 52881, 2398, 45449, 50289, or 52872 protein to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

In still another embodiment, an antisense nucleic acid of the invention is a ribozyme. A ribozyme having specificity for a 1983, 52881, 2398, 45449, 50289, or 52872-encoding nucleic acid can include one or more sequences complementary to the nucleotide sequence of a 1983, 52881, 2398, 45449, 50289, or 52872 cDNA disclosed herein (i.e., SEQ ID NO:1, SEQ ID NO:3, SEQ ID NO:4, SEQ ID NO:6, SEQ ID NO:7, SEQ ID NO:9, SEQ ID NO:10, SEQ ID NO:12, SEQ ID NO:13, SEQ ID NO:15, SEQ ID NO:16, or SEQ ID NO:18), and a sequence having known catalytic sequence responsible for mRNA cleavage (see U.S. Pat. No. 5,093,246 or Haselhoff and Gerlach (1988) Nature 334:585-591). For example, a derivative of a *Tetrahymena* L-19 IVS RNA can be constructed in which the nucleotide sequence of the active site is complementary to the nucleotide sequence to be cleaved in a 1983, 52881, 2398, 45449, 50289, or 52872-encoding mRNA. See, e.g., Cech et al. U.S. Patent No. 4,987,071; and Cech et al. U.S. Patent No. 5,116,742. Alternatively, 1983, 52881, 2398, 45449, 50289, or 52872 mRNA can be used to select a catalytic RNA having a specific ribonuclease activity from a pool of RNA molecules. See, e.g., Bartel, D. and Szostak, J.W. (1993) Science 261:1411-1418.

1983, 52881, 2398, 45449, 50289, or 52872 gene expression can be inhibited by targeting nucleotide sequences complementary to the regulatory region of the 1983, 52881, 2398, 45449, 50289, or 52872 (e.g., the 1983, 52881, 2398, 45449, 50289, or 52872 promoter and/or enhancers) to form triple helical structures that prevent transcription of the 1983, 52881, 2398, 45449, 50289, or 52872 gene in target cells. See generally, Helene, C. (1991) Anticancer Drug Des. 6:569-84; Helene, C. i (1992) Ann. N.Y. Acad. Sci. 660:27-36; and Maher, L.J. (1992) Bioassays 14:807-15. The potential sequences that can be targeted for triple helix formation can be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

A 1983, 52881, 2398, 45449, 50289, or 52872 nucleic acid molecule can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al. (1996) Bioorganic & Medicinal Chemistry 4: 5-23). As used herein, the terms "peptide nucleic acid" or "PNA" refers to a nucleic acid mimic, e.g., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup B. *et al.* (1996) *supra*; Perry-O'Keefe et al. Proc. Natl. Acad. Sci. 93: 14670-675.

PNAs of 1983, 52881, 2398, 45449, 50289, or 52872 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 1983, 52881, 2398, 45449, 50289, or 52872 nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B. *et al.* (1996) *supra*)); or as probes or primers for DNA sequencing or hybridization (Hyrup B. *et al.* (1996) *supra*; Perry-O'Keefe *supra*).

In other embodiments, the oligonucleotide may include other appended groups such as peptides (e.g., for targeting host cell receptors *in vivo*), or agents facilitating transport across the cell membrane (see, e.g., Letsinger et al. (1989) Proc. Natl. Acad. Sci. USA 86:6553-6556; Lemaitre et al. (1987) Proc. Natl. Acad. Sci. USA 84:648-652; PCT Publication No. W088/09810) or the blood-brain barrier (see, e.g., PCT Publication No. W089/10134). In addition, oligonucleotides can be modified with hybridization-triggered cleavage agents (see, e.g., Krol et al, (1988) Bio-Techniques 6:958-976) or intercalating agents. (see, e.g., Zon (1988) Pharm. Res. 5:539-549). To this end, the oligonucleotide may be conjugated to another molecule, (e.g., a peptide, hybridization triggered crosslinking agent, transport agent, or hybridization-triggered cleavage agent).

The invention also includes molecular beacon oligonucleotide primer and probe molecules having at least one region which is complementary to a 1983, 52881, 2398, 45449, 50289, or 52872 nucleic acid of the invention, two complementary regions one having a fluorophore and one a quencher such that the molecular beacon is useful for quantitating the presence of the 1983, 52881, 2398, 45449, 50289, or 52872 nucleic acid of the invention in a sample. Molecular beacon nucleic acids are described, for example, in Lizardi et al., U.S. Patent No. 5,854,033; Nazarenko et al., U.S. Patent No. 5,866,336, and Livak et al., U.S. Patent 5,876,930.

### Isolated 1983, 52881, 2398, 45449, 50289, or 52872 Polypeptides

In another aspect, the invention features, an isolated 1983, 52881, 2398, 45449, 50289, or 52872 protein, or fragment, e.g., a biologically active portion, for use as immunogens or antigens to raise or test (or more generally to bind) anti-1983, 52881, 2398, 45449, 50289, or 52872 antibodies. 1983, 52881, 2398, 45449, 50289, or 52872 protein can be isolated from cells or tissue sources using standard protein purification techniques. 1983, 52881, 2398, 45449, 50289, or 52872 protein or fragments thereof can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, e.g., cultured cells, which result in substantially the same post-trauslational modifications present when expressed the polypeptide is expressed in a native cell, or in systems which result in the alteration or omission of post-translational modifications, e.g., glycosylation or cleavage, present when expressed in a native cell.

In a preferred embodiment, a 1983, 52881, 2398, 45449, 50289, or 52872 polypeptide has one or more of the following characteristics:
(i) it has the ability to regulate, sense and/or transmit an extracellular signal into a cell;
(ii) it has the ability to interact with (e.g., bind to) an extracellular signal, e.g., a neuropeptide, or a cell surface receptor;
(iii) it has the ability to modulate a pain response;
(iv) it has the ability to modulate angiogenesis;
(v) it has the ability to modulate the control of vascular tone;
(vi) it has the ability to mobilize an intracellular molecule that participates in a signal transduction pathway (e.g., adenylate cyclase or phosphatidylinositol 4,5-bisphosphate (PIP₂), inositol 1,4,5-triphosphate (IP₃));
(vii) it has the ability to modulate proliferation, migration, differentiation and/or survival of a cell;
(viii) it has the ability to modulate function, survival, morphology, proliferation and/or differentiation of cells of tissues in which 1983, 52881, 2398, 45449, 50289, or 52872 molecules are expressed;
(ix) it has a molecular weight, e.g., a deduced molecular weight, preferably ignoring any contribution of post translational modifications, amino acid composition or other physical characteristic of a 1983, 52881, 2398, 45449, 50289, or 52872 polypeptide, e.g., a polypeptide of SEQ ID NO:2, SEQ ID NO:5, SEQ ID NO:8, SEQ ID NO:11, SEQ ID NO:14, or SEQ ID NO:17;
(x) it has an overall sequence similarity (identity) of at least 60%, more preferably at least 70, 80, 90, or 95%, with a polypeptide of SEQ ID NO:2, SEQ ID NO:5, SEQ ID NO:8, SEQ ID NO:11, SEQ ID NO:14, or SEQ ID NO:17;
(xi) it has at least one transmembrane domains which is preferably about 70%, 80%, 90%, 95% or higher, identical with amino acid residues 388-407, 420-436, 455-479, 488-508, 525-549, 574-591, or 598-622 of SEQ ID NO:2; amino acid residues 11-34, 44-67, 85-106, 127-149, 172-196, or 245-269 of SEQ ID NO:5; amino acid residues 42-66, 78-99, 114-135, 154-176,202-224,241-259, or 291-310 of SEQ ID NO:8; amino acid residues 12-33, 68-90, or 123-147 of SEQ ID NO:11; amino acid residues 567-590, 600-623, 641-659, 679-702, 726-750, 762-782, or 799-810 of SEQ ID NO:14; or amino acid residues 43-67, 76-110, 117-136, 158-180, 204-228, 264-285, or 310-326 of SEQ ID NO:17;
(xii) it has a seven transmembrane receptor domain which is preferably about 70%, 80%, 90% or 95% or higher, identical with amino acids 379 to 626 of SEQ ID NO:2, amino acids 80 to 154 of SEQ ID NO:5, amino acids 58 to 303 of SEQ ID NO:8, amino acids 1 to 176 of SEQ ID NO:11, or amino acids 59 to 323 of SEQ ID NO:17;
(xiii) it has an ANF receptor ligand binding domain which is preferably about 70%, 80%, 90% or 95% or higher, identical with amino acids 61 to 470 of SEQ ID NO:14;
(xiv) it has an EGF-like domain which is preferably about 70%, 80%, 90% or 95% or higher, identical with amino acids 17 to 54 of SEQ ID NO:2;
(xv) it has a latrophilin/CL-1-like GPS domain which is preferably about 70%, 80%, 90% or 95% or higher, identical with amino acids 321 to 373 of SEQ ID NO:2; or
(xvi) it has at least 10, preferably 70%, 80%, 90%, 95% and most preferably 100% of the cysteines found in the amino acid sequence of the native protein.

In a preferred embodiment the 1983, 52881, 2398, 45449, 50289, or 52872 protein, or fragment thereof, differs from the corresponding sequence in SEQ ID NO:2, SEQ ID NO:5, SEQ ID NO:8, SEQ ID NO:11, SEQ ID NO:14, or SEQ ID NO:17. In one embodiment it differs by at least one but by less than 15, 10 or 5 amino acid residues. In another it differs from the corresponding sequence in SEQ ID NO:2, SEQ ID NO:5, SEQ ID NO: 8, SEQ ID NO:11, SEQ ID NO:14, or SEQ ID NO:17 by at least one residue but less than 20%, 15%, 10% or 5% of the residues in it differ from the corresponding sequence in SEQ ID NO:2, SEQ ID NO:5, SEQ ID NO:8, SEQ ID NO:11, SEQ ID NO:14, or SEQ ID NO:17. (If this comparison requires alignment the sequences should be aligned for maximum homology. "Looped" out sequences from deletions or insertions, or mismatches, are considered differences.) The differences are, preferably, differences or changes at a non essential residue or a conservative substitution. In a preferred embodiment the differences are not in residues amino acids 379 to 626 of SEQ ID NO:2, amino acids 80 to 154 of SEQ ID NO:5, amino acids 58 to 303 of SEQ ID NO:8, amino acids 1 to 176 of SEQ ID NO:11, amino acids 59 to 323 of SEQ ID NO:17, or amino acids 61 to 470 of SEQ ID NO:14. Other embodiments include a protein that contain one or more changes in amino acid sequence, e.g., a change in an amino acid residue which is not essential for activity. Such 1983, 52881, 2398, 45449, 50289, or 52872 proteins differ in amino acid sequence from SEQ ID NO:2, SEQ ID NO:5, SEQ ID NO:8, SEQ ID NO:11, SEQ ID NO:14, or SEQ ID NO:17, yet retain biological activity.

In one embodiment, the protein includes an amino acid sequence at least about 60%, 65%, 70%, 75%, 80%, 85%, 90%, 95%, 98%, 99%, or more homologous to SEQ ID NO:2, SEQ ID NO:5, SEQ ID NO:8, SEQ ID NO:11, SEQ ID NO:14, or SEQ ID NO:17.

A 1983, 52881, 2398, 45449, 50289, or 52872 protein or fragment is provided which varies from the sequence of SEQ ID NO:2 in regions defined by amino acids 379 to 626 of SEQ ID NO:2, amino acids 80 to 154 of SEQ ID NO:5, amino acids 58 to 303 of SEQ ID NO:8, amino acids 1 to 176 of SEQ ID NO:11, amino acids 59 to 323 of SEQ ID NO:17, or amino acids 61 to 470 of SEQ ID NO:14 by at least one but by less than 15, 10 or 5 amino acid residues in the protein or fragment but which does not differ from SEQ ID NO:2, SEQ ID NO:5, SEQ ID NO:8, SEQ ID NO:11, SEQ ID NO:14, or SEQ ID NO:17 in regions outside of those listed above. (If this comparison requires alignment the sequences should be aligned for maximum homology. "Looped" out sequences from deletions or insertions, or mismatches, are considered differences.) In some embodiments the difference is at a non essential residue or is a conservative substitution, while in others the difference is at an essential residue or is a non conservative substitution.

In one embodiment, a biologically active portion of a 1983, 52881, 2398, 45449, 50289, or 52872 protein includes a 1983, 52881, 2398, 45449, 50289, or 52872 transmembrane domain. Moreover, other biologically active portions, in which other regions of the protein are deleted, can be prepared by recombinant techniques and evaluated for one or more of the functional activities of a native 1983, 52881, 2398, 45449, 50289, or 52872 protein.

In a preferred embodiment, the 1983, 52881, 2398, 45449, 50289, or 52872 protein has an amino acid sequence shown in SEQ ID NO:2, SEQ ID NO:5, SEQ ID NO:8, SEQ ID NO:11, SEQ ID NO:14, or SEQ ID NO:17. In other embodiments, the 1983, 52881, 2398, 45449, 50289, or 52872 protein is substantially identical to SEQ ID NO:2, SEQ ID NO:5, SEQ ID NO:8, SEQ ID NO:11, SEQ ID NO:14, or SEQ ID NO:17. In yet another embodiment, the 1983, 52881, 2398, 45449, 50289, or 52872 protein is substantially identical to SEQ ID NO:2, SEQ ID NO:5, SEQ ID NO:8, SEQ ID N0:11, SEQ ID NO:14, or SEQ ID NO:17 and retains the functional activity of the protein of SEQ ID NO:2, SEQ ID NO:5, SEQ ID NO:8, SEQ ID NO:11, SEQ, ID NO:14, or SEQ ID NO:17, as described in detail in the subsections above.

### 1983, 52881, 2398, 45449, 50289 or 52872 Chimeric or Fusion Proteins

In another aspect, the invention provides 1983, 52881, 2398, 45449, 50289, or 52872 chimeric or fusion proteins. As used herein, a 1983, 52881, 2398, 45449, 50289, or 52872 "chimeric protein" or "fusion protein" includes a 1983, 52881, 2398, 45449, 50289, or 52872 polypeptide linked to a non-1983, 52881, 2398, 45449, 50289, or 52872 polypeptide. A "non-1983, 52881, 2398, 45449, 50289, or 52872 polypeptide" refers to a polypeptide having an amino acid sequence corresponding to a protein which is not substantially homologous to the 1983, 52881, 2398, 45449, 50289, or 52872 protein, e.g., a protein which is different from the 1983, 52881, 2398, 45449, 50289, or 52872 protein and which is derived from the same or a different organism. The 1983, 52881, 2398, 45449, 50289, or 52872 polypeptide of the fusion protein can correspond to all or a portion e.g., a fragment described herein of a 1983, 52881, 2398, 45449, 50289, or 52872 amino acid sequence. In a preferred embodiment, a 1983, 52881, 2398, 45449, 50289, or 52872 fusion protein includes at least one (or two) biologically active portion of a 1983, 52881, 2398, 45449, 50289, or 52872 protein. The non-1983, 52881, 2398, 45449, 50289, or 52872 polypeptide can be fused to the N-terminus or C-terminus of the 1983, 52881, 2398, 45449, 50289, or 52872 polypeptide.

The fusion protein can include a moiety which has a high affinity for a ligand. For example, the fusion protein can be a GST-1983, 52881, 2398, 45449, 50289, or 52872 fusion protein in which the 1983, 52881, 2398, 45449, 50289, or 52872 sequences are fused to the C-terminus of the GST sequences. Such fusion proteins can facilitate the purification of recombinant 1983, 52881, 2398, 45449, 50289, or 52872. Alternatively, the fusion protein can be a 1983, 52881, 2398, 45449, 50289, or 52872 protein containing a heterologous signal sequence at its N-terminus. In certain host cells (e.g., mammalian host cells), expression and/or secretion of 1983, 52881, 2398, 45449, 50289, or 52872 can be increased through use of a heterologous signal sequence.

Fusion proteins can include all or a part of a serum protein, e.g., an IgG constant region, or human serum albumin.

The 1983, 52881, 2398, 45449, 50289, or 52872 fusion proteins of the invention can be incorporated into pharmaceutical compositions and administered to a subject *in vivo*. The 1983, 52881, 2398, 45449, 50289, or 52872 fusion proteins can be used to affect the bioavailability of a 1983, 52881, 2398, 45449, 50289, or 52872 substrate. 1983, 52881, 2398, 45449, 50289, or 52872 fusion proteins may be useful therapeutically for the treatment of disorders caused by, for example, (i) aberrant modification or mutation of a gene encoding a 1983, 52881, 2398, 45449, 50289, or 52872 protein; (ii) mis-regulation of the 1983, 52881, 2398, 45449, 50289, or 52872 gene; and (iii) aberrant post-translational modification of a 1983, 52881, 2398, 45449, 50289, or 52872 protein.

Moreover, the 1983, 52881, 2398, 45449, 50289, or 52872-fusion proteins of the invention can be used as immunogens to produce anti-1983, 52881, 2398, 45449, 50289, or 52872 antibodies in a subject, to purify 1983, 52881, 2398, 45449, 50289, or 52872 ligands and in screening assays to identify molecules which inhibit the interaction of 1983, 52881, 2398, 45449, 50289, or 52872 with a 1983, 52881, 2398, 45449, 50289, or 52872 substrate.

Expression vectors are commercially available that already encode a fusion moiety (e.g., a GST polypeptide). A 1983, 52881, 2398, 45449, 50289, or 52872-encoding nucleic acid can be cloned into such an expression vector such that the fusion moiety is linked in-frame to the 1983, 52881, 2398, 45449, 50289, or 52872 protein.

### Variants of 1983, 52881, 2398, 45449, 50289, or 52872 Proteins

In another aspect, the invention also features a variant of a 1983, 52881, 2398, 45449, 50289, or 52872 polypeptide, e.g., which functions as an agonist (mimetics) or as an antagonist. Variants of the 1983, 52881, 2398, 45449, 50289, or 52872 proteins can be generated by mutagenesis, e.g., discrete point mutation, the insertion or deletion of sequences or the truncation of a 1983, 52881, 2398, 45449, 50289, or 52872 protein. An agonist of the 1983, 52881, 2398, 45449, 50289, or 52872 proteins can retain substantially the same, or a subset, of the biological activities of the naturally occurring form of a 1983, 52881, 2398, 45449, 50289, or 52872 protein. An antagonist of a 1983, 52881, 2398, 45449, 50289, or 52872 protein can inhibit one or more of the activities of the naturally occurring form of the 1983, 52881, 2398, 45449, 50289, or 52872 protein by, for example, competitively modulating a 1983, 52881, 2398, 45449, 50289, or 52872-mediated activity of a 1983, 52881, 2398, 45449, 50289, or 52872 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 1983, 52881, 2398, 45449, 50289, or 52872 protein.

Variants of a 1983, 52881, 2398, 45449, 50289, or 52872 protein can be identified by screening combinatorial libraries of mutants, e.g., truncation mutants, of a 1983, 52881, 2398, 45449, 50289, or 52872 protein for agonist or antagonist activity.

Libraries of fragments e.g., N terminal, C terminal, or internal fragments, of a 1983, 52881, 2398, 45449, 50289, or 52872 protein coding sequence can be used to generate a variegated population of fragments for screening and subsequent selection of variants of a 1983, 52881, 2398, 45449, 50289, or 52872 protein. Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of 1983, 52881, 2398, 45449, 50289, or 52872 proteins. Recursive ensemble mutagenesis (REM), a new technique which enhances the frequency of functional mutants in the libraries, can be used in combination with the screening assays to identify 1983, 52881, 2398, 45449, 50289, or 52872 variants (Arkin and Yourvan (1992) Proc. Natl. Acad Sci. USA 89:7811-7815; Delgrave et al. (1993) Protein Engineering 6:327-331).

Cell based assays can be exploited to analyze a variegated 1983, 52881, 2398, 45449, 50289, or 52872 library. For example, a library of expression vectors can be transfected into a cell line, e.g., a cell line, which ordinarily responds to 1983, 52881, 2398, 45449, 50289, or 52872 in a substrate-dependent manner. The transfected cells are then contacted with 1983, 52881, 2398, 45449, 50289, or 52872 and the effect of the expression of the mutant on signaling by the 1983, 52881, 2398, 45449, 50289, or 52872 substrate can be detected, e.g., by measuring changes in cell growth, differentiation, and/or enzymatic activity. Plasmid DNA can then be recovered from the cells which score for inhibition, or alternatively, potentiation of signaling by the 1983, 52881, 2398, 45449, 50289, or 52872 substrate, and the individual clones further characterized.

In another aspect, the invention features a method of making a 1983, 52881, 2398, 45449, 50289, or 52872 polypeptide, e.g., a peptide having a non-wild type activity, e.g., an antagonist, agonist, or super agonist of a naturally occurring 1983, 52881, 2398, 45449, 50289, or 52872 polypeptide, e.g., a naturally occurring 1983, 52881, 2398, 45449, 50289, or 52872 polypeptide. The method includes: altering the sequence of a 1983, 52881, 2398, 45449, 50289, or 52872 polypeptide, e.g., altering the sequence , e.g., by substitution or deletion of one or more residues of a non-conserved region, a domain or residue disclosed herein, and testing the altered polypeptide for the desired activity.

In another aspect, the invention features a method of making a fragment or analog of a 1983, 52881, 2398, 45449, 50289, or 52872 polypeptide a biological activity of a naturally occurring 1983, 52881, 2398, 45449, 50289, or 52872 polypeptide. The method includes: altering the sequence, e.g., by substitution or deletion of one or more residues, of a 1983, 52881, 2398, 45449, 50289, or 52872 polypeptide, e.g., altering the sequence of a non-conserved region, or a domain or residue described herein, and testing the altered polypeptide for the desired activity.

### Anti-1983, 52881, 2398, 45449, 50289, or 52872 Antibodies

In another aspect, the invention provides an anti-1983, 52881, 2398, 45449, 50289, or 52872 antibody. The term "antibody" as used herein refers to an immunoglobulin molecule or immunologically active portion thereof, i.e., an antigen-binding portion. Examples of immunologically active portions of immunoglobulin molecules include F(ab) and F(ab')₂ fragments which can be generated by treating the antibody with an enzyme such as pepsin.

The antibody can be a polyclonal, monoclonal, recombinant, e.g., a chimeric or humanized, fully human, non-human, e.g., murine, or single chain antibody. In a preferred embodiment it has effector function and can fix complement. The antibody can be coupled to a toxin or imaging agent.

A full-length 1983, 52881, 2398, 45449, 50289, or 52872 protein or, antigenic peptide fragment of 1983, 52881,2398, 45449, 50289, or 52872 can be used as an immunogen or can be used to identify anti-1983, 52881, 2398, 45449, 50289, or 52872 antibodies made with other immunogens, e.g., cells, membrane preparations, and the like. The antigenic peptide of 1983, 52881, 2398, 45449, 50289, or 52872 should include at least 8 amino acid residues of the amino acid sequence shown in SEQ ID NO:2, SEQ ID NO:5, SEQ ID NO:8, SEQ ID NO:11, SEQ ID NO:14, or SEQ ID NO:17 and encompasses an epitope of 1983, 52881, 2398, 45449, 50289, or 52872. Preferably, the antigenic peptide includes at least 10 amino acid residues, more preferably at least 15 amino acid residues, even more preferably at least 20 amino acid residues, and most preferably at least 30 amino acid residues.

Fragments of 1983 which include residues from about 15-35, from about 195-205, or from about 275-285 of SEQ ID NO:2 can be used to make, e.g., used as immunogens or used to characterize the specificity of an antibody, antibodies against hydrophilic regions of the 1983 protein. Similarly, fragments of 1983 which include residues from about 210-220, from about 290-300, or from about 365-375 of SEQ ID NO:2 can be used to make an antibody against a hydrophobic region of the 1983 protein. Fragments of 1983 which include residues from about amino acid residues 388-407, 420-436, 455-479, 488-508, 525-549, 574-591, or 598-622 of SEQ ID NO:2 can be used to make an antibody against a transmembrane domain of the 1983 protein. A fragment of 1983 which include residues from about 379 to 626 of SEQ ID NO:2 can be used to make an antibody against the seven transmembrane domain of the 1983 protein.

Fragments of 52881 which include residues from about 225-240, from about 475-490, or from about 540-555 of SEQ ID NO:5 can be used to make, e.g., used as immunogens or used to characterize the specificity of an antibody, antibodies against hydrophilic regions of the 52881 protein. Similarly, fragments of 52881 which include residues from about 280-300, from about 420-430, or from about 495-505 of SEQ ID NO:5 can be used to make an antibody against a hydrophobic region of the 52881 protein. Fragments of 52881 which include residues from about 11-34, 44-67, 85-106, 127-149, 172-196, or 245-269 of SEQ ID NO:5 can be used to make an antibody against a transmembrane domain of the 52881 protein. A fragment of 52881 which include residues from about 80 to 154 of SEQ ID NO:5 can be used to make an antibody against the seven transmembrane domain of the 52881 protein.

Fragments of 2398 which include residues from about 1-25, from about 70-80, or from about 320-330 of SEQ ID NO:8 can be used to make, e.g., used as immunogens or used to characterize the specificity of an antibody, antibodies against hydrophilic regions of the 2398 protein. Similarly, fragments of 2398 which include residues from about 265-275 or from about 285-295 of SEQ ID NO:8 can be used to make an antibody against a hydrophobic region of the 2398 protein. Fragments of 2398 which include residues from about amino acid residues 42-66, 78-99, 114-135, 154-176, 202-224, 241-259, or 291-310 of SEQ ID NO:8 can be used to make an antibody against a transmembrane domain of the 2398 protein. A fragment of 2398 which include residues from about 58 to 303 of SEQ ID NO:8 can be used to make an antibody against the seven transmembrane domain of the 2398 protein.

Fragments of 45449 which include residues from about 100-110 or from about 195-205 of SEQ ID NO:11 can be used to make, e.g., used as immunogens or used to characterize the specificity of an antibody, antibodies against hydrophilic regions of the 45449 protein. Similarly, fragments of 45449 which include residues from about 160-170 of SEQ ID NO:11 can be used to make an antibody against a hydrophobic region of the 45449 protein. Fragments of 45449 which include residues from about amino acid residues 12-33, 68-90, and 123-147 of SEQ ID NO:1 can be used to make an antibody against a transmembrane domain of the 45449 protein, A fragment of 45449 which include residues from about 1 to 176 of SEQ ID NO:11 can be used to make an antibody against the seven transmembrane domain of the 45449 protein.

Fragments of 50289 which include residues from about 50-60, from about 480-510, or from about 545-560 of SEQ ID NO:14 can be used to make, e.g., used as immunogens or used to characterize the specificity of an antibody, antibodies against hydrophilic regions of the 50289 protein. Similarly, fragments of 50289 which include residues from about 70-80, from about 150-165, or from about 220-240 of SEQ ID NO:14 can be used to make an antibody against a hydrophobic region of the 50289 protein. Fragments of 50289 which include from about amino acid residues 567-590, 600-623, 641-659, 679-702, 726-750, 762-782, or 799-810 of SEQ ID NO:14 can be used to make an antibody against a transmembrane domain of the 50289 protein. A fragment of 50289 which include residues from about 61 to 470 of SEQ ID NO:14 can be used to make an antibody against the ANF receptor ligand binding domain of the 50289 protein.

Fragments of 52872 which include residues about 295-300, about 345-360, or about 370-380 of SEQ ID NO:17 can be used to make, e.g., used as immunogens or used to characterize the specificity of an antibody, antibodies against hydrophilic regions of the 52872 protein. Similarly, fragments of 52872 which include residues about 45-65, about 165-180, or about 210-225 of SEQ ID NO:17 can be used to make an antibody against a hydrophobic region of the 52872 protein. Fragments of 52872 which include residues about 1-42, about 111-116, about 181-203, or about 286-309 of SEQ ID NO:17 can be used to make an antibody against an extracellular region of the 52872 protein. Fragments of 52872 which include residues about 68-75, about 137-157, about 229-263, or about 327-398 of SEQ ID NO:17 can be used to make an antibody against an intracellular region of the 52872 protein. Fragments of 52872 which include residues about 43-67, about 76-110, about 117-136, about 158-180, about 204-228, about 264-285, or about 310-326 of SEQ ID NO:17 can be used to make an antibody against a transmembrane segment of the 52872 protein. A fragment of 52872 which include residues from about 59 to 323 of SEQ ID NO:17 can be used to make an antibody against the seven transmembrane domain of the 52872 protein.

Antibodies reactive with, or specific for, any of these regions, or other regions or domains described herein are provided.

Preferred epitopes encompassed by the antigenic peptide are regions of 1983, 52881, 2398, 45449, 50289, or 52872 are located on the surface of the protein, e.g., hydrophilic regions, as well as regions with high antigenicity. For example, an Emini surface probability analysis of the human 1983, 52881, 2398, 45449, 50289, or 52872 protein sequence can be used to indicate the regions that have a particularly high probability of being localized to the surface of the 1983, 52881, 2398, 45449, 50289, or 52872 protein and are thus likely to constitute surface residues useful for targeting antibody production.

In a preferred embodiment the antibody can bind to the extracellular portion of the 1983, 52881, 2398, 45449, 50289, or 52872 protein, e.g., it can bind to a whole cell which expresses the 1983, 52881, 2398, 45449, 50289, or 52872 protein. In another embodiment, the antibody binds an intracellular portion of the 1983, 52881, 2398, 45449, 50289, or 52872 protein.

In a preferred embodiment the antibody binds an epitope on any domain or region on 1983, 52881, 2398, 45449, 50289, or 52872 proteins described herein.

Antibodies which bind only native 1983, 52881, 2398, 45449, 50289, or 52872 protein, only denatured or otherwise non-native 1983, 52881, 2398, 45449, 50289, or 52872 protein, or which bind both, are with in the invention. Antibodies with linear or conformational epitopes are within the invention. Conformational epitopes can sometimes be identified by identifying antibodies which bind to native but not denatured 1983, 52881, 2398, 45449, 50289, or 52872 protein.

Chimeric, humanized, but most preferably, completely human antibodies are desirable for applications which include repeated administration, e.g., therapeutic treatment (and some diagnostic applications) of human patients.

The anti-1983, 52881, 2398, 45449, 50289, or 52872 antibody can be a single chain antibody. A single-chain antibody (scFV) may be engineered (see, for example, Colcher, D. et al. (1999) Ann N Y Acad Sci 880:263-80; and Reiter, Y. (1996) Clin Cancer Res 2:245-52). The single chain antibody can be dimerized or multimerized to generate multivalent antibodies having specificities for different epitopes of the same target 1983, 52881, 2398, 45449, 50289, or 52872 protein.

In a preferred embodiment, the antibody has reduced or no ability to bind an Fc receptor. For example, it is a isotype or subtype, fragment or other mutant, which does not support binding to an Fc receptor, e.g., it has a mutagenized or deleted Fc receptor bind ing region.

An anti-1983, 52881, 2398, 45449, 50289, or 52872 antibody (e.g., monoclonal antibody) can be used to isolate 1983, 52881, 2398, 45449, 50289, or 52872 by standard techniques, such as affinity chromatography or immunoprecipitation. Moreover, an anti-1983, 52881, 2398, 45449, 50289, or 52872 antibody can be used to detect 1983, 52881, 23 98, 45449, 50289, or 52872 protein (e.g., in a cellular lysate or cell supernatant) in order to evaluate the abundance and pattern of expression of the protein. Anti-1983, 52881, 2398, 45449, 50289, or 52872 antibodies can be used diagnostically to monitor protein levels in tissue as part of a clinical testing procedure, e.g., to determine the efficacy of a given treatment regimen. Detection can be facilitated by coupling (i.e., physically linking) the antibody to a detectable substance (i.e., antibody labelling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include 125_{I}, 131_{I}, 35_{S} or 3_{H}.

The invention also includes a nucleic acid which encodes an anti-1983, 52881, 2398, 45449, 50289, or 52872 antibody, e.g., an anti-1983, 52881, 2398, 45449, 50289, or 52872 antibody described herein. Also included are vectors which include the nucleic acid and cells transformed with the nucleic acid, particularly cells which are useful for producing an antibody, e.g., mammalian cells, e.g. CHO or lymphatic cells.

The invention also includes cell lines, e.g., hybridomas, which make an anti-1983, 52881, 2398, 45449, 50289, or 52872 antibody, e.g., and antibody described herein, and method of using said cells to make a 1983, 52881, 2398, 45449, 50289, or 52872 antibody.

### Recombinant Expression Vectors Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include a 1983, 52881, 2398, 45449, 50289, or 52872 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 1983, 52881, 2398, 45449, 50289, or 52872 proteins, mutant forms of 1983, 52881, 2398, 45449, 50289, or 52872 proteins, fusion proteins, and the like).

The recombinant expression vectors of the invention can be designed for expression of 1983, 52881, 2398, 45449, 50289, or 52872 proteins in prokaryotic or eukaryotic cells. For example, polypeptides of the invention can be expressed in *E. coli*, insect cells (e.g., using baculovirus expression vectors), yeast cells or mammalian cells. Suitable host cells are discussed further in Goeddel, (1990) Gene Expression Technology: Methods in Enzymology 185, Academic Press, San Diego, CA. Alternatively, the recombinant expression vector can be transcribed and translated *in vitro,* for example using T7 promoter regulatory sequences and T7 polymerase.

Expression of proteins in prokaryotes is most often carried out in *E*. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 1983, 52881, 2398, 45449, 50289, or 52872 activity assays, (e.g., direct assays or competitive assays described in detail below), or to generate antibodies specific for 1983, 52881, 2398, 45449, 50289, or 52872 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (e.g., six weeks).

To maximize recombinant protein expression in *E. coli* is to express the protein in a host bacteria with an impaired capacity to proteolytically cleave the recombinant protein (Gottesman, S., (1990) Gene Expression Technology: Methods in Enzymology 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada et al., (1992) Nucleic Acids Res. 20:2111-2118). Such alteration of nucleic acid sequences of the invention can be carried out by standard DNA synthesis techniques.

The 1983, 52881, 2398, 45449, 50289, or 52872 expression vector can be a yeast expression vector, a vector for expression in insect cells, e.g., a baculovirus expression vector or a vector suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

In another embodiment, the promoter is an inducible promoter, e.g., a promoter regulated by a steroid hormone, by a polypeptide hormone (e.g., by means of a signal transduction pathway), or by a heterologous polypeptide (e.g., the tetracycline-inducible systems, "Tet-On" and "Tet-Off"; see, e.g., Clontech Inc., CA, Gossen and Bujard (1992) Proc. Natl. Acad. Sci. USA 89:5547, and Paillard (1989) Human Gene Therapy 9:983).

In another embodiment, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) Proc. Natl. Acad. Sci. USA 86:5473-5477), pancreas-specific promoters (Edlund et al. (1985) Science 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss (1990) Science 249:374-379) and the α-fetoprotein promoter (Campes and Tilghman (1989) Genes Dev. 3:537-546).

The invention further provides a recombinant expression vector comprising a DNA molecule of the invention cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus. For a discussion of the regulation of gene expression using antisense genes see Weintraub, H. et al., (1986) Antisense RNA as a molecular tool for genetic analysis, Reviews - Trends in Genetics 1:1.

Another aspect the invention provides a host cell which includes a nucleic acid molecule described herein, e.g., a 1983, 52881, 2398, 45449, 50289, or 52872 nucleic acid molecule within a recombinant expression vector or a 1983, 52881, 2398, 45449, 50289, or 52872 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a 1983, 52881, 2398, 45449, 50289, or 52872 protein can be expressed in bacterial cells such as *E. coli*, insect cells, yeast or mammalian cells (such as Chinese hamster ovary cells (CHO) or COS cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran-mediated transfection, lipofection, or electroporation.

A host cell of the invention can be used to produce (i.e., express) a 1983, 52881, 2398, 45449, 50289, or 52872 protein. Accordingly, the invention further provides methods for producing a 1983, 52881,2398, 45449, 50289, or 52872 protein using the host cells of the invention. In one embodiment, the method includes culturing the host cell of the invention (into which a recombinant expression vector encoding a 1983, 52881, 2398, 45449, 50289, or 52872 protein has been introduced) in a suitable medium such that a 1983, 52881, 2398, 45449, 50289, or 52872 protein is produced. In another embodiment, the method further includes isolating a 1983, 52881, 2398, 45449, 50289, or 52872 protein from the medium or the host cell.

In another aspect, the invention features, a cell or purified preparation of cells which include a 1983, 52881, 2398, 45449, 50289, or 52872 transgene, or which otherwise misexpress 1983, 52881, 2398, 45449, 50289, or 52872. The cell preparation can consist of human or non human cells, e.g., rodent cells, e.g., mouse or rat cells, rabbit cells, or pig cells. In preferred embodiments, the cell or cells include a 1983, 52881, 2398, 45449, 50289, or 52872 transgene, e.g., a heterologous form of a 1983, 52881, 2398, 45449, 50289, or 52872, e.g., a gene derived from humans (in the case of a non-human cell). The 1983, 52881, 2398, 45449, 50289, or 52872 transgene can be misexpressed, e.g., overexpressed or underexpressed. In other preferred embodiments, the cell or cells include a gene which misexpress an endogenous 1983, 52881, 2398, 45449, 50289, or 52872, e.g., a gene the expression of which is disrupted, e.g., a knockout. Such cells can serve as a model for studying disorders which are related to mutated or mis-expressed 1983, 52881, 2398, 45449, 50289, or 52872 alleles or for use in drug screening.

In another aspect, the invention features, a human cell, e.g., a hematopoietic stem cell, transformed with nucleic acid which encodes a subject 1983, 52881, 2398, 45449, 50289, or 52872 polypeptide.

Also provided are cells, preferably human cells, e.g., human hematopoietic or fibroblast cells, in which an endogenous 1983, 52881, 2398, 45449, 50289, or 52872 is under the control of a regulatory sequence that does not normally control the expression of the endogenous 1983, 52881, 2398, 45449, 50289, or 52872 gene. The expression characteristics of an endogenous gene within a cell, e.g., a cell line or microorganism, can be modified by inserting a heterologous DNA regulatory element into the genome of the cell such that the inserted regulatory element is operably linked to the endogenous 1983, 52881, 2398, 45449, 50289, or 52872 gene. For example, an endogenous 1983, 52881, 2398, 45449, 50289, or 52872 gene which is "transcriptionally silent," e.g., not normally expressed, or expressed only at very low levels, may be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published in May 16, 1991.

In a preferred embodiment, recombinant cells described herein can be used for replacement therapy in a subject. For example, a nucleic acid encoding a 1983, 52881, 2398, 45449, 50289, or 52872 polypeptide operably linked to an inducible promoter (e.g., a steroid hormone receptor-regulated promoter) is introduced into a human or nonhuman, e.g., mammalian, e.g., porcine recombinant cell. The cell is cultivated and encapsulated in a biocompatible material, such as poly-lysine alginate, and subsequently implanted into the subject. See, e.g., Lanza (1996) Nat. Biotechnol. 14:1107; Joki et al. (2001) Nat. Biotechnol. 19:35; and U.S. Patent No. 5,876,742. Production of 1983, 52881, 2398, 45449, 50289, or 52872 polypeptide can be regulated in the subject by administering an agent (e.g., a steroid hormone) to the subject. In another preferred embodiment, the implanted recombinant cells express and secrete an antibody specific for a 1983, 52881, 2398, 45449, 50289, or 52872 polypeptide. The antibody can be any antibody or any antibody derivative described herein.

### Transgenic Animals

The invention provides non-human transgenic animals. Such animals are useful for studying the function and/or activity of a 1983, 52881, 2398, 45449, 50289, or 52872 protein and for identifying and/or evaluating modulators of 1983, 52881, 2398, 45449, 50289, or 52872 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, aulphibians, and the like. A transgene is exogenous DNA or a rearrangement, e.g., a deletion of endogenous chromosomal DNA, which preferably is integrated into or occurs in the genome of the cells of a transgenic animal. A transgene can direct the expression of an encoded gene product in one or more cell types or tissues of the transgenic animal, other transgenes, e.g., a knockout, reduce expression. Thus, a transgenic animal can be one in which an endogenous 1983, 52881, 2398, 45449, 50289, or 52872 gene has been altered by, e.g., by homologous recombination between the endogenous gene and an exogenous DNA molecule introduced into a cell of the animal, e.g., an embryonic cell of the animal, prior to development of the animal.

Intronic sequences and polyadenylation signals can also be included in the transgene to increase the efficiency of expression of the transgene. A tissue-specific regulatory sequence(s) can be operably linked to a transgene of the invention to direct expression of a 1983, 52881, 2398, 45449, 50289, or 52872 protein to particular cells. A transgenic founder animal can be identified based upon the presence of a 1983, 52881, 2398, 45449, 50289, or 52872 transgene in its genome and/or expression of 1983, 52881, 2398, 45449, 50289, or 52872 mRNA in tissues or cells of the animals. A transgenic founder animal can then be used to breed additional animals carrying the transgene. Moreover, transgenic animals carrying a transgene encoding a 1983, 52881, 2398, 45449, 50289, or 52872 protein can further be bred to other transgenic animals carrying other transgenes.

1983, 52881, 2398, 45449, 50289, or 52872 proteins or polypeptides can be expressed in transgenic animals or plants, e.g., a nucleic acid encoding the protein or polypeptide can be introduced into the genome of an animal. In preferred embodiments the nucleic acid is placed under the control of a tissue specific promoter, e.g., a milk or egg specific promoter, and recovered from the milk or eggs produced by the animal. Suitable animals are mice, pigs, cows, goats, and sheep.

The invention also includes a population of cells from a transgenic animal, as discussed, e.g., below.

### Uses

The nucleic acid molecules, proteins, protein homologues, and antibodies described herein can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic).

The isolated nucleic acid molecules of the invention can be used, for example, to express a 1983, 52881, 2398, 45449, 50289, or 52872 protein (e.g., via a recombinant expression vector in a host cell in gene therapy applications), to detect a 1983, 52881, 2398, 45449, 50289, or 52872 mRNA (e.g., in a biological sample) or a genetic alteration in a 1983, 52881, 2398, 45449, 50289, or 52872 gene, and to modulate 1983, 52881, 2398, 45449, 50289, or 52872 activity, as described further below. The 1983, 52881, 2398, 45449, 50289, or 52872 proteins can be used to treat disorders characterized by insufficient or excessive production of a 1983, 52881, 2398, 45449, 50289, or 52872 substrate or production of 1983, 52881, 2398, 45449, 50289, or 52872 inhibitors. In addition, the 1983, 52881, 2398, 45449, 50289, or 52872 proteins can be used to screen for naturally occurring 1983, 52881, 2398, 45449, 50289, or 52872 substrates, to screen for drugs or compounds which modulate 1983, 52881, 2398, 45449, 50289, or 52872 activity, as well as to treat disorders characterized by insufficient or excessive production of 1983, 52881, 2398, 45449, 50289, or 52872 protein or production of 1983, 52881, 2398, 45449, 50289, or 52872 protein forms which have decreased, aberrant or unwanted activity compared to 1983, 52881, 2398, 45449, 50289, or 52872 wild type protein (e.g., a cardiovascular disorder or a pain related disorder). Moreover, the anti-1983, 52881, 2398, 45449, 50289, or 52872 antibodies of the invention can be used to detect and isolate 1983, 52881, 2398, 45449, 50289, or 52872 proteins, regulate the bioavailability of 1983, 52881, 2398, 45449, 50289, or 52872 proteins, and modulate 1983, 52881, 2398, 45449, 50289, or 52872 activity.

A method of evaluating a compound for the ability to interact with, e.g., bind, a subject 1983, 52881, 2398, 45449, 50289, or 52872 polypeptide is provided. The method includes: contacting the compound with the subject 1983, 52881, 2398, 45449, 50289, or 52872 polypeptide; and evaluating ability of the compound to interact with, e.g., to bind or form a complex with the subject 1983, 52881, 2398, 45449, 50289, or 52872 polypeptide. This method can be performed in vitro, e.g., in a cell free system, or in vivo, e.g., in a two-hybrid interaction trap assay. This method can be used to identify naturally occurring molecules which interact with subject 1983, 52881, 2398, 45449, 50289, or 52872 polypeptide. It can also be used to find natural or synthetic inhibitors of subject 1983, 52881, 2398, 45449, 50289, or 52872 polypeptide. Screening methods are discussed in more detail below.

### Screening Assays:

The invention provides methods (also referred to herein as "screening assays") for identifying modulators, i.e., candidate or test compounds or agents (e.g., proteins, peptides, peptidomimetics, peptoids, small molecules or other drugs) which bind to 1983, 52881, 2398, 45449, 50289, or 52872 proteins, have a stimulatory or inhibitory effect on, for example, 1983, 52881, 2398, 45449, 50289, or 52872 expression or 1983, 52881, 2398, 45449, 50289, or 52872 activity, or have a stimulatory or inhibitory effect on, for example, the expression or activity of a 1983, 52881, 2398, 45449, 50289, or 52872 substrate. Compounds thus identified can be used to modulate the activity of target gene products (e.g., 1983, 52881, 2398, 45449, 50289, or 52872 genes) in a therapeutic protocol, to elaborate the biological function of the target gene product, or to identify compounds that disrupt normal target gene interactions.

In one embodiment, the invention provides assays for screening candidate or test compounds which are substrates of a 1983, 52881, 2398, 45449, 50289, or 52872 protein or polypeptide or a biologically active portion thereof. In another embodiment, the invention provides assays for screening candidate or test compounds which bind to or modulate the activity of a 1983, 52881, 2398, 45449, 50289, or 52872 protein or polypeptide or a biologically active portion thereof.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann, R.N. et al. (1994) J. Med. Chem. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, K.S. (1997) Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner, USP 5,223,409), spores (Ladner USP '409), plasmids (Cull et al. (1992) Proc Natl Acad Sci USA 89:1865-1869) or on phage (Scott and Smith (1990) Science 249:386-390; Devlin (1990) Science 249:404-406; Cwirla et al. (1990) Proc. Natl. Acad. Sci. 87:6378-6382; Felici (1991) J. Mol. Biol. 222:301-310; Ladner *supra*.).

In one embodiment, an assay is a cell-based assay in which a cell which expresses a 1983, 52881, 2398, 45449, 50289, or 52872 protein or biologically active portion thereof is contacted with a test compound, and the ability of the test compound to modulate 1983, 52881, 2398, 45449, 50289, or 52872 activity is determined. Determining the ability of the test compound to modulate 1983, 52881, 2398, 45449, 50289, or 52872 activity can be accomplished by monitoring, for example, cell signaling, cell growth, or cell differentiation. The cell, for example, can be of mammalian origin, e.g., human.

The ability of the test compound to modulate 1983, 52881, 2398, 45449, 50289, or 52872 binding to a compound, e.g., a 1983, 52881, 2398, 45449, 50289, or 52872 substrate, or to bind to 1983, 52881, 2398, 45449, 50289, or 52872 can also be evaluated. This can be accomplished, for example, by coupling the compound, e.g., the substrate, with a radioisotope or enzymatic label such that binding of the compound, e.g., the substrate, to 1983, 52881, 2398, 45449, 50289, or 52872 can be determined by detecting the labeled compound, e.g., substrate, in a complex. Alternatively, 1983, 52881, 2398, 45449, 50289, or 52872 could be coupled with a radioisotope or enzymatic label to monitor the ability of a test compound to modulate 1983, 52881, 2398, 45449, 50289, or 52872 binding to a 1983, 52881, 2398, 45449, 50289, or 52872 substrate in a complex. For example, compounds (e.g., 1983, 52881, 2398, 45449, 50289, or 52872 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 1983, 52881, 2398, 45449, 50289, or 52872 substrate) to interact with 1983, 52881, 2398, 45449, 50289, or 52872 with or without the labeling of any of the interactants can be evaluated. For example, a microphysiometer can be used to detect the interaction of a compound with 1983, 52881, 2398, 45449, 50289, or 52872 without the labeling of either the compound or the 1983, 52881, 2398, 45449, 50289, or 52872. McConnell, H. M. et al. (1992) Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 1983, 52881, 2398, 45449, 50289, or 52872.

In yet another embodiment, a cell-free assay is provided in which a 1983, 52881, 2398, 45449, 50289, or 52872 protein or biologically active portion thereof is contacted with a test compound and the ability of the test compound to bind to the 1983, 52881, 2398, 45449, 50289, or 52872 protein or biologically active portion thereof is evaluated. Preferred biologically active portions of the 1983, 52881, 2398, 45449, 50289, or 52872 proteins to be used in assays of the present invention include fragments which participate in interactions with non-1983, 52881, 2398, 45449, 50289, or 52872 molecules, e.g., fragments with high surface probability scores.

Soluble and/or membrane-bound forms of isolated proteins (e.g., 1983, 52881, 2398, 45449, 50289, or 52872 proteins or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, e.g., using fluorescence energy transfer (FET) (see, for example, Lakowicz et al., U.S. Patent No. 5,631,169; Stavrianopoulos, et al., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 1983, 52881, 2398, 45449, 50289, or 52872 protein to bind to a target molecule can be accomplished using real-time Biomolecular Interaction Analysis (BIA) (see, e.g., Sjolander, S. and Urbaniczky, C. (1991) Anal. Chem. 63:2338-2345 and Szabo et al. (1995) Curr. Opin. Struct. Biol. 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 1983, 52881, 2398, 45449, 50289, or 52872, an anti-1983, 52881, 2398, 45449, 50289, or 52872 antibody or its target molecule to facilitate separation of complexed from uncomplexed forms of one or both of the proteins, as well as to accommodate automation of the assay. Binding of a test compound to a 1983, 52881, 2398, 45449, 50289, or 52872 protein, or interaction of a 1983, 52881, 2398, 45449, 50289, or 52872 protein with a target molecule in the presence and absence of a candidate compound, can be accomplished in any vessel suitable for containing the reactants. Examples of such vessels include microtiter plates, test tubes, and microcentrifuge tubes. In one embodiment, a fusion protein can be provided which adds a domain that allows one or both of the proteins to be bound to a matrix. For example, glutathione-S-transferase/1983, 52881, 2398, 45449, 50289, or 52872 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 1983, 52881, 2398, 45449, 50289, or 52872 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 1983, 52881, 2398, 45449, 50289, or 52872 binding or activity determined using standard techniques.

Other techniques for immobilizing either a 1983, 52881, 2398, 45449, 50289, or 52872 protein or a target molecule on matrices include using conjugation of biotin and streptavidin. Biotinylated 1983, 52881, 2398, 45449, 50289, or 52872 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with 1983, 52881, 2398, 45449, 50289, or 52872 protein or target molecules but which do not interfere with binding of the 1983, 52881, 2398, 45449, 50289, or 52872 protein to its target molecule. Such antibodies can be derivatized to the wells of the plate, and unbound target or 1983, 52881, 2398, 45449, 50289, or 52872 protein trapped in the wells by antibody conjugation. Methods for detecting such complexes, in addition to those described above for the GST-immobilized complexes, include immunodetection of complexes using antibodies reactive with the 1983, 52881, 2398, 45449, 50289, or 52872 protein or target molecule, as well as enzyme-linked assays which rely on detecting an enzymatic activity associated with the 1983, 52881, 2398, 45449, 50289, or 52872 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A.P., (1993) Trends Biochem Sci 18:284-7); chromatography (gel filtration chromatography, ion-exchange chromatography); electrophoresis (see, e.g., Ausubel, F. et al., eds. Current Protocols in Molecular Biology 1999, J. Wiley: New York.); and immunoprecipitation (see, for example, Ausubel, F. et al., eds. (1999) Current Protocols in Molecular Biology, J. Wiley: New York). Such resins and chromatographic techniques are known to one skilled in the art (see, e.g., Heegaard, N.H., (1998) J Mol Recognit 11:141-8; Hage, D.S., and Tweed, S.A. (1997) J Chromatogr B Biomed Sci Appl. 699:499-525). Further, fluorescence energy transfer may also be conveniently utilized, as described herein, to detect binding without further purification of the complex from solution.

In a preferred embodiment, the assay includes contacting the 1983, 52881, 2398, 45449, 50289, or 52872 protein or biologically active portion thereof with a known compound which binds 1983, 52881, 2398, 45449, 50289, or 52872 to form an assay mixture, contacting the assay mixture with a test compound, and determining the ability of the test compound to interact with a 1983, 52881, 2398, 45449, 50289, or 52872 protein, wherein determining the ability of the test compound to interact with a 1983, 52881, 2398, 45449, 50289, or 52872 protein includes determining the ability of the test compound to preferentially bind to 1983, 52881, 2398, 45449, 50289, or 52872 or biologically active portion thereof, or to modulate the activity of a target molecule, as compared to the known compound.

The target gene products of the invention can, *in vivo*, interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 1983, 52881, 2398, 45449, 50289, or 52872 genes herein identified. In an alternative embodiment, the invention provides methods for determining the ability of the test compound to modulate the activity of a 1983, 52881, 2398, 45449, 50289, or 52872 protein through modulation of the activity of a downstream effector of a 1983, 52881, 2398, 45449, 50289, or 52872 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 1983, 52881, 2398, 45449, 50289, or 52872 proteins can be used as "bait proteins" in a two-hybrid assay or three-hybrid assay (see, e.g., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with 1983, 52881, 2398, 45449, 50289, or 52872 (" 1983, 52881, 2398, 45449, 50289, or 52872-binding proteins" or "1983, 52881, 2398, 45449, 50289, or 52872-bp") and are involved in 1983, 52881, 2398, 45449, 50289, or 52872 activity. Such 1983, 52881, 2398, 45449, 50289, or 52872-bps can be activators or inhibitors of signals by the 1983, 52881, 2398, 45449, 50289, or 52872 proteins or 1983, 52881, 2398, 45449, 50289, or 52872 targets as, for example, downstream elements of a 1983, 52881, 2398, 45449, 50289, or 52872-mediated signaling pathway.

The two-hybrid system is based on the modular nature of most transcription factors, which consist of separable DNA-binding and activation domains. Briefly, the assay utilizes two different DNA constructs. In one construct, the gene that codes for a 1983, 52881, 2398, 45449, 50289, or 52872 protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 1983, 52881, 2398, 45449, 50289, or 52872 protein can be the fused to the activator domain.) If the "bait" and the "prey" proteins are able to interact, *in vivo*, forming a 1983, 52881, 2398, 45449, 50289, or 52872-dependent complex, the DNA-binding and activation domains of the transcription factor are brought into close proximity. This proximity allows transcription of a reporter gene (e.g., lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 1983, 52881, 2398, 45449, 50289, or 52872 protein.

In another embodiment, modulators of 1983, 52881, 2398, 45449, 50289, or 52872 expression are identified. For example, a cell or cell free mixture is contacted with a candidate compound and the expression of 1983, 52881, 2398, 45449, 50289, or 52872 mRNA or protein evaluated relative to the level of expression of 1983, 52881, 2398, 45449, 50289, or 52872 mRNA or protein in the absence of the candidate compound. When expression of 1983, 52881, 2398, 45449, 50289, or 52872 mRNA or protein is greater in the presence of the candidate compound than in its absence, the candidate compound is identified as a stimulator of 1983, 52881, 2398, 45449, 50289, or 52872 mRNA or protein expression. Alternatively, when expression of 1983, 52881, 2398, 45449, 50289, or 52872 RNA or protein is less (statistically significantly less) in the presence of the candidate compound than in its absence, the candidate compound is identified as an inhibitor of 1983, 52881, 2398, 45449, 50289, or 52872 mRNA or protein expression. The level of 1983, 52881, 2398, 45449, 50289, or 52872 mRNA or protein expression can be determined by methods described herein for detecting 1983, 52881, 2398, 45449, 50289, or 52872 mRNA or protein.

In another aspect, the invention pertains to a combination of two or more of the assays described herein. For example, a modulating agent can be identified using a cell-based or a cell free assay, and the ability of the agent to modulate the activity of a 1983, 52881, 2398, 45449, 50289, or 52872 protein can be confirmed *in vivo*, e.g., in an animal model.

This invention further pertains to novel agents identified by the above-described screening assays. Accordingly, it is within the scope of this invention to further use an agent identified as described herein (e.g., a 1983, 52881, 2398, 45449, 50289, or 52872 modulating agent, an antisense 1983, 52881, 2398, 45449, 50289, or 52872 nucleic acid molecule, a 1983, 52881, 2398, 45449, 50289, or 52872-specific antibody, or a 1983, 52881, 2398, 45449, 50289, or 52872-binding partner) in an appropriate animal model to determine the efficacy, toxicity, side effects, or mechanism of action, of treatment with such an agent. Furthermore, novel agents identified by the above-described screening assays can be used for treatments as described herein.

### Detection Assays

Portions or fragments of the nucleic acid sequences identified herein can be used as polynucleotide reagents. For example, these sequences can be used to: (i) map their respective genes on a chromosome e.g., to locate gene regions associated with genetic disease or to associate 1983, 52881, 2398, 45449, 50289, or 52872 with a disease; (ii) identify an individual from a minute biological sample (tissue typing); and (iii) aid in forensic identification of a biological sample. These applications are described in the subsections below.

### Chromosome Mapping

The 1983, 52881, 2398, 45449, 50289, or 52872 nucleotide sequences or portions thereof can be used to map the location of the 1983, 52881, 2398, 45449, 50289, or 52872 genes on a chromosome. This process is called chromosome mapping. Chromosome mapping is useful in correlating the 1983, 52881, 2398, 45449, 50289, or 52872 sequences with genes associated with disease.

Briefly, 1983, 52881, 2398, 45449, 50289, or 52872 genes can be mapped to chromosomes by preparing PCR primers (preferably 15-25 bp in length) from the 1983, 52881, 2398, 45449, 50289, or 52872 nucleotide sequences. These primers can then be used for PCR screening of somatic cell hybrids containing individual human chromosomes. Only those hybrids containing the human gene corresponding to the 1983, 52881, 2398, 45449, 50289, or 52872 sequences will yield an amplified fragment.

A panel of somatic cell hybrids in which each cell line contains either a single human chromosome or a small number of human chromosomes, and a full set of mouse chromosomes, can allow easy mapping of individual genes to specific human chromosomes. (D'Eustachio P. et al. (1983) Science 220:919-924).

Other mapping strategies e.g., in situ hybridization (described in Fan, Y. et al. (1990) Proc. Natl. Acad. Sci. USA, 87:6223-27), pre-screening with labeled flow-sorted chromosomes, and pre-selection by hybridization to chromosome specific cDNA libraries can be used to map 1983, 52881, 2398, 45449, 50289, or 52872 to a chromosomal location.

Fluorescence *in situ* hybridization (FISH) of a DNA sequence to a metaphase chromosomal spread can further be used to provide a precise chromosomal location in one step. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma et al., Human Chromosomes: A Manual of Basic Techniques ((1988) Pergamon Press, New York).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. et al. (1987) Nature, 325:783-787.

Moreover, differences in the DNA sequences between individuals affected and unaffected with a disease associated with the 1983, 52881, 2398, 45449, 50289, or 52872 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

1983, 52881, 2398, 45449, 50289, or 52872 sequences can be used to identify individuals from biological samples using, e.g., restriction fragment length polymorphism (RFLP). In this technique, an individual's genomic DNA is digested with one or more restriction enzymes, the fragments separated, e.g., in a Southern blot, and probed to yield bands for identification. The sequences of the present invention are useful as additional DNA markers for RFLP (described in U.S. Patent 5,272,057).

Furthermore, the sequences of the present invention can also be used to determine the actual base-by-base DNA sequence of selected portions of an individual's genome. Thus, the 1983, 52881, 2398, 45449, 50289, or 52872 nucleotide sequences described herein can be used to prepare two PCR primers from the 5' and 3' ends of the sequences. These primers can then be used to amplify an individual's DNA and subsequently sequence it. Panels of corresponding DNA sequences from individuals, prepared in this manner, can provide unique individual identifications, as each individual will have a unique set of such DNA sequences due to allelic differences.

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1, SEQ ID NO:4, SEQ ID NO:7, SEQ ID NO:10, SEQ ID NO:13, or SEQ ID NO:16 can provide positive individual identification with a panel of perhaps 10 to 1,000 primers which each yield a noncoding amplified sequence of 100 bases. If predicted coding sequences, such as those in SEQ ID NO:3 are used, a more appropriate number of primers for positive individual identification would be 500-2,000.

If a panel of reagents from 1983, 52881, 2398, 45449, 50289, or 52872 nucleotide sequences described herein is used to generate a unique identification database for an individual, those same reagents can later be used to identify tissue from that individual. Using the unique identification database, positive identification of the individual, living or dead, can be made from extremely small tissue samples.

### Use of Partial 1983, 52881, 2398, 45449, 50289, or 52872 Sequences in Forensic Biology

DNA-based identification techniques can also be used in forensic biology. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1, SEQ ID NO:4, SEQ ID NO:7, SEQ ID NO:10, SEQ ID NO:13, or SEQ ID NO:16 (e.g., fragments derived from the noncoding regions of SEQ ID NO:1, SEQ ID NO:4, SEQ ID NO:7, SEQ ID NO:10, SEQ ID NO:13, or SEQ ID NO:16 having a length of at least 20 bases, preferably at least 30 bases) are particularly appropriate for this use.

The 1983, 52881, 2398, 45449, 50289, or 52872 nucleotide sequences described herein can further be used to provide polynucleotide reagents, e.g., labeled or labelable probes which can be used in, for example, an *in situ* hybridization technique, to identify a specific tissue. This can be very useful in cases where a forensic pathologist is presented with a tissue of unknown origin. Panels of such 1983, 52881, 2398, 45449, 50289, or 52872 probes can be used to identify tissue by species and/or by organ type.

In a similar fashion, these reagents, e.g., 1983, 52881, 2398, 45449, 50289, or 52872 primers or probes can be used to screen tissue culture for contamination (i.e. screen for the presence of a mixture of different types of cells in a culture).

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 1983, 52881, 2398, 45449, 50289, or 52872.

Such disorders include, e.g., a disorder associated with the misexpression of 1983, 52881, 2398, 45449, 50289, or 52872 gene, e.g., cardiovascular disorders, pain, pain related disorders, and inflammatory disorders.

The method includes one or more of the following:
detecting, in a tissue of the subject, the presence or absence of a mutation which affects the expression of the 1983, 52881, 2398, 45449, 50289, or 52872 gene, or detecting the presence or absence of a mutation in a region which controls the expression of the gene, e.g., a mutation in the 5' control region;
detecting, in a tissue of the subject, the presence or absence of a mutation which alters the structure of the 1983, 52881, 2398, 45449, 50289, or 52872 gene;
detecting, in a tissue of the subject, the misexpression of the 1983, 52881, 2398, 45449, 50289, or 52872 gene, at the mRNA level, e.g., detecting a non-wild type level of a mRNA;
detecting, in a tissue of the subject, the misexpression of the gene, at the protein level, e.g., detecting a non-wild type level of a 1983, 52881, 2398, 45449, 50289, or 52872 polypeptide.

In preferred embodiments the method includes: ascertaining the existence of at least one of: a deletion of one or more nucleotides from the 1983, 52881, 2398, 45449, 50289, or 52872 gene; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, SEQ ID NO:4, SEQ ID NO:7, SEQ ID NO:10, SEQ ID NO:13, or SEQ ID NO:16, or naturally occurring mutants thereof or 5' or 3' flanking sequences naturally associated with the 1983, 52881, 2398, 45449, 50289, or 52872 gene; (ii) exposing the probe/primer to nucleic acid of the tissue; and detecting, by hybridization, e.g., *in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 1983, 52881, 2398, 45449, 50289, or 52872 gene; the presence of a non-wild type splicing pattern of a messenger RNA transcript of the gene; or a non-wild type level of 1983, 52881, 2398, 45449, 50289, or 52872.

Methods of the invention can be used prenatally or to determine if a subject's offspring will be at risk for a disorder.

In preferred embodiments the method includes determining the structure of a 1983, 52881, 2398, 45449, 50289, or 52872 gene, an abnormal structure being indicative of risk for the disorder.

In preferred embodiments the method includes contacting a sample from the subject with an antibody to the 1983, 52881, 2398, 45449, 50289, or 52872 protein or a nucleic acid, which hybridizes specifically with the gene. There and other embodiments are discussed below.

### Diagnostic and Prognostic Assays

Diagnostic and prognostic assays of the invention include method for assessing the expression level of 1983, 52881, 2398, 45449, 50289 and 52872 molecules and for identifying variations and mutations in the sequence of 1983, 52881, 2398, 45449, 50289 and 52872 molecules.

### Expression Monitoring and Profiling

The presence, level, or absence of a 1983, 52881, 2398, 45449, 50289 or 52872 protein or nucleic acid in a biological sample can be evaluated by obtaining a biological sample from a test subject and contacting the biological sample with a compound or an agent capable of detecting 1983, 52881, 2398, 45449, 50289 and 52872 protein or nucleic acid (e.g., mRNA, genomic DNA) that encodes 1983, 52881, 2398, 45449, 50289 and 52872 protein such that the presence of 1983, 52881, 2398, 45449, 50289 and 52872 protein or nucleic acid is detected in the biological sample. The term "biological sample" includes tissues, cells and biological fluids isolated from a subject, as well as tissues, cells and fluids present within a subject. A preferred biological sample is serum. The level of expression ofthe 1983, 52881, 2398, 45449, 50289 and 52872 gene can be measured in a number of ways, including, but not limited to: measuring the mRNA encoded by the 1983, 52881, 2398, 45449, 50289 and 52872 genes; measuring the amount of protein encoded by the 1983, 52881, 2398, 45449, 50289 and 52872 genes; or measuring the activity of the protein encoded by the 1983, 52881, 2398, 45449, 50289 and 52872 genes.

The level of mRNA corresponding to the 1983, 52881, 2398, 45449, 50289 and 52872 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 1983, 52881, 2398, 45449, 50289 and 52872 nucleic acid, such as the nucleic acid of SEQ ID NO:1, 4 or 7, or a portion thereof, such as an oligonucleotide of at least 7, 15, 30, 50, 100, 250 or 500 nucleotides in length and sufficient to specifically hybridize under stringent conditions to 1983, 52881, 2398, 45449, 50289 and 52872 mRNA or genomic DNA. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array described below. The probe can be disposed on an address of an array, e.g., an array described below. A skilled artisan can adapt known mRNA detection methods for use in detecting the level of mRNA encoded by the 1983, 52881, 2398, 45449, 50289 and 52872 genes.

The level of mRNA in a sample that is encoded by one of 1983, 52881, 2398, 45449, 50289 and 52872 can be evaluated with nucleic acid amplification, e.g., by rtPCR (Mullis, 1987, U.S. Patent No. 4,683,202), ligase chain reaction (Barany, 1991, Proc. Natl. Acad. Sci. USA 88:189-193), self sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 1983, 52881, 2398, 45449, 50289 or 52872 gene being analyzed.

In another embodiment, the methods further contacting a control sample with a compound or agent capable of detecting 1983, 52881, 2398, 45449, 50289 and 52872 mRNA, or genomic DNA, and comparing the presence of 1983, 52881, 2398, 45449, 50289 and 52872 mRNA or genomic DNA in the control sample with the presence of 1983, 52881, 2398, 45449, 50289 and 52872 mRNA or genomic DNA in the test sample.

A variety of methods can be used to determine the level of protein encoded by 1983, 52881, 2398, 45449, 50289 and 52872. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 1983, 52881, 2398, 45449, 50289 and 52872 protein in a biological sample *in vitro* as well as *in vivo*. *In vitro* techniques for detection of 1983, 52881, 2398, 45449, 50289 and 52872 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 1983, 52881, 2398, 45449, 50289 and 52872 protein include introducing into a subject a labeled anti-1983, 52881, 2398, 45449, 50289 and 52872 antibody. For example, the antibody can be labeled with a radioactive marker whose presence and location in a subject can be detected by standard imaging techniques. In another embodiment, the sample is labeled, e.g., biotinylated and then contacted to the antibody, e.g., an anti-1983, 52881, 2398, 45449, 50289 or 52872 antibody positioned on an antibody array (as described below). The sample can be detected, e.g., with avidin coupled to a fluorescent label.

In another embodiment, the methods further include contacting the control sample with a compound or agent capable of detecting 1983, 52881, 2398, 45449, 50289 or 52872 protein, and comparing the presence of 1983, 52881, 2398, 45449, 50289 or 52872 protein in the control sample with the presence of 1983, 52881, 2398, 45449, 50289 or 52872 protein in the test sample.

The invention also includes kits for detecting the presence of 1983, 52881, 2398, 45449, 50289 and 52872 in a biological sample. For example, the kit can include a compound or agent capable of detecting 1983, 52881, 2398, 45449, 50289 or 52872 protein or mRNA in a biological sample; and a standard. The compound or agent can be packaged in a suitable container. The kit can further comprise instructions for using the kit to detect 1983, 52881, 2398, 45449, 50289 or 52872 protein or nucleic acid.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 1983, 52881, 2398, 45449, 50289 and 52872 expression or activity. As used herein, the term "unwanted" includes an unwanted phenomenon involved in a biological response such as pain or deregulated cell proliferation.

In one embodiment, a disease or disorder associated with aberrant or unwanted 1983, 52881, 2398, 45449, 50289 and 52872 expression or activity is identified. A test sample is obtained from a subject and 1983, 52881, 2398, 45449, 50289 and 52872 protein or nucleic acid (e.g., mRNA or genomic DNA) is evaluated, wherein the level, e.g., the presence or absence, of 1983, 52881, 2398, 45449, 50289 and 52872 protein or nucleic acid is diagnostic for a subject having or at risk of developing a disease or disorder associated with aberrant or unwanted 1983, 52881, 2398, 45449, 50289 and 52872 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 1983, 52881, 2398, 45449, 50289 and 52872 expression or activity. For example, such methods can be used to determine whether a subject can be effectively treated with an agent that modulates 1983, 52881, 2398, 45449, 50289 and 52872 expression or activity.

In another aspect, the invention features a computer medium having a plurality of digitally encoded data records. Each data record includes a value representing the level of expression of 1983, 52881, 2398, 45449, 50289 and 52872 in a sample, and a descriptor of the sample. The descriptor of the sample can be an identifier of the sample, a subject from which the sample was derived (e.g., a patient), a diagnosis, or a treatment (e.g., a preferred treatment). In a preferred embodiment, the data record further includes values representing the level of expression of genes other than 1983, 52881, 2398, 45449, 50289 and 52872 (e.g., other genes associated with a 1983, 52881, 2398, 45449, 50289 and 52872-disorder, or other genes on an array). The data record can be structured as a table, e.g., a table that is part of a database such as a relational database (e.g., a SQL database of the Oracle or Sybase database environments).

Also featured is a method of evaluating a sample. The method includes providing a sample, e.g., from the subject, and determining a gene expression profile of the sample, wherein the profile includes a value representing the level of 1983, 52881, 2398, 45449, 50289 and 52872 expression. The method can further include comparing the value or the profile (i.e., multiple values) to a reference value or reference profile. The gene expression profile of the sample can be obtained by any of the methods described herein (e.g., by providing a nucleic acid from the sample and contacting the nucleic acid to an array). The method can be used to diagnose a DISORDERA disorder in a subject wherein an increase in 1983, 52881, 2398, 45449, 50289 and 52872 expression is an indication that the subject has or is disposed to having a disorders as described herein. The method can be used to monitor a treatment for such disorders in a subject. For example, the gene expression profile can be determined for a sample from a subject undergoing treatment. The profile can be compared to a reference profile or to a profile obtained from the subject prior to treatment or prior to onset of the disorder (see, e.g., Golub *et al*. (1999) *Science* 286:531).

In yet another aspect, the invention features a method of evaluating a test compound (see also, "Screening Assays", above). The method includes providing a cell and a test compound; contacting the test compound to the cell; obtaining a subject expression profile for the contacted cell; and comparing the subject expression profile to one or more reference profiles. The profiles include a value representing the level of 1983, 52881, 2398, 45449, 50289 and 52872 expression. In a preferred embodiment, the subject expression profile is compared to a target profile, e.g., a profile for a normal cell or for desired condition of a cell. The test compound is evaluated favorably if the subject expression profile is more similar to the target profile than an expression profile obtained from an un-contacted cell.

In another aspect, the invention features a method of evaluating a subject. The method includes: a) obtaining a sample from a subject, e.g., from a caregiver, e.g., a caregiver who obtains the sample from the subject; b) determining a subject expression profile for the sample. Optionally, the method further includes either or both of steps: c) comparing the subject expression profile to one or more reference expression profiles; and d) selecting the reference profile most similar to the subject reference profile. The subject expression profile and the reference profiles include a value representing the level of 1983, 52881, 2398, 45449, 50289 or 52872 expression. A variety of routine statistical measures can be used to compare two reference profiles. One possible metric is the length of the distance vector that is the difference between the two profiles. Each of the subject and reference profile is represented as a multi-dimensional vector, wherein each dimension is a value in the profile.

The method can further include transmitting a result to a caregiver. The result can be the subject expression profile, a result of a comparison of the subject expression profile with another profile, a most similar reference profile, or a descriptor of any of the aforementioned. The result can be transmitted across a computer network, e.g., the result can be in the form of a computer transmission, e.g., a computer data signal embedded in a carrier wave.

Also featured is a computer medium having executable code for effecting the following steps: receive a subject expression profile; access a database of reference expression profiles; and either i) select a matching reference profile most similar to the subject expression profile or ii) determine at least one comparison score for the similarity of the subject expression profile to at least one reference profile. The subject expression profile, and the reference expression profiles each include a value representing the level of 1983, 52881, 2398, 45449, 50289 or 52872 expression.

### Arrays and Uses Thereof

In another aspect, the invention features an array that includes a substrate having a plurality of addresses. At least one address of the plurality includes a capture probe that binds specifically to a 1983, 52881, 2398, 45449, 50289 or 52872 molecule (e.g., a 1983, 52881, 2398, 45449, 50289 or 52872 nucleic acid or a 1983, 52881, 2398, 45449, 50289 or 52872 polypeptide). The array can have a density of at least than 10, 50, 100, 200, 500, 1,000, 2,000, or 10,000 or more addresses/cm², and ranges between. In a preferred embodiment, the plurality of addresses includes at least 10, 100, 500, 1,000, 5,000, 10,000, 50,000 addresses. In a preferred embodiment, the plurality of addresses includes equal to or less than 10, 100, 500, 1,000, 5,000, 10,000, or 50,000 addresses. The substrate can be a two-dimensional substrate such as a glass slide, a wafer (e.g., silica or plastic), a mass spectroscopy plate, or a three-dimensional substrate such as a gel pad. Addresses in addition to address of the plurality can be disposed on the array.

In a preferred embodiment, at least one address of the plurality includes a nucleic acid capture probe that hybridizes specifically to a 1983, 52881, 2398, 45449, 50289 or 52872 nucleic acid, e.g., the sense or anti-sense strand. In one preferred embodiment, a subset of addresses of the plurality of addresses has a nucleic acid capture probe for 1983, 52881, 2398, 45449, 50289 or 52872. Each address of the subset can include a capture probe that hybridizes to a different region of a 1983, 52881, 2398, 45449, 50289 and 52872 nucleic acid. In another preferred embodiment, addresses of the subset include a capture probe for a 1983, 52881, 2398, 45449, 50289 and 52872 nucleic acid. Each address of the subset is unique, overlapping, and complementary to a different variant of 1983, 52881, 2398, 45449, 50289 or 52872 (e.g., an allelic variant, or all possible hypothetical variants). The array can be used to sequence 1983, 52881, 2398, 45449, 50289 or 52872 by hybridization (see, e.g., U.S. Patent No. 5,695,940).

An array can be generated by various methods, e.g., by photolithographic methods (see, e.g., U.S. Patent Nos. 5,143,854; 5,510,270; and 5,527,681), mechanical methods (e.g., directed-flow methods as described in U.S. Patent No. 5,384,261), pin-based methods (e.g., as described in U.S. Pat. No. 5,288,514), and bead-based techniques (e.g., as described in PCT US/93/04145).

In another preferred embodiment, at least one address of the plurality includes a polypeptide capture probe that binds specifically to a 1983, 52881, 2398, 45449, 50289 or 52872 polypeptide or fragment thereof. The polypeptide can be a naturally-occurring interaction partner of 1983, 52881, 2398, 45449, 50289 or 52872 polypeptide. Preferably, the polypeptide is an antibody, e.g., an antibody described herein (see "Anti-1983, 52881, 2398, 45449, 50289 and 52872 Antibodies," above), such as a monoclonal antibody or a single-chain antibody.

In another aspect, the invention features a method of analyzing the expression of 1983, 52881, 2398, 45449, 50289 or 52872. The method includes providing an array as described above; contacting the array with a sample and detecting binding of a 1983, 52881, 2398, 45449, 50289 or 52872-molecule (e.g., nucleic acid or polypeptide) to the array. In a preferred embodiment, the array is a nucleic acid array. Optionally the method further includes amplifying nucleic acid from the sample prior or during contact with the array.

In another embodiment, the array can be used to assay gene expression in a tissue to ascertain tissue specificity of genes in the array, particularly the expression of 1983, 52881, 2398, 45449, 50289 or 52872. If a sufficient number of diverse samples is analyzed, clustering (e.g., hierarchical clustering, k-means clustering, Bayesian clustering and the like) can be used to identify other genes which are co-regulated with 1983, 52881, 2398, 45449, 50289 or 52872. For example, the array can be used for the quantitation of the expression of multiple genes. Thus, not only tissue specificity, but also the level of expression of a battery of genes in the tissue is ascertained. Quantitative data can be used to group (e.g., cluster) genes on the basis of their tissue expression *per se* and level of expression in that tissue.

For example, array analysis of gene expression can be used to assess the effect of cell-cell interactions on 1983, 52881, 2398, 45449, 50289 or 52872 expression. A first tissue can be perturbed and nucleic acid from a second tissue that interacts with the first tissue can be analyzed. In this context, the effect of one cell type on another cell type in response to a biological stimulus can be determined, e.g., to monitor the effect of cell-cell interaction at the level of gene expression.

In another embodiment, cells are contacted with a therapeutic agent. The expression profile of the cells is determined using the array, and the expression profile is compared to the profile of like cells not contacted with the agent. For example, the assay can be used to determine or analyze the molecular basis of an undesirable effect of the therapeutic agent. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor expression of one or more genes in the array with respect to time. For example, samples obtained from different time points can be probed with the array. Such analysis can identify and/or characterize the development of a 1983, 52881, 2398, 45449, 50289 or 52872-associated disease or disorder; and processes, such as a cellular transformation associated with a 1983, 52881, 2398, 45449, 50289 or 52872-associated disease or disorder. The method can also evaluate the treatment and/or progression of a 1983, 52881, 2398, 45449, 50289 or 52872-associated disease or disorder

The array is also useful for ascertaining differential expression patterns of one or more genes in normal and abnormal cells. This provides a battery of genes (*e*.*g*., including 1983, 52881, 2398, 45449, 50289 and 52872) that could serve as a molecular target for diagnosis or therapeutic intervention.

In another aspect, the invention features an array having a plurality of addresses. Each address of the plurality includes a unique polypeptide. At least one address of the plurality has disposed thereon a 1983, 52881, 2398, 45449, 50289 or 52872 polypeptide or fragment thereof. Methods of producing polypeptide arrays are described in the art, e.g., in De Wildt et al. (2000). Nature Biotech. 18, 989-994; Lueking et al. (1999). Anal. Biochem. 270, 103-111; Ge, H. (2000). Nucleic Acids Res. 28, e3, I-VII; MacBeath, G., and Schreiber, S.L. (2000). Science 289, 1760-1763; and WO 99/51773A1. In a preferred embodiment, each addresses of the plurality has disposed thereon a polypeptide at least 60, 70, 80, 85, 90, 95 or 99 % identical to a 1983, 52881, 2398, 45449, 50289 or 52872 polypeptide or fragment thereof. For example, multiple variants of a 1983, 52881, 2398, 45449, 50289 and 52872 polypeptide (e.g., encoded by allelic variants, site-directed mutants, random mutants, or combinatorial mutants) can be disposed at individual addresses of the plurality. Addresses in addition to the address of the plurality can be disposed on the array.

The polypeptide array can be used to detect a 1983, 52881, 2398, 45449, 50289 or 52872 binding compound, e.g., an antibody in a sample from a subject with specificity for a 1983, 52881, 2398, 45449, 50289 and 52872 polypeptide or the presence of a 1983, 52881, 2398, 45449, 50289 or 52872-binding protein or ligand.

The array is also useful for ascertaining the effect of the expression of a gene on the expression of other genes in the same cell or in different cells (e.g., ascertaining the effect of 1983, 52881, 2398, 45449, 50289 or 52872 expression on the expression of other genes). This provides, for example, for a selection of alternate molecular targets for therapeutic intervention if the ultimate or downstream target cannot be regulated.

In another aspect, the invention features a method of analyzing a plurality of probes. The method is useful, e.g., for analyzing gene expression. The method includes: providing a two dimensional array having a plurality of addresses, each address of the plurality being positionally distinguishable from each other address of the plurality having a unique capture probe, e.g., wherein the capture probes are from a cell or subject which express 1983, 52881, 2398, 45449, 50289 or 52872 or from a cell or subject in which a 1983, 52881, 2398, 45449, 50289 or 52872 mediated response has been elicited, e.g., by contact of the cell with 1983, 52881, 2398, 45449, 50289 or 52872 nucleic acid or protein, or administration to the cell or subject 1983, 52881, 2398, 45449, 50289 or 52872 nucleic acid or protein; providing a two dimensional array having a plurality of addresses, each address of the plurality being positionally distinguishable from each other address of the plurality, and each address ofthe plurality having a unique capture probe, e.g., wherein the capture probes are from a cell or subject which does not express 1983, 52881, 2398, 45449, 50289 or 52872 (or does not express as highly as in the case of the 1983, 52881, 2398, 45449, 50289 or 52872 positive plurality of capture probes) or from a cell or subject which in which a 1983, 52881, 2398, 45449, 50289 or 52872 mediated response has not been elicited (or has been elicited to a lesser extent than in the first sample); contacting the array with one or more inquiry probes (which is preferably other than a 1983, 52881, 2398, 45449, 50289 or 52872 nucleic acid, polypeptide, or antibody), and thereby evaluating the plurality of capture probes. Binding, e.g., in the case of a nucleic acid, hybridization with a capture probe at an address of the plurality, is detected, e.g., by signal generated from a label attached to the nucleic acid, polypeptide, or antibody.

In another aspect, the invention features a method of analyzing a plurality of probes or a sample. The method is useful, e.g., for analyzing gene expression. The method includes: providing a two dimensional array having a plurality of addresses, each address of the plurality being positionally distinguishable from each other address of the plurality having a unique capture probe, contacting the array with a first sample from a cell or subject which express or mis-express 1983, 52881,2398, 45449, 50289 or 52872 or from a cell or subject in which a 1983, 52881, 2398, 45449, 50289 or 52872-mediated response has been elicited, e.g., by contact of the cell with 1983, 52881, 2398, 45449, 50289 or 52872 nucleic acid or protein, or administration to the cell or subject 1983, 52881, 2398, 45449, 50289 or 52872 nucleic acid or protein; providing a two dimensional array having a plurality of addresses, each address ofthe plurality being positionally distinguishable from each other address of the plurality, and each address of the plurality having a unique capture probe, and contacting the array with a second sample from a cell or subject which does not express 1983, 52881, 2398, 45449, 50289 or 52872 (or does not express as highly as in the case of the 1983, 52881, 2398, 45449, 50289 or 52872 positive plurality of capture probes) or from a cell or subject which in which a 1983, 52881, 2398, 45449, 50289 or 52872 mediated response has not been elicited (or has been elicited to a lesser extent than in the first sample); and comparing the binding of the first sample with the binding of the second sample. Binding, e.g., in the case of a nucleic acid, hybridization with a capture probe at an address of the plurality, is detected, e.g., by signal generated from a label attached to the nucleic acid, polypeptide, or antibody. The same array can be used for both samples or different arrays can be used. If different arrays are used the plurality of addresses with capture probes should be present on both arrays.

In another aspect, the invention features a method of analyzing 1983, 52881, 2398, 45449, 50289 or 52872, e.g., analyzing structure, function, or relatedness to other nucleic acid or amino acid sequences. The method includes: providing a 1983, 52881, 2398, 45449, 50289 or 52872 nucleic acid or amino acid sequence; comparing the 1983, 52881, 2398, 45449, 50289 or 52872 sequence with one or more preferably a plurality of sequences from a collection of sequences, e.g., a nucleic acid or protein sequence database; to thereby analyze 1983, 52881, 2398, 45449, 50289 or 52872.

### Detection of Variations or Mutations

The methods of the invention can also be used to detect genetic alterations in a 1983, 52881, 2398, 45449, 50289 or 52872 gene, thereby determining if a subject with the altered gene is at risk for a disorder characterized by mis-regulation in 1983, 52881, 2398, 45449, 50289 or 52872 protein activity or nucleic acid expression, such as an immune disorder, a neurodegenerative disorder, or a cardiovascular disorder. In preferred embodiments, the methods include detecting, in a sample from the subject, the presence or absence of a genetic alteration characterized by at least one of an alteration affecting the integrity of a gene encoding a 1983, 52881, 2398, 45449, 50289 or 52872-protein, or the mis-expression of the 1983, 52881, 2398, 45449, 50289 or 52872 gene. For example, such genetic alterations can be detected by ascertaining the existence of at least one of 1) a deletion of one or more nucleotides from a 1983, 52881, 2398, 45449, 50289 or 52872 gene; 2) an addition of one or more nucleotides to a 1983, 52881, 2398, 45449, 50289 or 52872 gene; 3) a substitution of one or more nucleotides of a 1983, 52881, 2398, 45449, 50289 or 52872 gene, 4) a chromosomal rearrangement of a 1983, 52881, 2398, 45449, 50289 or 52872 gene; 5) an alteration in the level of a messenger RNA transcript of a 1983, 52881, 2398, 45449, 50289 or 52872 gene, 6) aberrant modification of a 1983, 52881, 2398, 45449, 50289 or 52872 gene, such as of the methylation pattern of the genomic DNA, 7) the presence of a non-wild type splicing pattern of a messenger RNA transcript of a 1983, 52881, 2398, 45449, 50289 or 52872 gene, 8) a non-wild type level of a 1983, 52881, 2398, 45449, 50289 or 52872-protein, 9) allelic loss of a 1983, 52881, 2398, 45449, 50289 or 52872 gene, and 10) inappropriate post-translational modification of a 1983, 52881, 2398, 45449, 50289 or 52872-protein.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 1983, 52881, 2398, 45449, 50289 or 52872-gene. This method can include the steps of collecting a sample of cells from a subject, isolating nucleic acid (e.g., genomic, mRNA or both) from the sample, contacting the nucleic acid sample with one or more primers which specifically hybridize to a 1983, 52881, 2398; 45449, 50289 or 52872 gene under conditions such that hybridization and amplification of the 1983, 52881, 2398, 45449, 50289 or 52872-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., (1990) Proc. Natl. Acad. Sci. USA 87:1874-1878), transcriptional amplification system (Kwoh, D.Y. et al., (1989) Proc. Natl. Acad. Sci. USA 86:1173-1177), Q-Beta Replicase (Lizardi, P.M. et al. (1988) Bio-Technology 6:1197), or other nucleic acid amplification methods, followed by the detection of the amplified molecules using techniques known to those of skill in the art.

In another embodiment, mutations in a 1983, 52881, 2398, 45449, 50289 or 52872 gene from a sample cell can be identified by detecting alterations in restriction enzyme cleavage patterns. For example, sample and control DNA is isolated, amplified (optionally), digested with one or more restriction endonucleases, and fragment length sizes are determined, e.g., by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 1983, 52881, 2398, 45449, 50289 or 52872 can be identified by hybridizing a sample and control nucleic acids, e.g., DNA or RNA, two- dimensional arrays, e.g., chip based arrays. Such arrays include a plurality of addresses, each of which is positionally distinguishable from the other. A different probe is located at each address of the plurality. The arrays can have a high density of addresses, e.g., can contain hundreds or thousands of oligonucleotides probes (Cronin, M.T. et al. (1996) Human Mutation 7: 244-255; Kozal, M.J. et al. (1996) Nature Medicine 2: 753-759). For example, genetic mutations in 1983, 52881, 2398, 45449, 50289 and 52872 can be identified in two dimensional arrays containing light-generated DNA probes as described in Cronin, M.T. *et al*. *supra*. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 1983, 52881, 2398, 45449, 50289 or 52872 gene and detect mutations by comparing the sequence of the sample 1983, 52881, 2398, 45449, 50289 or 52872 with the corresponding wild-type (control) sequence. Automated sequencing procedures can be utilized when performing the diagnostic assays ((1995) Biotechniques 19:448), including sequencing by mass spectrometry.

Other methods for detecting mutations in the 1983, 52881, 2398, 45449, 50289 or 52872 gene include methods in which protection from cleavage agents is used to detect mismatched bases in RNA/RNA or RNA/DNA heteroduplexes (Myers et al. (1985) Science 230:1242; Cotton et al. (1988) Pro. Natl Acad Sci USA 85:4397; Saleeba et al. (1992) Methods Enzymol. 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 1983, 52881, 2398, 45449, 50289 and 52872 cDNAs obtained from samples of cells. For example, the mutY enzyme of *E. coli* cleaves A at G/A mismatches and the thymidine DNA glycosylase from HeLa cells cleaves T at G/T mismatches (Hsu et al. (1994) Carcinogenesis 15:1657-1662; U.S. Patent No. 5,459,039).

In other embodiments, alterations in electrophoretic mobility will be used to identify mutations in 1983, 52881, 2398, 45449, 50289 or 52872 genes. For example, single strand conformation polymorphism (SSCP) may be used to detect differences in electrophoretic mobility between mutant and wild type nucleic acids (Orita et al. (1989) Proc Natl. Acad. Sci USA: 86:2766, see also Cotton (1993) Mutat. Res. 285:125-144; and Hayashi (1992) Genet. Anal. Tech. Appl. 9:73-79). Single-stranded DNA fragments of sample and control 1983, 52881, 2398, 45449, 50289 and 52872 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension (Saiki et al. (1986) Nature 324:163); Saiki et al. (1989) Proc. Notl Acad. Sci USA 86:6230). A further method of detecting point mutations is the chemical ligation of oligonucleotides as described in Xu et al. ((2001) Nature Biotechnol. 19:148). Adjacent oligonucleotides, one of which selectively anneals to the query site, are ligated together if the nucleotide at the query site of the sample nucleic acid is complementary to the query oligonucleotide; ligation can be monitored, e.g., by fluorescent dyes coupled to the oligonucleotides.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

In another aspect, the invention features a set of oligonucleotides. The set includes a plurality of oligonucleotides, each of which is at least partially complementary (e.g., at least 50%, 60%, 70%, 80%, 90%, 92%, 95%, 97%, 98%, or 99% complementary) to a 1983, 52881, 2398, 45449, 50289 or 52872 nucleic acid.

In a preferred embodiment the set includes a first and a second oligonucleotide. The first and second oligonucleotide can hybridize to the same or to different locations of SEQ ID NO: 1, 3, 4, 6, 7 or 9, or the complement of SEQ ID NO:1, 3, 4, 6, 7 or 9. Different locations can be different but overlapping or or nonoverlapping on the same strand. The first and second oligonucleotide can hybridize to sites on the same or on different strands.

The set can be useful, e.g., for identifying SNP's, or identifying specific alleles of 1983, 52881, 2398, 45449, 50289 or 52872. In a preferred embodiment, each oligonucleotide of the set has a different nucleotide at an interrogation position. In one embodiment, the set includes two oligonucleotides, each complementary to a different allele at a locus, e.g., a biallelic or polymorphic, locus.

In another embodiment, the set includes four oligonucleotides, each having a different nucleotide (e.g., adenine, guanine, cytosine, or thymidine) at the interrogation position. The interrogation position can be a SNP or the site of a mutation. In another preferred embodiment, the oligonucleotides of the plurality are identical in sequence to one another (except for differences in length). The oligonucleotides can be provided with differential labels, such that an oligonucleotide that hybridizes to one allele provides a signal that is distinguishable from an oligonucleotide that hybridizes to a second allele. In still another embodiment, at least one of the oligonucleotides of the set has a nucleotide change at a position in addition to a query position, e.g., a destabilizing mutation to decrease the Tₘ of the oligonucleotide. In another embodiment, at least one oligonucleotide of the set has a non-natural nucleotide, e.g., inosine. In a preferred embodiment, the oligonucleotides are attached to a solid support, e.g., to different addresses of an array or to different beads or nanoparticles.

In a preferred embodiment the set of oligo nucleotides can be used to specifically amplify, e.g., by PCR, or detect, a 1983, 52881, 2398, 45449, 50289 or 52872 nucleic acid.

The methods described herein may be performed, for example, by utilizing prepackaged diagnostic kits comprising at least one probe nucleic acid or antibody reagent described herein, which may be conveniently used, e.g., in clinical settings to diagnose patients exhibiting symptoms or family history of a disease or illness involving a 1983, 52881, 2398, 45449, 50289 or 52872 gene.

### Use of 1983, 52881, 2399, 45449, 50289, or 52872 Molecules as Surrogate Markers

The 1983, 52881, 2398, 45449, 50289, or 52872 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity ofthe 1983, 52881, 2398, 45449, 50289, or 52872 molecules of the invention may be detected, and may be correlated with one or more biological states in vivo. For example, the 1983, 52881, 2398, 45449, 50289, or 52872 molecules of the invention may serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (e.g., with the presence or absence of a tumor). The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies (e.g., early stage tumors), or when an assessment of disease progression is desired before a potentially dangerous clinical endpoint is reached (e.g., an assessment of cardiovascular disease may be made using cholesterol levels as a surrogate marker, and an analysis of HIV infection may be made using HIV RNA levels as a surrogate marker, well in advance of the undesirable clinical outcomes of myocardial infarction or fully-developed AIDS). Examples of the use of surrogate markers in the art include: Koomen et al. (2000) J. Mass. Spectrom. 35: 258-264; and James (1994) AIDS Treatment News Archive 209.

The 1983, 52881, 2398, 45449, 50289, or 52872 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug in vivo. Pharmacodynamic markers are of particular use in increasing the sensitivity of detection of drug effects, particularly when the drug is administered in low doses. Since even a small amount of a drug may be sufficient to activate multiple rounds of marker (e.g., a 1983, 52881, 2398, 45449, 50289, or 52872 marker) transcription or expression, the amplified marker may be in a quantity which is more readily detectable than the drug itself. Also, the marker may be more easily detected due to the nature of the marker itself; for example, using the methods described herein, anti-1983, 52881, 2398, 45449, 50289, or 52872 antibodies may be employed in an immune-based detection system for a 1983, 52881, 2398, 45449, 50289, or 52872 protein marker, or 1983, 52881, 2398, 45449, 50289, or 52872-specific radiolabeled probes may be used to detect a 1983, 52881, 2398, 45449, 50289, or 52872 mRNA marker. Furthermore, the use of a pharmacodynamic marker may offer mechanism-based prediction of risk due to drug treatment beyond the range of possible direct observations. Examples of the use of pharmacodynamic markers in the art include: Matsuda et al. US 6,033,862; Hattis et al. (1991) Env. Health Perspect. 90: 229-238; Schentag (1999) Am. J. Health-Syst. Pharm. 56 Suppl. 3: S21-S24; and Nicolau (1999) Am, J. Health-Syst. Pharm. 56 Suppl. 3: S 16-S20.

The 1983, 52881, 2398, 45449, 50289, or 52872 molecules of the invention are also useful as pharmacogenomic markers. As used herein, a "pharmacogenomic marker" is an objective biochemical marker which correlates with a specific clinical drug response or susceptibility in a subject (see, e.g., McLeod et al. (1999) Eur. J. Cancer 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, may be selected. For example, based on the presence or quantity of RNA, or protein (e.g., 1983, 52881, 2398, 45449, 50289, or 52872 protein or RNA) for specific tumor markers in a subject, a drug or course of treatment may be selected that is optimized for the treatment of the specific tumor likely to be present in the subject. Similarly, the presence or absence of a specific sequence mutation in 1983, 52881, 2398, 45449, 50289, or 52872 DNA may correlate 1983, 52881, 2398, 45449, 50289, or 52872 drug response. The use of pharmacogenomic markers therefore permits the application of the most appropriate treatment for each subject without having to administer the therapy.

### Pharmaceutical Composition

The nucleic acid and polypeptides, fragments thereof, as well as anti-1983, 52881, 2398, 45449, 50289, or 52872 antibodies (also referred to herein as "active compounds") of the invention can be incorporated into pharmaceutical compositions. Such compositions typically include the nucleic acid molecule, protein, or antibody and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank et al. ((1997) J. Acquired Immune Deficiency Syndromes and Human Retrovirology 14:193*).*

The present invention encompasses agents which modulate expression or activity. An agent may, for example, be a small molecule. For example, such small molecules include, but are not limited to, peptides, peptidomimetics (e.g., peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet ofthe subject, the time of administration, the route of administration, the rate of excretion, any drug combination, and the degree of expression or activity to be modulated.

An antibody (or fragment thereof) may be conjugated to a therapeutic moiety such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-delrydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### Methods of Treatment:

The present invention provides for both prophylactic and therapeutic methods of treating a subject at risk of (or susceptible to) a disorder or having a disorder associated with aberrant or unwanted 1983, 52881, 2398, 45449, 50289, or 52872 expression or activity. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides.

With regards to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 1983, 52881, 2398, 45449, 50289, or 52872 molecules of the present invention or 1983, 52881, 2398, 45449, 50289, or 52872 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

In one aspect, the invention provides a method for preventing in a subject, a disease or condition associated with an aberrant or unwanted 1983, 52881, 2398, 45449, 50289, or 52872 expression or activity, by administering to the subject a 1983, 52881, 2398, 45449, 50289, or 52872 or an agent which modulates 1983, 52881, 2398, 45449, 50289, or 52872 expression or at least one 1983, 52881, 2398, 45449, 50289, or 52872 activity. Subjects at risk for a disease which is caused or contributed to by aberrant or unwanted 1983, 52881, 2398, 45449, 50289, or 52872 expression or activity can be identified by, for example, any or a combination of diagnostic or prognostic assays as described herein. Administration of a prophylactic agent can occur prior to the manifestation of symptoms characteristic of the 1983, 52881, 2398, 45449, 50289, or 52872 aberrance, such that a disease or disorder is prevented or, alternatively, delayed in its progression. Depending on the type of 1983, 52881,2398, 45449, 50289, or 52872 aberrance, for example, a 1983, 52881, 2398, 45449, 50289, or 52872, 1983, 52881, 2398, 45449, 50289, or 52872 agonist or 1983, 52881, 2398, 45449, 50289, or 52872 antagonist agent can be used for treating the subject. The appropriate agent can be determined based on screening assays described herein.

It is possible that some 1983, 52881, 2398, 45449, 50289, or 52872 disorders can be caused, at least in part, by an abnormal level of gene product, or by the presence of a gene product exhibiting abnormal activity. As such, the reduction in the level and/or activity of such gene products would bring about the amelioration of disorder symptoms.

As discussed, successful treatment of 1983, 52881, 2398, 45449, 50289, or 52872 disorders can be brought about by techniques that serve to inhibit the expression or activity of target gene products. For example, compounds, e.g., an agent identified using an assays described above, that proves to exhibit negative modulatory activity, can be used in accordance with the invention to prevent and/or ameliorate symptoms of 1983, 52881, 2398, 45449, 50289, or 52872 disorders. Such molecules can include, but are not limited to peptides, phosphopeptides, small organic or inorganic molecules, or antibodies (including, for example, polyclonal, monoclonal, humanized, anti-idiotypic, chimeric or single chain antibodies, and Fab, F(ab')₂ and Fab expression library fragments, scFV molecules, and epitope-binding fragments thereof.

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to co-administer normal target gene protein into the cell or tissue in order to maintain the requisite level of cellular or tissue target gene activity.

Another method by which nucleic acid molecules may be utilized in treating or preventing a disease characterized by 1983, 52881, 2398, 45449, 50289, or 52872 expression is through the use of aptamer molecules specific for 1983, 52881, 2398, 45449, 50289, or 52872 protein. Aptamers are nucleic acid molecules having a tertiary structure which permits them to specifically bind to protein ligands (see, e.g., Osborne, et al. (1997) Curr. Opin. Chem Biol. 1: 5-9; and Patel, D.J. (1997) Curr Opin Chem Biol 1:32-46). Since nucleic acid molecules may in many cases be more conveniently introduced into target cells than therapeutic protein molecules may be, aptamers offer a method by which 1983, 52881, 2398, 45449, 50289, or 52872 protein activity may be specifically decreased without the introduction of drugs or other molecules which may have pluripotent effects.

Antibodies can be generated that are both specific for target gene product and that reduce target gene product activity. Such antibodies may, therefore, by administered in instances whereby negative modulatory techniques are appropriate for the treatment of 1983, 52881, 2398, 45449, 50289, or 52872 disorders. For a description of antibodies, see the Antibody section above.

In circumstances wherein injection of an animal or a human subject with a 1983, 52881, 2398, 45449, 50289, or 52872 protein or epitope for stimulating antibody production is harmful to the subject, it is possible to generate an immune response against 1983, 52881, 2398, 45449, 50289, or 52872 through the use of anti-idiotypic antibodies (see, for example, Herlyn, D. (1999) Ann Med 31:66-78; and Bhattacharya-Chatterjee, M., and Foon, K.A. (1998) Cancer Treat Res. 94:51-68). If an anti-idiotypic antibody is introduced into a mammal or human subject, it should stimulate the production of anti-anti-idiotypic antibodies, which should be specific to the 1983, 52881, 2398, 45449, 50289, or 52872 protein. Vaccines directed to a disease characterized by 1983, 52881, 2398, 45449, 50289, or 52872 expression may also be generated in this fashion.

In instances where the target antigen is intracellular and whole antibodies are used, internalizing antibodies may be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see e.g., Marasco et al. (1993) Proc. Natl. Acad. Sci. USA 90:7889-7893).

The identified compounds that inhibit target gene expression, synthesis and/or activity can be administered to a patient at therapeutically effective doses to prevent, treat or ameliorate 1983, 52881, 2398, 45449, 50289, or 52872 disorders. A therapeutically effective dose refers to that amount of the compound sufficient to result in amelioration of symptoms of the disorders. Toxicity and therapeutic efficacy of such compounds can be determined by standard pharmaceutical procedures as described above.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of determination of effective dose for an individual is the ability to directly assay levels of "free" and "bound" compound in the serum of the test subject. Such assays may utilize antibody mimics and/or "biosensors" that have been created through molecular imprinting techniques. The compound which is able to modulate 1983, 52881, 2398, 45449, 50289, or 52872 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell, R. J. et al (1996) Current Opinion in Biotechnology 7:89-94 and in Shea, K.J. (1994) Trends in Polymer Science 2:166-173. Such "imprinted" affinity matrixes are amenable to ligand-binding assays, whereby the immobilized monoclonal antibody component is replaced by an appropriately imprinted matrix. An example of the use of such matrixes in this way can be seen in Vlatakis, G. et al (1993) Nature 361:645-647. Through the use of isotope-labeling, the "free" concentration of compound which modulates the expression or activity of 1983, 52881, 2398, 45449, 50289, or 52872 can be readily monitored and used in calculations of IC₅₀.

Such "imprinted" affinity matrixes can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. An rudimentary example of such a "biosensor" is discussed in Kriz, D. et al (1995) Analytical Chemistry 67:2142-2144.

Another aspect of the invention pertains to methods of modulating 1983, 52881, 2398, 45449, 50289, or 52872 expression or activity for therapeutic purposes. Accordingly, in an exemplary embodiment, the modulatory method of the invention involves contacting a cell with a 1983, 52881, 2398, 45449, 50289, or 52872 or agent that modulates one or more of the activities of 1983, 52881, 2398, 45449, 50289, or 52872 protein activity associated with the cell. An agent that modulates 1983, 52881, 2398, 45449, 50289, or 52872 protein activity can be an agent as described herein, such as a nucleic acid or a protein, a naturally-occurring target molecule of a 1983, 52881, 2398, 45449, 50289, or 52872 protein (e.g., a 1983, 52881, 2398, 45449, 50289, or 52872 substrate or receptor), a 1983, 52881, 2398, 45449, 50289, or 52872 antibody, a 1983, 52881, 2398, 45449, 50289, or 52872 agonist or antagonist, a peptidomimetic of a 1983, 52881, 2398, 45449, 50289, or 52872 agonist or antagonist, or other small molecule.

In one embodiment, the agent stimulates one or 1983, 52881, 2398, 45449, 50289, or 52872 activities. Examples of such stimulatory agents include active 1983, 52881, 2398, 45449, 50289, or 52872 protein and a nucleic acid molecule encoding 1983, 52881, 2398, 45449, 50289, or 52872. In another embodiment, the agent inhibits one or more 1983, 52881, 2398, 45449, 50289, or 52872 activities. Examples of such inhibitory agents include antisense 1983, 52881, 2398, 45449, 50289, or 52872 nucleic acid molecules, anti1983, 52881, 2398, 45449, 50289, or 52872 antibodies, and 1983, 52881, 2398, 45449, 50289, or 52872 inhibitors. These modulatory methods can be performed *in vitro* (e.g., by culturing the cell with the agent) or, alternatively, *in vivo* (e.g., by administering the agent to a subject). As such, the present invention provides methods of treating an individual afflicted with a disease or disorder characterized by aberrant or unwanted expression or activity of a 1983, 52881, 2398, 45449, 50289, or 52872 protein or nucleic acid molecule. In one embodiment, the method involves administering an agent (e.g., an agent identified by a screening assay described herein), or combination of agents that modulates (e.g., up regulates or down regulates) 1983, 52881, 2398, 45449, 50289, or 52872 expression or activity. In another embodiment, the method involves administering a 1983, 52881, 2398, 45449, 50289, or 52872 protein or nucleic acid molecule as therapy to compensate for reduced, aberrant, or unwanted 1983, 52881, 2398, 45449, 50289, or 52872 expression or activity.

Stimulation of 1983, 52881, 2398, 45449, 50289, or 52872 activity is desirable in situations in which 1983, 52881, 2398, 45449, 50289, or 52872 is abnormally downregulated and/or in which increased 1983, 52881, 2398, 45449, 50289, or 52872 activity is likely to have a beneficial effect. For example, stimulation of 1983, 52881, 2398, 45449, 50289, or 52872 activity is desirable in situations in which a 1983, 52881, 2398, 45449, 50289, or 52872 is downregulated and/or in which increased 1983, 52881, 2398, 45449, 50289, or 52872 activity is likely to have a beneficial effect. Likewise, inhibition of 1983, 52881, 2398, 45449, 50289, or 52872 activity is desirable in situations in which 1983, 52881, 2398, 45449, 50289, or 52872 is abnormally upregulated and/or in which decreased 1983, 52881, 2398, 45449, 50289, or 52872 activity is likely to have a beneficial effect.

### Pharmacogenomics

The 1983, 52881, 2398, 45449, 50289, or 52872 molecules of the present invention, as well as agents, or modulators which have a stimulatory or inhibitory effect on 1983, 52881, 2398, 45449, 50289, or 52872 activity (e.g., 1983, 52881, 2398, 45449, 50289, or 52872 gene expression) as identified by a screening assay described herein can be administered to individuals to treat (prophylactically or therapeutically) 1983, 52881, 2398, 45449, 50289, or 52872 associated disorders (e.g., a cardiovascular disorder) associated with aberrant or unwanted 1983, 52881, 2398, 45449, 50289, or 52872 activity. In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a 1983, 52881, 2398, 45449, 50289, or 52872 molecule or 1983, 52881, 2398, 45449, 50289, or 52872 modulator as well as tailoring the dosage and/or therapeutic regimen of treatment with a 1983, 52881, 2398, 45449, 50289, or 52872 molecule or 1983, 52881, 2398, 45449, 50289, or 52872 modulator.

Pharmacogenomics deals with clinically significant hereditary variations in the response to drugs due to altered drug disposition and abnormal action in affected persons. See, for example, Eichelbaum, M. et al. (1996) Clin. Exp. Pharmacol. Physiol. 23:983-985 and Linder, M.W. et al. (1997) Clim. Chem. 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individual, taking into account traits that may be common among such genetically similar individuals.

Alternatively, a method termed the "candidate gene approach", can be utilized to identify genes that predict drug response. According to this method, if a gene that encodes a drug's target is known (e.g., a 1983, 52881, 2398, 45449, 50289, or 52872 protein of the present invention), all common variants of that gene can be fairly easily identified in the population and it can be determined if having one version of the gene versus another is associated with a particular drug response.

Alternatively, a method termed the "gene expression profiling", can be utilized to identify genes that predict drug response. For example, the gene expression of an animal dosed with a drug (e.g., a 1983, 52881, 2398, 45449, 50289, or 52872 molecule or 1983, 52881, 2398, 45449, 50289, or 52872 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 1983, 52881, 2398, 45449, 50289, or 52872 molecule or 1983, 52881, 2398, 45449, 50289, or 52872 modulator, such as a modulator identified by one of the exemplary screening assays described herein.

The present invention further provides methods for identifying new agents, or combinations, that are based on identifying agents that modulate the activity of one or more of the gene products encoded by one or more ofthe 1983, 52881, 2398, 45449, 50289, or 52872 genes of the present invention, wherein these products may be associated with resistance of the cells to a therapeutic agent. Specifically, the activity of the proteins encoded by the 1983, 52881, 2398, 45449, 50289, or 52872 genes of the present invention can be used as a basis for identifying agents for overcoming agent resistance. By blocking the activity of one or more of the resistance proteins, target cells, e.g., human cells, will become sensitive to treatment with an agent that the unmodified target cells were resistant to.

Monitoring the influence of agents (e.g., drugs) on the expression or activity of a 1983, 52881, 2398, 45449, 50289, or 52872 protein can be applied in clinical trials. For example, the effectiveness of an agent determined by a screening assay as described herein to increase 1983, 52881, 2398, 45449, 50289, or 52872 gene expression, protein levels, or upregulate 1983, 52881, 2398, 45449, 50289, or 52872 activity, can be monitored in clinical trials of subjects exhibiting decreased 1983, 52881, 2398, 45449, 50289, or 52872 gene expression, protein levels, or downregulated 1983, 52881, 2398, 45449, 50289, or 52872 activity. Alternatively, the effectiveness of an agent determined by a screening assay to decrease 1983, 52881, 2398, 45449, 50289, or 52872 gene expression, protein levels, or downregulate 1983, 52881, 2398, 45449, 50289, or 52872 activity, can be monitored in clinical trials of subjects exhibiting increased 1983, 52881, 2398, 45449, 50289, or 52872 gene expression, protein levels, or upregulated 1983, 52881, 2398, 45449, 50289, or 52872 activity. In such clinical trials, the expression or activity of a 1983, 52881, 2398, 45449, 50289, or 52872 gene, and preferably, other genes that have been implicated in, for example, a 1983, 52881, 2398, 45449, 50289, or 52872-associated disorder can be used as a "read out" or markers of the phenotype of a particular cell.

### Informatics

The sequence of a 1983, 52881, 2398, 45449, 50289 or 52872 molecule is provided in a variety of media to facilitate use thereof. A sequence can be provided as a manufacture, other than an isolated nucleic acid or amino acid molecule, which contains a 1983, 52881, 2398, 45449, 50289 or 52872. Such a manufacture can provide a nucleotide or amino acid sequence, e.g., an open reading frame, in a form which allows examination of the manufacture using means not directly applicable to examining the nucleotide or amino acid sequences, or a subset thereof, as they exists in nature or in purified form. The sequence information can include, but is not limited to, 1983, 52881,2398, 45449, 50289 or 52872 full-length nucleotide and/or amino acid sequences, partial nucleotide and/or amino acid sequences, polymorphic sequences including single nucleotide polymorphisms (SNPs), epitope sequence, and the like. In a preferred embodiment, the manufacture is a machine-readable medium, e.g., a magnetic, optical, chemical or mechanical information storage device. As used herein, "machine-readable media" refers to any medium that can be read and accessed directly by a machine, e.g., a digital computer or analogue computer. Non-limiting examples of a computer include a desktop PC, laptop, mainframe, server (e.g., a web server, network server, or server farm), handheld digital assistant, pager, mobile telephone, and the like. The computer can be stand-alone or connected to a communications network, e.g., a local area network (such as a VPN or intranet), a wide area network (e.g., an Extranet or the Internet), or a telephone network (e.g., a wireless, DSL, or ISDN network).

Machine-readable media include, but are not limited to: magnetic storage media, such as floppy discs, hard disc storage medium, and magnetic tape; optical storage media such as CD-ROM; electrical storage media such as RAM, ROM, EPROM, EEPROM, flash memory, and the like; and hybrids of these categories such as magnetic/optical storage media.

A variety of data storage structures are available to a skilled artisan for creating a machine-readable medium having recorded thereon a nucleotide or amino acid sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. The skilled artisan can readily adapt any number of data processor structuring formats (e.g., text file or database) in order to obtain computer readable medium having recorded thereon the nucleotide sequence information of the present invention.

In a preferred embodiment, the sequence information is stored in a relational database (such as Sybase or Oracle). The database can have a first table for storing sequence (nucleic acid and/or amino acid sequence) information. The sequence information can be stored in one field (e.g., a first column) of a table row and an identifier for the sequence can be store in another field (e.g., a second column) of the table row. The database can have a second table, e.g., storing annotations. The second table can have a field for the sequence identifier, a field for a descriptor or annotation text (e.g., the descriptor can refer to a functionality of the sequence, a field for the initial position in the sequence to which the annotation refers, and a field for the ultimate position in the sequence to which the annotation refers. Non-limiting examples for annotation to nucleic acid sequences include polymorphisms (e.g., SNP's) translational regulatory sites and splice junctions. Non-limiting examples for annotations to amino acid sequence include polypeptide domains, e.g., a domain described herein; active sites and other functional amino acids; and modification sites.

By providing the nucleotide or amino acid sequences of the invention in computer readable form, the skilled artisan can routinely access the sequence information for a variety of purposes. For example, one skilled in the art can use the nucleotide or amino acid sequences of the invention in computer readable form to compare a target sequence or target structural motif with the sequence information stored within the data storage means. A search is used to identify fragments or regions of the sequences of the invention which match a particular target sequence or target motif. The search can be a BLAST search or other routine sequence comparison, e.g., a search described herein.

Thus, in one aspect, the invention features a method of analyzing 1983, 52881, 2398, 45449, 50289 or 52872, e.g., analyzing structure, function, or relatedness to one or more other nucleic acid or amino acid sequences. The method includes: providing a 1983, 52881, 2398, 45449, 50289 or 52872 nucleic acid or amino acid sequence; comparing the 1983, 52881, 2398, 45449, 50289 or 52872 sequence with a second sequence, e.g., one or more preferably a plurality of sequences from a collection of sequences, e.g., a nucleic acid or protein sequence database to thereby analyze 1983, 52881, 2398, 45449, 50289 or 52872. The method can be performed in a machine, e.g., a computer, or manually by a skilled artisan.

The method can include evaluating the sequence identity between a 1983, 52881, 2398, 45449, 50289 or 52872 sequence and a database sequence. The method can be performed by accessing the database at a second site, e.g., over the Internet.

As used herein, a "target sequence" can be any DNA or amino acid sequence of six or more nucleotides or two or more amino acids. A skilled artisan can readily recognize that the longer a target sequence is, the less likely a target sequence will be present as a random occurrence in the database. Typical sequence lengths of a target sequence are from about 10 to 100 amino acids or from about 30 to 300 nucleotide residues. However, it is well recognized that commercially important fragments, such as sequence fragments involved in gene expression and protein processing, may be of shorter length.

Computer software is publicly available which allows a skilled artisan to access sequence information provided in a computer readable medium for analysis and comparison to other sequences. A variety of known algorithms are disclosed publicly and a variety of commercially available software for conducting search means are and can be used in the computer-based systems of the present invention. Examples of such software include, but are not limited to, MacPattern (EMBL), BLASTN and BLASTX (NCBI).

Thus, the invention features a method of making a computer readable record of a sequence of a 1983, 52881, 2398, 45449, 50289 or 52872 sequence which includes recording the sequence on a computer readable matrix. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention features a method of analyzing a sequence. The method includes: providing a 1983, 52881, 2398, 45449, 50289 or 52872 sequence, or record, in machine-readable form; comparing a second sequence to the 1983, 52881, 2398, 45449, 50289 or 52872 sequence; thereby analyzing a sequence. Comparison can include comparing to sequences for sequence identity or determining if one sequence is included within the other, e.g., determining if the 1983, 52881, 2398, 45449, 50289 or 52872 sequence includes a sequence being compared. In a preferred embodiment the 1983, 52881, 2398, 45449, 50289 or 52872 or second sequence is stored on a first computer, e.g., at a first site and the comparison is performed, read, or recorded on a second computer, e.g., at a second site. E.g., the 1983, 52881, 2398, 45449, 50289 or 52872 or second sequence can be stored in a public or proprietary database in one computer, and the results of the comparison performed, read, or recorded on a second computer. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention provides a machine-readable medium for holding instructions for performing a method for determining whether a subject has a 1983, 52881, 2398, 45449, 50289 or 52872-associated disease or disorder or a pre-disposition to a 1983, 52881, 2398, 45449, 50289 or 52872-associated disease or disorder, wherein the method comprises the steps of determining 1983, 52881, 2398, 45449, 50289 or 52872 sequence information associated with the subject and based on the 1983, 52881, 2398, 45449, 50289 or 52872 sequence information, determining whether the subject has a 1983, 52881, 2398, 45449, 50289 or 52872-associated disease or disorder or a pre-disposition to a 1983, 52881, 2398, 45449, 50289 or 52872-associated disease or disorder and/or recommending a particular treatment for the disease, disorder or pre-disease condition.

The invention further provides in an electronic system and/or in a network, a method for determining whether a subject has a 1983, 52881, 2398, 45449, 50289 or 52872-associated disease or disorder or a pre-disposition to a disease associated with a 1983, 52881, 2398, 45449, 50289 or 52872 wherein the method comprises the steps of determining 1983, 52881, 2398, 45449, 50289 or 52872 sequence information associated with the subject, and based on the 1983, 52881, 2398, 45449, 50289 or 52872 sequence information, determining whether the subject has a 1983, 52881, 2398, 45449, 50289 or 52872-associated disease or disorder or a pre-disposition to a 1983, 52881, 2398, 45449, 50289 or 52872-associated disease or disorder, and/or recommending a particular treatment for the disease, disorder or pre-disease condition. In a preferred embodiment, the method further includes the step of receiving information, e.g., phenotypic or genotypic information, associated with the subject and/or acquiring from a network phenotypic information associated with the subject. The information can be stored in a database, e.g., a relational database. In another embodiment, the method further includes accessing the database, e.g., for records relating to other subjects, comparing the 1983, 52881, 2398, 45449, 50289 or 52872 sequence of the subject to the 1983, 52881, 2398, 45449, 50289 or 52872 sequences in the database to thereby determine whether the subject as a 1983, 52881, 2398, 45449, 50289 or 52872-associated disease or disorder, or a pre-disposition for such.

The present invention also provides in a network, a method for determining whether a subject has a 1983, 52881, 2398, 45449, 50289 or 52872 associated disease or disorder or a pre-disposition to a 1983, 52881, 2398, 45449, 50289 or 52872-associated disease or disorder associated with 1983, 52881, 2398, 45449, 50289 or 52872, said method comprising the steps of receiving 1983, 52881, 2398, 45449, 50289 or 52872 sequence information from the subject and/or information related thereto, receiving phenotypic information associated with the subject, acquiring information from the network corresponding to 1983, 52881, 2398, 45449, 50289 or 52872 and/or corresponding to a 1983, 52881, 2398, 45449, 50289 or 52872-associated disease or disorder (e.g., a 1983, 52881, 2398, 45449, 50289 or 52872- mediated disorder as described herein), and based on one or more of the phenotypic information, the 1983, 52881, 2398, 45449, 50289 or 52872 information (*e*.*g*., sequence information and/or information related thereto), and the acquired information, determining whether the subject has a 1983, 52881, 2398, 45449, 50289 or 52872-associated disease or disorder or a pre-disposition to a 1983, 52881, 2398, 45449, 50289 or 52872-associated disease or disorder. The method may further comprise the step of recommending a particular treatment for the disease, disorder or pre-disease condition.

The present invention also provides a method for determining whether a subject has a 1983, 52881, 2398, 45449, 50289 or 52872 -associated disease or disorder or a pre-disposition to a 1983, 52881, 2398, 45449, 50289 or 52872-associated disease or disorder, said method comprising the steps of receiving information related to 1983, 52881, 2398, 45449, 50289 or 52872 (e.g., sequence information and/or information related thereto), receiving phenotypic information associated with the subject, acquiring information from the network related to 1983, 52881, 2398, 45449, 50289 or 52872 and/or related to a 1983, 52881, 2398, 45449, 50289 or 52872-associated disease or disorder, and based on one or more of the phenotypic information, the 1983, 52881, 2398, 45449, 50289 or 52872 information, and the acquired information, determining whether the subject has a 1983, 52881, 2398, 45449, 50289 or 52872-associated disease or disorder or a pre-disposition to a 1983, 52881, 2398, 45449, 50289 or 52872-associated disease or disorder. The method may further comprise the step of recommending a particular treatment for the disease, disorder or pre-disease condition.

This invention is further illustrated by the following examples which should not be construed as limiting. The contents of all references, patents and published patent applications cited throughout this application are incorporated herein by reference.

### EXAMPLES

### Example 1: Identification and Characterization of Human 1983, 52881, 2398, 45449, 50289, and 52872 cDNAs

The human 1983 nucleotide sequence (Figures 1A-1D; SEQ ID NO:1), which is approximately 3127 nucleotides long including untranslated regions, contains a predicted methionine-initiated coding sequence of about 1938 nucleotides, including the termination codon (nucleotides indicated as coding of SEQ ID NO:1 in Figures 1A-1D; SEQ ID NO:3). The coding sequence encodes a 645 amino acid protein (Figure 2; SEQ ID NO:2).

The human 52881 sequence (Figures 6A-6D; SEQ ID NO:4), which is approximately 4238 nucleotides long including untranslated regions, contains a predicted methionine-initiated coding sequence of about 1830 nucleotides, including the termination codon (nucleotides indicated as coding of SEQ ID NO:4 in Figures 6A-6D; SEQ ID NO:6). The coding sequence encodes a 609 amino acid protein (Figure 7; SEQ ID NO:5).

The human 2398 nucleotide sequence (Figures 9A-9B; SEQ ID NO:7), which is approximately 1113 nucleotides long including untranslated regions, contains a predicted methionine-initiated coding sequence of about 1053 nucleotides, including the termination codon (nucleotides indicated as coding of SEQ ID NO:7 in Figures 9A-9B; SEQ ID NO:9). The coding sequence encodes a 350 amino acid protein (Figure 10; SEQ ID NO:8).

The human 45449 nucleotide sequence (Figures 12A-12B; SEQ ID NO:10), which is approximately 1109 nucleotides long including untranslated regions, contains a predicted methionine-initiated coding sequence of about 672 nucleotides, including the termination codon (nucleotides indicated as coding of SEQ ID NO:10 in Figures 12A-12B; SEQ ID NO:12). The coding sequence encodes a 223 amino acid protein (Figure 13; SEQ ID NO:11).

The human 50289 nucleotide sequence (Figures 15A-15E; SEQ ID NO:13), which is approximately 3489 nucleotides long including untranslated regions, contains a predicted methionine-initiated coding sequence of about 2559 nucleotides, including the termination codon (nucleotides indicated as coding of SEQ ID NO:13 in Figures 15A-15E; SEQ ID NO:15). The coding sequence encodes a 852 amino acid protein (Figure 16; SEQ ID NO:14).

The human 52872 sequence (Figures 18A-18B; SEQ ID NO:16), which is approximately 1609 nucleotides long including untranslated regions, contains a predicted methionine-initiated coding sequence of about 1197 nucleotides, including the termination codon (nucleotides indicated as coding of SEQ ID NO:16 in Figures 18A-18B; SEQ ID NO:18). The coding sequence encodes a 398 amino acid protein (Figure 19; SEQ ID NO:17).

### Example 2: Tissue Distribution of 52872 mRNA

Endogenous human 52872 gene expression was determined using the Perkin-Elmer/ABI 7700 Sequence Detection System which employs TaqMan technology. Briefly, TaqMan technology relies on standard RT-PCR with the addition of a third gene-specific oligonucleotide (referred to as a probe) which has a fluorescent dye coupled to its 5' end (typically 6-FAM) and a quenching dye at the 3' end (typically TAMRA). When the fluorescently tagged oligonucleotide is intact, the fluorescent signal from the 5' dye is quenched. As PCR proceeds, the 5' to 3' nucleolytic activity of Taq polymerase digests the labeled primer, producing a free nucleotide labeled with 6-FAM, which is now detected as a fluorescent signal. The PCR cycle where fluorescence is first released and detected is directly proportional to the starting amount of the gene of interest in the test sample, thus providing a way of quantitating the initial template concentration. Samples can be internally controlled by the addition of a second set of primers/probe specific for a housekeeping gene such as GAPDH which has been labeled with a different fluorophore on the 5' end (typically VIC).

To determine the level of 52872 in various human tissues a primer/probe set was designed using Primer Express (Perkin-Elmer) software and primary cDNA sequence information. Total RNA was prepared from a series of human tissues using an RNeasy kit from Qiagen. First strand cDNA was prepared from 1 µg total RNA using an oligo-dT primer and Superscript II reverse transcriptase (Gibco/BRL). cDNA obtained from approximately 50 ng total RNA was used per TaqMan reaction. 52872 mRNA levels were analyzed in a variety of samples of human tissues, and in rodent models of pain response.

Figure 21 shows relative 52872 expression in mRNA derived from human tissue samples. The samples are derived from human adrenal gland, brain, heart, kidney, liver, lung, mammary gland, placenta, prostate, pituitary gland, muscle, small intestine, spleen, stomach, testes, thymus, trachea, uterus, spinal cord, skin, and dorsal root ganglion (DRG). The highest 52872 mRNA expression was observed in brain, placenta, testes, thymus, spinal cord, and DRG.

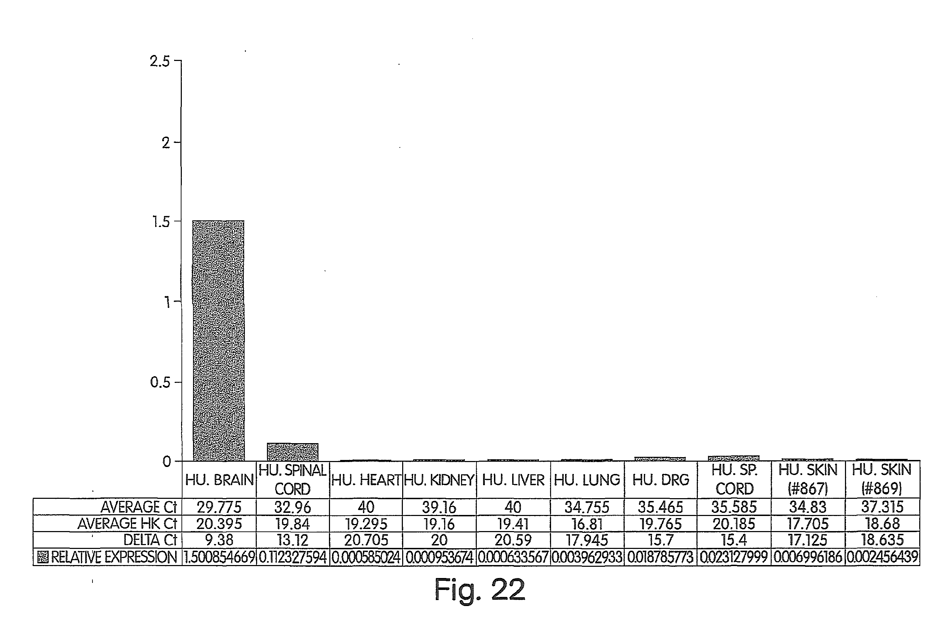

Figure 22 shows relative 52872 expression in mRNA derived from human tissue samples. The samples are derived from human brain, spinal cord, heart, kidney, liver, lung, DRG, spinal cord, and skin. The highest 52872 mRNA expression was observed in brain and spinal cord.

In situ hybridization showed expression of 52872 in the brain cortex, striatum, thalamus, spinal cord, and dorsal horni. Low level expression was detected in a small population of medium size DRG neurons.

Figure 23 shows relative 52872 expression in mRNA derived from monkey and human tissue samples. The monkey samples were derived from cortex, DRG, spinal cord, sciatic nerve, kidney, hairy skin, heart, and liver. The human samples were derived from brain, spinal cord, heart, kidney, liver, and lung. Highest expression in monkey tissues was detected in cortex and spinal cord. Highest expression in human tissues was detected in brain.

Taqman experiments in rodent models of pain response showed that the 52872 gene is regulated in three different pain response models. Figure 24 shows the upregulation of 52872 expression in DRG following CFA injection (28 days), axotomy (7 days), and CCI (7 days). Figure 25 shows the upregulation of 52872 expression in the spinal cord following CFA injection (28 days), axotomy (1-7 days), and CCI (1-14 days).

### Example 3: Expression of 52881 in Endothelial Cells

Human umbilical vein endothelial cells (HUVEC) were gown under a variety of conditions and the levels of 52881 expression were determined by (Figure 26). The relative levels of 52881 mRNA in endothelial cells was determined by microarray hybridization.

In lanes 2-4, HUVEC were cultured on plastic tissue culture plates. The cells were treated as follows: no added growth factor (lane 2); IL-1β added (lane 3); and VEGF added (lane 4). In lanes 5-7, HUVEC were plated and grown on Matrigel (Becton Dickinson). 52881 expression levels were determined at various time points following the plating, each of which represents a stage of vascular-like tube formation that occurs when endothelial cells are cultured on Matrigel: 2 hours after plating (lane 5; early stage of active tube formation); 6 hours after plating (lane 6; active tube formation); and 16 hours after plating (lane 7; late stage of active tube formation). Expression of 52881 in 293 cells (non-endothelial) is depicted in lane 1. As shown in Figure 26, 52881 is expressed in cultured endothelial cells and is down-regulated during the formation of vascular tube-like structures that are induced by plating on Matrigel.

### Example 4: Tissue Distribution of 1983, 2398, 45449, and 50289 mRNA

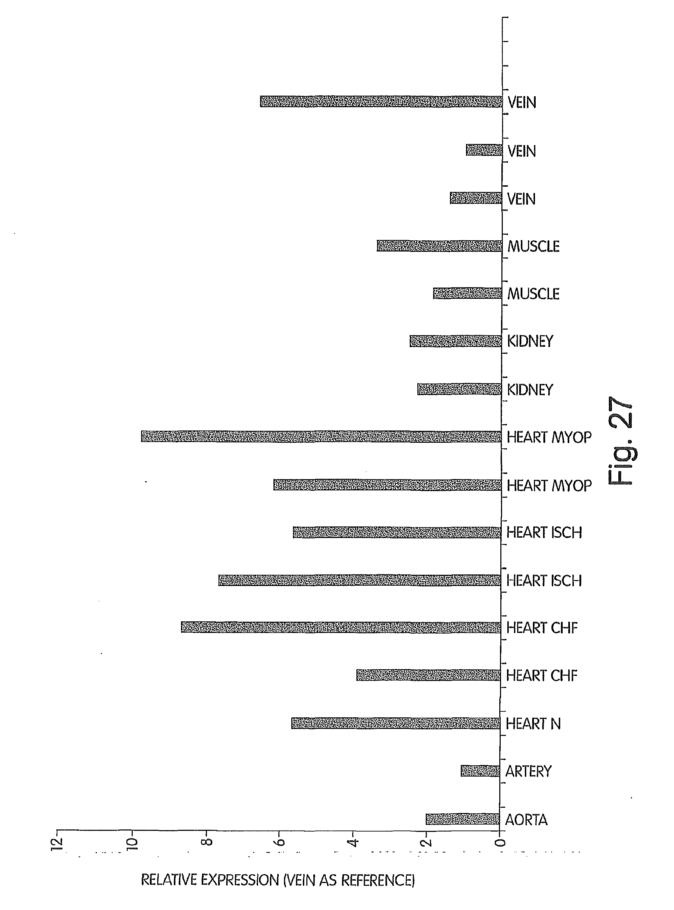

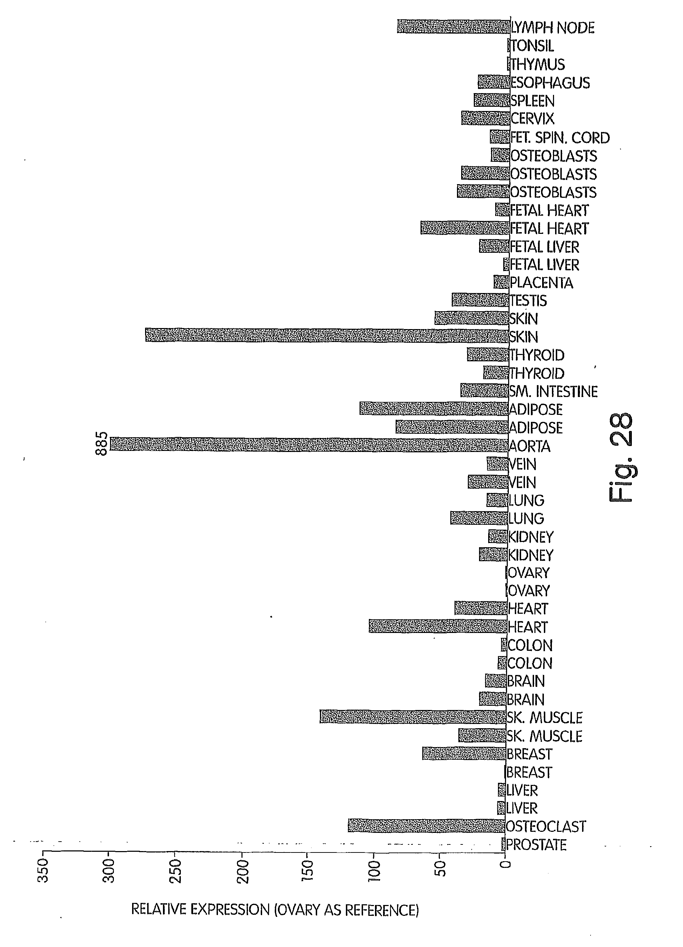

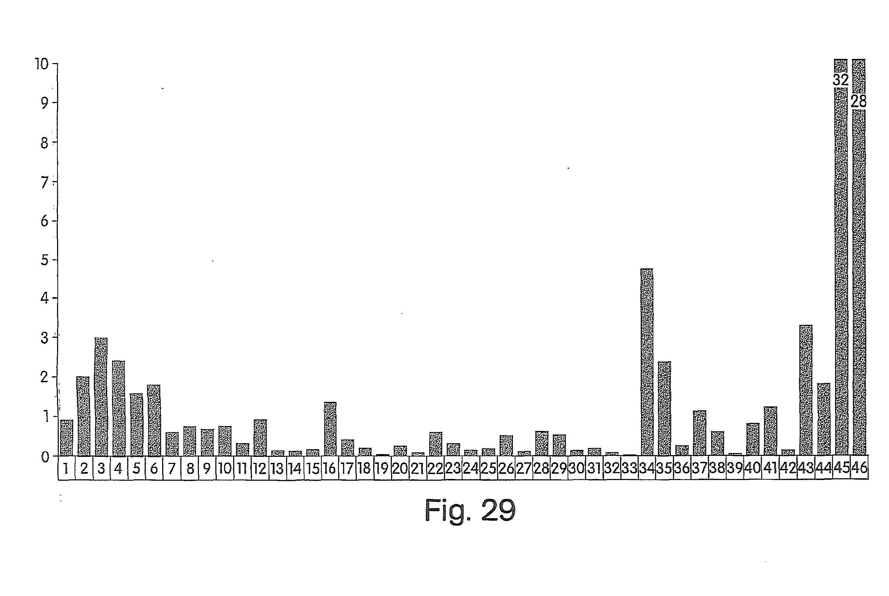

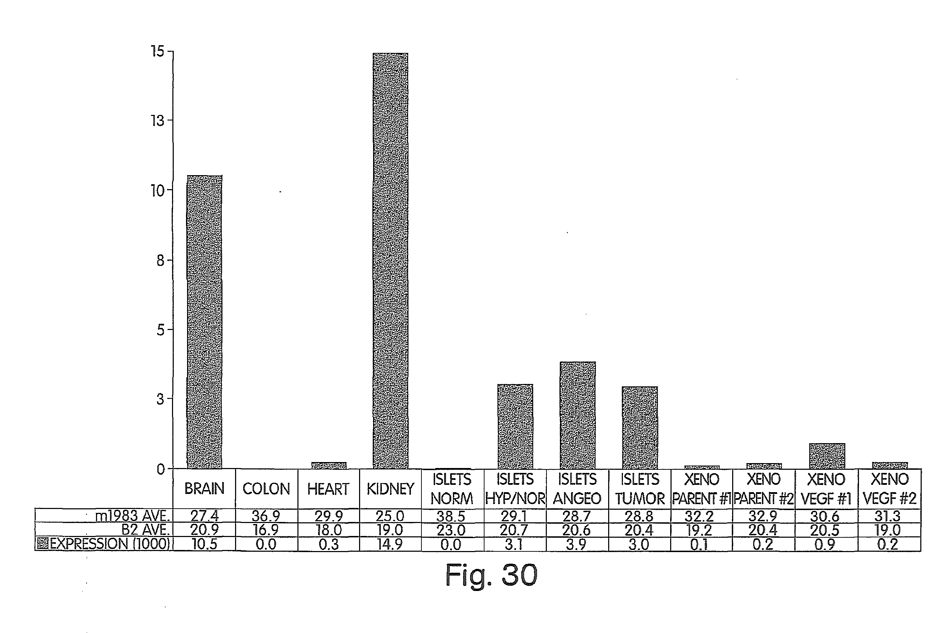

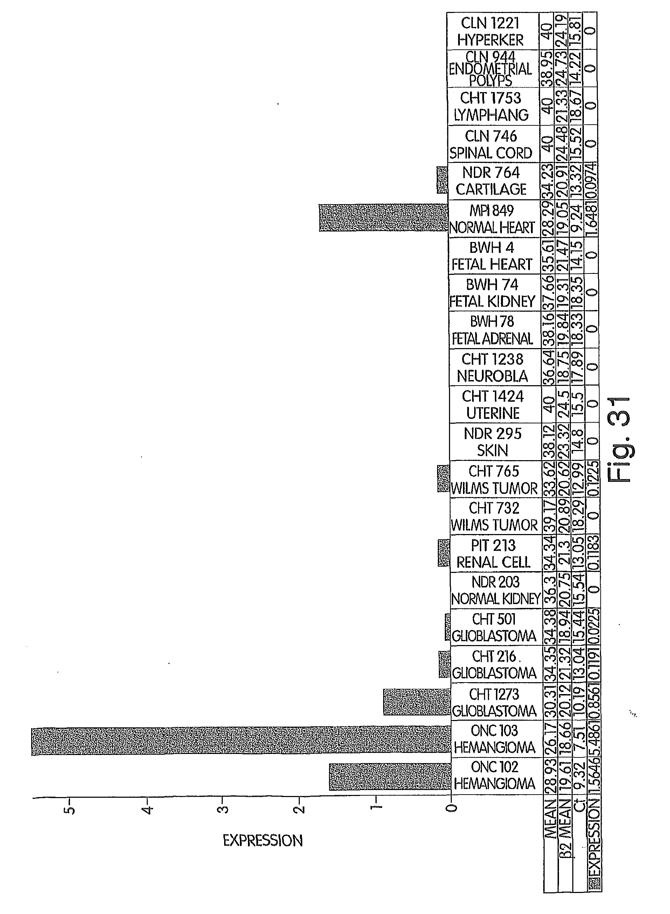

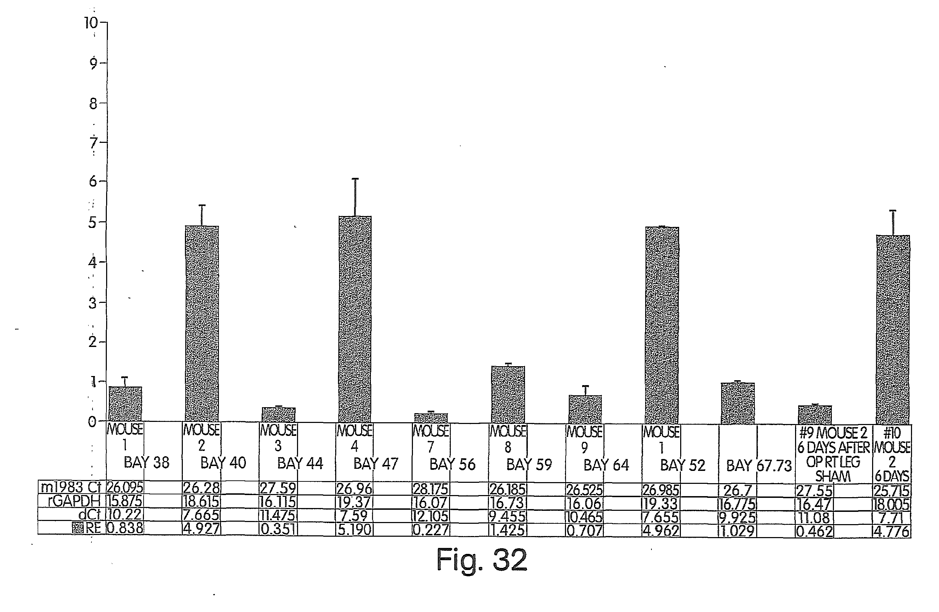

Human 1983 gene expression was evaluated using TaqMan technology as described herein. The 1983 mRNA was expressed in the heart, e.g., the diseased heart (e.g., heart tissue from humans with cardiac myopathy, or congestive heart failure) (Figure 27). The 1983 mRNA was also expressed in blood vessels, e.g., aorta, veins, human umbilical cord vein-derived endothelial cells (HUVEC), human microvascular endothelial cells (HMVEC), and endothelial cells, as well as in the skin (Figure 28 and 29). The tissues examined in Figure 29 are as follows, from left to right: (1) normal aorta; (2) normal fetal heart; (3) normal heart; (4) heart/CHF; (5) normal vein; (6) SMC (aortic); (7) normal spinal cord; (8) normal brain cortex; (9) normal brain hypothalamus; (10) glial cells (astrocytes); (11) brain/glioblastoma; (12) normal breast; (13) breast tumor (IDC); (14) normal ovary; (15) ovary tumor; (16) pancreas; (17) normal prostate; (18) tumor prostate; (19) normal colon; (20) colon tumor; (21) colon (IBD); (22) normal kidney; (23) normal liver; (24) liver fibrosis; (25) normal fetal liver; (26) normal lung; (27) lung tumor; (28) lung (COPD); (29) normal spleen; (30) normal tonsil; (31) normal lymph node; (32) normal thymus; (33) epithelial cells (prostate); (34) endothelial cells (aortic); (35) normal skeletal muscle; (36) fibroblasts (dermal); (37) normal skin; (38) normal adipose; (39) primary osteoblasts; (40) undifferentiated osteoblasts; (41) differentiated osteoblasts; (42) osteoclasts; (43) aorta SMC (early); (44) aorta SMC (late); (45) HYVEC (shear); and (46) HUVEC (static). 1983 mRNA was also found at relatively high levels in the brain and the kidney (Figure 30) and in hemangioma (Figure 31). Figure 32 depicts relative 1983 mRNA levels in the mouse hindlimb.

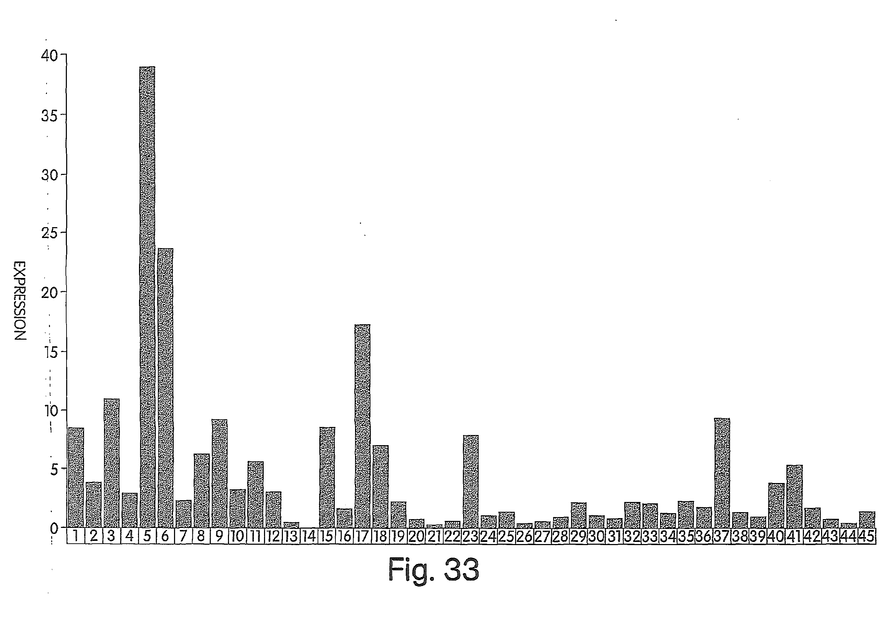

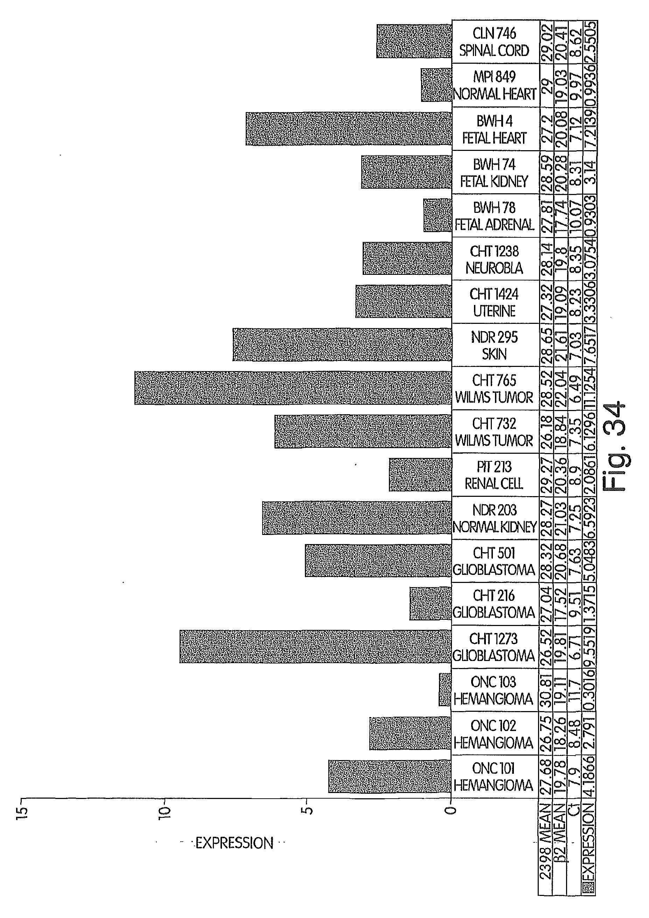

Human 2398 gene expression was evaluated using TaqMan technology as described herein. The 2398 mRNA was expressed in vessels, e.g., static HUVEC, shear HUVEC, as well as in the brain and dermal cells (Figure 33). The tissues examined in Figure 33 are as follows, from left to right: (1) normal artery; (2) normal vein; (3) aortic SMC (early); (4) coronary SMC; (5) static HUVEC; (6) shear HUVEC; (7) normal heart; (8) heart CHF; (9) kidney; (10) skeletal muscle; (11) normal adipose; (12) pancreas; (13) primary osteoblasts; (14) differentiated osteoclasts; (15) normal skin; (16) normal spinal cord; (17) normal brain cortex; (18) brain hypothalamus; (19) nerve; (20) dorsal root ganglion (DRG); (21) resting PBMC; (22) glioblastoma; (23) normal breast; (24) breast tumor; (25) normal ovary; (26) ovary tumor; (27) normal prostate; (28) prostate tumor; (29) normal colon; (30) colon tumor; (31) normal lung; (32) lung tumor; (33) lung COPD; (34) colon IBD; (35) normal liver; (36) liver fibrosis; (37) dermal cells (fibroblasts); (38) normal spleen; (39) normal tonsil; (40) lymph node; (41) small intestine; (42) skin (decubitis); (43) synovium; (44) bone marrow mononuclear cells; and (45) activated PBMC. Figure 34 depicts relative 2398 mRNA levels in tissues and cell samples rich in vascular cells.

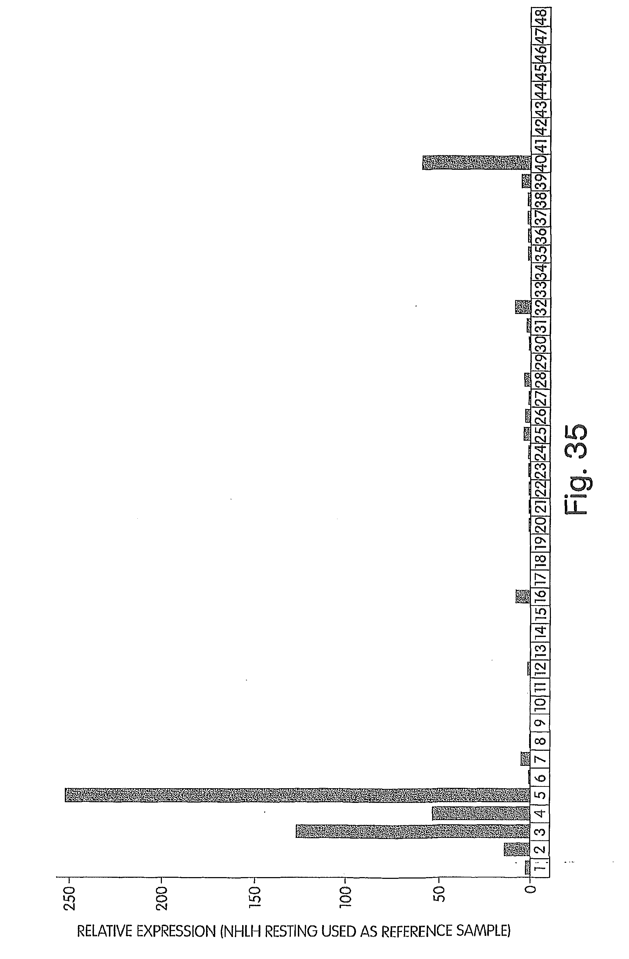

Human 45449 gene expression was evaluated using TaqMan technology as described herein. The 45449 mRNA was expressed in heart and brain, as well as in HepG2-A cells (Figure 35). The tissues examined in Figure 35 are as follows, from left to right: (1) lung; (2) kidney; (3) brain; (4) granulocytes; (5) heart; (6) spleen; (7) fetal liver; (8) pooled liver; (9) NHDF resting; (10) NHLF/CTN 48 hours; (11) NHLF/TGF 48 hours; (12) NHLH resting; (13) NHLF/TGF 48 hours; (14) passage stellates; (15) LF/NDR 190; (16) LF/NDR 191; (17) LF/NDR 194; (18) LF/NDR 113; (19) lymph nodes; (20) tonsils; (21) TH1 24 hours; (22) TH2 24 hours; (23) TH1 24 hours; (24) TH2 24 hours; (25) CD4; (26) CD8; (27) CD14 resting; (28) PBMC mock; (29) CD19; (30) CD3 resting; (31) bone marrow mononuclear cells LP26; (32) mPB CD34+; (33) ABM CD34+; (34) core blood CD34+; (35) erythroid; (36) meg LP16; (37) Neut d14; (38) mBM CD15+/CD11b-; (39) BM/GPA; (40) HepG2A; (41) HepG2.2.15-A; (42) HBV-Liver MAI-1; (43) HL60; (44) K562; (45) Molt-4; (46) Hep3B Nor; (47) Hep3B Hypox; and (48) NTC.

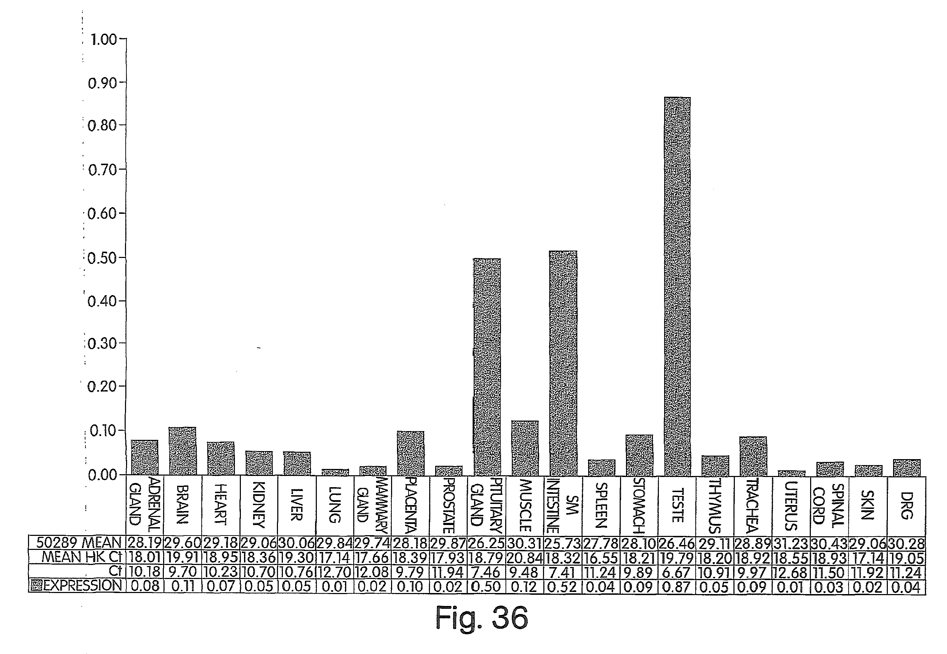

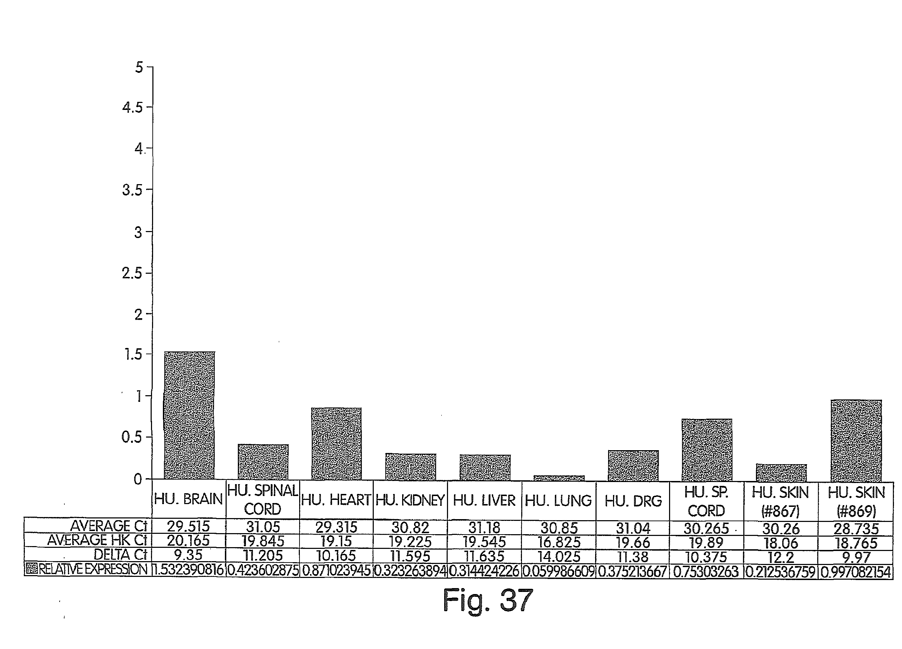

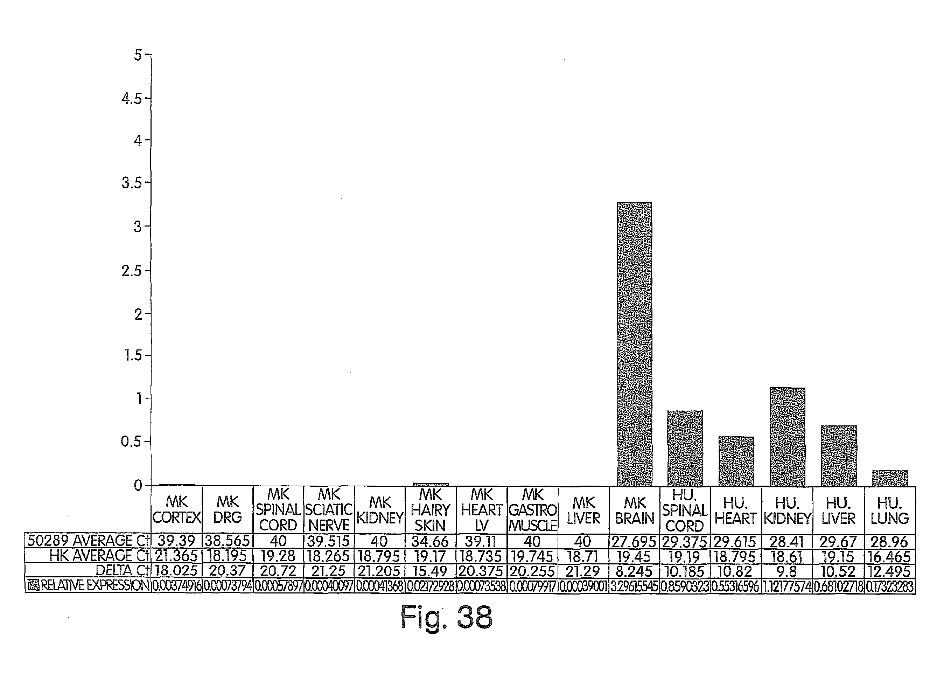

Human 50289 gene expression was evaluated using TaqMan technology as described herein. The 50289 mRNA was expressed at elevated levels in, e.g., testes, small intestine, and the pituitary gland (Figures 36-38).

### Example 5: Tissue Distribution of 1983, 52881, 2398, 45449, 50289, or 52872 mRNA

Northern blot hybridizations with various RNA samples can be performed under standard conditions and washed under stringent conditions, i.e., 0.2xSSC at 65°C. A DNA probe corresponding to all or a portion of the 1983, 52881, 2398, 45449, 50289, or 52872 cDNA (SEQ ID NO:1) can be used. The DNA was radioactively labeled with ³²P-dCTP using the Prime-It Kit (Stratagene, La Jolla, CA) according to the instructions of the supplier. Filters containing mRNA from mouse hematopoietic and endocrine tissues, and cancer cell lines (Clontech, Palo Alto, CA) can be probed in ExpressHyb hybridization solution (Clontech) and washed at high stringency according to manufacturer's recommendations.

### Example 6: Recombinant Expression of 1983, 52881, 2398, 45449, 50289, or 52872 in Bacterial Cells

In this example, 1983, 52881, 2398, 45449, 50289, or 52872 is expressed as a recombinant glutathione-S-transferase (GST) fusion polypeptide in *E. coli* and the fusion polypeptide is isolated and characterized. Specifically, 1983, 52881, 2398, 45449, 50289, or 52872 is fused to GST and this fusion polypeptide is expressed in *E. coli*, e.g., strain PEB199. Expression ofthe GST-1983, 52881, 2398, 45449, 50289, or 52872 fusion protein in PEB 199 is induced with IPTG. The recombinant fusion polypeptide is purified from crude bacterial lysates of the induced PEB 199 strain by affinity chromatography on glutathione beads. Using polyacrylamide gel electrophoretic analysis of the polypeptide purified from the bacterial lysates, the molecular weight of the resultant fusion polypeptide is determined.

### Example 7: Expression of Recombinant 1983, 52881, 2398, 45449, 50289, or 52872 Protein in COS Cells

To express the 1983, 52881, 2398, 45449, 50289, or 52872 gene in COS cells, the pcDNA/Amp vector by Invitrogen Corporation (San Diego, CA) is used. This vector contains an SV40 origin of replication, an ampicillin resistance gene, an *E. coli* replication origin, a CMV promoter followed by a polylinker region, and an SV40 intron and polyadenylation site. A DNA fragment encoding the entire 1983, 52881, 2398, 45449, 50289, or 52872 protein and an HA tag (Wilson et al. (1984) Cell 37:767) or a FLAG tag fused in-frame to its 3' end of the fragment is cloned into the polylinker region of the vector, thereby placing the expression of the recombinant protein under the control of the CMV promoter.

To construct the plasmid, the 1983, 52881, 2398, 45449, 50289, or 52872 DNA sequence is amplified by PCR using two primers. The 5' primer contains the restriction site of interest followed by approximately twenty nucleotides of the 1983, 52881, 2398, 45449, 50289, or 52872 coding sequence starting from the initiation codon; the 3' end sequence contains complementary sequences to the other restriction site of interest, a translation stop codon, the HA tag or FLAG tag and the last 20 nucleotides of the 1983, 52881, 2398, 45449, 50289, or 52872 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the 1983, 52881, 2398, 45449, 50289, or 52872_gene is inserted in the correct orientation. The ligation mixture is transformed into *E. coli* cells (strains HB101, DH5α, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.
COS cells are subsequently transfected with the 1983, 52881, 2398, 45449, 50289, or 52872-pcDNA/Amp plasmid DNA using the calcium phosphate or calcium chloride coprecipitation methods, DEAE-dextran-mediated transfection, lipofection, or electroporation. Other suitable methods for transfecting host cells can be found in Sambrook, J., Fritsh, E. F., and Maniatis, T. (1989) Molecular Cloning: A Laboratory Manual. 2nd, ed., Cold Spring Harbor Laboratory, Cold Spring Harbor Laboratory Press, Cold Spring Harbor, NY. The expression of the 1983, 52881, 2398, 45449, 50289, or 52872 polypeptide is detected by radiolabelling (³⁵S-methionine or ³⁵S-cysteine available from NEN, Boston, MA, can be used) and immunoprecipitation (Harlow, E. and Lane, D. (1988) Antibodies: A Laboratory Manual, Cold Spring Harbor Laboratory Press, Cold Spring Harbor, NY) using an HA specific monoclonal antibody. Briefly, the cells are labeled for 8 hours with ³⁵S-methionine (or ³⁵S-cysteine). The culture media are then collected and the cells are lysed using detergents (RIPA buffer, 150 mM NaCl, 1% NP-40, 0.1% SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the 1983, 52881,2398, 45449, 50289, or 52872 coding sequence is cloned directly into the polylinker of the pCDNA/Amp vector using the appropriate restriction sites. The resulting plasmid is transfected into COS cells in the manner described above, and the expression of the 1983, 52881, 2398, 45449, 50289, or 52872 polypeptide is detected by radiolabelling and immunoprecipitation using a 1983, 52881, 2398, 45449, 50289, or 52872 specific monoclonal antibody.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
(a) a nucleic acid molecule comprising a nucleotide sequence which is at least 80% identical to the nucleotide sequence of SEQ ID NO:4, SEQ ID NO:6, SEQ ID NO:1, SEQ ID NO:3, SEQ ID NO:7, SEQ ID NO:9, SEQ ID NO:10, SEQ ID NO:12, SEQ ID NO:13, SEQ ID NO:15, SEQ ID NO:16, or SEQ ID NO:18;
(b) a nucleic acid molecule comprising a fragment of at least 500 nucleotides of the nucleotide sequence of SEQ ID NO:4, SEQ ID NO:6, SEQ ID NO:1, SEQ ID NO:3, SEQ ID NO:7, SEQ ID NO:9, SEQ ID NO:10, SEQ ID NO:12, SEQ ID NO:13, SEQ ID NO:15, SEQ ID NO:16, or SEQ ID NO:18;
(c) a nucleic acid molecule which encodes a polypeptide comprising the amino acid sequence of SEQ ID NO:5, SEQ ID NO:2, SEQ ID NO:8, SEQ ID NO:11, SEQ ID NO:14, or SEQ ID NO:17;
(d) a nucleic acid molecule which encodes a fragment of a polypeptide comprising the amino acid sequence of SEQ ID NO:5, SEQ ID NO:2, SEQ ID NO:8, SEQ ID NO:11, SEQ ID NO:14, or SEQ ID NO:17, wherein the fragment comprises at least 15 contiguous amino acids of SEQ ID NO:5, SEQ ID NO:2, SEQ ID NO:8, SEQ ID NO:11, SEQ ID NO:14, or SEQ ID NO:17;
(e) a nucleic acid molecule which encodes a naturally occurring allelic variant of a polypeptide comprising the amino acid sequence of SEQ ID NO:5, SEQ ID NO:2, SEQ ID NO:8, SEQ ID NO:11, SEQ ID NO:14, or SEQ ID NO:17, wherein the nucleic acid molecule hybridizes to a nucleic acid molecule comprising SEQ ID NO:4, SEQ ID NO:6, SEQ ID NO:1, SEQ ID NO:3, SEQ ID NO:7. SEQ ID NO:9, SEQ ID NO:10, SEQ ID NO:12, SEQ ID NO:13, SEQ ID NO:15, SEQ ID NO:16, SEQ ID NO:18, or a complement thereof, under stringent conditions;
(f) a nucleic acid comprising the nucleotide sequence of SEQ ID NO:4, SEQ ID NO:6, SEQ ID NO:1, SEQ ID NO:3, SEQ ID NO:7, SEQ ID NO:9, SEQ ID NO:10, SEQ ID NO:12, SEQ ID NO:13, SEQ ID NO:15, SEQ ID NO:16, or SEQ ID NO:18; and
(g) a nucleic acid molecule which encodes a polypeptide comprising the amino acid sequence of SEQ ID NO:5, SEQ ID NO:2, SEQ ID NO:8, SEQ ID NO:11, SEQ ID NO:14, or SEQ ID NO:17.

2. The nucleic acid molecule of claim 1 further comprising vector nucleic acid sequences.

3. The nucleic acid molecule of claim 1 further comprising nucleic acid sequences encoding a heterologous polypeptide.

4. A host cell, preferably a mammalian host cell, more preferably a non-human mammalian host cell, which contains the nucleic acid molecule of claim 1.

5. An isolated polypeptide selected from the group consisting of:
(a) a polypeptide which is encoded by a nucleic acid molecule comprising a nucleotide sequence which is at least 60% identical to a nucleic acid comprising the nucleotide sequence of SEQ ID NO:4, SEQ ID NO:6, SEQ ID NO:1, SEQ ID NO:3, SEQ ID NO:7, SEQ ID NO:9, SEQ ID NO:10, SEQ ID NO:12, SEQ ID NO:13, SEQ ID NO:15, SEQ ID NO:16, or SEQ ID NO:18;
(b) a naturally occurring allelic variant of a polypeptide comprising the amino acid sequence of SEQ ID NO:5, SEQ ID NO:2, SSEQ ID NO:8, SEQ ID NO:11, SEQ ID NO:14, or SEQ ID NO:17, wherein the polypeptide is encoded by a nucleic acid molecule which hybridizes to a nucleic acid molecule comprising SEQ ID NO:4, SEQ ID NO:6, SEQ ID NO:1, SEQ ID NO:3, SEQ ID NO:7, SEQ ID NO:9, SEQ ID NO:10, SEQ ID NO:12, SEQ ID NO:13, SEQ ID NO:15, SEQ ID NO:16, SEQ ID NO:18, or a complement thereof under stringent conditions;
(c) a fragment of a polypeptide comprising the amino acid sequence of SEQ ID NO:5, SEQ ID NO:2, SEQ ID NO:8, SEQ ID NO:11, SEQ ID NO:14, or SEQ ID NO:17, wherein the fragment comprises at least 15 contiguous amino acids of SEQ ID NO: 5, SEQ ID NO:2, SEQ ID NO:8, SEQ ID NO:11, SEQ ID NO:14, or SEQ ID NO:17; and
(d) a polypeptide comprising the amino acid sequence of SEQ ID NO:5, SEQ ID NO:2, SEQ ID NO:8, SEQ ID NO:11, SEQ ID NO:14, or SEQ ID NO:17.

6. The polypeptide of claim 5 further comprising heterologous amino acid sequences.

7. An antibody which selectively binds to a polypeptide of claim 5.

8. A method for producing a polypeptide selected from the group consisting of:
(a) a polypeptide comprising the amino acid sequence of SEQ ID NO:5, SEQ ID NO:2, SEQ ID NO:8, SEQ ID NO:11, SEQ ID NO:14, or SEQ ID NO:17;
(b) a polypeptide comprising a fragment of the amino acid sequence of SEQ ID NO:5, SEQ ID NO:2, SEQ ID NO:8, SEQ ID NO:11, SEQ ID NO:14, or SEQ ID NO:17; wherein the fragment comprises at least 15 contiguous amino acids of SEQ ID NO:5, SEQ ID NO:2, SEQ ID NO:8, SEQ ID NO:11, SEQ ID NO:14, or SEQ ID NO:17; and
(c) a naturally occurring allelic variant of a polypeptide comprising the amino acid sequence of SEQ ID NO:5, SEQ ID NO:2, SEQ ID NO:8, SEQ ID NO:11, SEQ ID NO:14, or SEQ ID NO:17, wherein the polypeptide is encoded by a nucleic acid molecule which hybridizes to a nucleic acid molecule comprising SEQ ID NO:4, SEQ ID NO:6, SEQ ID NO:1, SEQ ID NO:3, SEQ ID NO:7, SEQ ID NO:9, SEQ ID NO:10, SEQ ID NO:12, SEQ ID NO:13, SEQ ID NO:15, SEQ ID NO:16, SEQ ID NO:18, or a complement thereof under stringent conditions;
comprising culturing the host cell of claim 4 under conditions in which the nucleic acid molecule is expressed.

9. A method for detecting the presence of a polypeptide of claim 5 in a sample, comprising:
(a) contacting the sample with a compound, preferably an antibody, which selectively binds to a polypeptide of claim 5; and
(b) determining whether the compound binds to the polypeptide in the sample.

10. A kit comprising a compound which selectively binds to a polypeptide of claim 5 and instructions for use.

11. A method for detecting the presence of a nucleic acid molecule of claim 1 in a sample, comprising the steps of:
(a) contacting the sample with a nucleic acid probe or primer which selectively hybridizes to the nucleic acid molecule; and
(b) determining whether the nucleic acid probe or primer binds to a nucleic acid molecule in the sample;
preferably wherein the sample comprises mRNA molecules and is contacted with a nucleic acid probe.

12. A kit comprising a compound which selectively hybridizes to a nucleic acid molecule of claim 1 and instructions for use.

13. A method for identifying a compound which binds to a polypeptide of claim 5 comprising the steps of:
(a) contacting a polypeptide, or a cell expressing a polypeptide of claim 5 with a test compound; and
(b) determining whether the polypeptide binds to the test compound;
preferably wherein the binding of the test compound to the polypeptide is detected by a method selected from the group consisting of:
(i) detection of binding by direct detecting of test compound/polypeptide binding;
(ii) detection of binding using a competition binding assay; and
(iii) detection of binding using an assay for 52881, 1983, 2398, 45449, 50289, or 52872-mediated signal transduction.

14. A method for modulating the activity of a polypeptide of claim 5 comprising contacting a polypeptide or a cell expressing a polypeptide of claim 5 with a compound which binds to the polypeptide in a sufficient concentration to modulate the activity of the polypeptide.

15. A method for identifying a compound which modulates the activity of a polypeptide of claim 5, comprising:
(a) contacting a polypeptide of claim 5 with a test compound; and
(b) determining the effect of the test compound on the activity of the polypeptide to thereby identify a compound which modulates the activity of the polypeptide.

16. A method of treating or preventing a cardiovascular disorder in a subject, the method comprising administering to the subject an agent that modulates the activity or expression of a 52881 polypeptide or nucleic acid, in an amount effective to treat or prevent the cardiovascular disorder;
preferably wherein the agent is a peptide, a phosphopeptide, a small molecule, an antibody, or any combination thereof;
or preferably wherein the agent is an antisense, a ribozyme, a triple helix molecule, a 52881 nucleic acid, or any combination thereof.

17. A method for identifying an agent that modulates the activity or expression of a 52881 polypeptide or nucleic acid, comprising contacting the 52881 polypeptide or nucleic acid with an agent, and determining the effect of the agent on the activity or expression of the polypeptide or nucleic acid;
preferably wherein the agent is a peptide, a phosphopeptide, a small molecule, an antibody, or any combination thereof;
or preferably wherein the agent is an antisense, a ribozyme, a triple helix molecule, a 52881 nucleic acid, or any combination thereof.

18. The method of claim 17, wherein the effect of the agent on the activity or expression of the polypeptide or nucleic acid is determined in a cardiovascular cell.


SEQUENCE LISTING

<110> Millennium Pharmaceuticals, Inc.

<120> 1983, 52881, 2398, 45449, 50289, AND
  52872, NOVEL G PROTEIN-COUPLED RECEPTORS AND USES THEREFOR

<130> PC785839EPA

<140> 09160573.3
<141> 2002-09-03

<150> US 60/186,059
<151> 2000-02-29

<160> 26

<170> FastSEQ for Windows Version 4.0

<210> 1
<211> 3127
<212> DNA
<213> Homo sapiens

<220>
<221> CDS
<222> (306)...(2240)

<400> 1
aattcccggg tcgacccacg cgtccgggaa agtgtccgga ggaagccggc gcgtcacgga     60
ggacggatcc gggaccctgc cgtgcccgcc cagcggcgcc cggtcccggg tccacagccg    120
ccctcactcc gmcgcgctct ccgccaccgc caccactgcg gccaccgcca atgaaacgcc    180
tcccgctcct agtggttttt tccactttgt tgaattgttc ctatactcaa aattgcacca    240
agacaccttg tctcccaaat gcaaaatgtg aaatacgcaa tggaattgaa gcctgctatt    300
gcaac atg gga ttt tca gga aat ggt gtc aca att tgt gaa gat gat aat    350
gaa tgt gga aat tta act cag tcc tgt ggc gaa aat gct aat tgc act      398
aac aca gaa gga agt tat tat tgt atg tgt gta cct ggc ttc aga tcc      446
agc agt aac caa gac agg ttt atc act aat gat gga acc gtc tgt ata      494
gaa aat gtg aat gca aac tgc cat tta gat aat gtc tgt ata gct gca      542
aat att aat aaa act tta aca aaa atc aga tcc ata aaa gaa cct gtg      590
gct ttg cta caa gaa gtc tat aga aat tct gtg aca gat ctt tca cca      638
aca gat ata att aca tat ata gaa ata tta gct gaa tca tct tca tta        686
cta ggt tac aag aac aac act atc tca gcc aag gac acc ctt tct aac        734
tca act ctt act gaa ttt gta aaa acc gtg aat aat ttt gtt caa agg        782
gat aca ttt gta gtt tgg gac aag tta tct gtg aat cat agg aga aca        830
cat ctt aca aaa ctc atg cac act gtt gaa caa gct act tta agg ata        878
tcc cag agc ttc caa aag acc aca gag ttt gat aca aat tca acg gat        926
ata gct ctc aaa gtt ttc ttt ttt gat tca tat aac atg aaa cat att        974
cat cct cat atg aat atg gat gga gac tac ata aat ata ttt cca aag       1022
aga aaa gct gca tat gat tca aat ggc aat gtt gca gtt gca ttt tta       1070
tat tat aag agt att ggt cct ttg ctt tca tca tct gac aac ttc tta       1118
ttg aaa cct caa aat tat gat aat tct gaa gag gag gaa aga gtc ata       1166
tct tca gta att tca gtc tca atg agc tca aac cca ccc aca tta tat       1214
gaa ctt gaa aaa ata aca ttt aca tta agt cat cga aag gtc aca gat       1262
agg tat agg agt cta tgt gca ttt tgg aat tac tca cct gat acc atg       1310
aat ggc agc tgg tct tca gag ggc tgt gag ctg aca tac tca aat gag       1358
acc cac acc tca tgc cgc tgt aat cac ctg aca cat ttt gca att ttg 1406
atg tcc tct ggt cct tcc att ggt att aaa gat tat aat att ctt aca 1454
agg atc act caa cta gga ata att att tca ctg att tgt ctt gcc ata 1502
tgc att ttt acc ttc tgg ttc ttc agt gaa att caa agc acc agg aca 1550
aca att cac aaa aat ctt tgc tgt agc cta ttt ctt gct gaa ctt gtt 1598
ttt ctt gtt ggg atc aat aca aat act aat aag ctc ttc tgt tca atc 1646
att gcc gga ctg cta cac tac ttc ttt tta gct gct ttt gca tgg atg 1694
tgc att gaa ggc ata cat ctc tat ctc att gtt gtg ggt gtc atc tac 1742
aac aag gga ttt ttg cac aag aat ttt tat atc ttt ggc tat cta agc 1790
cca gcc gtg gta gtt gga ttt tcg gca gca cta gga tac aga tat tat 1838
ggc aca acc aaa gta tgt tgg ctt agc acc gaa aac aac ttt att tgg 1886
agt ttt ata gga cca gca tgc cta atc att ctt gtt aat ctc ttg gct 1934
ttt gga gtc atc ata tac aaa gtt ttt cgt cac act gca ggg ttg aaa 1982
cca gaa gtt agt tgc ttt gag aac ata agg tct tgt gca aga gga gcc 2030
ctc gct ctt ctg ttc ctt ctc ggc acc acc tgg atc ttt ggg gtt ctc 2078
cat gtt gtg cac gca tca gtg gtt aca gct tac ctc ttc aca gtc agc 2126
aat gct ttc cag ggg atg ttc att ttt tta ttc ctg tgt gtt tta tct 2174
aga aag att caa gaa gaa tat tac aga ttg ttc aaa aat gtc ccc tgt 2222
tgt ttt gga tgt tta agg taaacataga gaatggtgga taattacaac 2270
tgcacaaaaa taaaaattcc aagctgtgga tgaccaatgt ataaaaatga ctcatcaaat 2330
tatccaatta ttaactacta gacaaaaagt attttaaatc agtttttctg tttatgctat 2390
aggaactgta gataataagg taaaattatg tatcatatag atatactatg tttttctatg 2450
tgaaatagtt ctgtcaaaaa tagtattgca gatatttgga aagtaattgg tttctcagga 2510
gtgatatcac tgcacccaag gaaagatttt ctttctaaca cgagaagtat atgaatgtcc 2570
tgaaggaaac cactggcttg atatttctgt gactcgtgtt gcctttgaaa ctagtcccct 2630
accacctcgg taatgagctc cattacagaa agtggaacat aagagaatga aggggcagaa 2690
tatcaaacag tgaaaaggga atgataagat gtattttgaa tgaactgttt tttctgtaga 2750
ctagctgaga aattgttgac ataaaataaa gaattgaaga aacacatttt accattttgt 2810
gaattgttct gaacttaaat gtccactaaa acaacttaga cttctgtttg ctaaatctgt 2870
ttctttttct aatattctaa aaaaaaaaaa aaggtttacc tccacaaatt gaaaaaaaaa 2930
aagtgaaaaa aatctgtttc taaggttaga ctgagatata tactatttcc ttacttattt 2990
cacagattgt gactttggat agttaatcag taaaatataa atgtgtcaag atataatatt 3050
gtttatacct atcaatgtaa aaacagtgta ataaagctga agtattctat taaaaaaaaa 3110
aaaaaaaaaa aaaaaaa 3127

<210> 2
<211> 645
<212> PRT
<213> Homo sapiens

<400> 2

<210> 3

<211> 1938
<212> DNA
<213> Homo sapiens

<400> 3
atgggatttt caggaaatgg tgtcacaatt tgtgaagatg ataatgaatg tggaaattta      60
actcagtcct gtggcgaaaa tgctaattgc actaacacag aaggaagtta ttattgtatg     120
tgtgtacctg gcttcagatc cagcagtaac caagacaggt ttatcactaa tgatggaacc     180
gtctgtatag aaaatgtgaa tgcaaactgc catttagata atgtctgtat agctgcaaat     240
attaataaaa ctttaacaaa aatcagatcc ataaaagaac ctgtggcttt gctacaagaa     300
gtctatagaa attctgtgac agatctttca ccaacagata taattacata tatagaaata     360
ttagctgaat catcttcatt actaggttac aagaacaaca ctatctcagc caaggacacc     420
ctttctaact caactcttac tgaatttgta aaaaccgtga ataattttgt tcaaagggat      480
acatttgtag tttgggacaa gttatctgtg aatcatagga gaacacatct tacaaaactc     540
atgcacactg ttgaacaagc tactttaagg atatcccaga gcttccaaaa gaccacagag     600
tttgatacaa attcaacgga tatagctctc aaagttttct tttttgattc atataacatg     660
aaacatattc atcctcatat gaatatggat ggagactaca taaatatatt tccaaagaga     720
aaagctgcat atgattcaaa tggcaatgtt gcagttgcat ttttatatta taagagtatt     780
ggtcctttgc tttcatcatc tgacaacttc ttattgaaac ctcaaaatta tgataattct     840
gaagaggagg aaagagtcat atcttcagta atttcagtct caatgagctc aaacccaccc     900
acattatatg aacttgaaaa aataacattt acattaagtc atcgaaaggt cacagatagg     960
tataggagtc tatgtgcatt ttggaattac tcacctgata ccatgaatgg cagctggtct    1020
tcagagggct gtgagctgac atactcaaat gagacccaca cctcatgccg ctgtaatcac    1080
ctgacacatt ttgcaatttt gatgtcctct ggtccttcca ttggtattaa agattataat    1140
attcttacaa ggatcactca actaggaata attatttcac tgatttgtct tgccatatgc    1200
atttttacct tctggttctt cagtgaaatt caaagcacca ggacaacaat tcacaaaaat    1260
ctttgctgta gcctatttct tgctgaactt gtttttcttg ttgggatcaa tacaaatact    1320
aataagctct tctgttcaat cattgccgga ctgctacact acttcttttt agctgctttt    1380
gcatggatgt gcattgaagg catacatctc tatctcattg ttgtgggtgt catctacaac    1440
aagggatttt tgcacaagaa tttttatatc tttggctatc taagcccagc cgtggtagtt    1500
ggattttcgg cagcactagg atacagatat tatggcacaa ccaaagtatg ttggcttagc    1560
accgaaaaca actttatttg gagttttata ggaccagcat gcctaatcat tcttgttaat    1620
ctcttggctt ttggagtcat catatacaaa gtttttcgtc acactgcagg gttgaaacca    1680
gaagttagtt gctttgagaa cataaggtct tgtgcaagag gagccctcgc tcttctgttc    1740
cttctcggca ccacctggat ctttggggtt ctccatgttg tgcacgcatc agtggttaca    1800
gcttacctct tcacagtcag caatgctttc caggggatgt tcattttttt attcctgtgt    1860
gttttatcta gaaagattca agaagaatat tacagattgt tcaaaaatgt cccctgttgt    1920
tttggatgtt taaggtaa                                                  1938

<210> 4
<211> 4238
<212> DNA
<213> Homo sapiens

<220>
<221> CDS
<222> (403)...(2229)

<221> misc_feature
<222> (1)...(4238)
<223> n = A,T,C or G

<400> 4
   attcccgggt cgaacccacg cgtccgccca cgcgtccgcc cacgcgtccg gctgagcgaa      60
   gcgcggcggc ggcggcggcg cctaggggag ggaggggcgg cggggccgag cccacctagc     120
   ggagcgcgcc ggccgccggt ggccgccgcc agcatgcccc ggcccgcggg ccgctccgcc     180
   gccagccacc cccgcggccc tcggcggcct gcgctcggcc cgggggcgcg ggaaccgcag     240
   ccggagccgg aggcgggagc agcgagccgg agccccgggc gctcgaatgc aggatgctcg     300
   tggtccccag catccttgag ccaccaggag tgagggctgc tgctccctga gacctggctc     360
   caaggaggat gccacagccg cctgccagct ccggtctgca cc atg agt gat gag       414
                                                  Met Ser Asp Glu
   1
   cgg cgg ctg cct ggc agt gca gtg ggc tgg ctg gta tgt ggg ggc ctc 462
   Arg Arg Leu Pro Gly Ser Ala Val Gly Trp Leu Val Cys Gly Gly Leu
   5 10 15 20
   tcc ctg ctg gcc aat gcc tgg ggc atc ctc agc gtt ggc gcc aag cag 510
   Ser Leu Leu Ala Asn Ala Trp Gly Ile Leu Ser Val Gly Ala Lys Gln
   25 30 35
   aag aag tgg aag ccc ttg gag ttc ctg ctg tgt acg ctc gcg gcc acc 558
   Lys Lys Trp Lys Pro Leu Glu Phe Leu Leu Cys Thr Leu Ala Ala Thr
   40 45 50
   cac atg cta aat gtg gcc gtg ccc atc gcc acc tac tcc gtg gtg cag 606
   His Met Leu Asn Val Ala Val Pro Ile Ala Thr Tyr Ser Val Val Gln
   55 60 65
   ctg cgg cgg cag cgc ccc gac ttc gag tgg aat gag ggt ctc tgc aag 654
   Leu Arg Arg Gln Arg Pro Asp Phe Glu Trp Asn Glu Gly Leu Cys Lys
   70 75 80
   gtc ttc gtg tcc acc ttc tac acc ctc acc ctg gcc acc tgt ttc tct 702
   Val Phe Val Ser Thr Phe Tyr Thr Leu Thr Leu Ala Thr Cys Phe Ser
   85 90 95 100
   gtc acc tcc ctc tcc tac cac cgc atg tgg atg gtc tgc tgg cct gtc 750
   Val Thr Ser Leu Ser Tyr His Arg Met Trp Met Val Cys Trp Pro Val
   105 110 115
   aac tac cgg ctg agc aat gcc aag aag cag gcg gtg cac aca gtc atg 798
   Asn Tyr Arg Leu Ser Asn Ala Lys Lys Gln Ala Val His Thr Val Met
   120 125 130
   ggt atc tgg atg gtg tcc ttc atc ctg tcg gcc ctg cct gcc gtt ggc 846
   Gly Ile Trp Met Val Ser Phe Ile Leu Ser Ala Leu Pro Ala Val Gly
   135 140 145
   tgg cac gac acc agc gag cgc ttc tac acc cat ggc tgc cgc ttc atc 894
   Trp His Asp Thr Ser Glu Arg Phe Tyr Thr His Gly Cys Arg Phe Ile
   150 155 160
   gtg gct gag atc ggc ctg ggc ttt ggc gtc tgc ttc ctg ctg ctg gtg 942
   Val Ala Glu Ile Gly Leu Gly Phe Gly Val Cys Phe Leu Leu Leu Val
   165 170 175 180
   ggc ggc agc gtg gcc atg ggc gtg atc tgc aca gcc atc gcc ctc ttc 990
   Gly Gly Ser Val Ala Met Gly Val Ile Cys Thr Ala Ile Ala Leu Phe
   185 190 195
   cag acg ctg gcc gtg cag gtg ggg cgc cag gcc gac cac cgc gcc ttc 1038
   Gln Thr Leu Ala Val Gln Val Gly Arg Gln Ala Asp His Arg Ala Phe
   200 205 210
   acc gtg ccc acc atc gtg gtg gag gac gcg cag ggc aag cgg cgc tcc 1086
   Thr Val Pro Thr Ile Val Val Glu Asp Ala Gln Gly Lys Arg Arg Ser
   215 220 225
   tcc atc gat ggc tcg gag ccc gcc aaa acc tct ctg cag acc acg ggc 1134
   Ser Ile Asp Gly Ser Glu Pro Ala Lys Thr Ser Leu Gln Thr Thr Gly
   230 235 240
   ctc gtg acc acc ata gtc ttc atc tac gac tgc ctc atg ggc ttc cct 1182
   Leu Val Thr Thr Ile Val Phe Ile Tyr Asp Cys Leu Met Gly Phe Pro
   245 250 255 260
   gtg ctg gtg gtg agc ttc agc agc ctg cgg gcc gac gcc tca gcg ccc 1230
   Val Leu Val Val Ser Phe Ser Ser Leu Arg Ala Asp Ala Ser Ala Pro
   265 270 275
   tgg atg gca ctc tgc gtg ctg tgg tgc tcc gtg gcc cag gcc ctg ctg 1278
   Trp Met Ala Leu Cys Val Leu Trp Cys Ser Val Ala Gln Ala Leu Leu
   280 285 290
   ctg cct gtg ttc ctc tgg gcc tgc gac cgc tac cgg gct gac ctc aaa 1326
   Leu Pro Val Phe Leu Trp Ala Cys Asp Arg Tyr Arg Ala Asp Leu Lys
   295 300 305
   gct gtc cgg gag aag tgc atg gcc ctc atg gcc aac gac gag gag tca 1374
   Ala Val Arg Glu Lys Cys Met Ala Leu Met Ala Asn Asp Glu Glu Ser
   310 315 320
   gac gat gag acc agc ctg gaa ggt ggc atc tcc ccg gac ctg gtg ttg 1422
   Asp Asp Glu Thr Ser Leu Glu Gly Gly Ile Ser Pro Asp Leu Val Leu
   325 330 335 340
   gag cgc tcc ctg gac tat ggc tat gga ggt gat ttt gtg gcc cta gat 1470
   Glu Arg Ser Leu Asp Tyr Gly Tyr Gly Gly Asp Phe Val Ala Leu Asp
   345 350 355
   agg atg gcc aag tat gag atc tcc gcc ctg gag ggg ggc ctg ccc cag 1518
   Arg Met Ala Lys Tyr Glu Ile Ser Ala Leu Glu Gly Gly Leu Pro Gln
   360 365 370
   ctc tac cca ctg cgg ccc ttg cag gag gac aag atg caa tac ctg cag 1566
   Leu Tyr Pro Leu Arg Pro Leu Gln Glu Asp Lys Met Gln Tyr Leu Gln
   375 380 385
   gtc ccg ccc acg cgg cgc ttc tcc cac gac gat gcg gac gtg tgg gcc 1614
   Val Pro Pro Thr Arg Arg Phe Ser His Asp Asp Ala Asp Val Trp Ala
   390 395 400
   gcc gtc ccg ctg ccc gcc ttc ctg ccg cgc tgg ggc tcc ggc gag gac 1662
   Ala Val Pro Leu Pro Ala Phe Leu Pro Arg Trp Gly Ser Gly Glu Asp
   405 410 415 420
   ctg gcc gcc ctg gcg cac ctg gtg ctg cct gcc ggg ccc gag cgg cgc 1710
   Leu Ala Ala Leu Ala His Leu Val Leu Pro Ala Gly Pro Glu Arg Arg
   425 430 435
   cgc gcc agc ctc ctg gcc ttc gcg gag gac gca cca ccg tcc cgc gcg 1758
   Arg Ala Ser Leu Leu Ala Phe Ala Glu Asp Ala Pro Pro Ser Arg Ala
   440 445 450
   cgc cgc cgc tcg gcc gag agc ctg ctg tcg ctg cgg ccc tcg gcc ctg 1806
   Arg Arg Arg Ser Ala Glu Ser Leu Leu Ser Leu Arg Pro Ser Ala Leu
   455 460 465
   gat agc ggc ccg cgg gga gcc cgc gac tcg ccc ccc ggc agc ccg cgc 1854
   Asp Ser Gly Pro Arg Gly Ala Arg Asp Ser Pro Pro Gly Ser Pro Arg
   470 475 480
   cgc cgc ccc ggg ccc ggc ccc cgc tcc gcc tcg gcc tcg ctg ctg ccc 1902
   Arg Arg Pro Gly Pro Gly Pro Arg Ser Ala Ser Ala Ser Leu Leu Pro
   485 490 495 500
   gac gcc ttc gcc ctg acc gcc ttc gag tgc gag cca cag gcc ctg cgc 1950
   Asp Ala Phe Ala Leu Thr Ala Phe Glu Cys Glu Pro Gln Ala Leu Arg
   505 510 515
   cgc ccg ccc ggg ccc ttc ccc gct gcg ccc gcc gcc ccc gac ggc gca 1998
   Arg Pro Pro Gly Pro Phe Pro Ala Ala Pro Ala Ala Pro Asp Gly Ala
   520 525 530
   gat ccc gga gag gcc ccg acg ccc cca agc agc gcc cag cgg agc cca 2046
   Asp Pro Gly Glu Ala Pro Thr Pro Pro Ser Ser Ala Gln Arg Ser Pro
   535 540 545
   ggg cca cgc ccc tct gcg cac tcg cac gcc ggc tct ctg cgc ccc ggc 2094
   Gly Pro Arg Pro Ser Ala His Ser His Ala Gly Ser Leu Arg Pro Gly
   550 555 560
   ctg agc gcg tcg tgg ggc gag ccc ggg ggg ctg cgc gcg gcg ggc ggc 2142
   Leu Ser Ala Ser Trp Gly Glu Pro Gly Gly Leu Arg Ala Ala Gly Gly
   565 570 575 580
   ggc ggc agc acc agc agc ttc ctg agt tcc ccc tcc gag tcc tcg ggc 2190
   Gly Gly Ser Thr Ser Ser Phe Leu Ser Ser Pro Ser Glu Ser Ser Gly
   585 590 595
   tac gcc acg ctg cac tcg gac tcg ctg ggc tcc gcg tcc taggaccgcc 2239
   Tyr Ala Thr Leu His Ser Asp Ser Leu Gly Ser Ala Ser
   600 605
   ggcgcctccc cacggacgcc aggcaggcca ggccgctctc cggggccgca gcaccaaaga 2299
   cgcccgcctc ccccccgcgc gcagacatgc gccacccctc ccaggggtga gggggcngtt 2359
   ggcctcagcg tttgtcttcc ggctcctccc agctggcctt gtcccagggg cgacggctgc 2419
   cccggacgac tgcgctgggc accgcatgtc ccgggccgag tgaggtcggg cctggggagc 2479
   tgagtgacat cccaagcttg ggcttgggta gtgagtgaca tgtgcacacg tccagctgcg 2539
   ccatcaccag ccctgcgcaa caggacgtcg ggagcaggga acctgagaca gggccactgc 2599
   gggatcggac aaagccccgc tttggagagg ctgagctgga gccattggcc tccccagggg 2659
   ctttccaccc acactgcacc ataaccgcca cacccttcgg ggggggggag ggtacagaag 2719
   ggtctctaag cacaggggtg ttcagagccc gaacaagctt tgatcaggtt tccctgcttc 2779
   cgacctgtcc tgcctcagtt tccccatctg tgatgagcag gtgaccacgt taactctcag 2839
   ggctgtttga agtctcttgg ttgtaggccc tcgccactga gtggcccagg tgtgagaggt 2899
   agtctagagc cctctgcggc ctttgtggag gtccgttctc agcatgtggc ctggtgtggc 2959
   tccctaggcc tgaggcctcc cactctcagt gccccctgcc cccttgggaa cccacactcc 3019
   accccagcta agcacaacac tgtggaccac caccaatggc acctgagcca ccttgaccac 3079
   cattagtgtc cccaccctca ttactctgcc gtcgttgcct gtccccacca catcatctcc 3139
   ctccatggct cccaccatga cgctggcagg tgcaagcgga gagcacgagg gcagcagggc 3199
   ctggccttcc gtgtccctgc gtcatcccca gcctcactcc ccacccgtgc aggcctggcc 3259
   aggaagggat cctggcaggg gcttccaggt tctcagctca aggcctggtc ccgggcaggc 3319
   gtccaacccc tgggagcaat gtatttcttt gccttccatc ctgggcagac cccttacagg 3379
   cctgggcatt gccatgggcc ctgggtcttc ccaggctaag gagaaccagg aacagctata 3439
   accttgagct agtgaaatag ggtagatgan gaaggctgtc tcctccagac ccctacccct 3499
   acacagtggc cccacaatat gaagacctgg ggtaattcca aggtgagcat agagcctgcc 3559
   tgtgcccagt tccttctggc cctcaggtgg ccaagcccat ctcttcatcc ttcagatagg 3619
   gtcccactcc cagaagaagc tgctggggtg ggggtgggag gctgcctaag cctgtctgtg 3679
   cttcagaggc ccctccagtc cctggctgtg gggtaactgg gggtatgagc tgtggccaca 3739
   ggtgagcaag gcagggaact gcaatccagc cctggccgcg ggagggccca tctctggcca 3799
   atgctgctgt gccttcaagg actgacaagt tacgtagggg cagaggtcgc cagctagcca 3859
   gtgtctcctc catctggggg gcgtctgtcc acttgtcacc ttaggttttc actcatttgt 3919
   caccttgggg ttttgctctg tgtgtttcat atccaacggc aatacttgca gggggacaga 3979
   gtcctctaaa tactccaatc ctgcggtttt tacaaacata aagggggara mcccaartgg 4039
   argaccctkg gccyggarct ccytcccaaa ctttgtccag catccagcct gttccctggg 4099
   ctcactgggg agggagttgt cttcatagca cacccagagc cagggatccc tttgtagttt 4159
   tttgacaacg gagcatttct cttctgtaca ggacccaata aaaacttcct tatgaaaaaa 4219
   aaaaaaaaag ggcggccgc 4238

<210> 5
<211> 609
<212> PRT
<213> Homo sapiens

<400> 5
Met Ser Asp Glu Arg Arg Leu Pro Gly Ser Ala Val Gly Trp Leu Val
1               5                   10                  15
Cys Gly Gly Leu Ser Leu Leu Ala Asn Ala Trp Gly Ile Leu Ser Val
            20                  25                  30
Gly Ala Lys Gln Lys Lys Trp Lys Pro Leu Glu Phe Leu Leu Cys Thr
        35                  40                  45
Leu Ala Ala Thr His Met Leu Asn Val Ala Val Pro Ile Ala Thr Tyr
    50                  55                  60
Ser Val Val Gln Leu Arg Arg Gln Arg Pro Asp Phe Glu Trp Asn Glu
65                  70                  75                  80
Gly Leu Cys Lys Val Phe Val Ser Thr Phe Tyr Thr Leu Thr Leu Ala
                85                  90                  95
Thr Cys Phe Ser Val Thr Ser Leu Ser Tyr His Arg Met Trp Met Val
            100                 105                 110
Cys Trp Pro Val Asn Tyr Arg Leu Ser Asn Ala Lys Lys Gln Ala Val
        115                 120                 125
His Thr Val Met Gly Ile Trp Met Val Ser Phe Ile Leu Ser Ala Leu
    130                 135                 140
Pro Ala Val Gly Trp His Asp Thr Ser Glu Arg Phe Tyr Thr His Gly
145                 150                 155                 160
Cys Arg Phe Ile Val Ala Glu Ile Gly Leu Gly Phe Gly Val Cys Phe
                165                 170                 175
Leu Leu Leu Val Gly Gly Ser Val Ala Met Gly Val Ile Cys Thr Ala
            180                 185                 190
Ile Ala Leu Phe Gln Thr Leu Ala Val Gln Val Gly Arg Gln Ala Asp
        195                 200                 205
His Arg Ala Phe Thr Val Pro Thr Ile Val Val Glu Asp Ala Gln Gly
    210                 215                 220
Lys Arg Arg Ser Ser Ile Asp Gly Ser Glu Pro Ala Lys Thr Ser Leu
225                 230                 235                 240
Gln Thr Thr Gly Leu Val Thr Thr Ile Val Phe Ile Tyr Asp Cys Leu
                245                 250                 255
Met Gly Phe Pro Val Leu Val Val Ser Phe Ser Ser Leu Arg Ala Asp
            260                 265                 270
Ala Ser Ala Pro Trp Met Ala Leu Cys Val Leu Trp Cys Ser Val Ala
        275                 280                 285
Gln Ala Leu Leu Leu Pro Val Phe Leu Trp Ala Cys Asp Arg Tyr Arg
    290                 295                 300
Ala Asp Leu Lys Ala Val Arg Glu Lys Cys Met Ala Leu Met Ala Asn
305                 310                 315                 320
Asp Glu Glu Ser Asp Asp Glu Thr Ser Leu Glu Gly Gly Ile Ser Pro
                325                 330                 335
Asp Leu Val Leu Glu Arg Ser Leu Asp Tyr Gly Tyr Gly Gly Asp Phe
            340                 345                 350
Val Ala Leu Asp Arg Met Ala Lys Tyr Glu Ile Ser Ala Leu Glu Gly
        355                 360                 365
Gly Leu Pro Gln Leu Tyr Pro Leu Arg Pro Leu Gln Glu Asp Lys Met
    370                 375                 380
Gln Tyr Leu Gln Val Pro Pro Thr Arg Arg Phe Ser His Asp Asp Ala

385                 390                 395                 400
Asp Val Trp Ala Ala Val Pro Leu Pro Ala Phe Leu Pro Arg Trp Gly
                405                 410                 415
Ser Gly Glu Asp Leu Ala Ala Leu Ala His Leu Val Leu Pro Ala Gly
            420                 425                 430
Pro Glu Arg Arg Arg Ala Ser Leu Leu Ala Phe Ala Glu Asp Ala Pro
        435                 440                 445
Pro Ser Arg Ala Arg Arg Arg Ser Ala Glu Ser Leu Leu Ser Leu Arg
    450                 455                 460
Pro Ser Ala Leu Asp Ser Gly Pro Arg Gly Ala Arg Asp Ser Pro Pro
465                 470                 475                 480
Gly Ser Pro Arg Arg Arg Pro Gly Pro Gly Pro Arg Ser Ala Ser Ala
                485                 490                 495
Ser Leu Leu Pro Asp Ala Phe Ala Leu Thr Ala Phe Glu Cys Glu Pro
            500                 505                 510
Gln Ala Leu Arg Arg Pro Pro Gly Pro Phe Pro Ala Ala Pro Ala Ala
        515                 520                 525
Pro Asp Gly Ala Asp Pro Gly Glu Ala Pro Thr Pro Pro Ser Ser Ala
    530                 535                 540
Gln Arg Ser Pro Gly Pro Arg Pro Ser Ala His Ser His Ala Gly Ser
545                 550                 555                 560
Leu Arg Pro Gly Leu Ser Ala Ser Trp Gly Glu Pro Gly Gly Leu Arg
                565                 570                 575
Ala Ala Gly Gly Gly Gly Ser Thr Ser Ser Phe Leu Ser Ser Pro Ser
            580                 585                 590
Glu Ser Ser Gly Tyr Ala Thr Leu His Ser Asp Ser Leu Gly Ser Ala
        595                 600                 605
Ser

<210> 6
<211> 1830
<212> DNA
<213> Homo sapiens

<400> 6
atgagtgatg agcggcggct gcctggcagt gcagtgggct ggctggtatg tgggggcctc      60
tccctgctgg ccaatgcctg gggcatcctc agcgttggcg ccaagcagaa gaagtggaag     120
cccttggagt tcctgctgtg tacgctcgcg gccacccaca tgctaaatgt ggccgtgccc     180
atcgccacct actccgtggt gcagctgcgg cggcagcgcc ccgacttcga gtggaatgag     240
ggtctctgca aggtcttcgt gtccaccttc tacaccctca ccctggccac ctgtttctct     300
gtcacctccc tctcctacca ccgcatgtgg atggtctgct ggcctgtcaa ctaccggctg     360
agcaatgcca agaagcaggc ggtgcacaca gtcatgggta tctggatggt gtccttcatc     420
ctgtcggccc tgcctgccgt tggctggcac gacaccagcg agcgcttcta cacccatggc     480
tgccgcttca tcgtggctga gatcggcctg ggctttggcg tctgcttcct gctgctggtg     540
ggcggcagcg tggccatggg cgtgatctgc acagccatcg ccctcttcca gacgctggcc     600
gtgcaggtgg ggcgccaggc cgaccaccgc gccttcaccg tgcccaccat cgtggtggag     660
gacgcgcagg gcaagcggcg ctcctccatc gatggctcgg agcccgccaa aacctctctg     720
cagaccacgg gcctcgtgac caccatagtc ttcatctacg actgcctcat gggcttccct     780
gtgctggtgg tgagcttcag cagcctgcgg gccgacgcct cagcgccctg gatggcactc     840
tgcgtgctgt ggtgctccgt ggcccaggcc ctgctgctgc ctgtgttcct ctgggcctgc     900
gaccgctacc gggctgacct caaagctgtc cgggagaagt gcatggccct catggccaac     960
gacgaggagt cagacgatga gaccagcctg gaaggtggca tctccccgga cctggtgttg    1020
gagcgctccc tggactatgg ctatggaggt gattttgtgg ccctagatag gatggccaag    1080
tatgagatct ccgccctgga ggggggcctg ccccagctct acccactgcg gcccttgcag    1140
gaggacaaga tgcaatacct gcaggtcccg cccacgcggc gcttctccca cgacgatgcg    1200
gacgtgtggg ccgccgtccc gctgcccgcc ttcctgccgc gctggggctc cggcgaggac    1260
ctggccgccc tggcgcacct ggtgctgcct gccgggcccg agcggcgccg cgccagcctc    1320
ctggccttcg cggaggacgc accaccgtcc cgcgcgcgcc gccgctcggc cgagagcctg    1380
ctgtcgctgc ggccctcggc cctggatagc ggcccgcggg gagcccgcga ctcgcccccc    1440
ggcagcccgc gccgccgccc cgggcccggc ccccgctccg cctcggcctc gctgctgccc    1500

gacgccttcg ccctgaccgc cttcgagtgc gagccacagg ccctgcgccg cccgcccggg 1560
cccttccccg ctgcgcccgc cgcccccgac ggcgcagatc ccggagaggc cccgacgccc 1620
ccaagcagcg cccagcggag cccagggcca cgcccctctg cgcactcgca cgccggctct 1680
ctgcgccccg gcctgagcgc gtcgtggggc gagcccgggg ggctgcgcgc ggcgggcggc 1740
ggcggcagca ccagcagctt cctgagttcc ccctccgagt cctcgggcta cgccacgctg 1800
cactcggact cgctgggctc cgcgtcctag 1830

<210> 7
<211> 1113
<212> DNA
<213> Homo sapiens

<220>
<221> CDS
<222> (1)...(1050)

<400> 7
atg gct ttg gaa cag aac cag tca aca gat tat tat tat gag gaa aat 48
Met Ala Leu Glu Gln Asn Gln Ser Thr Asp Tyr Tyr Tyr Glu Glu Asn
1               5                   10                  15

gaa atg aat ggc act tat gac tac agt caa tat gaa ctg atc tgt atc 96
Glu Met Asn Gly Thr Tyr Asp Tyr Ser Gln Tyr Glu Leu Ile Cys Ile
            20                  25                  30

aaa gaa gat gtc aga gaa ttt gca aaa gtt ttc ctc cct gta ttc ctc 144
Lys Glu Asp Val Arg Glu Phe Ala Lys Val Phe Leu Pro Val Phe Leu
        35                  40                  45

aca ata gtt ttc gtc att gga ctt gca ggc aat tcc atg gta gtg gca 192
Thr Ile Val Phe Val Ile Gly Leu Ala Gly Asn Ser Met Val Val Ala
    50                  55                  60

att tat gcc tat tac aag aaa cag aga acc aaa aca gat gtg tac atc 240
Ile Tyr Ala Tyr Tyr Lys Lys Gln Arg Thr Lys Thr Asp Val Tyr Ile
65                  70                  75                  80

ctg aat ttg gct gta gca gat tta ctc ctt cta ttc act ctg cct ttt 288
Leu Asn Leu Ala Val Ala Asp Leu Leu Leu Leu Phe Thr Leu Pro Phe
                85                  90                  95

tgg gct gtt aat gca gtt cat ggg tgg gtt tta ggg aaa ata atg tgc 336
Trp Ala Val Asn Ala Val His Gly Trp Val Leu Gly Lys Ile Met Cys
            100                 105                 110

aaa ata act tca gcc ttg tac aca cta aac ttt gtc tct gga atg cag 384
Lys Ile Thr Ser Ala Leu Tyr Thr Leu Asn Phe Val Ser Gly Met Gln
        115                 120                 125

ttt ctg gct tgt atc agc ata gac aga tat gtg gca gta act aaa gtc 432
Phe Leu Ala Cys Ile Ser Ile Asp Arg Tyr Val Ala Val Thr Lys Val
    130                 135                 140

ccc agc caa tca gga gtg gga aaa cca tgc tgg atc atc tgt ttc tgt 480
Pro Ser Gln Ser Gly Val Gly Lys Pro Cys Trp Ile Ile Cys Phe Cys
145                 150                 155                 160

gtc tgg atg gct gcc atc ttg ctg agc ata ccc cag ctg gtt ttt tat 528
Val Trp Met Ala Ala Ile Leu Leu Ser Ile Pro Gln Leu Val Phe Tyr
                165                 170                 175

aca gta aat gac aat gct agg tgc att ccc att ttc ccc cgc tac cta 576
Thr Val Asn Asp Asn Ala Arg Cys Ile Pro Ile Phe Pro Arg Tyr Leu
            180                 185                 190

gga aca tca atg aaa gca ttg att caa atg cta gag atc tgc att gga 624
Gly Thr Ser Met Lys Ala Leu Ile Gln Met Leu Glu Ile Cys Ile Gly
        195                 200                 205

ttt gta gta ccc ttt ctt att atg ggg gtg tgc tac ttt atc aca gca 672
Phe Val Val Pro Phe Leu Ile Met Gly Val Cys Tyr Phe Ile Thr Ala
    210                 215                 220

agg aca ctc atg aag atg cca aac att aaa ata tct cga ccc cta aaa 720
Arg Thr Leu Met Lys Met Pro Asn Ile Lys Ile Ser Arg Pro Leu Lys
225                 230                 235                 240

gtt ctg ctc aca gtc gtt ata gtt ttc att gtc act caa ctg cct tat 768
Val Leu Leu Thr Val Val Ile Val Phe Ile Val Thr Gln Leu Pro Tyr
                245                 250                 255

aac att gtc aag ttc tgc cga gcc ata gac atc atc tac tcc ctg atc 816
Asn Ile Val Lys Phe Cys Arg Ala Ile Asp Ile Ile Tyr Ser Leu Ile
            260                 265                 270

acc agc tgc aac atg agc aaa cgc atg gac atc gcc atc caa gtc aca 864
Thr Ser Cys Asn Met Ser Lys Arg Met Asp Ile Ala Ile Gln Val Thr
        275                 280                 285

gaa agc atc gca ctc ttt cac agc tgc ctc aac cca atc ctt tat gtt 912
Glu Ser Ile Ala Leu Phe His Ser Cys Leu Asn Pro Ile Leu Tyr Val
    290                 295                 300

ttt atg gga gca tct ttc aaa aac tac gtt atg aaa gtg gcc aag aaa 960
Phe Met Gly Ala Ser Phe Lys Asn Tyr Val Met Lys Val Ala Lys Lys
305                 310                 315                 320

tat ggg tcc tgg aga aga cag aga caa agt gtg gag gag ttt cct ttt 1008
Tyr Gly Ser Trp Arg Arg Gln Arg Gln Ser Val Glu Glu Phe Pro Phe
                325                 330                 335

gat tct gag ggt cct aca gag cca acc agt act ttt agc att 1050
Asp Ser Glu Gly Pro Thr Glu Pro Thr Ser Thr Phe Ser Ile
            340                 345                 350

taaaggtaaa actgctctgc cttttgcttg gatacatatg aatgatgctt tcccctcaaa 1110
taa 1113

<210> 8
<211> 350
<212> PRT
<213> Homo sapiens

<400> 8
Met Ala Leu Glu Gln Asn Gln Ser Thr Asp Tyr Tyr Tyr Glu Glu Asn
1               5                   10                  15
Glu Met Asn Gly Thr Tyr Asp Tyr Ser Gln Tyr Glu Leu Ile Cys Ile
            20                  25                  30
Lys Glu Asp Val Arg Glu Phe Ala Lys Val Phe Leu Pro Val Phe Leu
        35                  40                  45
Thr Ile Val Phe Val Ile Gly Leu Ala Gly Asn Ser Met Val Val Ala
    50                  55                  60

Ile Tyr Ala Tyr Tyr Lys Lys Gln Arg Thr Lys Thr Asp Val Tyr Ile
65                  70                  75                  80
Leu Asn Leu Ala Val Ala Asp Leu Leu Leu Leu Phe Thr Leu Pro Phe
                85                  90                  95
Trp Ala Val Asn Ala Val His Gly Trp Val Leu Gly Lys Ile Met Cys
            100                 105                 110
Lys Ile Thr Ser Ala Leu Tyr Thr Leu Asn Phe Val Ser Gly Met Gln
        115                 120                 125
Phe Leu Ala Cys Ile Ser Ile Asp Arg Tyr Val Ala Val Thr Lys Val
    130                 135                 140
Pro Ser Gln Ser Gly Val Gly Lys Pro Cys Trp Ile Ile Cys Phe Cys
145                 150                 155                 160
Val Trp Met Ala Ala Ile Leu Leu Ser Ile Pro Gln Leu Val Phe Tyr
                165                 170                 175
Thr Val Asn Asp Asn Ala Arg Cys Ile Pro Ile Phe Pro Arg Tyr Leu
            180                 185                 190
Gly Thr Ser Met Lys Ala Leu Ile Gln Met Leu Glu Ile Cys Ile Gly
        195                 200                 205
Phe Val Val Pro Phe Leu Ile Met Gly Val Cys Tyr Phe Ile Thr Ala
    210                 215                 220
Arg Thr Leu Met Lys Met Pro Asn Ile Lys Ile Ser Arg Pro Leu Lys
225                 230                 235                 240
Val Leu Leu Thr Val Val Ile Val Phe Ile Val Thr Gln Leu Pro Tyr
                245                 250                 255
Asn Ile Val Lys Phe Cys Arg Ala Ile Asp Ile Ile Tyr Ser Leu Ile
            260                 265                 270
Thr Ser Cys Asn Met Ser Lys Arg Met Asp Ile Ala Ile Gln Val Thr
        275                 280                 285
Glu Ser Ile Ala Leu Phe His Ser Cys Leu Asn Pro Ile Leu Tyr Val
    290                 295                 300
Phe Met Gly Ala Ser Phe Lys Asn Tyr Val Met Lys Val Ala Lys Lys
305                 310                 315                 320
Tyr Gly Ser Trp Arg Arg Gln Arg Gln Ser Val Glu Glu Phe Pro Phe
                325                 330                 335
Asp Ser Glu Gly Pro Thr Glu Pro Thr Ser Thr Phe Ser Ile
            340                 345                 350

<210> 9
<211> 1053
<212> DNA
<213> Homo sapiens

<400> 9
atggctttgg aacagaacca gtcaacagat tattattatg aggaaaatga aatgaatggc      60
acttatgact acagtcaata tgaactgatc tgtatcaaag aagatgtcag agaatttgca     120
aaagttttcc tccctgtatt cctcacaata gttttcgtca ttggacttgc aggcaattcc     180
atggtagtgg caatttatgc ctattacaag aaacagagaa ccaaaacaga tgtgtacatc     240
ctgaatttgg ctgtagcaga tttactcctt ctattcactc tgcctttttg ggctgttaat     300
gcagttcatg ggtgggtttt agggaaaata atgtgcaaaa taacttcagc cttgtacaca     360
ctaaactttg tctctggaat gcagtttctg gcttgtatca gcatagacag atatgtggca     420
gtaactaaag tccccagcca atcaggagtg ggaaaaccat gctggatcat ctgtttctgt     480
gtctggatgg ctgccatctt gctgagcata ccccagctgg ttttttatac agtaaatgac     540
aatgctaggt gcattcccat tttcccccgc tacctaggaa catcaatgaa agcattgatt     600
caaatgctag agatctgcat tggatttgta gtaccctttc ttattatggg ggtgtgctac     660
tttatcacag caaggacact catgaagatg ccaaacatta aaatatctcg acccctaaaa     720
gttctgctca cagtcgttat agttttcatt gtcactcaac tgccttataa cattgtcaag     780
ttctgccgag ccatagacat catctactcc ctgatcacca gctgcaacat gagcaaacgc     840
atggacatcg ccatccaagt cacagaaagc atcgcactct ttcacagctg cctcaaccca     900
atcctttatg tttttatggg agcatctttc aaaaactacg ttatgaaagt ggccaagaaa     960
tatgggtcct ggagaagaca gagacaaagt gtggaggagt ttccttttga ttctgagggt    1020
cctacagagc caaccagtac ttttagcatt taa                                 1053

<210> 10
<211> 1109
<212> DNA
<213> Homo sapiens

<220>
<221> CDS
<222> (326)...(994)

<400> 10
   ccatgcgtcc acgcgtccgg gaagaggaaa gaggtgccct gctgatgata tatgtaatca 60
   tgacttagtg acatatcaca agcataggaa gcttctggtt cagtggtcct caaatgcaac 120
   tcacaaaata tcatctgggg aagttctgaa ttaagtcgtt gagagtgtta cctgggcatc 180
   aggatattta aaatgagaat ctacagttgt aggcaagccg ttcaccatca tctcttgctt 240
   ttgtcaccgc tgggtgtttg gctggatcgg ctgccgctgg tatggatggg ctggattttt 300
   cyttggctgt ggaagcctta tcacc atg act gct gtc agc ctg gat cga tat 352
   Met Thr Ala Val Ser Leu Asp Arg Tyr
   1 5
   ttg aaa atc tgc tat tta tct tat cac gcc tac atc tgc ctg gca gcc 400
   Leu Lys Ile Cys Tyr Leu Ser Tyr His Ala Tyr Ile Cys Leu Ala Ala
   10 15 20 25
   atc tgg gcc tat gct tcc ttc tgg acc acc atg ccc ttg gta ggt ctg 448
   Ile Trp Ala Tyr Ala Ser Phe Trp Thr Thr Met Pro Leu Val Gly Leu
   30 35 40
   ggg gac tac gta cct gag ccc ttc gga acc tcg tgc acc ctg gac tgg 496
   Gly Asp Tyr Val Pro Glu Pro Phe Gly Thr Ser Cys Thr Leu Asp Trp
   45 50 55
   tgg ctg gcc cag gcc tcg gta ggg ggc cag gtt ttc atc ctg aac atc 544
   Trp Leu Ala Gln Ala Ser Val Gly Gly Gln Val Phe Ile Leu Asn Ile
   60 65 70
   ctc ttc ttc tgc ctc ttg ctc cca acg gct gtg atc gtg ttc tcc tac 592
   Leu Phe Phe Cys Leu Leu Leu Pro Thr Ala Val Ile Val Phe Ser Tyr
   75 80 85
   gta aag atc att gcc aag gtt aag tcc tct tcc aaa gaa gta gct cat 640
   Val Lys Ile Ile Ala Lys Val Lys Ser Ser Ser Lys Glu Val Ala His
   90 95 100 105
   ttc gac agt cgg atc cat agc agc cat gtg ctg gaa atg aaa ctg aca 688
   Phe Asp Ser Arg Ile His Ser Ser His Val Leu Glu Met Lys Leu Thr
   110 115 120
   aag gta gcg atg ttg att tgt gct gga ttc ctg att gcc tgg att cct 736
   Lys Val Ala Met Leu Ile Cys Ala Gly Phe Leu Ile Ala Trp Ile Pro
   125 130 135
   tat gca gtg gtg tct gtg tgg tca gct ttt gga agg cca gac tcc att 784
   Tyr Ala Val Val Ser Val Trp Ser Ala Phe Gly Arg Pro Asp Ser Ile
   140 145 150
   ccc ata cag ctc tct gtg gtg cca acc cta ctt gca aaa tct gca gcg 832
   Pro Ile Gln Leu Ser Val Val Pro Thr Leu Leu Ala Lys Ser Ala Ala
   155 160 165
   atg tac aat ccc atc att tac caa gtt att gat tac aaa ttt gcc tgt 880
   Met Tyr Asn Pro Ile Ile Tyr Gln Val Ile Asp Tyr Lys Phe Ala Cys
   170                 175                 180                 185
   tgc caa act ggt ggt ttg aaa gca acc aag aag aag tct ctg gaa ggc      928
   Cys Gln Thr Gly Gly Leu Lys Ala Thr Lys Lys Lys Ser Leu Glu Gly
                   190                 195                 200
   ttc agg ctg cac acc gta acc aca gtc agg aag tct tct gct gtg ctg      976
   Phe Arg Leu His Thr Val Thr Thr Val Arg Lys Ser Ser Ala Val Leu
               205                 210                 215
   gaa att cat gaa gag gta tgaagatgga tacagcatca ctatggacac            1024
   Glu Ile His Glu Glu Val
           220
   tcgtattcac ttatttgcct cttcactgct gtaaacattt gattgtggcc ccaaaaaaaa   1084
   aaaaaaaaaa aaaaaaaatt gcggc                                          1109

<210> 11
<211> 223
<212> PRT
<213> Homo sapiens

<400> 11
Met Thr Ala Val Ser Leu Asp Arg Tyr Leu Lys Ile Cys Tyr Leu Ser
 1               5                  10                  15
Tyr His Ala Tyr Ile Cys Leu Ala Ala Ile Trp Ala Tyr Ala Ser Phe
            20                  25                  30
Trp Thr Thr Met Pro Leu Val Gly Leu Gly Asp Tyr Val Pro Glu Pro
        35                  40                  45
Phe Gly Thr Ser Cys Thr Leu Asp Trp Trp Leu Ala Gln Ala Ser Val
    50                  55                  60
Gly Gly Gln Val Phe Ile Leu Asn Ile Leu Phe Phe Cys Leu Leu Leu
65                  70                  75                  80
Pro Thr Ala Val Ile Val Phe Ser Tyr Val Lys Ile Ile Ala Lys Val
                85                  90                  95
Lys Ser Ser Ser Lys Glu Val Ala His Phe Asp Ser Arg Ile His Ser
            100                 105                 110
Ser His Val Leu Glu Met Lys Leu Thr Lys Val Ala Met Leu Ile Cys
        115                 120                 125
Ala Gly Phe Leu Ile Ala Trp Ile Pro Tyr Ala Val Val Ser Val Trp
    130                 135                 140
Ser Ala Phe Gly Arg Pro Asp Ser Ile Pro Ile Gln Leu Ser Val Val
145                 150                 155                 160
Pro Thr Leu Leu Ala Lys Ser Ala Ala Met Tyr Asn Pro Ile Ile Tyr
                165                 170                 175
Gln Val Ile Asp Tyr Lys Phe Ala Cys Cys Gln Thr Gly Gly Leu Lys
            180                 185                 190
Ala Thr Lys Lys Lys Ser Leu Glu Gly Phe Arg Leu His Thr Val Thr
        195                 200                 205
Thr Val Arg Lys Ser Ser Ala Val Leu Glu Ile His Glu Glu Val
    210                 215                 220

<210> 12
<211> 672
<212> DNA
<213> Homo sapiens

<400> 12
atgactgctg tcagcctgga tcgatatttg aaaatctgct atttatctta tcacgcctac      60
atctgcctgg cagccatctg ggcctatgct tccttctgga ccaccatgcc cttggtaggt     120

ctgggggact acgtacctga gcccttcgga acctcgtgca ccctggactg gtggctggcc 180
caggcctcgg tagggggcca ggttttcatc ctgaacatcc tcttcttctg cctcttgctc 240
ccaacggctg tgatcgtgtt ctcctacgta aagatcattg ccaaggttaa gtcctcttcc 300
aaagaagtag ctcatttcga cagtcggatc catagcagcc atgtgctgga aatgaaactg 360
acaaaggtag cgatgttgat ttgtgctgga ttcctgattg cctggattcc ttatgcagtg 420
gtgtctgtgt ggtcagcttt tggaaggcca gactccattc ccatacagct ctctgtggtg 480
ccaaccctac ttgcaaaatc tgcagcgatg tacaatccca tcatttacca agttattgat 540
tacaaatttg cctgttgcca aactggtggt ttgaaagcaa ccaagaagaa gtctctggaa 600
ggcttcaggc tgcacaccgt aaccacagtc aggaagtctt ctgctgtgct ggaaattcat 660
gaagaggtat ga 672

<210> 13
<211> 3489
<212> DNA
<213> Homo sapiens

<220>
<221> CDS
<222> (52)...(2607)

<221> misc_feature
<222> (1)...(3489)
<223> n = A,T,C or G

<400> 13
ttactatagg gagtcgaccc actgcgtccg ctgttggaag ttgcctctgc c atg ctg 57
Met Leu
1

ggc cct gct gtc ctg ggc ctc agc ctc tgg gct ctc ctg cac cct ggg 105
Gly Pro Ala Val Leu Gly Leu Ser Leu Trp Ala Leu Leu His Pro Gly
        5                   10                  15

acg ggg gcc cca ttg tgc ctg tca cag caa ctt agg atg aag ggg gac 153
Thr Gly Ala Pro Leu Cys Leu Ser Gln Gln Leu Arg Met Lys Gly Asp
    20                  25                  30

tac gtg ctg ggg ggg ctg ttc ccc ctg ggc gag gcc gag gag gct ggc 201
Tyr Val Leu Gly Gly Leu Phe Pro Leu Gly Glu Ala Glu Glu Ala Gly
35                  40                  45                  50

ctc cgc agc cgg aca cgg ccc agc agc cct gtg tgc acc agg ttc tcc 249
Leu Arg Ser Arg Thr Arg Pro Ser Ser Pro Val Cys Thr Arg Phe Ser
                55                  60                  65

tca aac ggc ctg ctc tgg gca ctg gcc atg aaa atg gcc gtg gag gag 297
Ser Asn Gly Leu Leu Trp Ala Leu Ala Met Lys Met Ala Val Glu Glu
            70                  75                  80

atc aac aac aag tcg gat ctg ctg ccc ggg ctg cgc ctg ggc tac gac 345
Ile Asn Asn Lys Ser Asp Leu Leu Pro Gly Leu Arg Leu Gly Tyr Asp
        85                  90                  95

ctc ttt gat acg tgc tcg gag cct gtg gtg gcc atg aag ccc agc ctc 393
Leu Phe Asp Thr Cys Ser Glu Pro Val Val Ala Met Lys Pro Ser Leu
    100                 105                 110

atg ttc ctg gcc aag gca ggc agc cgc gac atc gcc gcc tac tgc aac 441
Met Phe Leu Ala Lys Ala Gly Ser Arg Asp Ile Ala Ala Tyr Cys Asn
115                 120                 125                 130

tac acg cag tac cag ccc cgt gtg ctg gct gtc atc ggg ccc cac tcg 489
Tyr Thr Gln Tyr Gln Pro Arg Val Leu Ala Val Ile Gly Pro His Ser
                135                 140                 145

tca gag ctc gcc atg gtc acc ggc aag ttc ttc agc ttc ttc ctc atg 537
Ser Glu Leu Ala Met Val Thr Gly Lys Phe Phe Ser Phe Phe Leu Met
            150                 155                 160

ccc cag gtc agc tac ggt gct agc atg gag ctg ctg agc gcc cgg gag 585
Pro Gln Val Ser Tyr Gly Ala Ser Met Glu Leu Leu Ser Ala Arg Glu
        165                 170                 175

acc ttc ccc tcc ttc ttc cgc acc gtg ccc agc gac cgt gtg cag ctg 633
Thr Phe Pro Ser Phe Phe Arg Thr Val Pro Ser Asp Arg Val Gln Leu
    180                 185                 190

acg gcc gcc gcg gag ctg ctg cag gag ttc ggc tgg aac tgg gtg gcc 681
Thr Ala Ala Ala Glu Leu Leu Gln Glu Phe Gly Trp Asn Trp Val Ala
195                 200                 205                 210

gcc ctg ggc agc gac gac gag tac ggc cgg cag ggc ctg agc atc ttc 729
Ala Leu Gly Ser Asp Asp Glu Tyr Gly Arg Gln Gly Leu Ser Ile Phe
                215                 220                 225

tcg gcc ctg gcc gcg gca cgc ggc atc tgc atc gcg cac gag ggc ctg 777
Ser Ala Leu Ala Ala Ala Arg Gly Ile Cys Ile Ala His Glu Gly Leu
            230                 235                 240

gtg ccg ctg ccc cgt gcc gat gac tcg cgg ctg ggg aag gtg cag gac 825
Val Pro Leu Pro Arg Ala Asp Asp Ser Arg Leu Gly Lys Val Gln Asp
        245                 250                 255

gtc ctg cac cag gtg aac cag agc agc gtg cag gtg gtg ctg ctg ttc 873
Val Leu His Gln Val Asn Gln Ser Ser Val Gln Val Val Leu Leu Phe
    260                 265                 270

gcc tcc gtg cac gcc gcc cac gcc ctc ttc aac tac agc atc agc agc 921
Ala Ser Val His Ala Ala His Ala Leu Phe Asn Tyr Ser Ile Ser Ser
275                 280                 285                 290

agg ctc tcg ccc aag gtg tgg gtg gcc agc gag gcc tgg ctg acc tct 969
Arg Leu Ser Pro Lys Val Trp Val Ala Ser Glu Ala Trp Leu Thr Ser
                295                 300                 305

gac ctg gtc atg ggg ctg ccc ggc atg gcc cag atg ggc acg gtg ctt 1017
Asp Leu Val Met Gly Leu Pro Gly Met Ala Gln Met Gly Thr Val Leu
            310                 315                 320

ggc ttc ctc cag agg ggt gcc cag ctg cac gag ttc ccc cag tac gtg 1065
Gly Phe Leu Gln Arg Gly Ala Gln Leu His Glu Phe Pro Gln Tyr Val
        325                 330                 335

aag acg cac ctg gcc ctg gcc acc gac ccg gcc ttc tgc tct gcc ctg 1113
Lys Thr His Leu Ala Leu Ala Thr Asp Pro Ala Phe Cys Ser Ala Leu
    340                 345                 350

ggc gag agg gag cag ggt ctg gag gag gac gtg gtg ggc cag cgc tgc 1161
Gly Glu Arg Glu Gln Gly Leu Glu Glu Asp Val Val Gly Gln Arg Cys
355                 360                 365                 370

ccg cag tgt gac tgc atc acg ctg cag aac gtg agc gca ggg cta aat 1209

Pro Gln Cys Asp Cys Ile Thr Leu Gln Asn Val Ser Ala Gly Leu Asn
                375                 380                 385

cac cac cag acg ttc tct gtc tac gca gct gtg tat agc gtg gcc cag      1257
His His Gln Thr Phe Ser Val Tyr Ala Ala Val Tyr Ser Val Ala Gln
            390                 395                 400

gcc ctg cac aac act ctt cag tgc aac gcc tca ggc tgc ccc gcg cag      1305
Ala Leu His Asn Thr Leu Gln Cys Asn Ala Ser Gly Cys Pro Ala Gln
        405                 410                 415

gac ccc gtg aag ccc tgg cag ctc ctg gag aac atg tac aac ctg acc      1353
Asp Pro Val Lys Pro Trp Gln Leu Leu Glu Asn Met Tyr Asn Leu Thr
    420                 425                 430

ttc cac gtg ggc ggg ctg ccg ctg cgg ttc gac agc agc gga aac gtg      1401
Phe His Val Gly Gly Leu Pro Leu Arg Phe Asp Ser Ser Gly Asn Val
435                 440                 445                 450

gac atg gag tac gac ctg aag ctg tgg gtg tgg cag ggc tca gtg ccc      1449
Asp Met Glu Tyr Asp Leu Lys Leu Trp Val Trp Gln Gly Ser Val Pro
                455                 460                 465

agg ctc cac gac gtg ggc agg ttc aac ggc agc ctc agg aca gag cgc      1497
Arg Leu His Asp Val Gly Arg Phe Asn Gly Ser Leu Arg Thr Glu Arg
            470                 475                 480

ctg aag atc cgc tgg cac acg tct gac aac cag aag ccc gtg tcc cga      1545
Leu Lys Ile Arg Trp His Thr Ser Asp Asn Gln Lys Pro Val Ser Arg
        485                 490                 495

tgc tcg cgg cag tgc cag gag ggc cag gtg cgc cgg gtc aag ggg ttc      1593
Cys Ser Arg Gln Cys Gln Glu Gly Gln Val Arg Arg Val Lys Gly Phe
    500                 505                 510

cac tcc tgc tgc tac gac tgt gtg gac tgc gag gcg ggc agc tac cgg      1641
His Ser Cys Cys Tyr Asp Cys Val Asp Cys Glu Ala Gly Ser Tyr Arg
515                 520                 525                 530

caa aac cca gac gac atc gcc tgc acc ttt tgt ggc cag gat gag tgg      1689
Gln Asn Pro Asp Asp Ile Ala Cys Thr Phe Cys Gly Gln Asp Glu Trp
                535                 540                 545

tcc ccg gag cga agc aca cgc tgc ttc cgc cgc agg tct cgg ttc ctg      1737
Ser Pro Glu Arg Ser Thr Arg Cys Phe Arg Arg Arg Ser Arg Phe Leu
            550                 555                 560

gca tgg ggc gag ccg gct gtg ctg ctg ctg ctc ctg ctg ctg agc ctg      1785
Ala Trp Gly Glu Pro Ala Val Leu Leu Leu Leu Leu Leu Leu Ser Leu
        565                 570                 575

gcg ctg ggc ctt gtg ctg gct gct ttg ggg ctg ttc gtt cac cat cgg      1833
Ala Leu Gly Leu Val Leu Ala Ala Leu Gly Leu Phe Val His His Arg
    580                 585                 590

gac agc cca ctg gtt cag gcc tcg ggg ggg ccc ctg gcc tgc ttt ggc      1881
Asp Ser Pro Leu Val Gln Ala Ser Gly Gly Pro Leu Ala Cys Phe Gly
595                 600                 605                 610

ctg gtg tgc ctg ggc ctg gtc tgc ctc agc gtc ctc ctg ttc cct ggc      1929
Leu Val Cys Leu Gly Leu Val Cys Leu Ser Val Leu Leu Phe Pro Gly

                615                 620                 625

cag ccc agc cct gcc cga tgc ctg gcc cag cag ccc ttg tcc cac ctc 1977
Gln Pro Ser Pro Ala Arg Cys Leu Ala Gln Gln Pro Leu Ser His Leu
            630                 635                 640

ccg ctc acg ggc tgc ctg agc aca ctc ttc ctg cag gcg gcc gag atc 2025
Pro Leu Thr Gly Cys Leu Ser Thr Leu Phe Leu Gln Ala Ala Glu Ile
        645                 650                 655

ttc gtg gag tca gaa ctg cct ctg agc tgg gca gac cgg ctg agt ggc 2073
Phe Val Glu Ser Glu Leu Pro Leu Ser Trp Ala Asp Arg Leu Ser Gly
    660                 665                 670

tgc ctg cgg ggg ccc tgg gcc tgg ctg gtg gtg ctg ctg gcc atg ctg 2121
Cys Leu Arg Gly Pro Trp Ala Trp Leu Val Val Leu Leu Ala Met Leu
675                 680                 685                 690

gtg gag gtc gca ctg tgc acc tgg tac ctg gtg gcc ttc ccg ccg gag 2169
Val Glu Val Ala Leu Cys Thr Trp Tyr Leu Val Ala Phe Pro Pro Glu
                695                 700                 705

gtg gtg acg gac tgg cac atg ctg ccc acg gag gcg ctg gtg cac tgc 2217
Val Val Thr Asp Trp His Met Leu Pro Thr Glu Ala Leu Val His Cys
            710                 715                 720

cgc aca cgc tcc tgg gtc agc ttc ggc cta gcg cac gcc acc aat gcc 2265
Arg Thr Arg Ser Trp Val Ser Phe Gly Leu Ala His Ala Thr Asn Ala
        725                 730                 735

acg ctg gcc ttt ctc tgc ttc ctg ggc act ttc ctg gtg cgg agc cag 2313
Thr Leu Ala Phe Leu Cys Phe Leu Gly Thr Phe Leu Val Arg Ser Gln
    740                 745                 750

ccg ggc cgc tac aac cgt gcc cgt ggc ctc acc ttt gcc atg ctg gcc 2361
Pro Gly Arg Tyr Asn Arg Ala Arg Gly Leu Thr Phe Ala Met Leu Ala
755                 760                 765                 770

tac ttc atc acc tgg gtc tcc ttt gtg ccc ctc ctg gcc aat gtg cag 2409
Tyr Phe Ile Thr Trp Val Ser Phe Val Pro Leu Leu Ala Asn Val Gln
                775                 780                 785

gtg gtc ctc agg ccc gcc gtg cag atg ggc gcc ctc ctg ctc tgt gtc 2457
Val Val Leu Arg Pro Ala Val Gln Met Gly Ala Leu Leu Leu Cys Val
            790                 795                 800

ctg ggc atc ctg gct gcc ttc cac ctg ccc agg tgt tac ctg ctc atg 2505
Leu Gly Ile Leu Ala Ala Phe His Leu Pro Arg Cys Tyr Leu Leu Met
        805                 810                 815

cgg cag cca ggg ctc aac acc ccc gag ttc ttc ctg gga ggg ggc cct 2553
Arg Gln Pro Gly Leu Asn Thr Pro Glu Phe Phe Leu Gly Gly Gly Pro
    820                 825                 830

ggg gat gcc caa ggc cag aat gac ggg aac aca gga aat cag ggg aaa 2601
Gly Asp Ala Gln Gly Gln Asn Asp Gly Asn Thr Gly Asn Gln Gly Lys
835                 840                 845                 850

cat gag tgacccaacc ctgtgatctc agccccggtg aacccagact tagctgcgat 2657
His Glu

cccccccaag ccagcaatga cccgtgtctc gctacagaga ccctcccgct ctaggttctg 2717
accccaggtt gtctcctgac cctgacccca cagtgagccc taggcctgga gcacgtggac 2777
acccctgtga ccatctgggc cccagagcca agctgtgtcc ctgtccctct gtgcccagac 2837
caggcctgcc caggtaaccc agacccactg ttctggaaag aggcccggag ggctcccagg 2897
gtacccgcaa cccacaccgt gagctcagga aaaggacgca gggaggcccc ggccagatgg 2957
ctggaagccc aaatcaggcc ctgccgacct gaccatgtcc caccagggcc cccatcctgc 3017
accctgccag gcaccacagc agtgggaggc caggtggggg cacacaggca tatgcccagg 3077
gcagagcccg ccgaggtggg ggtggcaccc agcttcctac tctgcccctt gcccagtggg 3137
tagacagcat catgactgtc accagtacca gggacagagc ccaggtgggg tgggggcggg 3197
gtccagcacc acggccagca ccgaccacca ggaccccgga gccagcacca tggacagaaa 3257
actgcccacc aggatctgac gccagcacgc cgccaggccc acacagggtc tccggtcaga 3317
gtcccagggt cagctcccag cagggcctag gggaggctgg accagctccc tgtgcctcat 3377
tccaaggcag cccagccgga gagaaggggc acaggccnca catctgtccc ataaaattaa 3437
acgcttttta gtgtttnaaa tannnnnaaa agggtttngg gcgcccgcta aa 3489

<210> 14
<211> 852
<212> PRT
<213> Homo sapiens

<400> 14
Met Leu Gly Pro Ala Val Leu Gly Leu Ser Leu Trp Ala Leu Leu His
1               5                   10                  15
Pro Gly Thr Gly Ala Pro Leu Cys Leu Ser Gln Gln Leu Arg Met Lys
            20                  25                  30
Gly Asp Tyr Val Leu Gly Gly Leu Phe Pro Leu Gly Glu Ala Glu Glu
        35                  40                  45
Ala Gly Leu Arg Ser Arg Thr Arg Pro Ser Ser Pro Val Cys Thr Arg
    50                  55                  60
Phe Ser Ser Asn Gly Leu Leu Trp Ala Leu Ala Met Lys Met Ala Val
65                  70                  75                  80
Glu Glu Ile Asn Asn Lys Ser Asp Leu Leu Pro Gly Leu Arg Leu Gly
                85                  90                  95
Tyr Asp Leu Phe Asp Thr Cys Ser Glu Pro Val Val Ala Met Lys Pro
            100                 105                 110
Ser Leu Met Phe Leu Ala Lys Ala Gly Ser Arg Asp Ile Ala Ala Tyr
        115                 120                 125
Cys Asn Tyr Thr Gln Tyr Gln Pro Arg Val Leu Ala Val Ile Gly Pro
    130                 135                 140
His Ser Ser Glu Leu Ala Met Val Thr Gly Lys Phe Phe Ser Phe Phe
145                 150                 155                 160
Leu Met Pro Gln Val Ser Tyr Gly Ala Ser Met Glu Leu Leu Ser Ala
                165                 170                 175
Arg Glu Thr Phe Pro Ser Phe Phe Arg Thr Val Pro Ser Asp Arg Val
            180                 185                 190
Gln Leu Thr Ala Ala Ala Glu Leu Leu Gln Glu Phe Gly Trp Asn Trp
        195                 200                 205
Val Ala Ala Leu Gly Ser Asp Asp Glu Tyr Gly Arg Gln Gly Leu Ser
    210                 215                 220
Ile Phe Ser Ala Leu Ala Ala Ala Arg Gly Ile Cys Ile Ala His Glu
225                 230                 235                 240
Gly Leu Val Pro Leu Pro Arg Ala Asp Asp Ser Arg Leu Gly Lys Val
                245                 250                 255
Gln Asp Val Leu His Gln Val Asn Gln Ser Ser Val Gln Val Val Leu
            260                 265                 270
Leu Phe Ala Ser Val His Ala Ala His Ala Leu Phe Asn Tyr Ser Ile
        275                 280                 285
Ser Ser Arg Leu Ser Pro Lys Val Trp Val Ala Ser Glu Ala Trp Leu
    290                 295                 300
Thr Ser Asp Leu Val Met Gly Leu Pro Gly Met Ala Gln Met Gly Thr

305                 310                 315                 320
Val Leu Gly Phe Leu Gln Arg Gly Ala Gln Leu His Glu Phe Pro Gln
                325                 330                 335
Tyr Val Lys Thr His Leu Ala Leu Ala Thr Asp Pro Ala Phe Cys Ser
            340                 345                 350
Ala Leu Gly Glu Arg Glu Gln Gly Leu Glu Glu Asp Val Val Gly Gln
        355                 360                 365
Arg Cys Pro Gln Cys Asp Cys Ile Thr Leu Gln Asn Val Ser Ala Gly
    370                 375                 380
Leu Asn His His Gln Thr Phe Ser Val Tyr Ala Ala Val Tyr Ser Val
385                 390                 395                 400
Ala Gln Ala Leu His Asn Thr Leu Gln Cys Asn Ala Ser Gly Cys Pro
                405                 410                 415
Ala Gln Asp Pro Val Lys Pro Trp Gln Leu Leu Glu Asn Met Tyr Asn
            420                 425                 430
Leu Thr Phe His Val Gly Gly Leu Pro Leu Arg Phe Asp Ser Ser Gly
        435                 440                 445
Asn Val Asp Met Glu Tyr Asp Leu Lys Leu Trp Val Trp Gln Gly Ser
    450                 455                 460
Val Pro Arg Leu His Asp Val Gly Arg Phe Asn Gly Ser Leu Arg Thr
465                 470                 475                 480
Glu Arg Leu Lys Ile Arg Trp His Thr Ser Asp Asn Gln Lys Pro Val
                485                 490                 495
Ser Arg Cys Ser Arg Gln Cys Gln Glu Gly Gln Val Arg Arg Val Lys
            500                 505                 510
Gly Phe His Ser Cys Cys Tyr Asp Cys Val Asp Cys Glu Ala Gly Ser
        515                 520                 525
Tyr Arg Gln Asn Pro Asp Asp Ile Ala Cys Thr Phe Cys Gly Gln Asp
    530                 535                 540
Glu Trp Ser Pro Glu Arg Ser Thr Arg Cys Phe Arg Arg Arg Ser Arg
545                 550                 555                 560
Phe Leu Ala Trp Gly Glu Pro Ala Val Leu Leu Leu Leu Leu Leu Leu
                565                 570                 575
Ser Leu Ala Leu Gly Leu Val Leu Ala Ala Leu Gly Leu Phe Val His
            580                 585                 590
His Arg Asp Ser Pro Leu Val Gln Ala Ser Gly Gly Pro Leu Ala Cys
        595                 600                 605
Phe Gly Leu Val Cys Leu Gly Leu Val Cys Leu Ser Val Leu Leu Phe
    610                 615                 620
Pro Gly Gln Pro Ser Pro Ala Arg Cys Leu Ala Gln Gln Pro Leu Ser
625                 630                 635                 640
His Leu Pro Leu Thr Gly Cys Leu Ser Thr Leu Phe Leu Gln Ala Ala
                645                 650                 655
Glu Ile Phe Val Glu Ser Glu Leu Pro Leu Ser Trp Ala Asp Arg Leu
            660                 665                 670
Ser Gly Cys Leu Arg Gly Pro Trp Ala Trp Leu Val Val Leu Leu Ala
        675                 680                 685
Met Leu Val Glu Val Ala Leu Cys Thr Trp Tyr Leu Val Ala Phe Pro
    690                 695                 700
Pro Glu Val Val Thr Asp Trp His Met Leu Pro Thr Glu Ala Leu Val
705                 710                 715                 720
His Cys Arg Thr Arg Ser Trp Val Ser Phe Gly Leu Ala His Ala Thr
                725                 730                 735
Asn Ala Thr Leu Ala Phe Leu Cys Phe Leu Gly Thr Phe Leu Val Arg
            740                 745                 750
Ser Gln Pro Gly Arg Tyr Asn Arg Ala Arg Gly Leu Thr Phe Ala Met
        755                 760                 765
Leu Ala Tyr Phe Ile Thr Trp Val Ser Phe Val Pro Leu Leu Ala Asn
    770                 775                 780
Val Gln Val Val Leu Arg Pro Ala Val Gln Met Gly Ala Leu Leu Leu
785                 790                 795                 800

Cys Val Leu Gly Ile Leu Ala Ala Phe His Leu Pro Arg Cys Tyr Leu
                805                 810                 815
Leu Met Arg Gln Pro Gly Leu Asn Thr Pro Glu Phe Phe Leu Gly Gly
                820                 825                 830
Gly Pro Gly Asp Ala Gln Gly Gln Asn Asp Gly Asn Thr Gly Asn Gln
        835                 840                 845
Gly Lys His Glu
    850

<210> 15
<211> 2559
<212> DNA
<213> Homo sapiens

<400> 15
atgctgggcc ctgctgtcct gggcctcagc ctctgggctc tcctgcaccc tgggacgggg      60
gccccattgt gcctgtcaca gcaacttagg atgaaggggg actacgtgct gggggggctg     120
ttccccctgg gcgaggccga ggaggctggc ctccgcagcc ggacacggcc cagcagccct     180
gtgtgcacca ggttctcctc aaacggcctg ctctgggcac tggccatgaa aatggccgtg     240
gaggagatca acaacaagtc ggatctgctg cccgggctgc gcctgggcta cgacctcttt     300
gatacgtgct cggagcctgt ggtggccatg aagcccagcc tcatgttcct ggccaaggca     360
ggcagccgcg acatcgccgc ctactgcaac tacacgcagt accagccccg tgtgctggct     420
gtcatcgggc cccactcgtc agagctcgcc atggtcaccg gcaagttctt cagcttcttc     480
ctcatgcccc aggtcagcta cggtgctagc atggagctgc tgagcgcccg ggagaccttc     540
ccctccttct tccgcaccgt gcccagcgac cgtgtgcagc tgacggccgc cgcggagctg     600
ctgcaggagt tcggctggaa ctgggtggcc gccctgggca gcgacgacga gtacggccgg     660
cagggcctga gcatcttctc ggccctggcc gcggcacgcg gcatctgcat cgcgcacgag     720
ggcctggtgc cgctgccccg tgccgatgac tcgcggctgg ggaaggtgca ggacgtcctg     780
caccaggtga accagagcag cgtgcaggtg gtgctgctgt tcgcctccgt gcacgccgcc     840
cacgccctct tcaactacag catcagcagc aggctctcgc ccaaggtgtg ggtggccagc     900
gaggcctggc tgacctctga cctggtcatg gggctgcccg gcatggccca gatgggcacg     960
gtgcttggct tcctccagag gggtgcccag ctgcacgagt tcccccagta cgtgaagacg    1020
cacctggccc tggccaccga cccggccttc tgctctgccc tgggcgagag ggagcagggt    1080
ctggaggagg acgtggtggg ccagcgctgc ccgcagtgtg actgcatcac gctgcagaac    1140
gtgagcgcag ggctaaatca ccaccagacg ttctctgtct acgcagctgt gtatagcgtg    1200
gcccaggccc tgcacaacac tcttcagtgc aacgcctcag gctgccccgc gcaggacccc    1260
gtgaagccct ggcagctcct ggagaacatg tacaacctga ccttccacgt gggcgggctg    1320
ccgctgcggt tcgacagcag cggaaacgtg gacatggagt acgacctgaa gctgtgggtg    1380
tggcagggct cagtgcccag gctccacgac gtgggcaggt tcaacggcag cctcaggaca    1440
gagcgcctga agatccgctg gcacacgtct gacaaccaga agcccgtgtc ccgatgctcg    1500
cggcagtgcc aggagggcca ggtgcgccgg gtcaaggggt tccactcctg ctgctacgac    1560
tgtgtggact gcgaggcggg cagctaccgg caaaacccag acgacatcgc ctgcaccttt    1620
tgtggccagg atgagtggtc cccggagcga agcacacgct gcttccgccg caggtctcgg    1680
ttcctggcat ggggcgagcc ggctgtgctg ctgctgctcc tgctgctgag cctggcgctg    1740
ggccttgtgc tggctgcttt ggggctgttc gttcaccatc gggacagccc actggttcag    1800
gcctcggggg ggcccctggc ctgctttggc ctggtgtgcc tgggcctggt ctgcctcagc    1860
gtcctcctgt tccctggcca gcccagccct gcccgatgcc tggcccagca gcccttgtcc    1920
cacctcccgc tcacgggctg cctgagcaca ctcttcctgc aggcggccga gatcttcgtg    1980
gagtcagaac tgcctctgag ctgggcagac cggctgagtg gctgcctgcg ggggccctgg    2040
gcctggctgg tggtgctgct ggccatgctg gtggaggtcg cactgtgcac ctggtacctg    2100
gtggccttcc cgccggaggt ggtgacggac tggcacatgc tgcccacgga ggcgctggtg    2160
cactgccgca cacgctcctg ggtcagcttc ggcctagcgc acgccaccaa tgccacgctg    2220
gcctttctct gcttcctggg cactttcctg gtgcggagcc agccgggccg ctacaaccgt    2280
gcccgtggcc tcacctttgc catgctggcc tacttcatca cctgggtctc ctttgtgccc    2340
ctcctggcca atgtgcaggt ggtcctcagg cccgccgtgc agatgggcgc cctcctgctc    2400
tgtgtcctgg gcatcctggc tgccttccac ctgcccaggt gttacctgct catgcggcag    2460
ccagggctca acacccccga gttcttcctg ggagggggcc ctggggatgc ccaaggccag    2520
aatgacggga acacaggaaa tcaggggaaa catgagtga                           2559

<210> 16
<211> 1609

<212> DNA
<213> Homo sapiens

<220>
<221> CDS
<222> (176)...(1369)

<400> 16
gtcgaccacg cgtccggagc ccagcacagc tgccctctgg accctgcgga ccccagcccg 60
agccccttcc tgagttccac aggcgcagcc cccgggcggt cgggcggagg ggtccccggg 120
gcggtgccag ggcgcaatcc tggagggcgg ccgggaggag gaggtgcgcg cggcc atg 178
Met
1

cac acc gtg gct acg tcc ggg ccc aac gcg tcc tgg ggg gca ccg gcc 226
His Thr Val Ala Thr Ser Gly Pro Asn Ala Ser Trp Gly Ala Pro Ala
            5                   10                  15

aac gcc tcc ggc tgc ccg ggc tgt ggc gcc aac gcc tcg gac ggc cca 274
Asn Ala Ser Gly Cys Pro Gly Cys Gly Ala Asn Ala Ser Asp Gly Pro
        20                  25                  30

gtc cct tcg ccg cgg gcc gtg gac gcc tgg ctc gtg ccg ctc ttc ttc 322
Val Pro Ser Pro Arg Ala Val Asp Ala Trp Leu Val Pro Leu Phe Phe
    35                  40                  45

gcg gcg ctg atg ctg ctg ggc ctg gtg ggg aac tcg ctg gtc atc tac 370
Ala Ala Leu Met Leu Leu Gly Leu Val Gly Asn Ser Leu Val Ile Tyr
50                  55                  60                  65

gtc atc tgc cgc cac aag ccg atg cgg acc gtg acc aac ttc tac atc 418
Val Ile Cys Arg His Lys Pro Met Arg Thr Val Thr Asn Phe Tyr Ile
                70                  75                  80

gcc aac ctg gcg gcc acg gac gtg acc ttc ctc ctg tgc tgc gtc ccc 466
Ala Asn Leu Ala Ala Thr Asp Val Thr Phe Leu Leu Cys Cys Val Pro
            85                  90                  95

ttc acg gcc ctg ctg tac ccg ctg ccc ggc tgg gtg ctg ggc gac ttc 514
Phe Thr Ala Leu Leu Tyr Pro Leu Pro Gly Trp Val Leu Gly Asp Phe
        100                 105                 110

atg tgc aag ttc gtc aac tac atc cag cag gtc tcg gtg cag gcc acg 562
Met Cys Lys Phe Val Asn Tyr Ile Gln Gln Val Ser Val Gln Ala Thr
    115                 120                 125

tgt gcc act ctg acc gcc atg agt gtg gac cgc tgg tac gtg acg gtg 610
Cys Ala Thr Leu Thr Ala Met Ser Val Asp Arg Trp Tyr Val Thr Val
130                 135                 140                 145

ttc ccg ttg cgc gcc ctg cac cgc cgc acg ccc cgc ctg gcg ctg gct 658
Phe Pro Leu Arg Ala Leu His Arg Arg Thr Pro Arg Leu Ala Leu Ala
                150                 155                 160

gtc agc ctc agc atc tgg gta ggc tct gcg gcg gtg tct gcg ccg gtg 706
Val Ser Leu Ser Ile Trp Val Gly Ser Ala Ala Val Ser Ala Pro Val
            165                 170                 175

ctc gcc ctg cac cgc ctg tca ccc ggg ccg cgc gcc tac tgc agt gag 754
Leu Ala Leu His Arg Leu Ser Pro Gly Pro Arg Ala Tyr Cys Ser Glu
        180                 185                 190

gcc ttc ccc agc cgc gcc ctg gag cgc gcc ttc gca ctg tac aac ctg      802
Ala Phe Pro Ser Arg Ala Leu Glu Arg Ala Phe Ala Leu Tyr Asn Leu
    195                 200                 205

ctg gcg ctg tac ctg ctg ccg ctc ctc gcc acc tgc gcc tgc tat gcg      850
Leu Ala Leu Tyr Leu Leu Pro Leu Leu Ala Thr Cys Ala Cys Tyr Ala
210                 215                 220                 225

gcc atg ctg cgc cac ctg ggc cgg gtc gcc gtg cgc ccc gcg ccc gcc      898
Ala Met Leu Arg His Leu Gly Arg Val Ala Val Arg Pro Ala Pro Ala
                230                 235                 240

gat agc gcc ctg cag ggg cag gtg ctg gca gag cgc gca ggc gcc gtg      946
Asp Ser Ala Leu Gln Gly Gln Val Leu Ala Glu Arg Ala Gly Ala Val
            245                 250                 255

cgg gcc aag gtc tcg cgg ctg gtg gcg gcc gtg gtc ctg ctc ttc gcc      994
Arg Ala Lys Val Ser Arg Leu Val Ala Ala Val Val Leu Leu Phe Ala
        260                 265                 270

gcc tgc tgg ggc ccc atc cag ctg ttc ctg gtg ctg cag gcg ctg ggc     1042
Ala Cys Trp Gly Pro Ile Gln Leu Phe Leu Val Leu Gln Ala Leu Gly
    275                 280                 285

ccc gcg ggc tcc tgg cac cca cgc agc tac gcc gcc tac gcg ctt aag     1090
Pro Ala Gly Ser Trp His Pro Arg Ser Tyr Ala Ala Tyr Ala Leu Lys
290                 295                 300                 305

acc tgg gct cac tgc atg tcc tac agc aac tcc gcg ctg aac ccg ctg     1138
Thr Trp Ala His Cys Met Ser Tyr Ser Asn Ser Ala Leu Asn Pro Leu
                310                 315                 320

ctc tac gcc ttc ctg ggc tcg cac ttc cga cag gcc ttc cgc cgc gtc     1186
Leu Tyr Ala Phe Leu Gly Ser His Phe Arg Gln Ala Phe Arg Arg Val
            325                 330                 335

tgc ccc tgc gcg ccg cgc cgc ccc cgc cgc ccc cgc cgg ccc gga ccc     1234
Cys Pro Cys Ala Pro Arg Arg Pro Arg Arg Pro Arg Arg Pro Gly Pro
        340                 345                 350

tcg gac ccc gca gcc cca cac gcg gag ctg cac cgc ctg ggg tcc cac     1282
Ser Asp Pro Ala Ala Pro His Ala Glu Leu His Arg Leu Gly Ser His
    355                 360                 365

ccg gcc ccc gcc agg gcg cag aag cca ggg agc agt ggg ctg gcc gcg     1330
Pro Ala Pro Ala Arg Ala Gln Lys Pro Gly Ser Ser Gly Leu Ala Ala
370                 375                 380                 385

cgc ggg ctg tgc gtc ctg ggg gag gac aac gcc cct ctc tgagcggacc      1379
Arg Gly Leu Cys Val Leu Gly Glu Asp Asn Ala Pro Leu
                390                 395

cggtgggaat ccgagcggct ccctcgggag cggggactgc tggaacagcg gctattcttc   1439
tgttattagt atttttctta ctgtccaaga tcaactgtgg aaatattttg gtctcttgtg   1499
acgttcggtg cagtttcgtt gtgaagtttg ctattgatat tgaaattatg acttctgtgt   1559
ttcctgaaat taaacatgtg tcaacaaaaa aaaaaaaaaa aaaagggcgg              1609

<210> 17
<211> 398
<212> PRT

<213> Homo sapiens

<400> 17
Met His Thr Val Ala Thr Ser Gly Pro Asn Ala Ser Trp Gly Ala Pro
1               5                   10                  15
Ala Asn Ala Ser Gly Cys Pro Gly Cys Gly Ala Asn Ala Ser Asp Gly
            20                  25                  30
Pro Val Pro Ser Pro Arg Ala Val Asp Ala Trp Leu Val Pro Leu Phe
        35                  40                  45
Phe Ala Ala Leu Met Leu Leu Gly Leu Val Gly Asn Ser Leu Val Ile
    50                  55                  60
Tyr Val Ile Cys Arg His Lys Pro Met Arg Thr Val Thr Asn Phe Tyr
65                  70                  75                  80
Ile Ala Asn Leu Ala Ala Thr Asp Val Thr Phe Leu Leu Cys Cys Val
                85                  90                  95
Pro Phe Thr Ala Leu Leu Tyr Pro Leu Pro Gly Trp Val Leu Gly Asp
            100                 105                 110
Phe Met Cys Lys Phe Val Asn Tyr Ile Gln Gln Val Ser Val Gln Ala
        115                 120                 125
Thr Cys Ala Thr Leu Thr Ala Met Ser Val Asp Arg Trp Tyr Val Thr
    130                 135                 140
Val Phe Pro Leu Arg Ala Leu His Arg Arg Thr Pro Arg Leu Ala Leu
145                 150                 155                 160
Ala Val Ser Leu Ser Ile Trp Val Gly Ser Ala Ala Val Ser Ala Pro
                165                 170                 175
Val Leu Ala Leu His Arg Leu Ser Pro Gly Pro Arg Ala Tyr Cys Ser
            180                 185                 190
Glu Ala Phe Pro Ser Arg Ala Leu Glu Arg Ala Phe Ala Leu Tyr Asn
        195                 200                 205
Leu Leu Ala Leu Tyr Leu Leu Pro Leu Leu Ala Thr Cys Ala Cys Tyr
    210                 215                 220
Ala Ala Met Leu Arg His Leu Gly Arg Val Ala Val Arg Pro Ala Pro
225                 230                 235                 240
Ala Asp Ser Ala Leu Gln Gly Gln Val Leu Ala Glu Arg Ala Gly Ala
                245                 250                 255
Val Arg Ala Lys Val Ser Arg Leu Val Ala Ala Val Val Leu Leu Phe
            260                 265                 270
Ala Ala Cys Trp Gly Pro Ile Gln Leu Phe Leu Val Leu Gln Ala Leu
        275                 280                 285
Gly Pro Ala Gly Ser Trp His Pro Arg Ser Tyr Ala Ala Tyr Ala Leu
    290                 295                 300
Lys Thr Trp Ala His Cys Met Ser Tyr Ser Asn Ser Ala Leu Asn Pro
305                 310                 315                 320
Leu Leu Tyr Ala Phe Leu Gly Ser His Phe Arg Gln Ala Phe Arg Arg
                325                 330                 335
Val Cys Pro Cys Ala Pro Arg Arg Pro Arg Arg Pro Arg Arg Pro Gly
            340                 345                 350
Pro Ser Asp Pro Ala Ala Pro His Ala Glu Leu His Arg Leu Gly Ser
        355                 360                 365
His Pro Ala Pro Ala Arg Ala Gln Lys Pro Gly Ser Ser Gly Leu Ala
    370                 375                 380
Ala Arg Gly Leu Cys Val Leu Gly Glu Asp Asn Ala Pro Leu
385                 390                 395

<210> 18
<211> 1197
<212> DNA
<213> Homo sapiens

<400> 18
atgcacaccg tggctacgtc cgggcccaac gcgtcctggg gggcaccggc caacgcctcc      60

ggctgcccgg gctgtggcgc caacgcctcg gacggcccag tcccttcgcc gcgggccgtg 120
gacgcctggc tcgtgccgct cttcttcgcg gcgctgatgc tgctgggcct ggtggggaac 180
tcgctggtca tctacgtcat ctgccgccac aagccgatgc ggaccgtgac caacttctac 240
atcgccaacc tggcggccac ggacgtgacc ttcctcctgt gctgcgtccc cttcacggcc 300
ctgctgtacc cgctgcccgg ctgggtgctg ggcgacttca tgtgcaagtt cgtcaactac 360
atccagcagg tctcggtgca ggccacgtgt gccactctga ccgccatgag tgtggaccgc 420
tggtacgtga cggtgttccc gttgcgcgcc ctgcaccgcc gcacgccccg cctggcgctg 480
gctgtcagcc tcagcatctg ggtaggctct gcggcggtgt ctgcgccggt gctcgccctg 540
caccgcctgt cacccgggcc gcgcgcctac tgcagtgagg ccttccccag ccgcgccctg 600
gagcgcgcct tcgcactgta caacctgctg gcgctgtacc tgctgccgct gctcgccacc 660
tgcgcctgct atgcggccat gctgcgccac ctgggccggg tcgccgtgcg ccccgcgccc 720
gccgatagcg ccctgcaggg gcaggtgctg gcagagcgcg caggcgccgt gcgggccaag 780
gtctcgcggc tggtggcggc cgtggtcctg ctcttcgccg cctgctgggg ccccatccag 840
ctgttcctgg tgctgcaggc gctgggcccc gcgggctcct ggcacccacg cagctacgcc 900
gcctacgcgc ttaagacctg ggctcactgc atgtcctaca gcaactccgc gctgaacccg 960
ctgctctacg ccttcctggg ctcgcacttc cgacaggcct tccgccgcgt ctgcccctgc 1020
gcgccgcgcc gcccccgccg cccccgccgg cccggaccct cggaccccgc agccccacac 1080
gcggagctgc accgcctggg gtcccacccg gcccccgcca gggcgcagaa gccagggagc 1140
agtgggctgg ccgcgcgcgg gctgtgcgtc ctgggggagg acaacgcccc tctctga 1197

<210> 19
<211> 268
<212> PRT
<213> Artificial Sequence

<220>
<223> consensus sequence

<400> 19
Ala Leu Leu Leu Lys Val Ile Tyr Thr Val Gly Tyr Ser Leu Ser Leu
1               5                   10                  15
Val Ala Leu Leu Leu Ala Ile Phe Ile Phe Leu Leu Phe Arg Arg Leu
            20                  25                  30
His Cys Thr Arg Asn Tyr Ile His Leu Asn Leu Phe Ile Ser Phe Ile
        35                  40                  45
Leu Arg Ala Leu Leu Phe Leu Ile Gly Asp Ala Val Leu Gln Asn Ser
    50                  55                  60
Val Gly Pro Glu Ser Leu His Cys Ser Asn Gln Val Gly Cys Lys Val
65                  70                  75                  80
Val Ala Val Phe Leu His Tyr Phe Phe Leu Ala Asn Phe Phe Trp Met
                85                  90                  95
Leu Val Glu Gly Leu Tyr Leu Tyr Thr Leu Leu Val Val Glu Val Phe
            100                 105                 110
Phe Ser Glu Arg Lys Arg Leu Arg Trp Tyr Ile Leu Ile Gly Trp Gly
        115                 120                 125
Val Pro Ala Val Phe Val Val Val Trp Ala Ile Val Arg Leu Ile Lys
    130                 135                 140
Pro Lys Gly Tyr Gly Glu Asp Asp Gly Cys Leu Trp Leu Ser Asn Glu
145                 150                 155                 160
Asp Asn Thr Gly Phe Trp Trp Ile Ile Lys Gly Pro Val Leu Leu Ala
                165                 170                 175
Ile Leu Val Asn Phe Ile Leu Phe Ile Asn Ile Leu Arg Ile Leu Val
            180                 185                 190
Thr Lys Leu Arg Glu Ser Asn Thr Gly Glu Ser Asp Gln Tyr Arg Leu
        195                 200                 205
Val Lys Ser Thr Leu Val Leu Leu Pro Leu Leu Gly Ile Thr Trp Ile
    210                 215                 220
Leu Phe Leu Phe Ala Pro Glu Asn Asp Ala Arg Gly Ile Ser Ser Val
225                 230                 235                 240
Val Phe Leu Tyr Leu Phe Leu Ile Leu Asn Ser Phe Gln Gly Phe Phe
                245                 250                 255

Val Ala Leu Leu Tyr Cys Phe Leu Asn Gly Glu Val
            260                 265

<210> 20
<211> 45
<212> PRT
<213> Artificial Sequence

<220>
<223> consensus sequence

<400> 20
Cys Ala Pro Asn Asn Pro Cys Ser Asn Gly Gly Thr Cys Val Asn Thr
 1               5                  10                  15
Pro Gly Gly Ser Ser Asp Asn Phe Gly Gly Tyr Thr Cys Glu Cys Pro
            20                  25                  30
Pro Gly Asp Tyr Tyr Leu Ser Tyr Thr Gly Lys Arg Cys
        35                  40                  45

<210> 21
<211> 54
<212> PRT
<213> Artificial Sequence

<220>
<223> consensus sequence

<400> 21
Ser Asn Pro Ile Cys Val Phe Trp Asp Glu Ser Glu Leu Ser Leu Gly
 1               5                  10                  15
Val Trp Ser Thr Asp Arg Gly Cys Glu Leu Val Glu Thr Ser Lys Pro
            20                  25                  30
Ser His Thr Thr Cys Ser Cys Asn His Leu Thr Ser Phe Ala Val Leu
        35                  40                  45
Met Asp Val Ser Pro Asn
    50

<210> 22
<211> 77
<212> PRT
<213> Artificial Sequence

<220>
<223> consensus sequence

<400> 22
Ser Ala Leu Cys Lys Leu Val Thr Ala Leu Asp Val Val Asn Met Tyr
 1               5                  10                  15
Ala Ser Ile Leu Leu Leu Thr Ala Ile Ser Ile Asp Arg Tyr Leu Ala
            20                  25                  30
Ile Val His Pro Leu Arg Tyr Arg Arg Arg Arg Thr Ser Pro Arg Arg
        35                  40                  45
Ala Lys Val Val Ile Leu Leu Val Trp Val Leu Ala Leu Leu Leu Ser
    50                  55                  60
Leu Pro Pro Leu Leu Phe Ser Trp Val Lys Thr Val Glu
65                  70                  75

<210> 23
<211> 259
<212> PRT
<213> Artificial Sequence

<220>
<223> consensus sequence

<400> 23
   Gly Asn Leu Leu Val Ile Leu Val Ile Leu Arg Thr Lys Lys Leu Arg
   1               5                   10                  15
   Thr Pro Thr Asn Ile Phe Ile Leu Asn Leu Ala Val Ala Asp Leu Leu
               20                  25                  30
   Phe Leu Leu Thr Leu Pro Pro Trp Ala Leu Tyr Tyr Leu Val Gly Gly
           35                  40                  45
   Ser Glu Asp Trp Pro Phe Gly Ser Ala Leu Cys Lys Leu Val Thr Ala
       50                  55                  60
   Leu Asp Val Val Asn Met Tyr Ala Ser Ile Leu Leu Leu Thr Ala Ile
   65                  70                  75                  80
   Ser Ile Asp Arg Tyr Leu Ala Ile Val His Pro Leu Arg Tyr Arg Arg
                   85                  90                  95
   Arg Arg Thr Ser Pro Arg Arg Ala Lys Val Val Ile Leu Leu Val Trp
               100                 105                 110
   Val Leu Ala Leu Leu Leu Ser Leu Pro Pro Leu Leu Phe Ser Trp Val
           115                 120                 125
   Lys Thr Val Glu Glu Gly Asn Gly Thr Leu Asn Val Asn Val Thr Val
       130                 135                 140
   Cys Leu Ile Asp Phe Pro Glu Glu Ser Thr Ala Ser Val Ser Thr Trp
   145                 150                 155                 160
   Leu Arg Ser Tyr Val Leu Leu Ser Thr Leu Val Gly Phe Leu Leu Pro
                   165                 170                 175
   Leu Leu Val Ile Leu Val Cys Tyr Thr Arg Ile Leu Arg Thr Leu Arg
               180                 185                 190
   Lys Ala Ala Lys Thr Leu Leu Val Val Val Val Val Phe Val Leu Cys
           195                 200                 205
   Trp Leu Pro Tyr Phe Ile Val Leu Leu Leu Asp Thr Leu Cys Leu Ser
       210                 215                 220
   Ile Ile Met Ser Ser Thr Cys Glu Leu Glu Arg Val Leu Pro Thr Ala
   225                 230                 235                 240
   Leu Leu Val Thr Leu Trp Leu Ala Tyr Val Asn Ser Cys Leu Asn Pro
                   245                 250                 255
   Ile Ile Tyr


<210> 24
<211> 183
<212> PRT
<213> Artificial Sequence

<220>
<223> consensus sequence

<400> 24
Leu Thr Ala Ile Ser Ile Asp Arg Tyr Leu Ala Ile Val His Pro Leu
1               5                   10                  15
Arg Tyr Arg Arg Arg Arg Thr Ser Pro Arg Arg Ala Lys Val Val Ile
            20                  25                  30
Leu Leu Val Trp Val Leu Ala Leu Leu Leu Ser Leu Pro Pro Leu Leu
        35                  40                  45
Phe Ser Trp Val Lys Thr Val Glu Glu Gly Asn Gly Thr Leu Asn Val
    50                  55                  60
Asn Val Thr Val Cys Leu Ile Asp Phe Pro Glu Glu Ser Thr Ala Ser
65                  70                  75                  80
Val Ser Thr Trp Leu Arg Ser Tyr Val Leu Leu Ser Thr Leu Val Gly
                85                  90                  95

Phe Leu Leu Pro Leu Leu Val Ile Leu Val Cys Tyr Thr Arg Ile Leu
            100                 105                 110
Arg Thr Leu Arg Lys Ala Ala Lys Thr Leu Leu Val Val Val Val Val
        115                 120                 125
Phe Val Leu Cys Trp Leu Pro Tyr Phe Ile Val Leu Leu Leu Asp Thr
    130                 135                 140
Leu Cys Leu Ser Ile Ile Met Ser Ser Thr Cys Glu Leu Glu Arg Val
145                 150                 155                 160
Leu Pro Thr Ala Leu Leu Val Thr Leu Trp Leu Ala Tyr Val Asn Ser
                165                 170                 175
Cys Leu Asn Pro Ile Ile Tyr
            180

<210> 25
<211> 464
<212> PRT
<213> Artificial Sequence

<220>
<223> consensus sequence

<400> 25
Gly Leu Ile Asn Glu Ala Val Arg Gly Ile Leu Arg Leu Glu Ala Met
 1               5                  10                  15
Leu Gly Ala Phe Asp Arg Ile Asn Ala Asp Pro Ala Leu Leu Pro Gly
            20                  25                  30
Leu Ala Leu Gly Leu Ala Ile Leu Asp Ile Asn Ser Leu Arg Thr Val
        35                  40                  45
Ala Leu Glu Gln Ser Phe Thr Phe Val Tyr Ala Leu Leu Ile Lys Ser
    50                  55                  60
Glu Cys Asp Cys Ser Ser Val Arg Cys Ala Ala Val Asp Leu Ala Leu
65                  70                  75                  80
Glu His Gly Val Ala Lys Lys Met Ile Val Gly Val Ile Gly Pro Ser
                85                  90                  95
Cys Ser Ser Ser Ala Ile Gln Val Ala Asn Leu Ala Ser Leu Leu Asn
            100                 105                 110
Ile Pro Met Ile Ser Tyr Ala Ser Thr Ala Pro Glu Leu Ser Asp Lys
        115                 120                 125
Asp Thr Arg Tyr Pro Thr Leu Ser Arg Thr Ile Pro Ser Asp Ala Lys
    130                 135                 140
Leu Gly Glu Ala Met Val Asp Asn Asn Ile Leu Lys His Phe Asn Trp
145                 150                 155                 160
Asn Arg Tyr Val Ser Leu Val Tyr Ser Asp Gly Asp Tyr Gly Glu Glu
                165                 170                 175
Gly Cys Glu Ala Leu Lys Glu Ala Leu Arg Glu Ala Gly Gly Ile Cys
            180                 185                 190
Ile Ala Leu Ser Val Lys Ile Gly Glu Phe Asp Arg Ala Asp Glu Glu
        195                 200                 205
Glu Phe Asp Gln Leu Leu Arg Glu Leu Lys Arg Arg Lys Pro Glu Ala
    210                 215                 220
Arg Val Val Val Met Cys Gly His Ser Ala Leu Leu Gly Gly Glu Thr
225                 230                 235                 240
Leu Arg Glu Leu Leu Glu Ala Ala Leu Arg Leu Gly Leu Thr Gly Glu
                245                 250                 255
Asp Tyr Val Phe Ile Ser Asp Asp Leu Phe Asn Lys Ser Leu Pro Ala
            260                 265                 270
Glu Pro Gly Tyr Glu Glu Val Ala Pro Gly Ala Ile Thr Ile Glu Leu
        275                 280                 285
Ala Asn Ala Ser Met Leu Arg Phe Ala Tyr Tyr Phe Val Leu Val Leu
    290                 295                 300
Thr Leu Asn Asn Pro Arg Asn Pro Trp Phe Leu Glu Phe Trp Lys Glu

305                 310                 315                 320
Asn Phe Ile Cys Ala Leu Gln Asp Phe Glu Thr Asn Lys Ser Asn Phe
                325                 330                 335
Arg Arg Lys Cys Thr Gly Val Glu Arg Ile Thr Ala Leu Leu Glu Pro
            340                 345                 350
Tyr Glu Val Glu Gly Lys Ala Gly Phe Val Tyr Asp Ala Val Tyr Leu
        355                 360                 365
Tyr Ala His Ala Leu His Asn Met Thr Leu Ala Leu Gly Gly Asp Cys
    370                 375                 380
Gly Leu Cys Pro Ala Met Lys Trp Val Asp Gly Glu Lys Leu Val Gln
385                 390                 395                 400
His Leu Arg Asn Val Thr Phe Glu Gly Val Thr Gly Pro Pro Val Thr
                405                 410                 415
Phe Asp Glu Asn Asn Gly Asp Arg Pro Gly Asp Tyr Val Leu Leu Asp
            420                 425                 430
Thr Gln Asn Thr Glu Thr Gly Gln Leu Lys Val Thr Leu Ser Gly Met
        435                 440                 445
Tyr Pro Ile Thr Phe Thr Tyr Asp Gly Val Gly Lys Trp Thr Glu Pro
    450                 455                 460

<210> 26
<211> 175
<212> PRT
<213> Artificial Sequence

<220>
<223> exemplary motif

<221> VARIANT
<222> 1-4, 6-53, 55-66, 68-137, 139-144, 146, 147, 150-170, 172,
      173, 175
<223> Xaa = any amino acid

<221> VARIANT
<222> 149
<223> Xaa = any aromatic amino acid

<400> 26
Xaa Xaa Xaa Xaa Cys Xaa Xaa Xaa Xaa Xaa Xaa Xaa Xaa Xaa Xaa Xaa
 1               5                  10                  15
Xaa Xaa Xaa Xaa Xaa Xaa Xaa Xaa Xaa Xaa Xaa Xaa Xaa Xaa Xaa Xaa
            20                  25                  30
Xaa Xaa Xaa Xaa Xaa Xaa Xaa Xaa Xaa Xaa Xaa Xaa Xaa Xaa Xaa Xaa
        35                  40                  45
Xaa Xaa Xaa Xaa Xaa Cys Xaa Xaa Xaa Xaa Xaa Xaa Xaa Xaa Xaa Xaa
    50                  55                  60
Xaa Xaa Cys Xaa Xaa Xaa Xaa Xaa Xaa Xaa Xaa Xaa Xaa Xaa Xaa Xaa
65                  70                  75                  80
Xaa Xaa Xaa Xaa Xaa Xaa Xaa Xaa Xaa Xaa Xaa Xaa Xaa Xaa Xaa Xaa
                85                  90                  95
Xaa Xaa Xaa Xaa Xaa Xaa Xaa Xaa Xaa Xaa Xaa Xaa Xaa Xaa Xaa Xaa
            100                 105                 110
Xaa Xaa Xaa Xaa Xaa Xaa Xaa Xaa Xaa Xaa Xaa Xaa Xaa Xaa Xaa Xaa
        115                 120                 125
Xaa Xaa Xaa Xaa Xaa Xaa Xaa Xaa Xaa Cys Xaa Xaa Xaa Xaa Xaa Xaa
    130                 135                 140
Cys Xaa Xaa Gly Xaa Xaa Xaa Xaa Xaa Xaa Xaa Xaa Xaa Xaa Xaa Xaa
145                 150                 155                 160
Xaa Xaa Xaa Xaa Xaa Xaa Xaa Xaa Xaa Xaa Gly Xaa Xaa Cys Xaa
                165                 170                 175